# EUROPEAN PATENT APPLICATION

(11) **EP 4 696 326 A1**
(43) Date of publication of application: **18.02.2026**
(21) Application number: 24788255.8
(22) Date of filing: 15.04.2024
(51) Int. Cl.: A61K 47/54, A61K 47/55, C07D 239/47, A61P 35/00

(54) **PIKFYVE PROTEIN KINASE DEGRADATION AGENT AND USE THEREOF**

(30) Priority: 13.04.2023 CN 202310395799
(71) Applicant: SHANGHAI INSTITUTE OF ORGANIC CHEMISTRY, CHINESE ACADEMY OF SCIENCES, Shanghai 200032 (CN); The Regents of the University of Michigan, Ann Arbor, MI 48109-2590 (US); Livzon Pharmaceutical Group Inc., Guangdong 519090 (CN)
(72) Inventor: DING, Ke, Shanghai 200032 (CN); CHINNAIYAN, Arul M., Ann Arbor 48109 Michigan (US); LI, Chungen, Shanghai 200032 (CN); WANG, Zhen, Shanghai 200032 (CN); QIAO, Yuanyuan, Ann Arbor 48109 Michigan (US)
(74) Representative: J A Kemp LLP
(86) International application number: PCT/CN2024/087809
(87) International publication number: WO 2024/213160

(57) **Abstract**

The present invention provides a PIKfyve protein degradation agent and use thereof. Specifically, the present invention provides a compound having a structure represented by formula (I), or a pharmaceutically acceptable salt thereof, or a stereoisomer thereof, or a prodrug molecule thereof. The compound can target and degrade PIKfyve proteins through a ubiquitin-proteasome pathway, and therefore can be used for treating indications mediated by aberrant expression of the PIKfyve proteins.

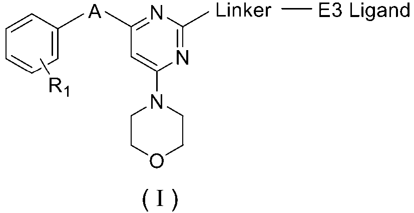

## Description

### Technical Field

The present invention relates to the field of pharmaceutical chemistry, and specifically relates to a class of compounds targeting ubiquitination and degradation of PIKfyve proteins, a pharmaceutical composition thereof, and use thereof.

### Background

PIKfyve is a class of lipid kinases that catalyze phosphorylation of phosphatidylinositol-3-phosphate (PI(3)P) to generate phosphatidylinositol-4,5-bisphosphate (PI(4,5)P2). PI(4,5)P2 plays a key role in the formation of cellular endosomes and lysosomes. PIKfyve is a key enzyme for cytomembrane homeostasis, intracellular transport, and autophagy pathway. Studies show that inhibiting or silencing PIKfyve in various tumor cells can effectively suppress the autophagy pathway thereof, thereby resulting in obvious accumulation of key autophagy protein LC3, resulting in obvious vacuolation of cells, and inhibiting proliferation thereof. At present, a plurality of PIKfyve inhibitors are widely used in studies on the treatment of tumors such as leukemia, prostate cancer, and lymphoma.

In addition, PIKfyve plays an important role in a Toll-like receptor signaling pathway, and is involved in innate immunity regulation of cells. Studies show that inhibiting PIKfyve can effectively suppress generation of IL-12 and IL-23 in immune cells such as B cells and macrophages. The PIKfyve inhibitor Apilimod shows specific inhibition to IL-12 and IL-23 both in vitro and in vivo, and is therefore used in studies related to autoimmune diseases and infectious diseases.

At present, targeted protein degradation technology is widely used in drug development and is considered a major breakthrough in the field of small molecule drugs. Among them, studies on proteolysis targeting chimera (PROTAC) are most mature. The molecular structure of PROTAC is composed of a target protein recognition ligand, a Linker, and an E3 ubiquitin ligase recognition ligand. PROTAC can effectively induce ubiquitination and labeling of target proteins and effectively degrade them through a proteasome pathway. Compared with conventional small molecule inhibitors, PROTAC molecules have advantages such as low dosage, low probability to generate drug resistance, and high safety. At present, multiple PROTAC molecules have entered clinical research.

### Summary of the Invention

An objective of the present invention is to provide a compound capable of degrading PIKfyve proteins through a ubiquitin-proteasome pathway and a pharmaceutical composition thereof.

A first aspect of the present invention provides a compound represented by formula (I), or a pharmaceutically acceptable salt thereof, or a stereoisomer thereof, or a prodrug molecule thereof, 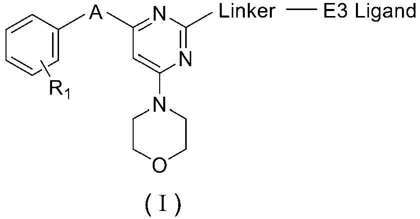 wherein
R₁ is selected from the group consisting of: H, halogen, cyano, a substituted or unsubstituted C₁-C₆ alkyl, a substituted or unsubstituted C₃-C₆ cycloalkyl, and a substituted or unsubstituted C₁-C₆ alkoxy;
A is selected from the group consisting of: -NH-, 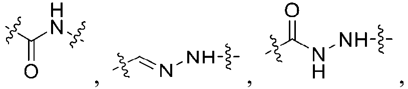 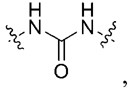 a 6-10-membered aryl, and a 5-10-membered heteroaryl; the A is each independently optionally substituted with 0-3 R₂, the R₂ is selected from the group consisting of: H, halogen, a substituted or unsubstituted C₁-C₆ alkyl, a substituted or unsubstituted C₃-C₆ cycloalkyl, and a substituted or unsubstituted C₁-C₆ alkoxy;
Linker has a structure represented by a formula below: 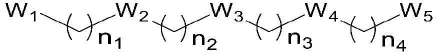
wherein W₁, W₂, W₃, W₄, and W₅ are each independently selected from the group consisting of: -O-, -S-, -NH-, -CH₂-, -CONH-, -NHCO-, -C=C-, -CH=CH-, -C(O)-, -P(=O)-, -S(O)₂-, -S(O)-, -P(O)₂(OH)-, -NH-S(O)-NH-, -C(O)O-, -OC(O)-, -NHCONH-, and a 3-12-membered ring having 0-4 heteroatoms; and n₁, n₂, n₃, and n₄ are each independently 0, 1, 2, 3, 4, 5, 6, 7, 8, 9, or 10; wherein the 3-12-membered ring may be a structure of a monocyclic ring, a spiro ring, a fused ring, or a bridged ring;
E3 Ligand is an E3 ubiquitin ligase ligand having a structure selected from the group consisting of: 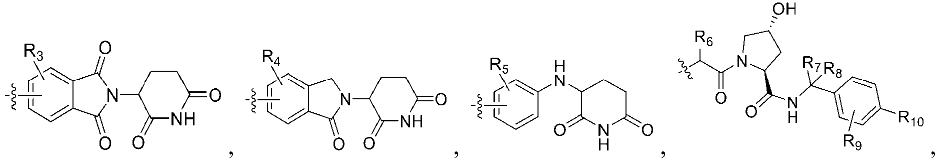 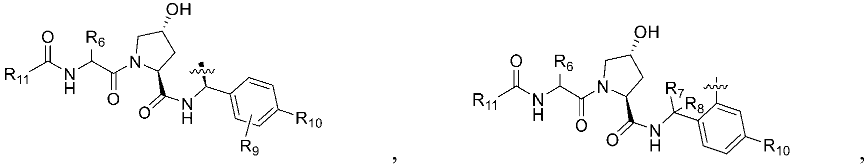 and 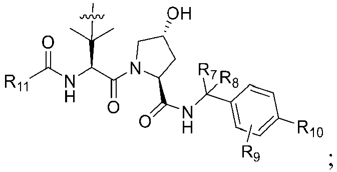
wherein R₃, R₄, R₅, R₆, R₇, R₈, R₉, and R₁₁ are each independently selected from the group consisting of: H, halogen, cyano, a substituted or unsubstituted C₁-C₆ alkyl, a substituted or unsubstituted C₃-C₆cycloalkyl, and a substituted or unsubstituted C₁-C₆ alkoxy; and
R₁₀ is selected from the group consisting of: 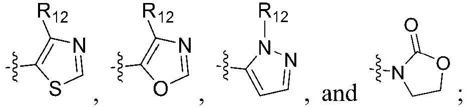 wherein each R₁₂ is each independently selected from the group consisting of: H, a substituted or unsubstituted C₁-C₆ alkyl, a substituted or unsubstituted C₃-C₆ cycloalkyl, and a substituted or unsubstituted C₁-C₆ alkoxy;
wherein the "substituted" in the "substituted or unsubstituted" means that one or more H atoms on the group are substituted with a substituent selected from the group consisting of: halogen, a deuterium atom, hydroxyl, cyano, a C1-C3 alkyl, a C1-C3 alkoxy, a C1-C3 haloalkyl, a C1-C3 haloalkoxy, a C1-C3 aldehyde group, a C1-C3 carboxyl, and -SF₅.

In some embodiments, the E3 Ligand has a structure selected from the group consisting of: 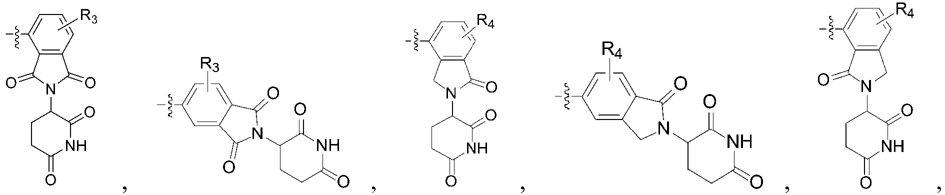 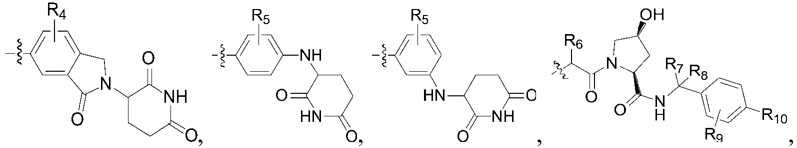 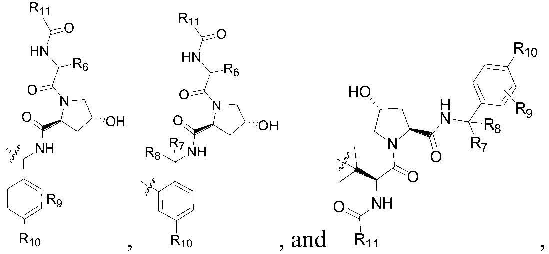 wherein the R₃, the R₄, the R₅, the R₆, the R₇, the R₈, the R₉, the R₁₀, and the R₁₁ are as defined in claim 1.

In some embodiments, the R₁ is a C₁-C₆ alkyl.

In some embodiments, the R₁ is methyl.

In some embodiments, the A is

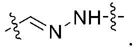

In some embodiments, the Linker has a structure represented by a formula below: 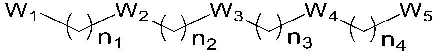 wherein the W₁, the W₂, the W₃, the W₄, and the W₅ are each independently selected from the group consisting of: -O-, -S-, -NH-, -CH₂-, -CONH-, -NHCO-, -C(O)-, -C(O)O-, -OC(O)-, - NHCONH-, and a 3-10-membered ring having 0-4 heteroatoms; and the n₁, the n₂, the n₃, and the n₄ are each independently 0, 1, 2, 3, 4, 5, 6, 7, 8, 9, or 10.

In some embodiments, the Linker has a structure selected from the group consisting of: 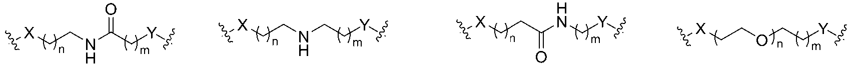 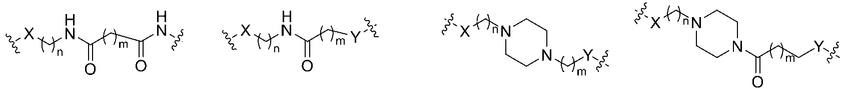 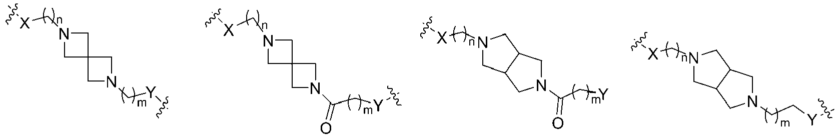 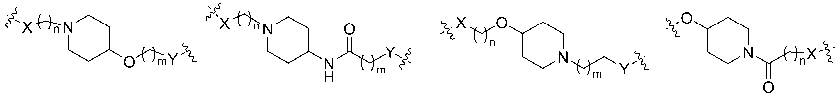 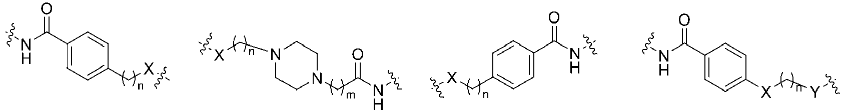 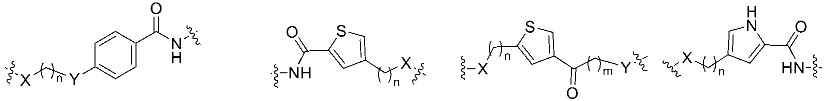 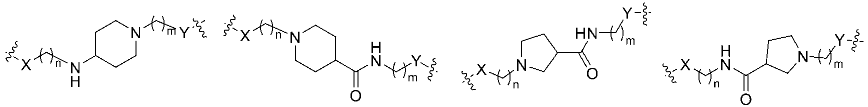 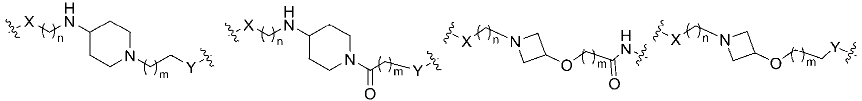 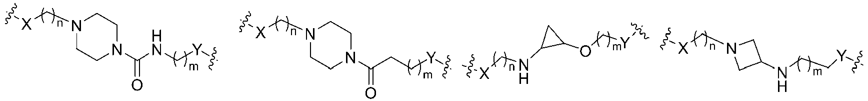 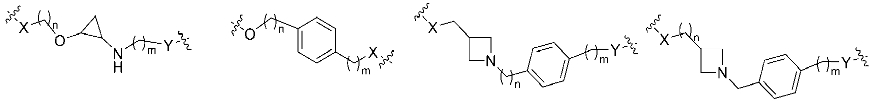 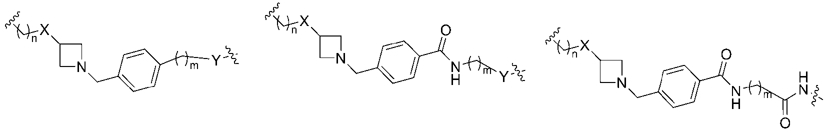 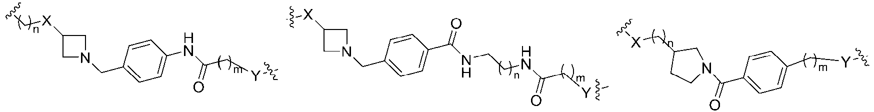 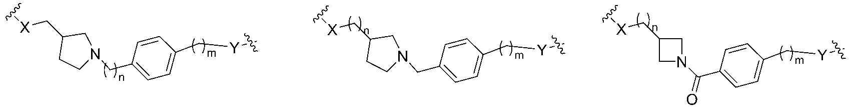 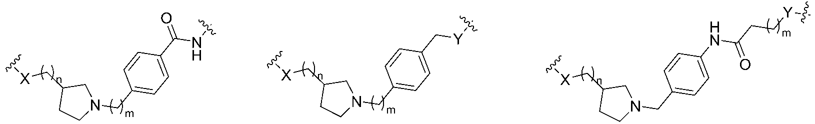 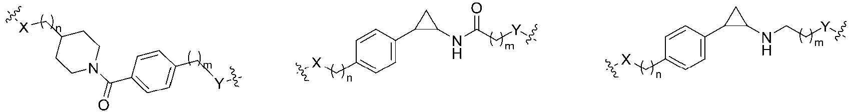 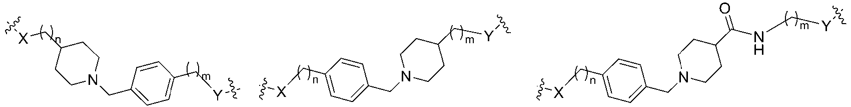 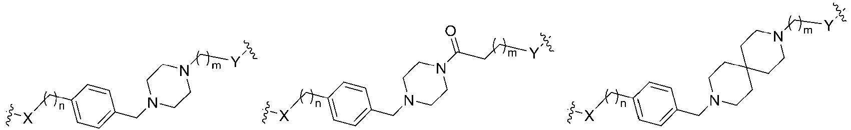 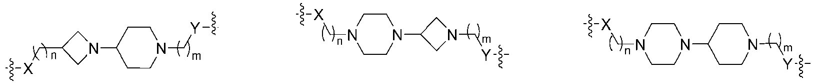 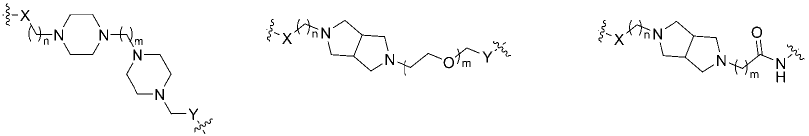 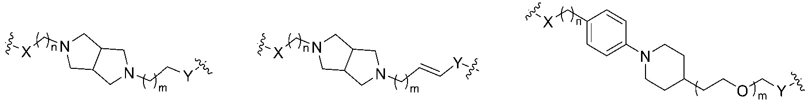 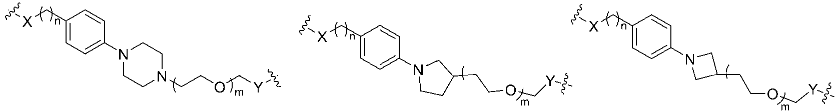 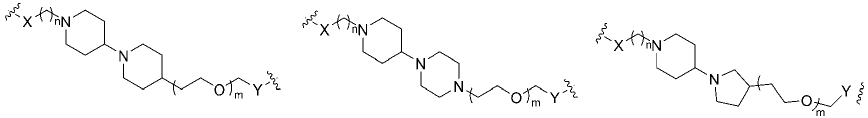 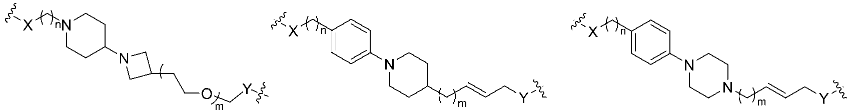 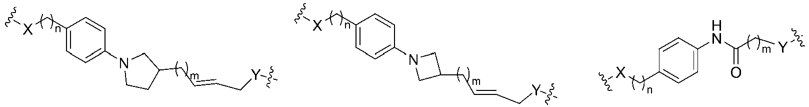 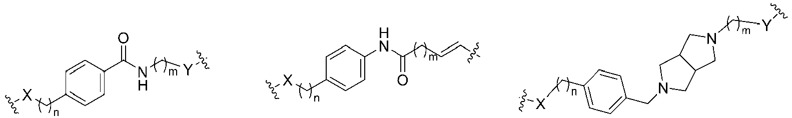 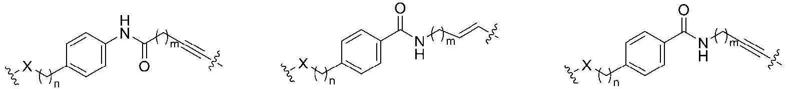 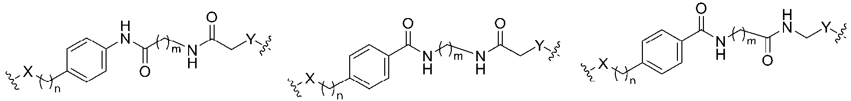 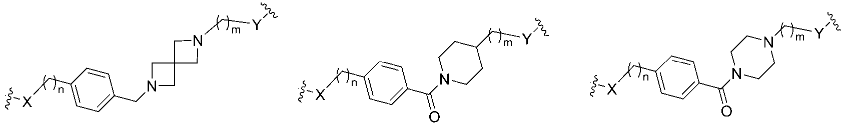 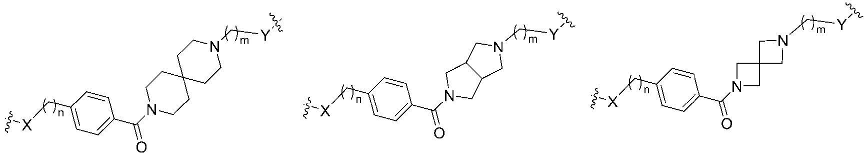 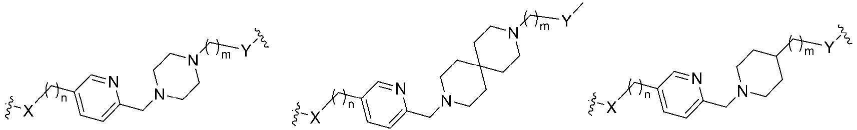 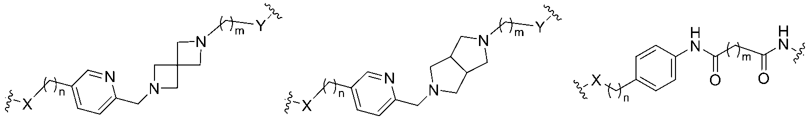 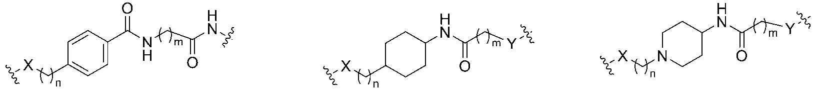 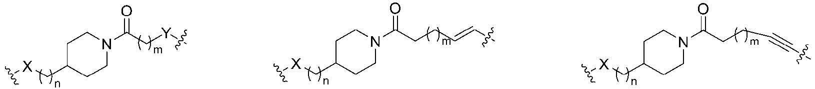 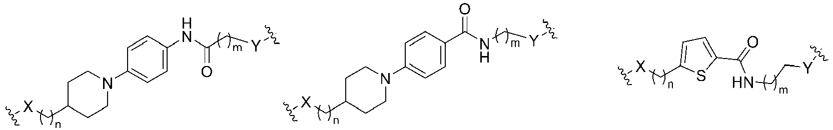 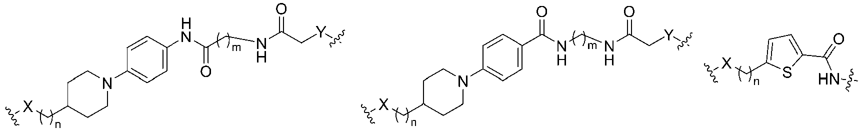 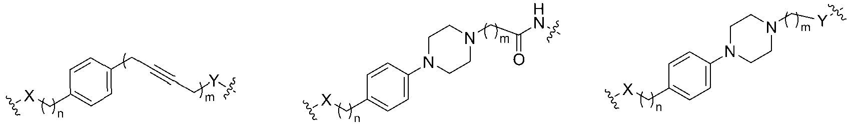 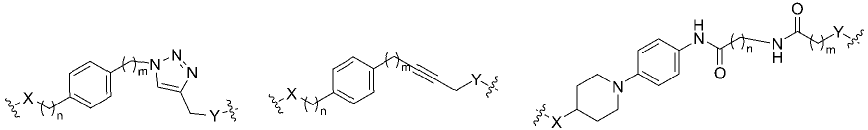 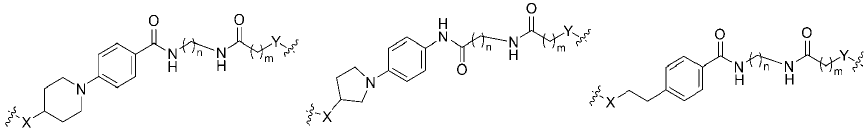 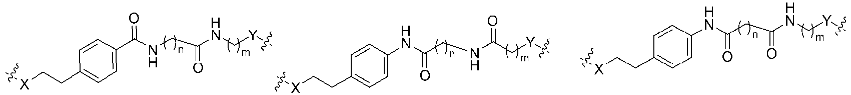 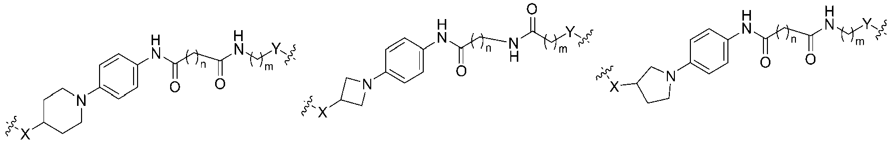 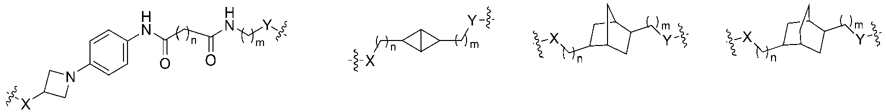 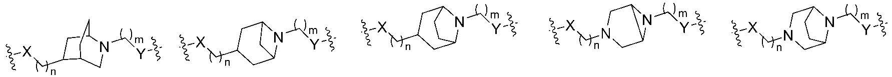 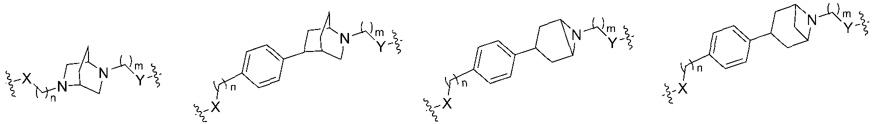 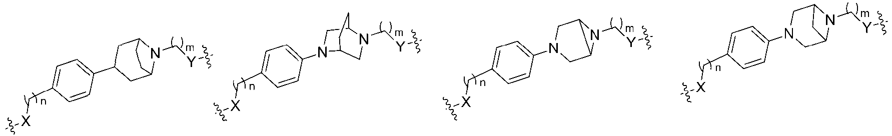 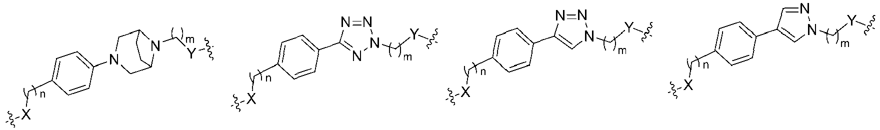 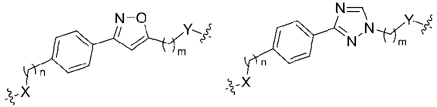 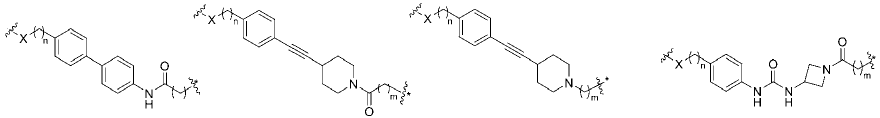 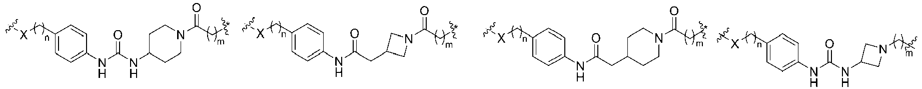 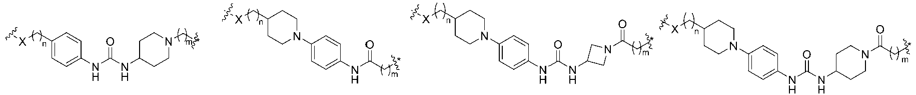 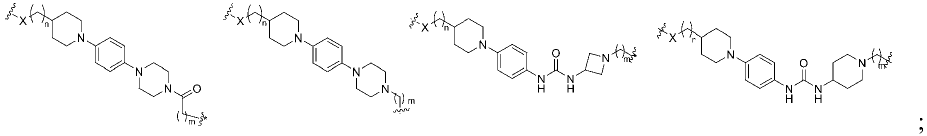 wherein each of X and Y is each independently selected from the group consisting of: -O-, -S-, -NH-, -CH₂-, and 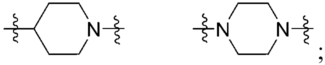 and each of n and m is each independently 0, 1, 2, 3, 4, 5, 6, 7, 8, 9, or 10.

In some embodiments, the linking group Linker is: 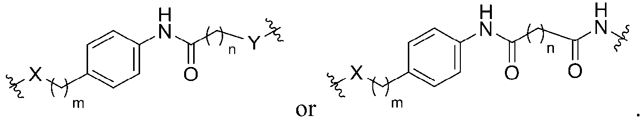 wherein X and Y are independently selected from -O- and -NH-; m is 0, 1, 2, 3, or 4; and n is 0, 1, 2, 3, 4, 5, 6, 7, 8, 9, or 10.

In some embodiments, the A is selected from the group consisting of: -NH-, 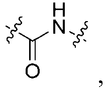 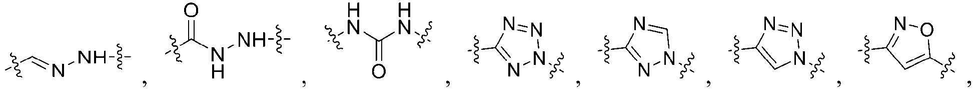 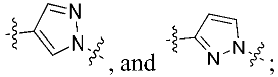 the A is each independently optionally substituted with 0-2 R₂, and the R₂ is as defined in claim 1.

In some embodiments, the compound is selected from the group consisting of:

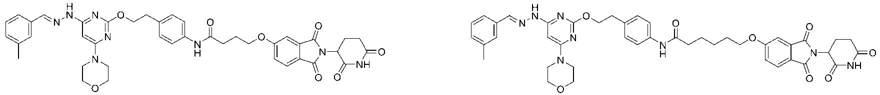

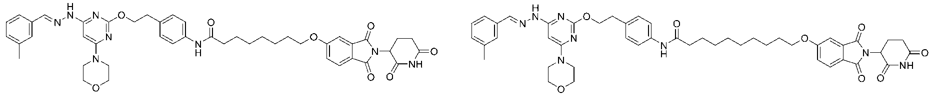

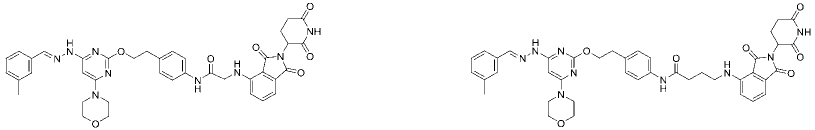

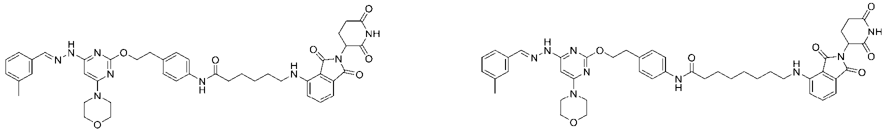

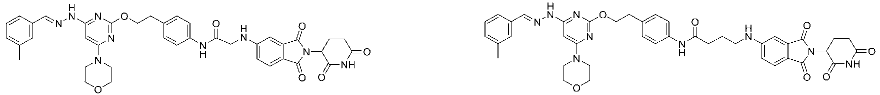

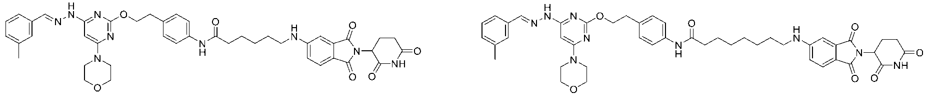

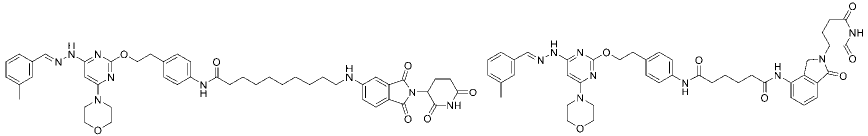

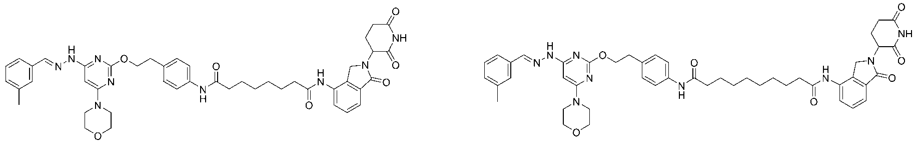

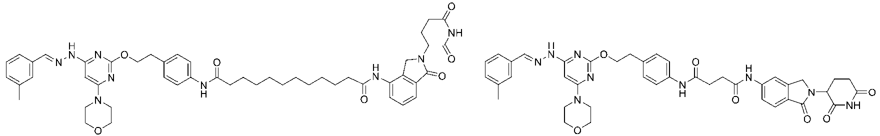

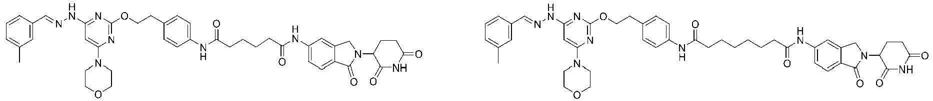

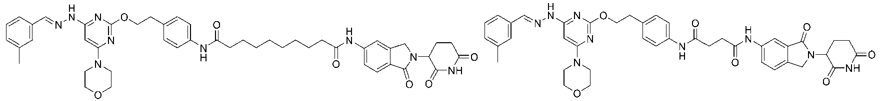

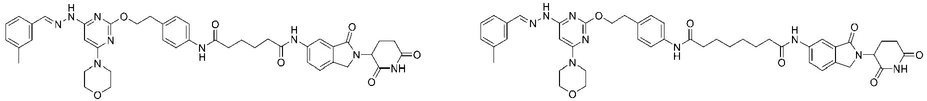

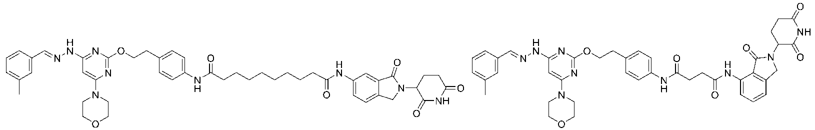

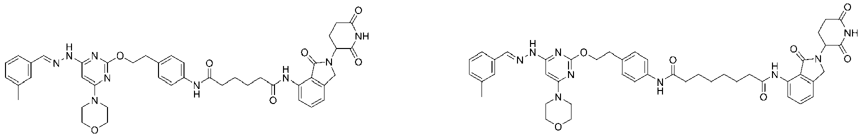

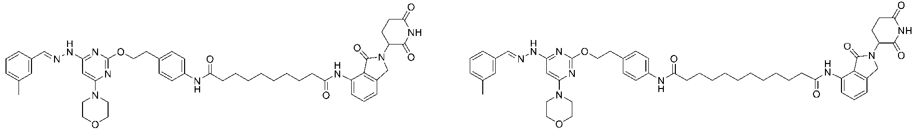

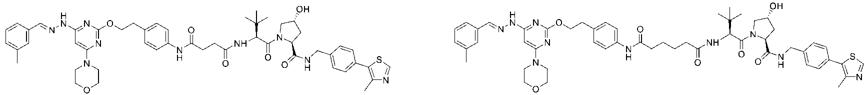

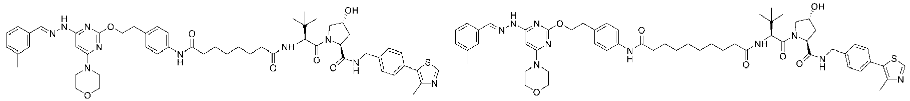

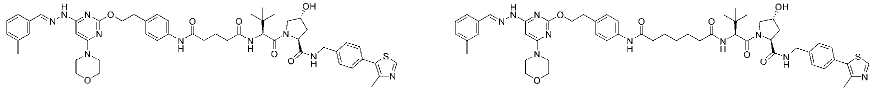

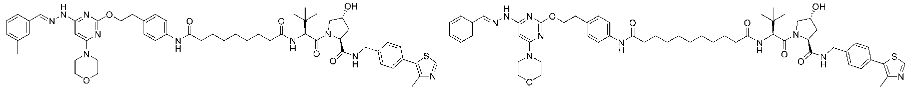

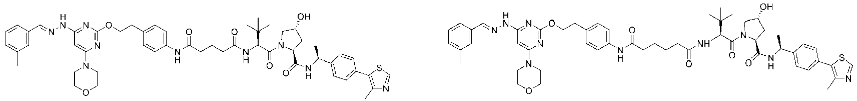

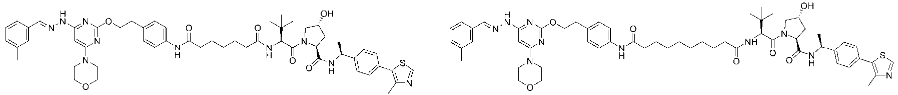

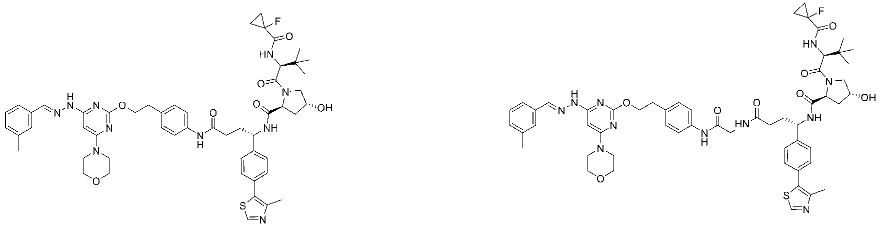

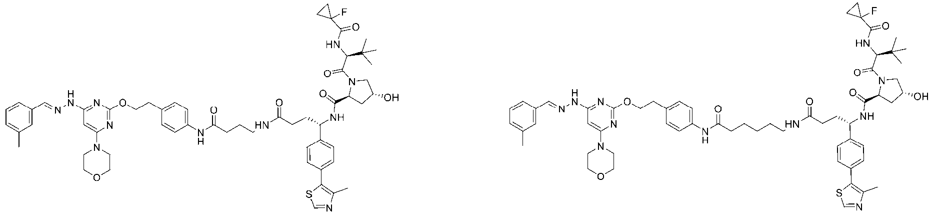

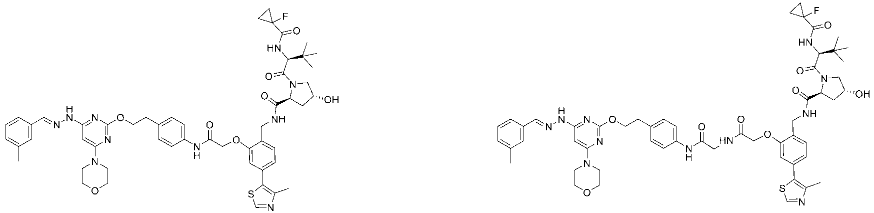

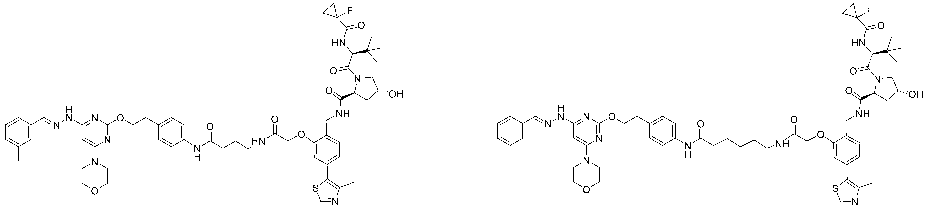

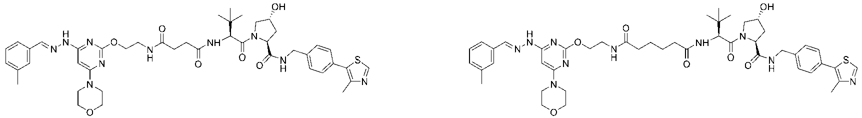

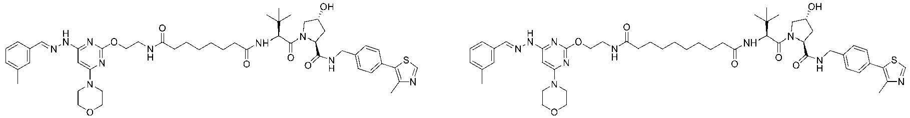

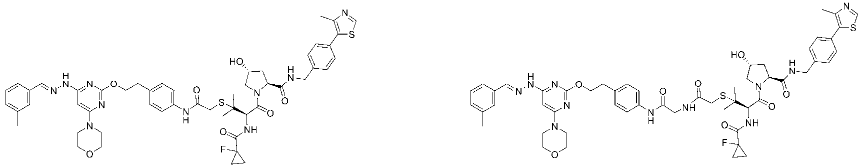

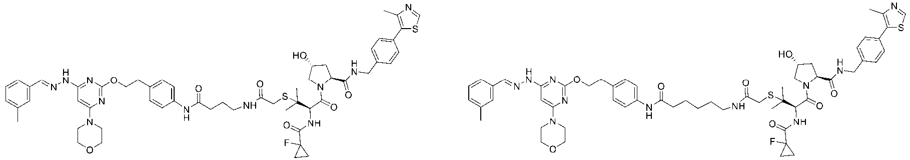

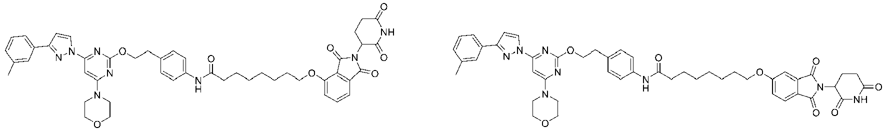

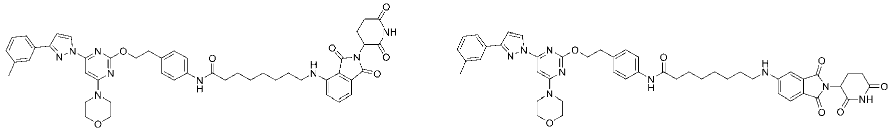

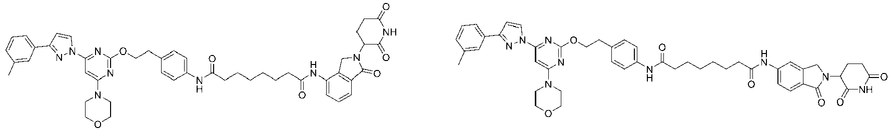

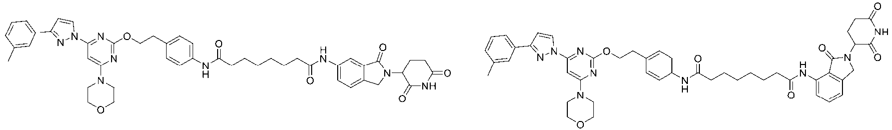

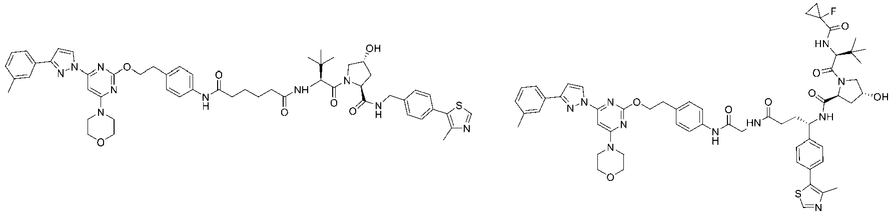

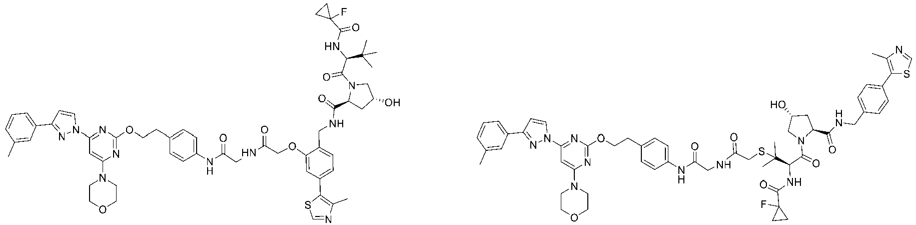

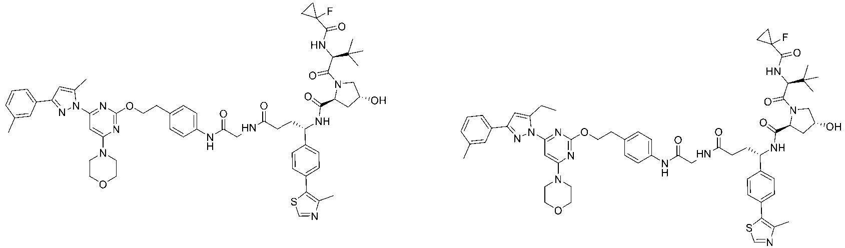

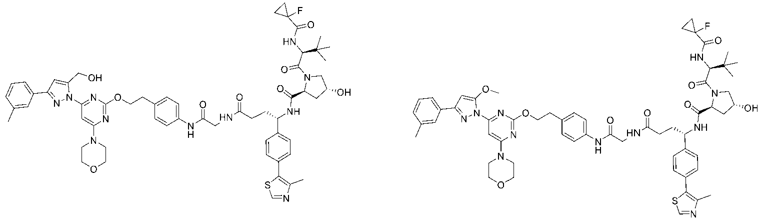

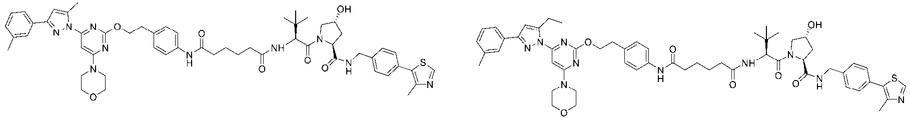

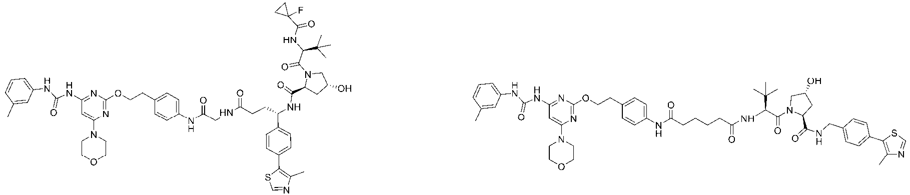

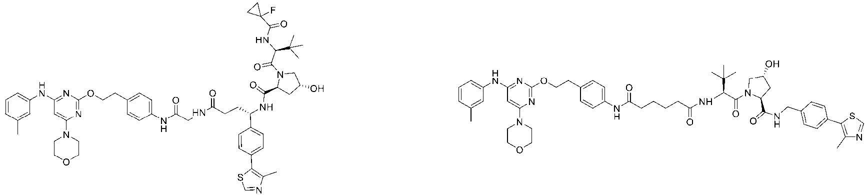

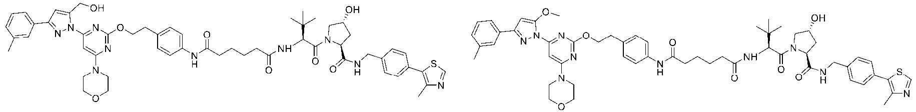

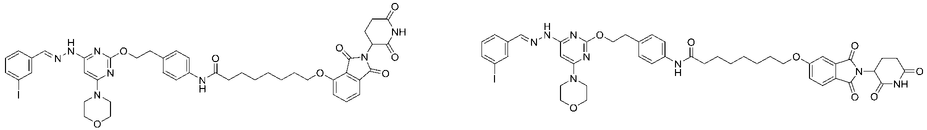

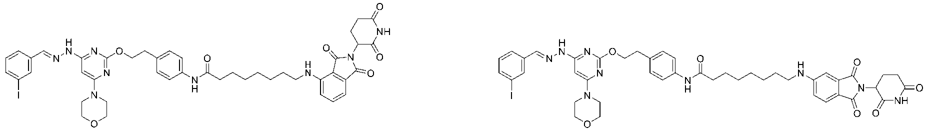

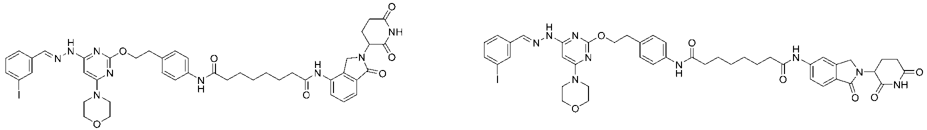

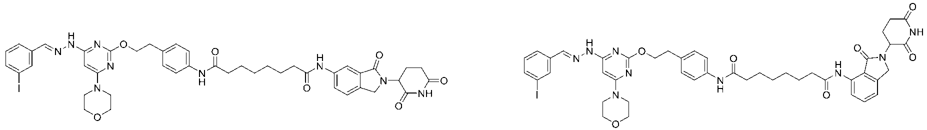

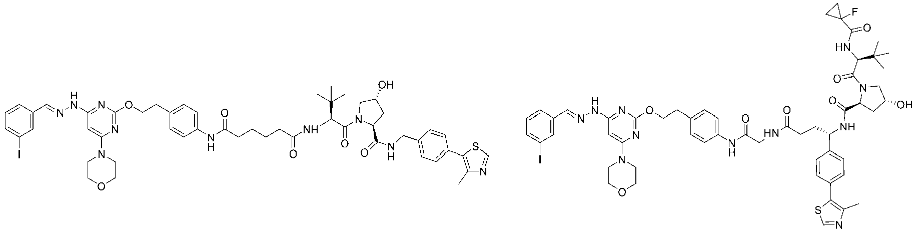

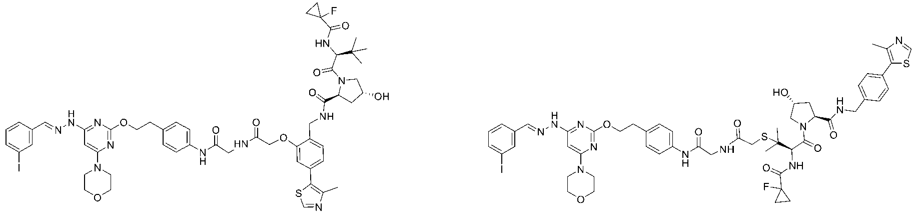

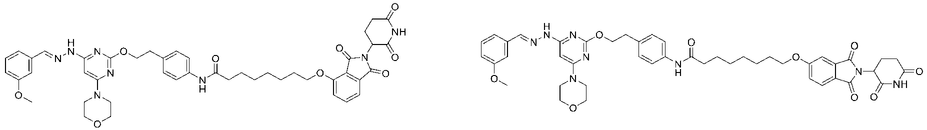

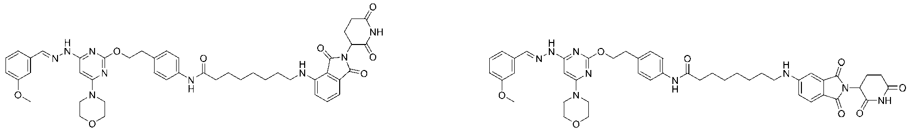

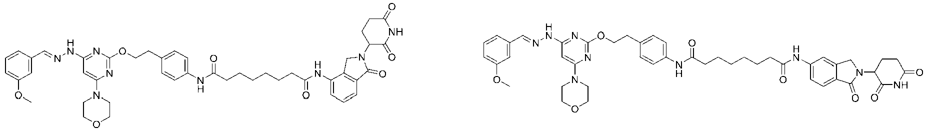

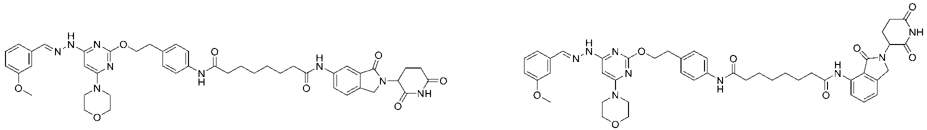

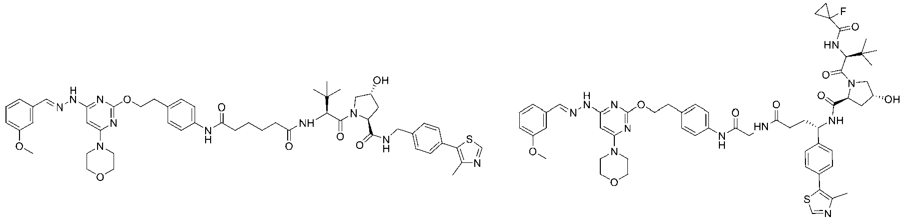

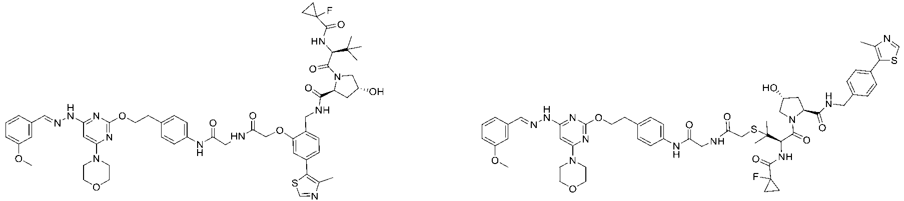

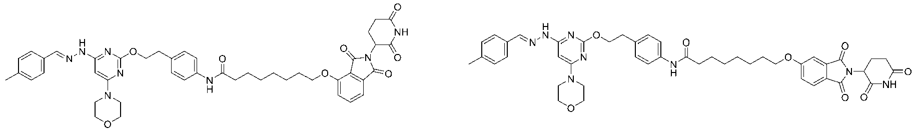

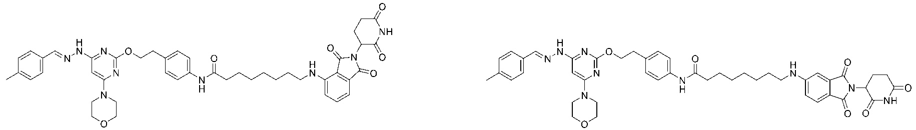

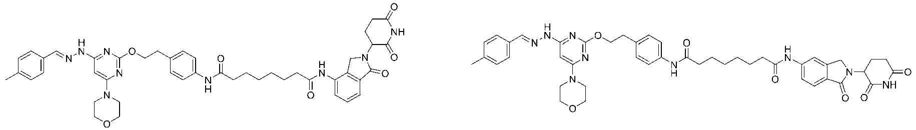

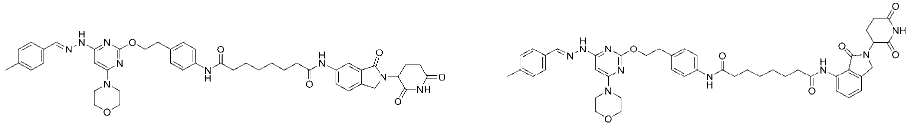

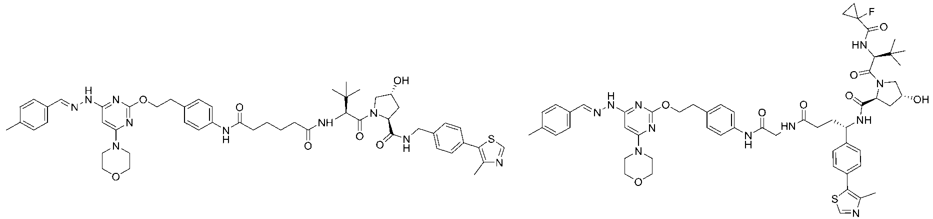

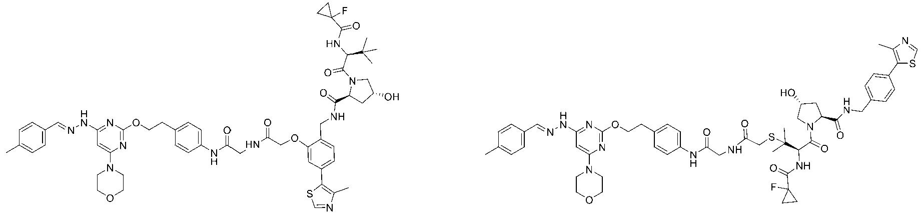

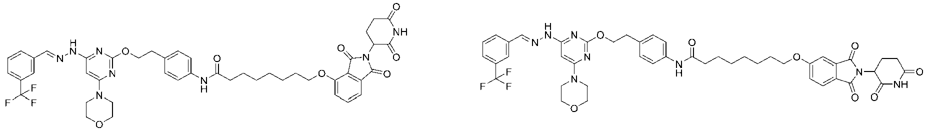

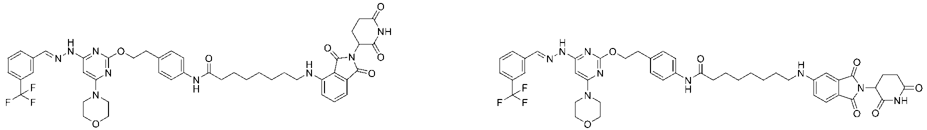

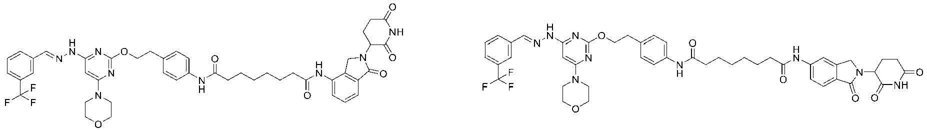

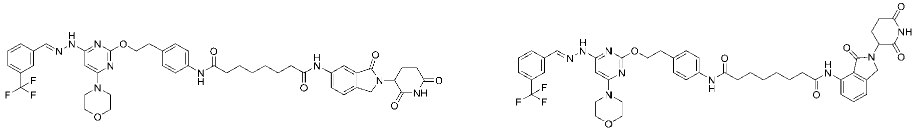

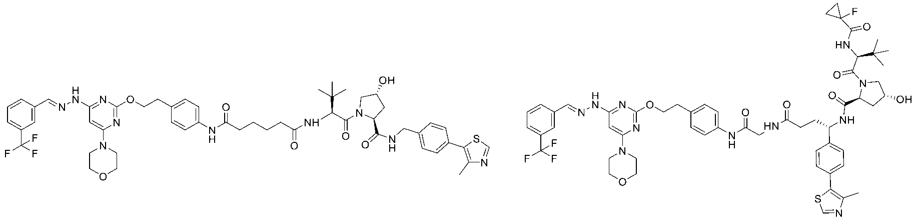

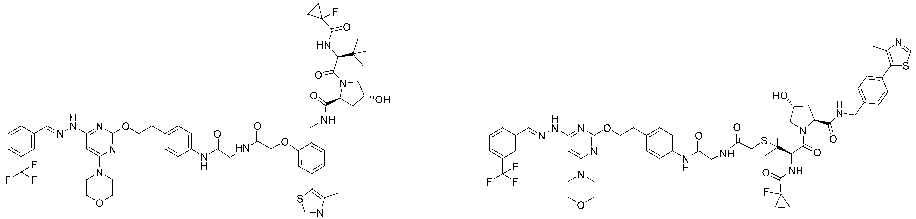

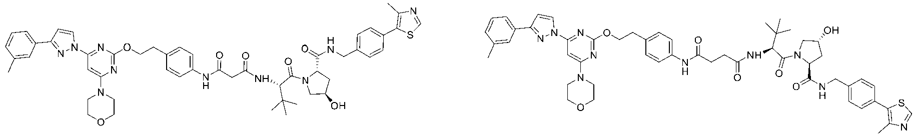

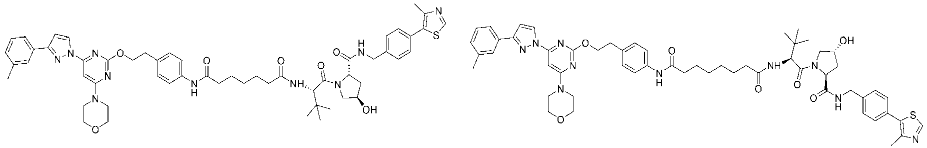

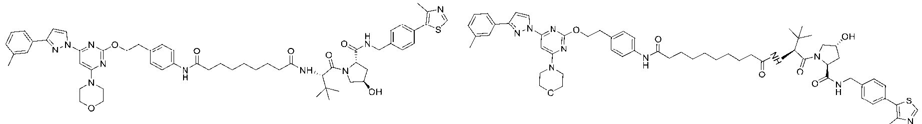

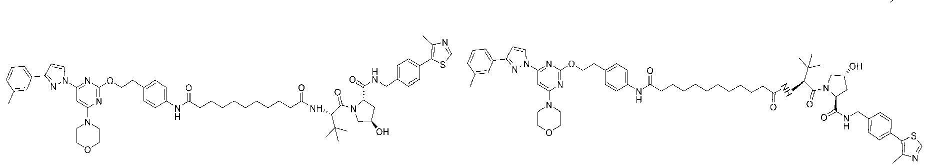

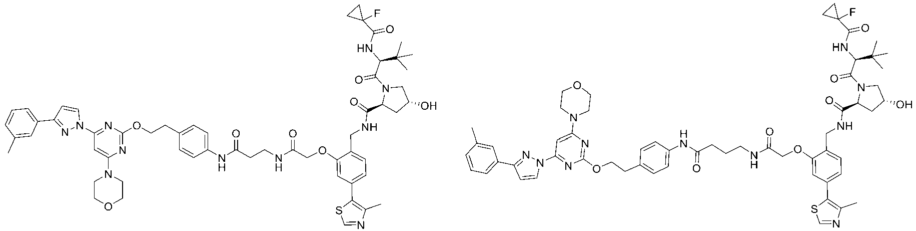

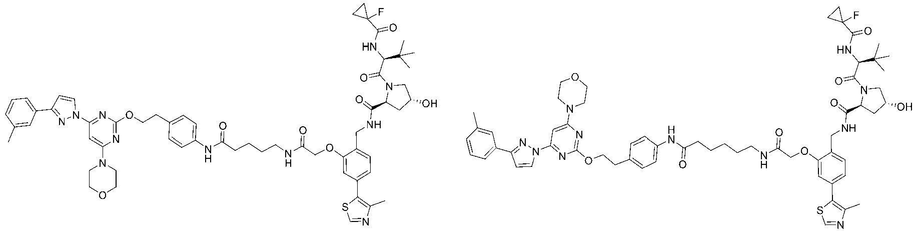

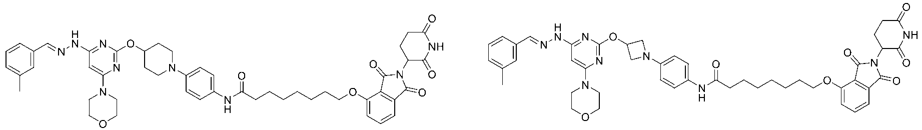

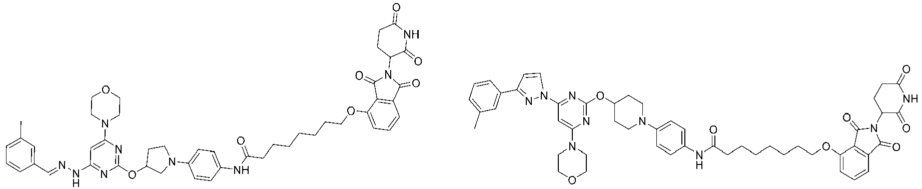

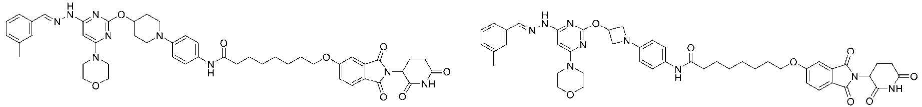

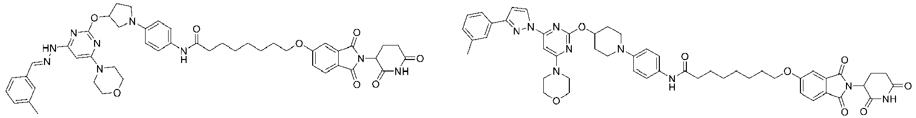

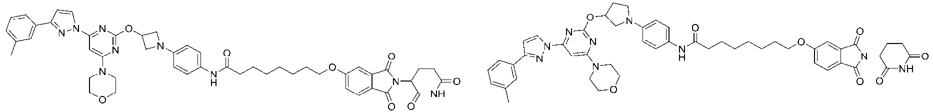

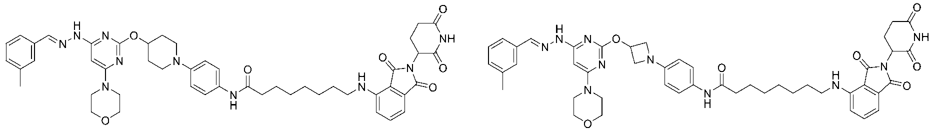

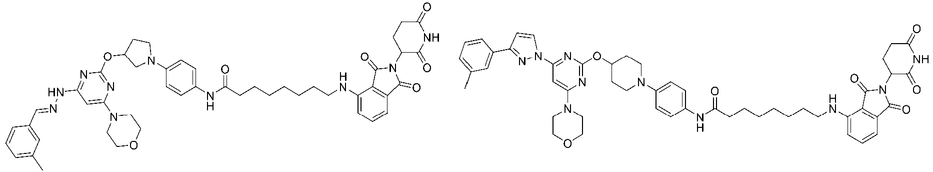

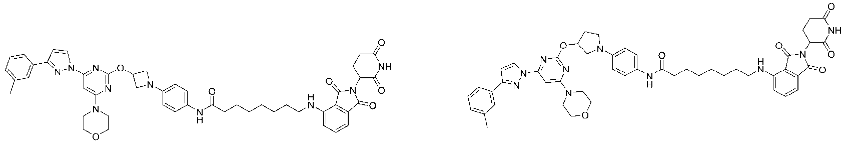

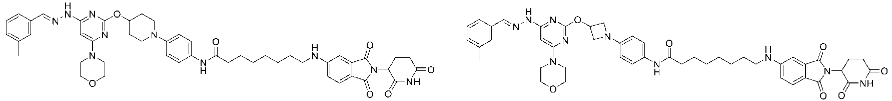

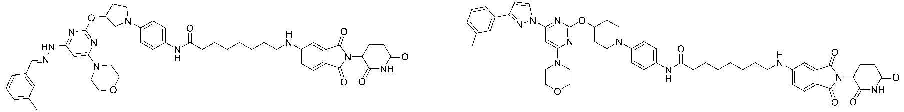

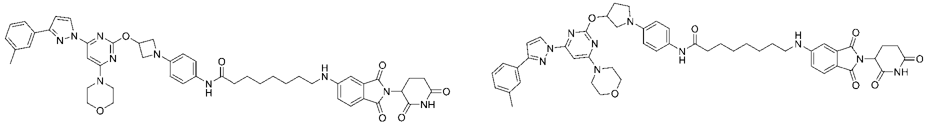

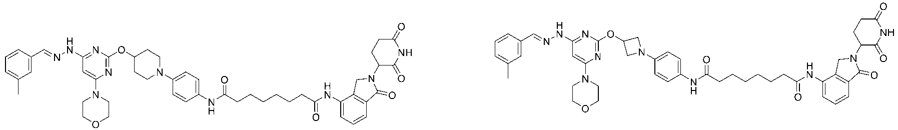

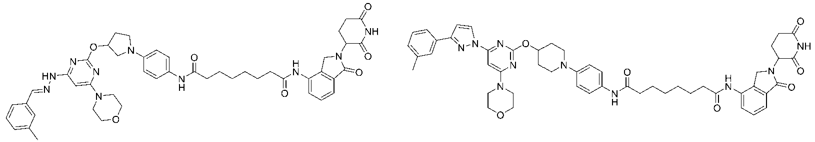

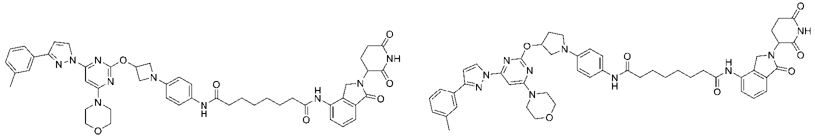

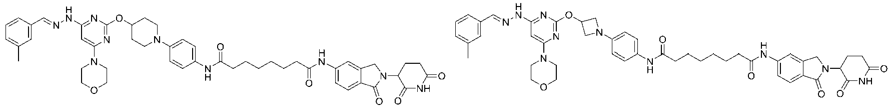

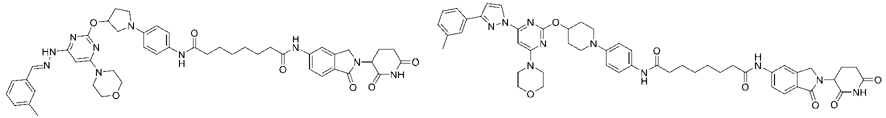

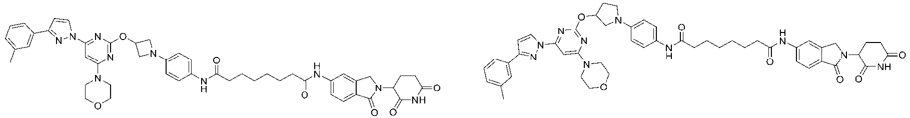

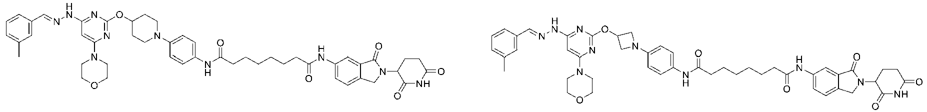

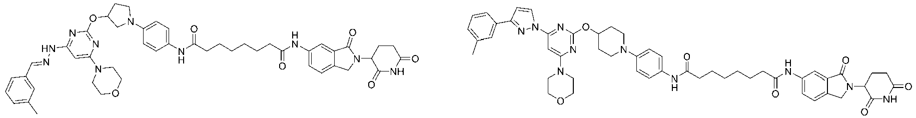

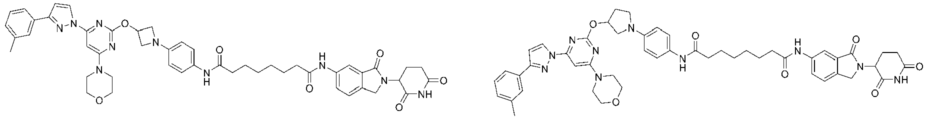

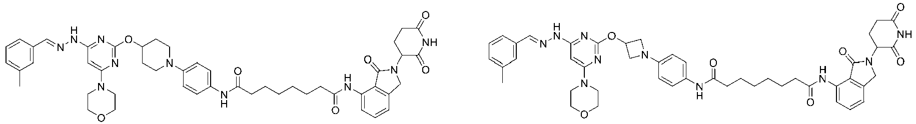

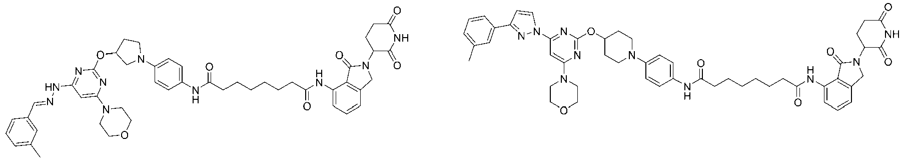

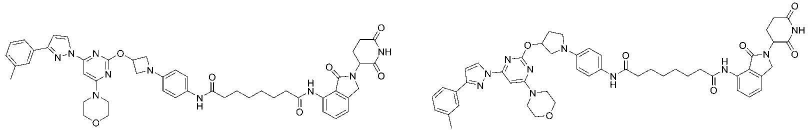

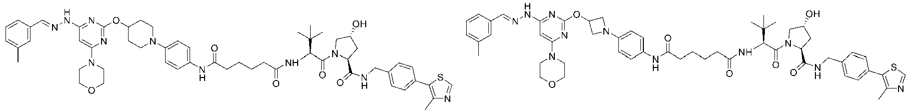

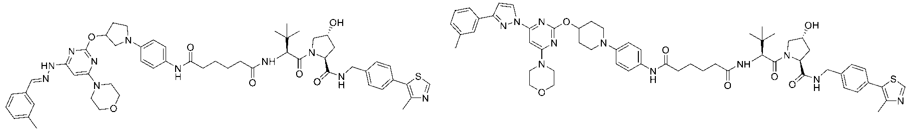

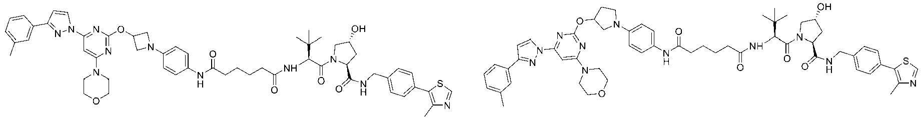

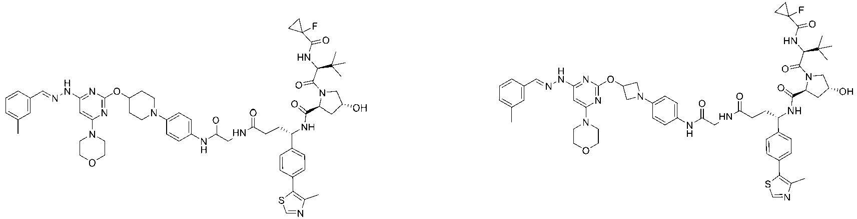

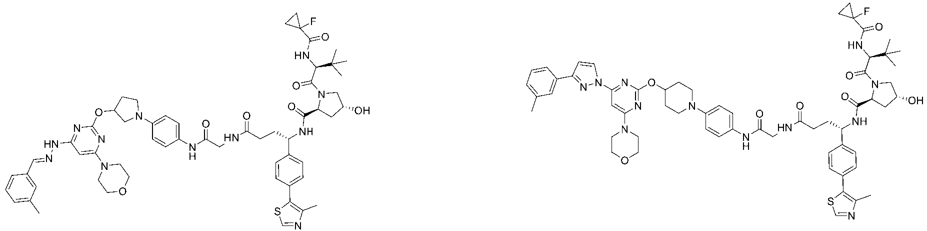

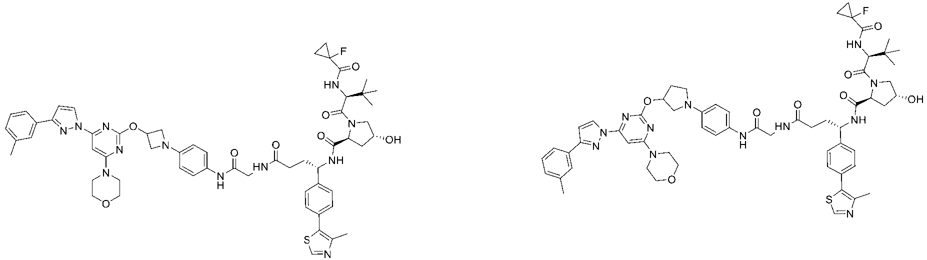

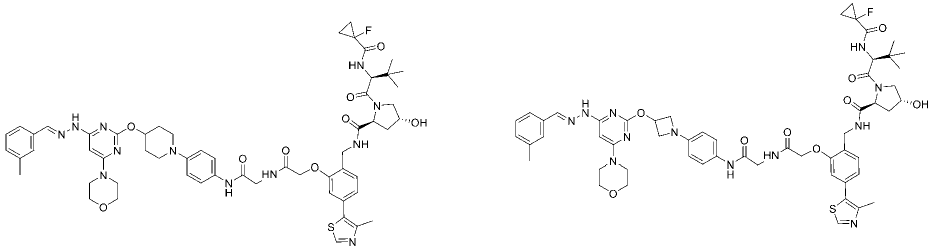

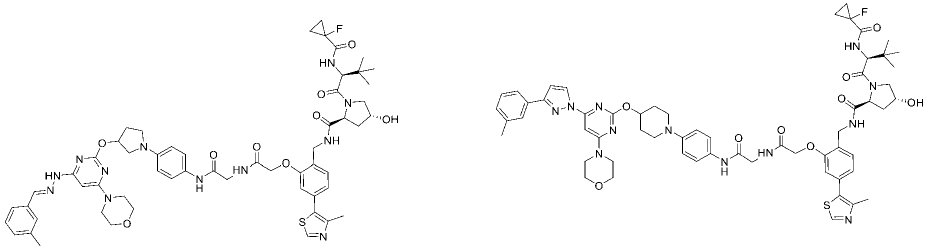

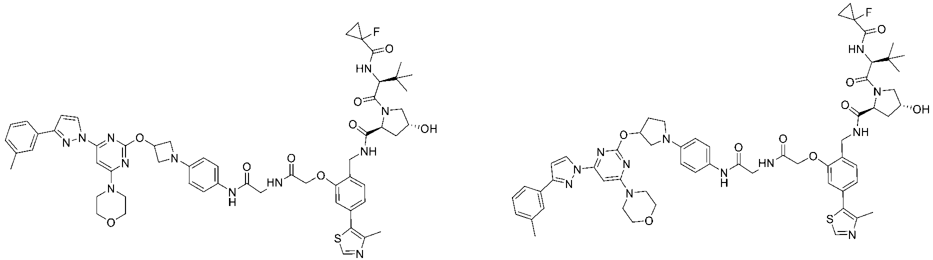

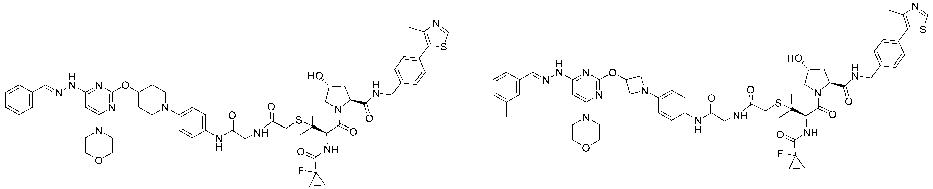

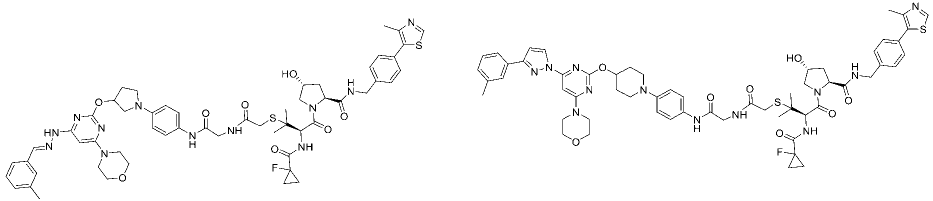

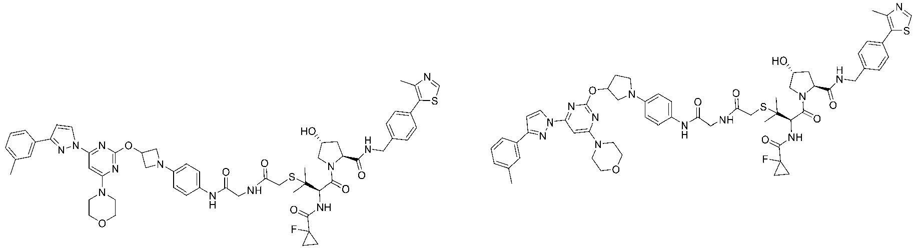

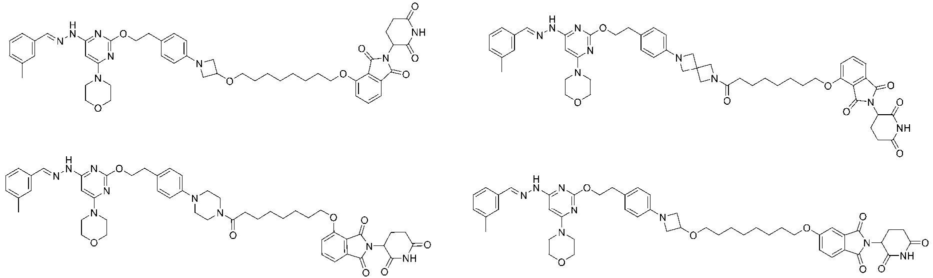

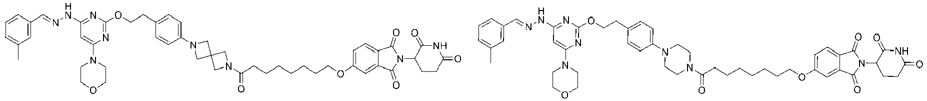

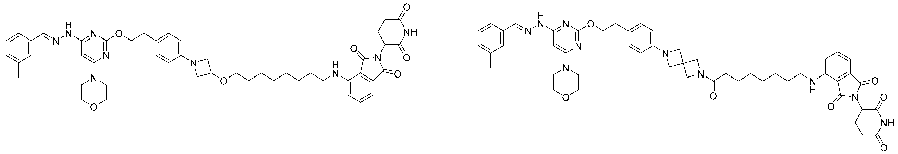

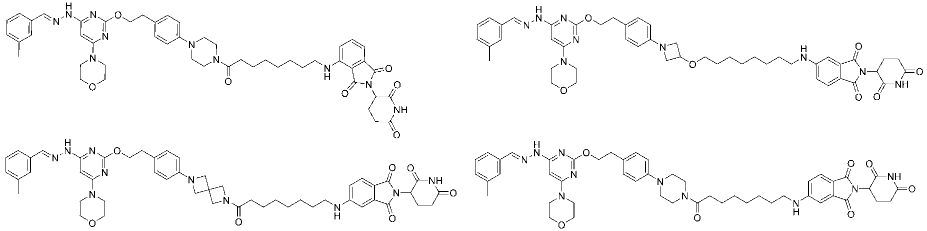

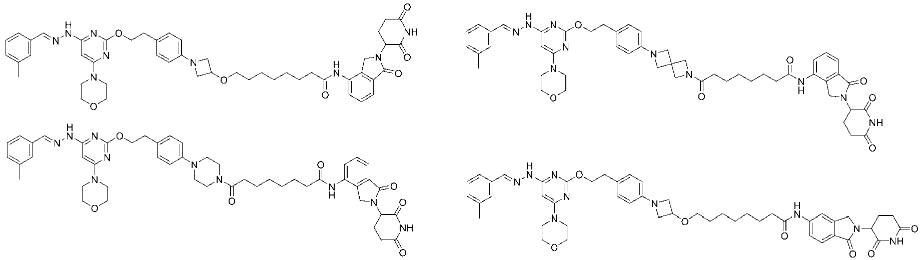

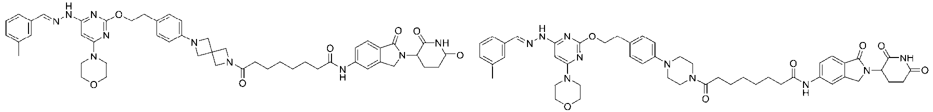

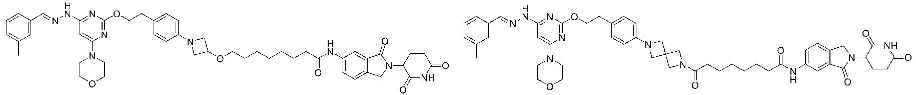

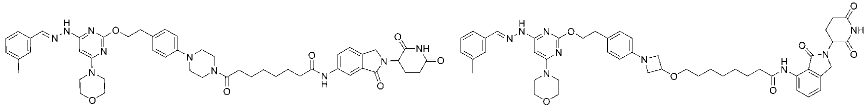

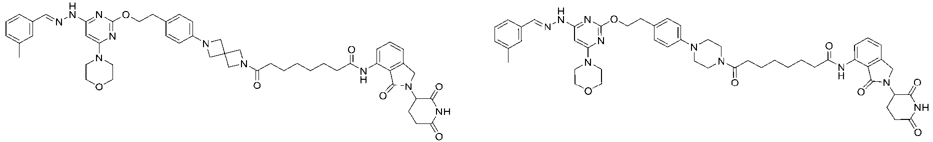

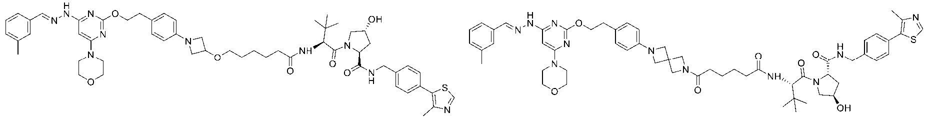

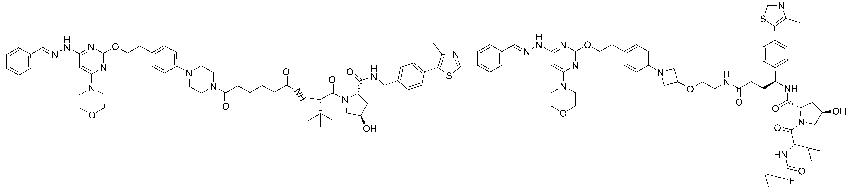

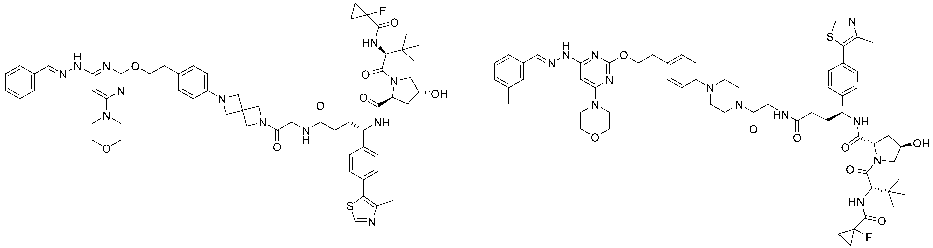

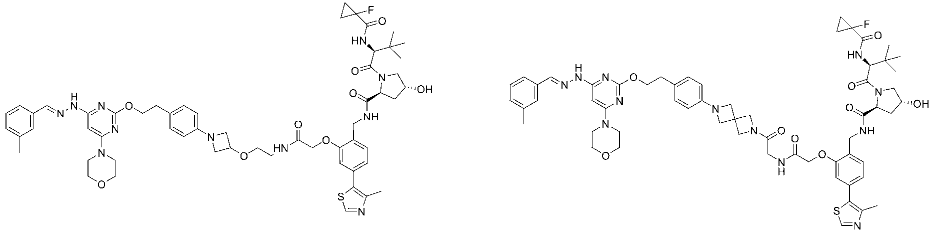

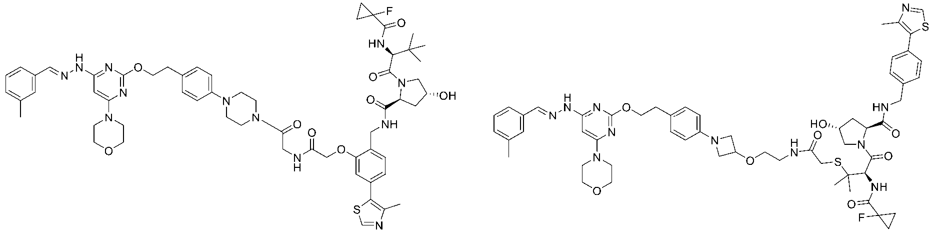

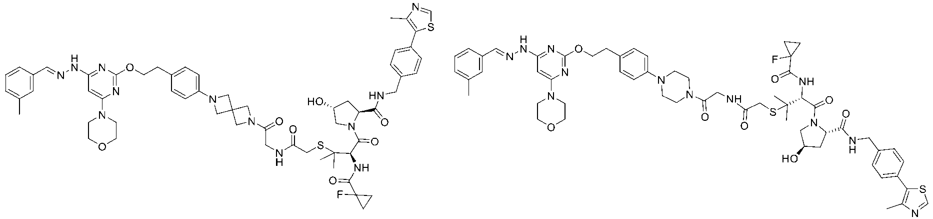

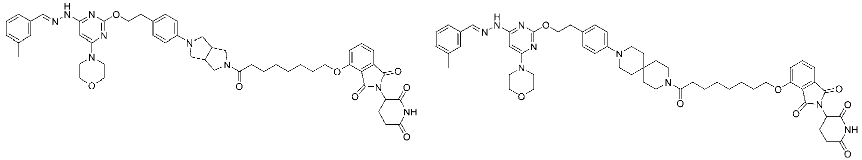

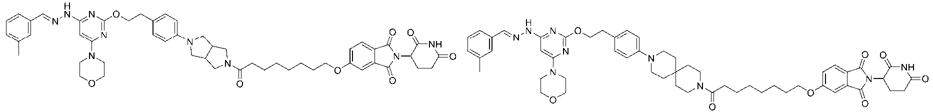

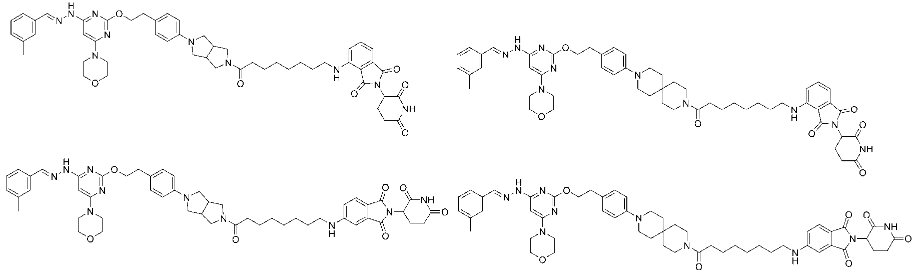

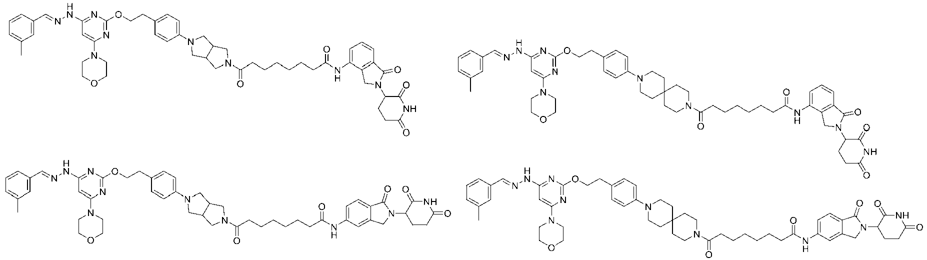

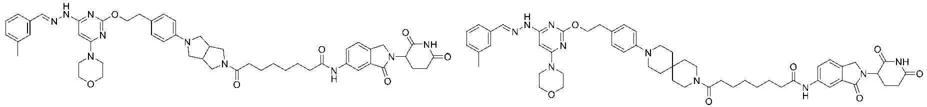

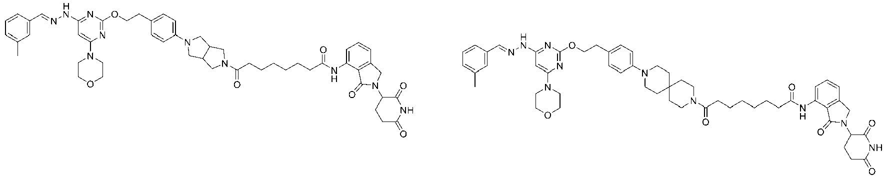

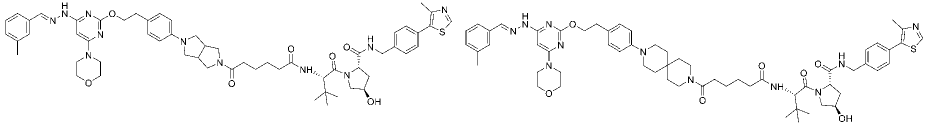

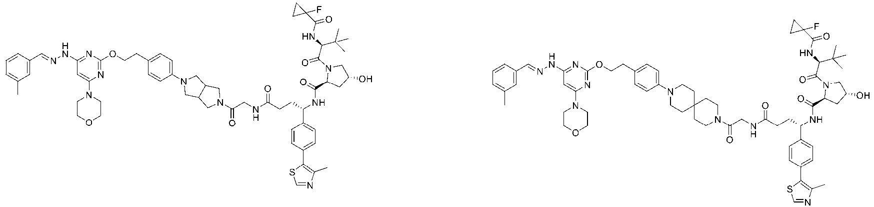

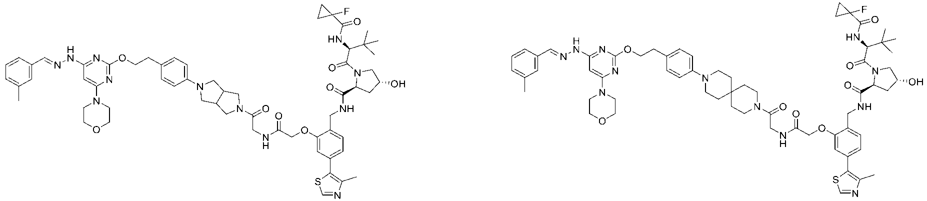

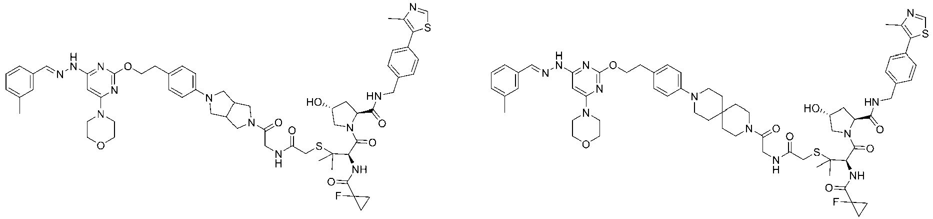

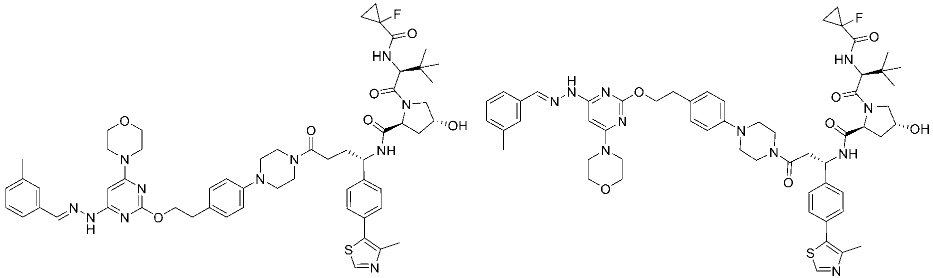

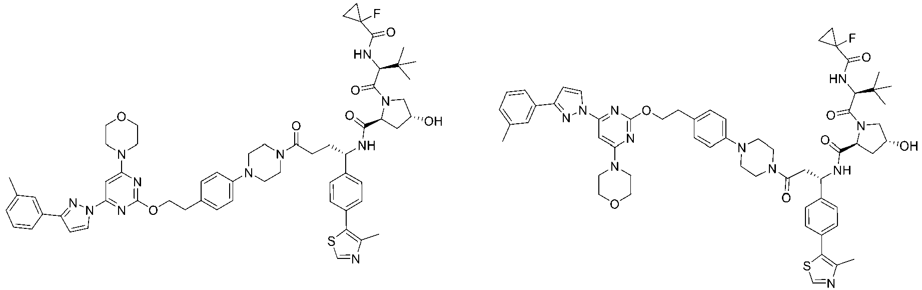

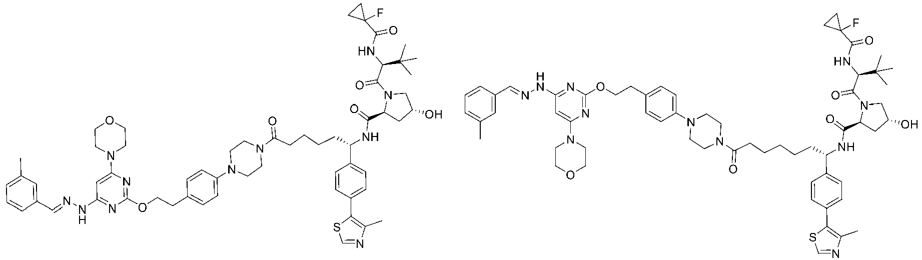

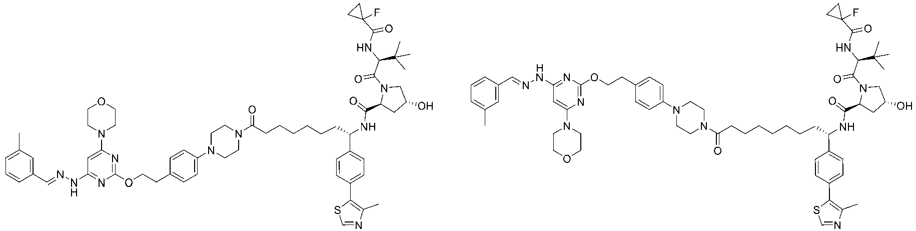

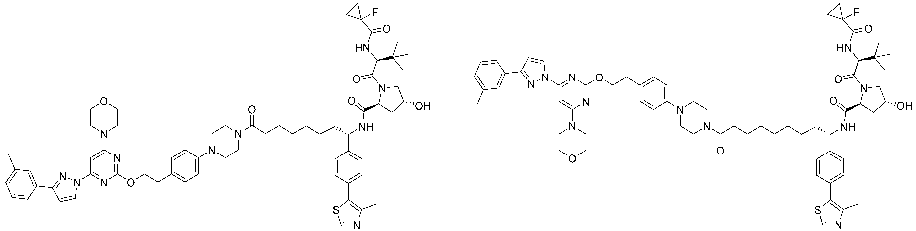

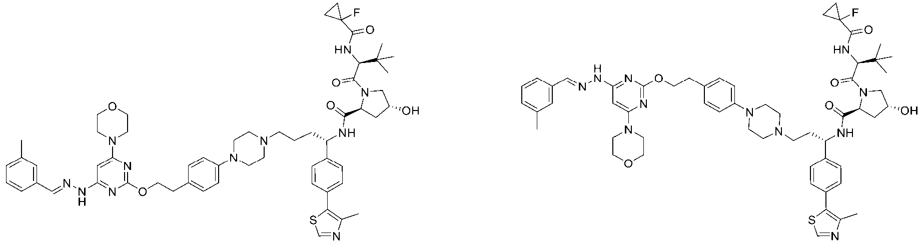

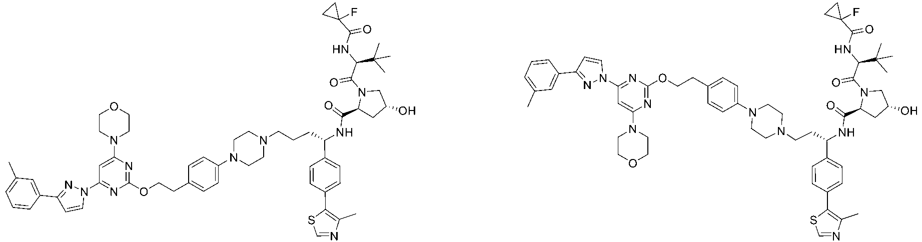

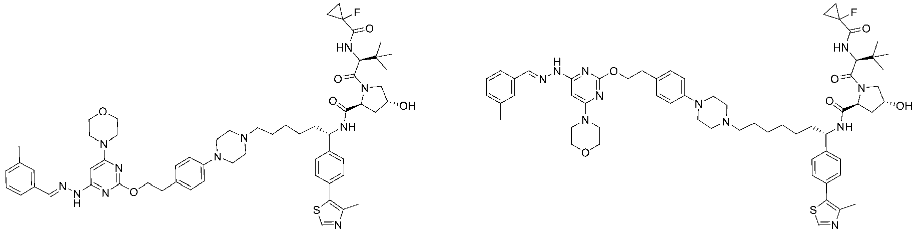

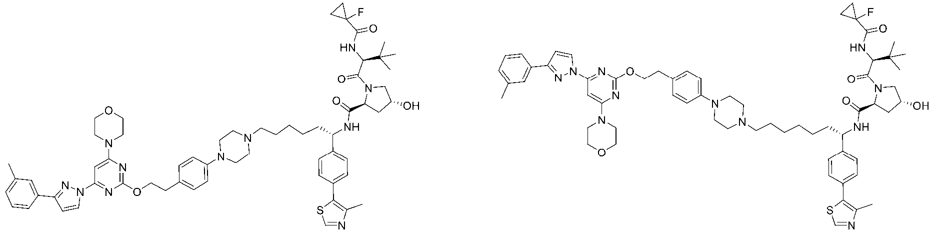

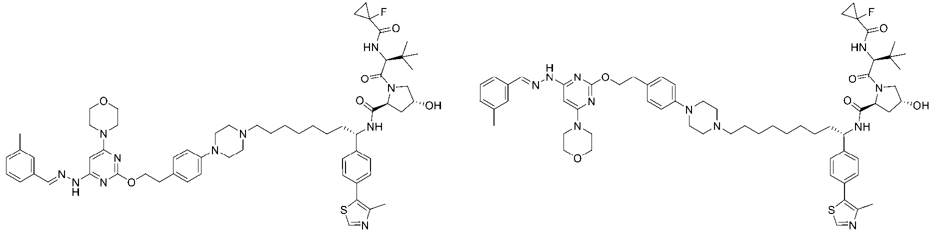

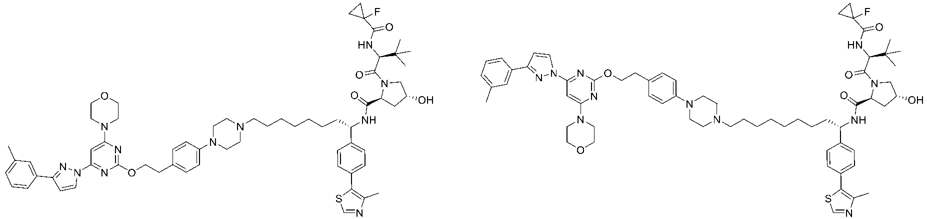

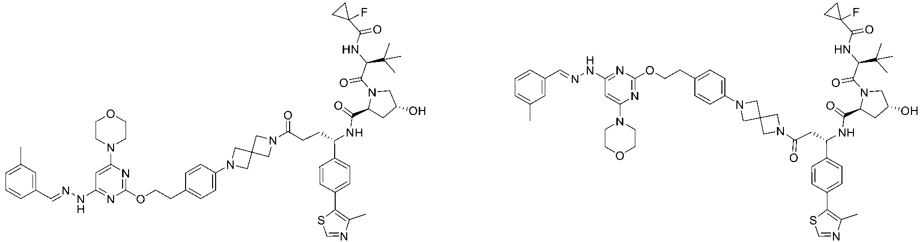

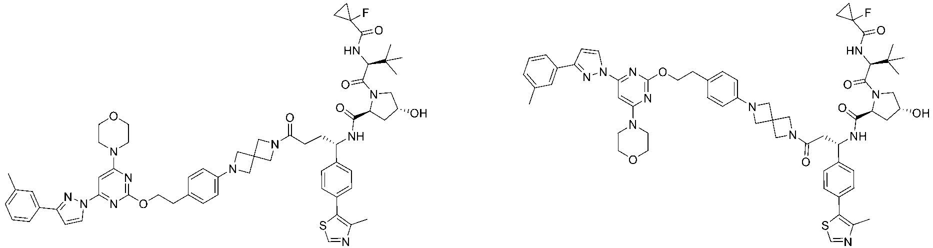

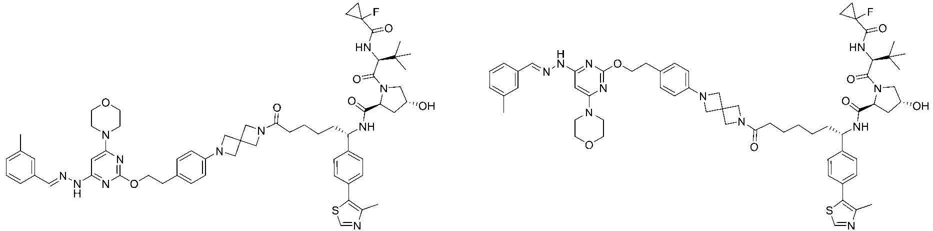

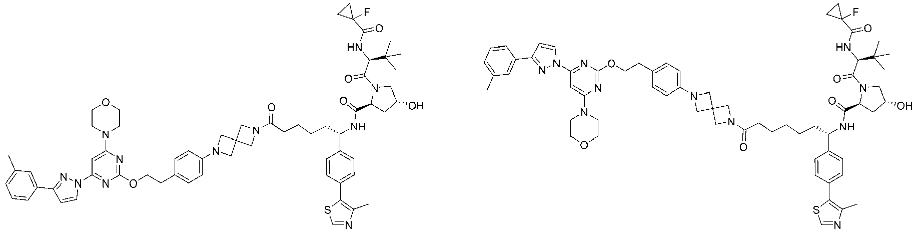

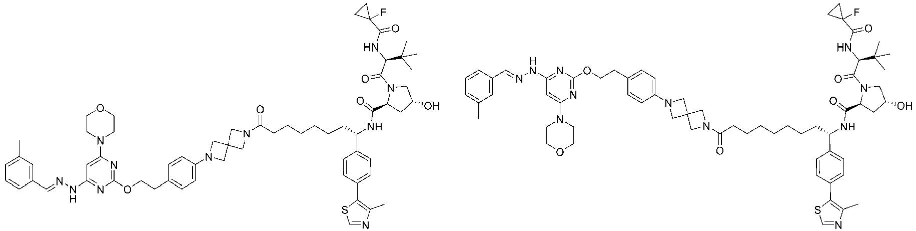

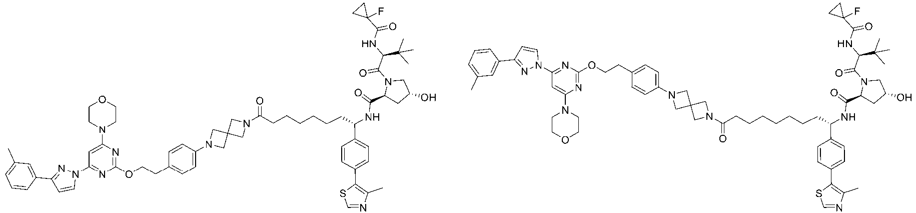

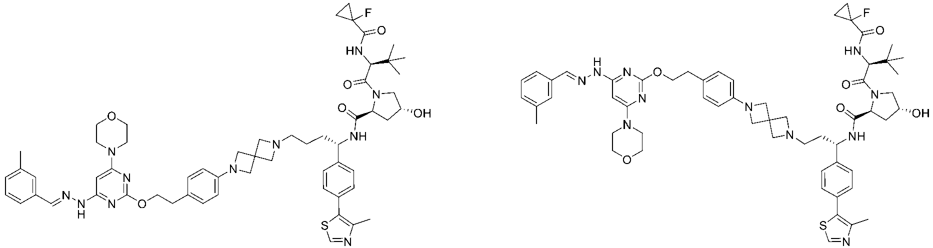

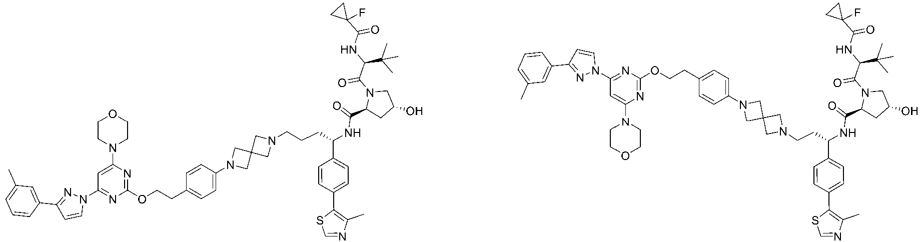

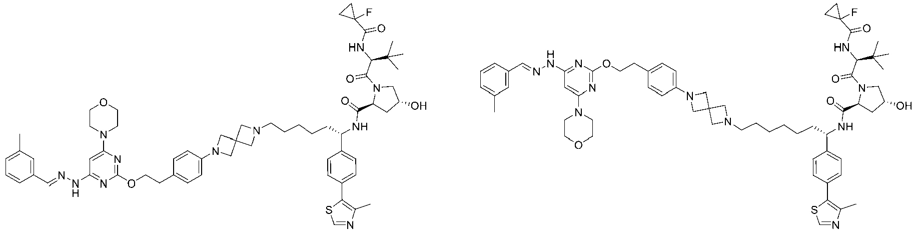

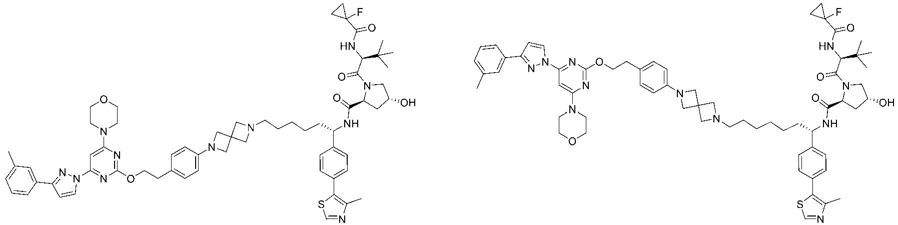

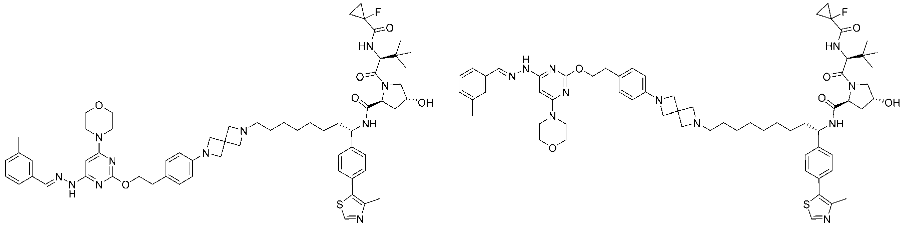

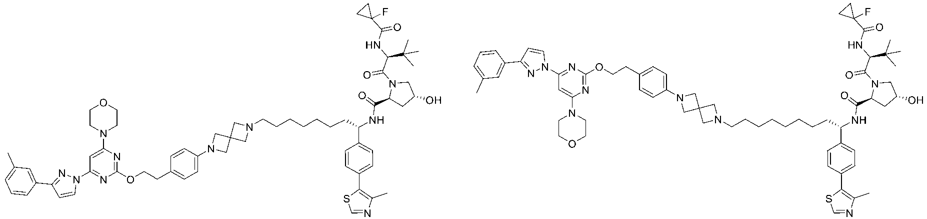

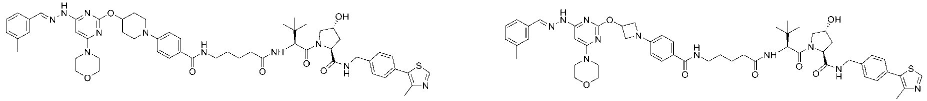

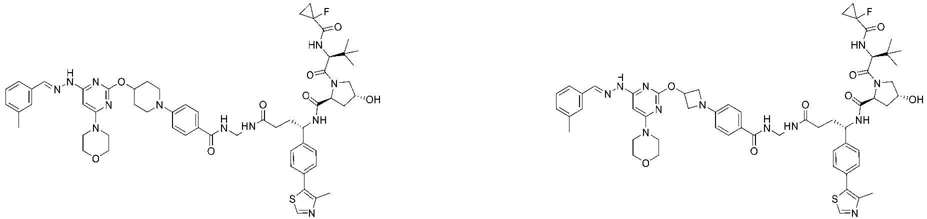

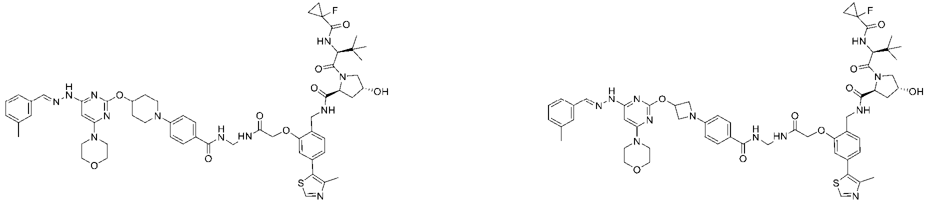

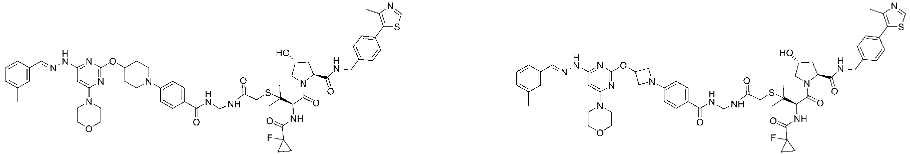

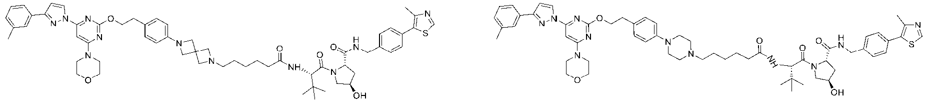

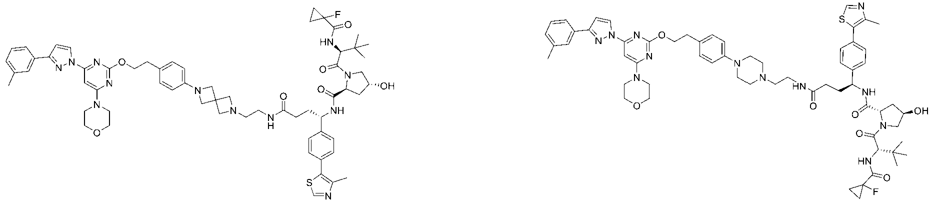

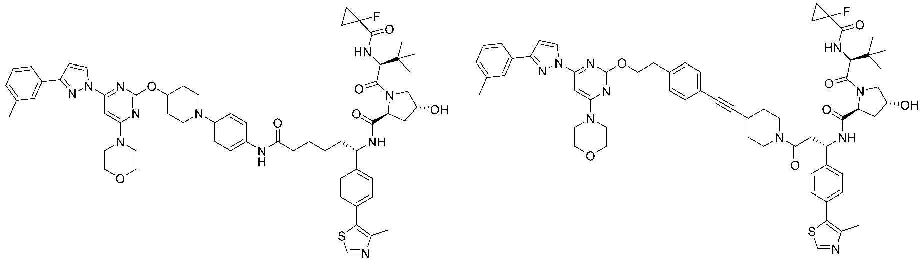

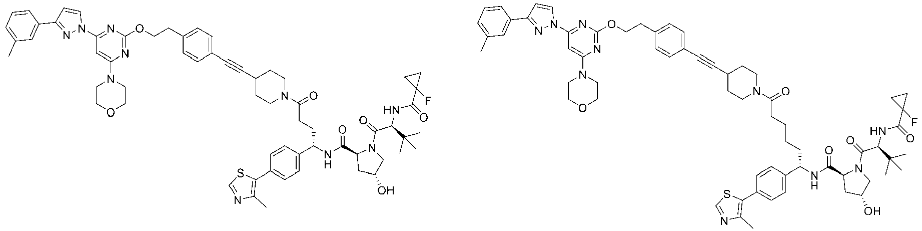

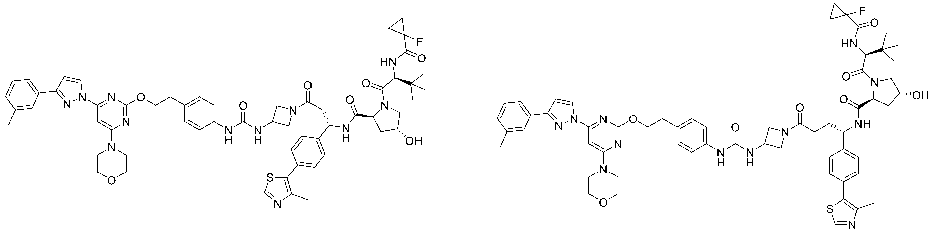

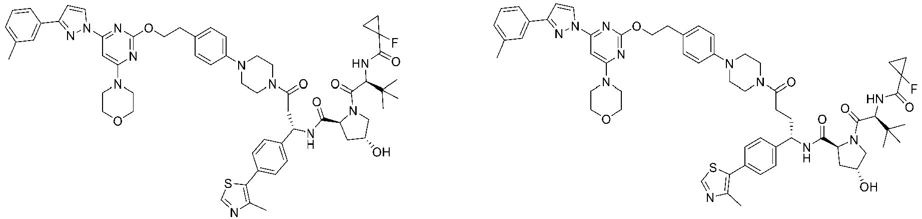

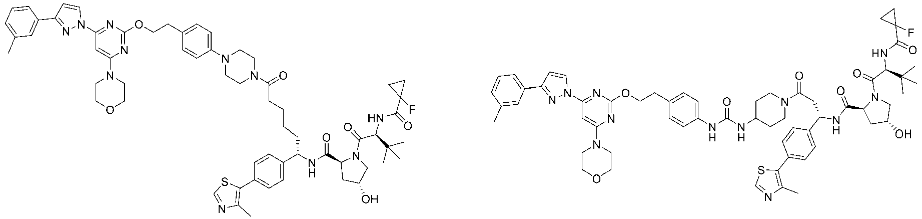

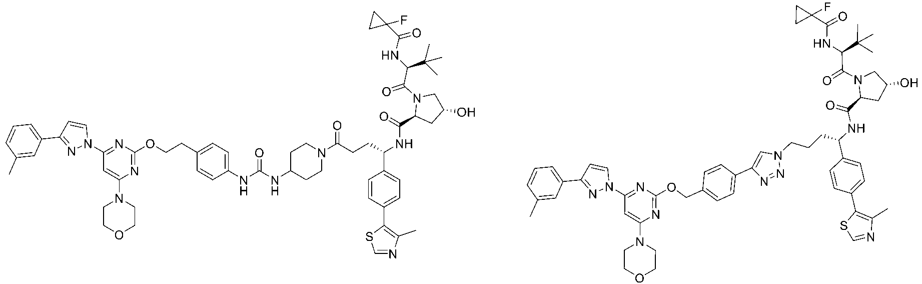

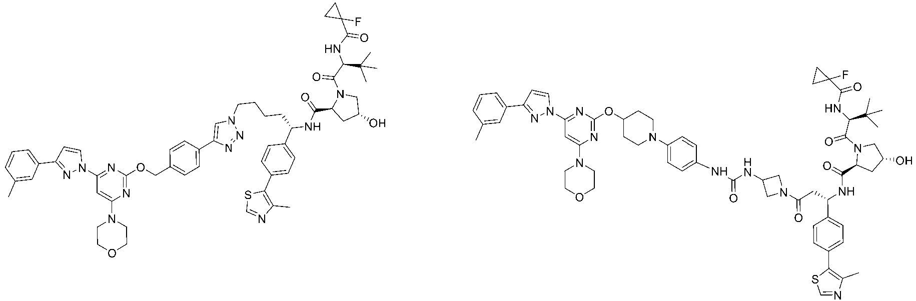

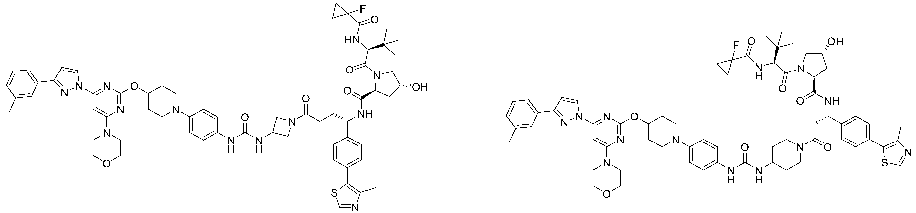

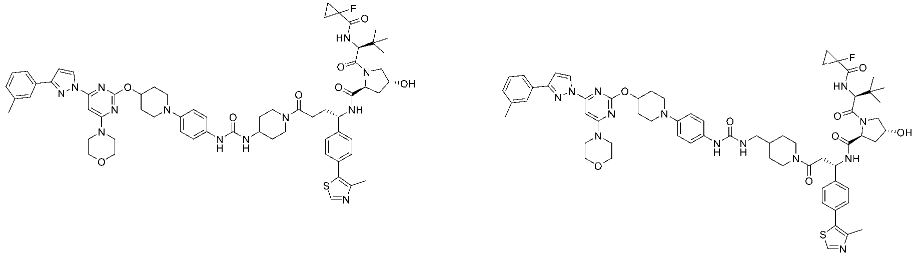

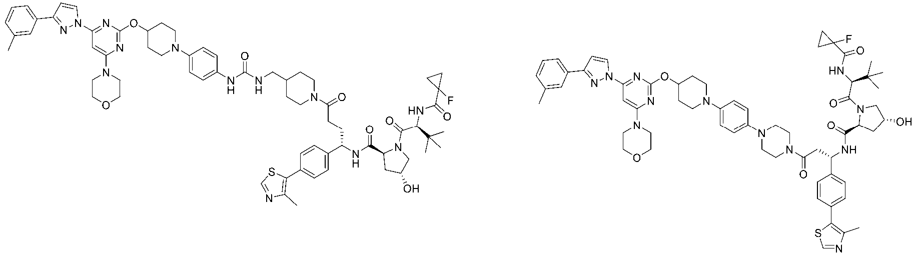

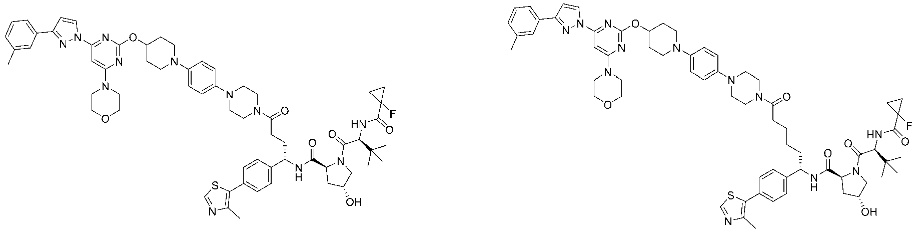

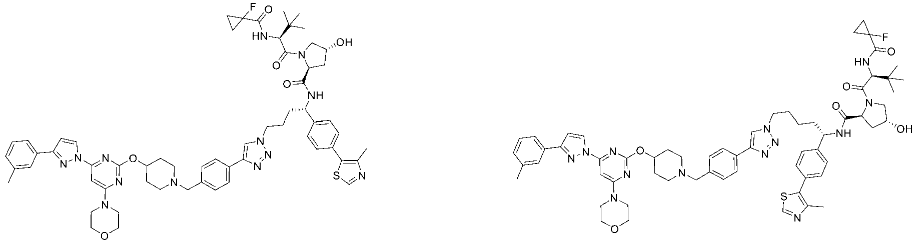

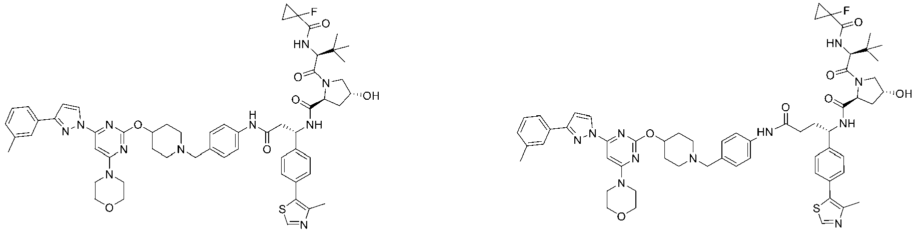

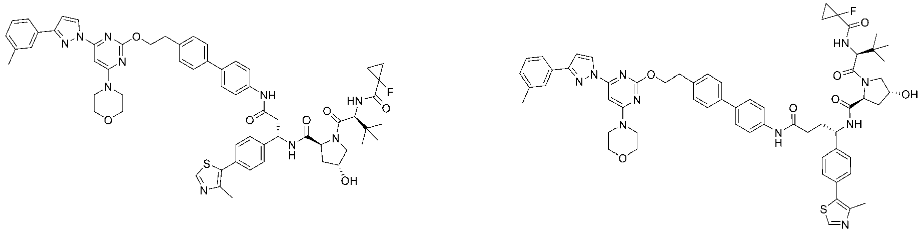

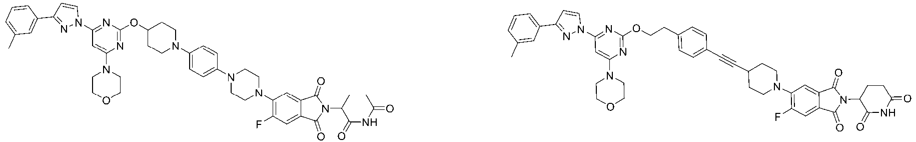

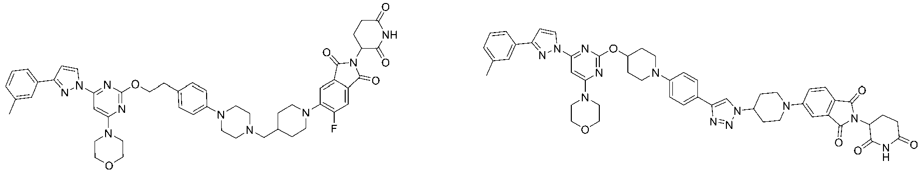

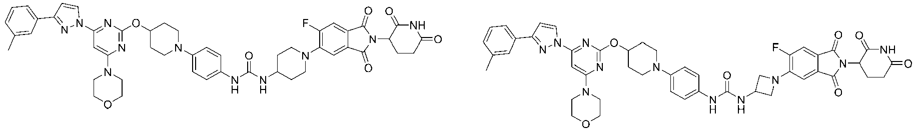

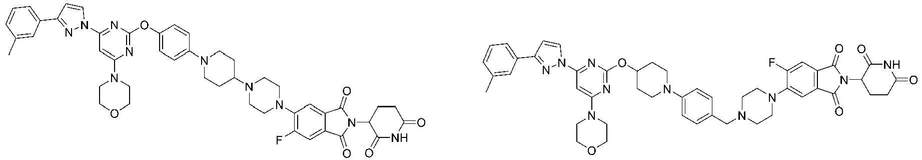

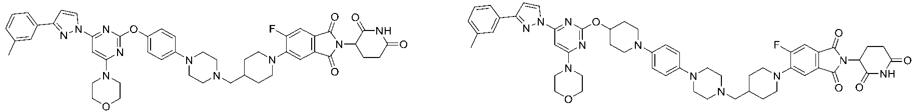

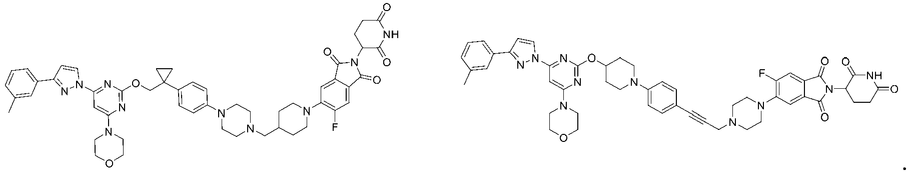

In another preferred example, the compound is selected from the group consisting of:

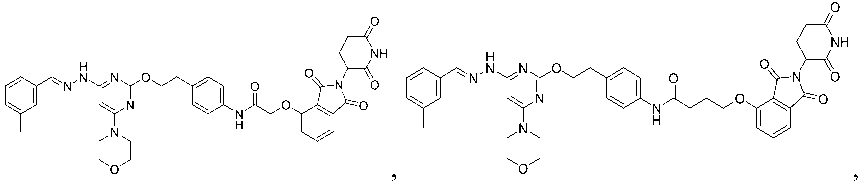

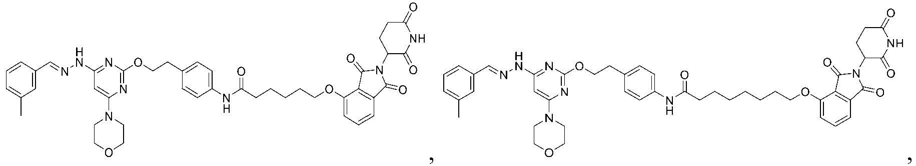

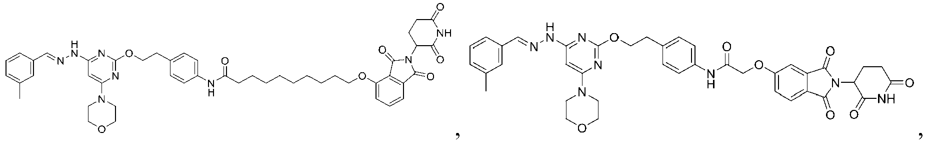

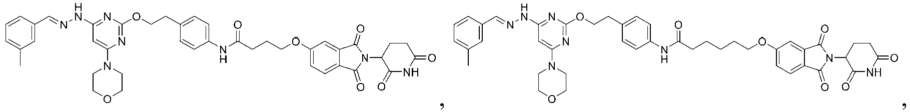

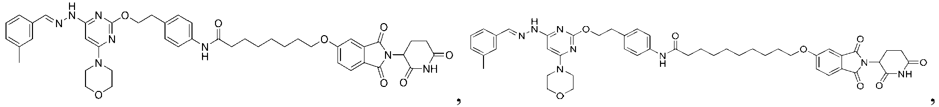

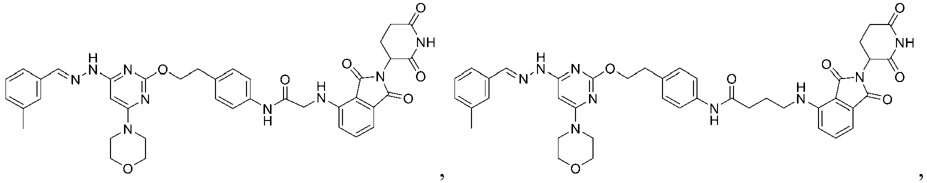

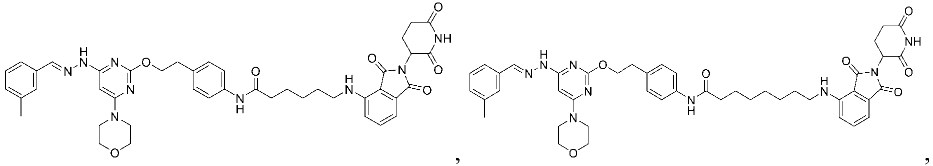

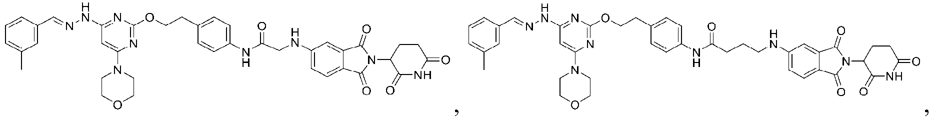

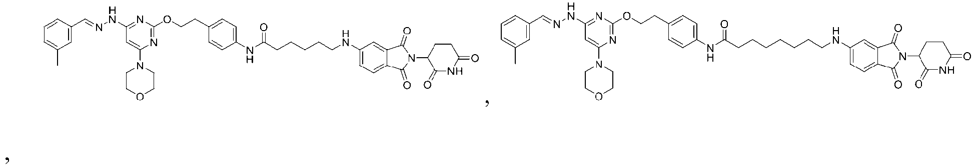

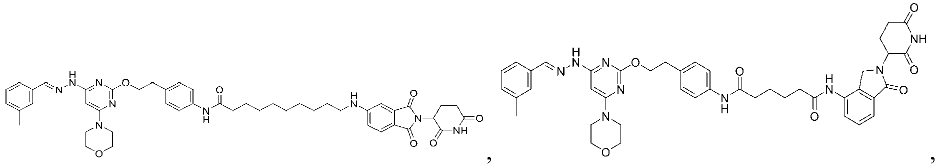

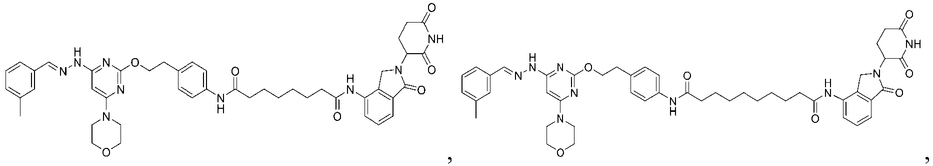

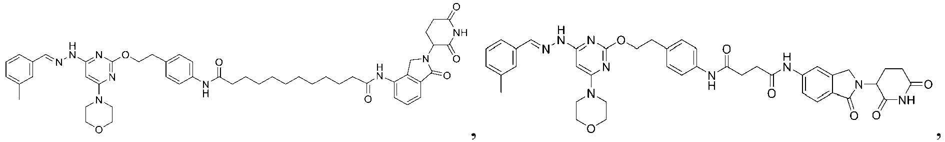

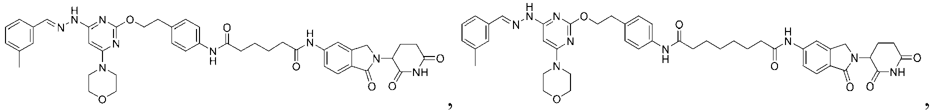

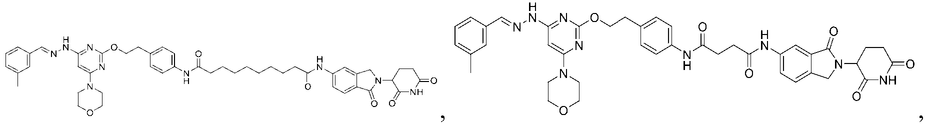

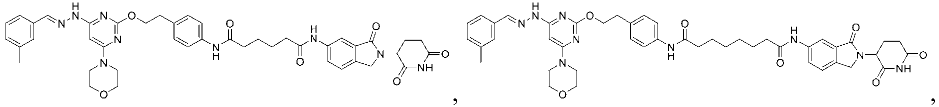

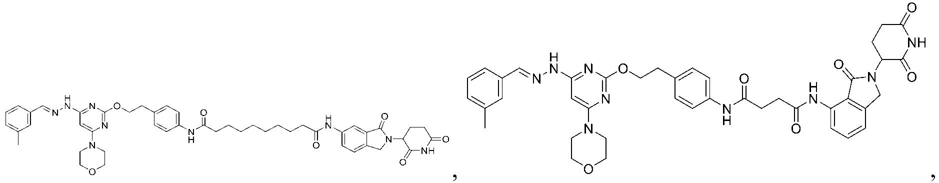

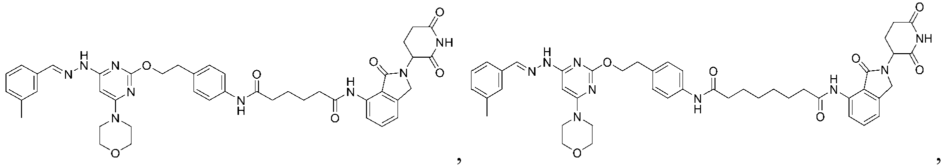

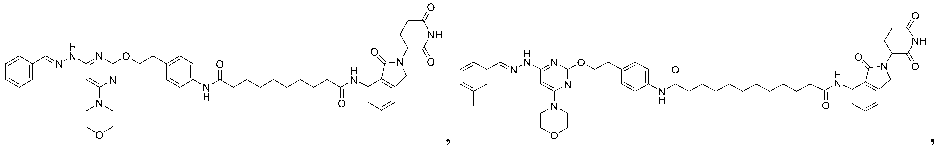

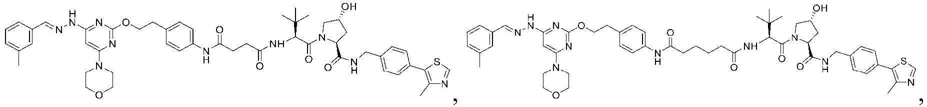

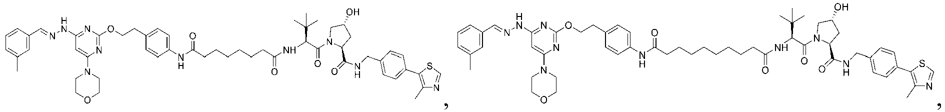

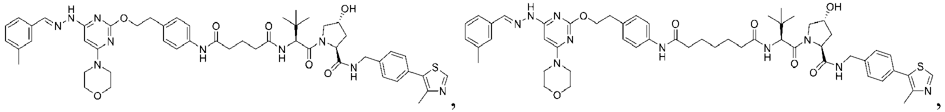

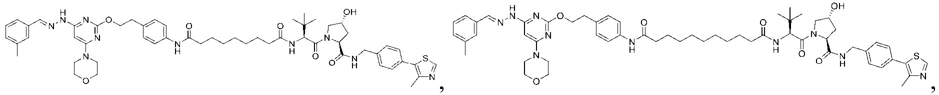

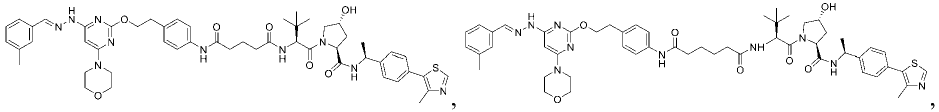

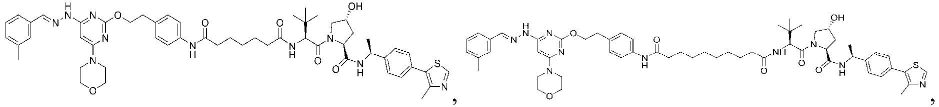

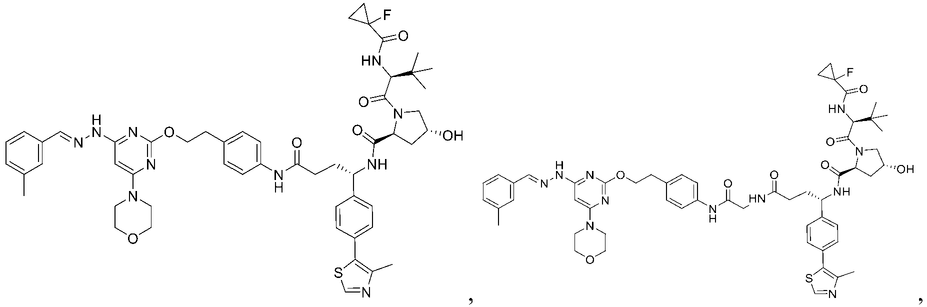

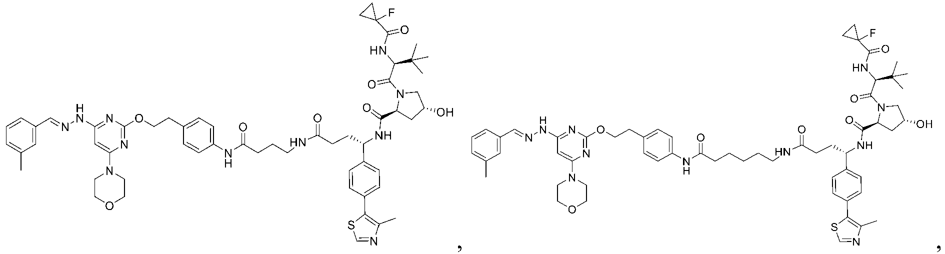

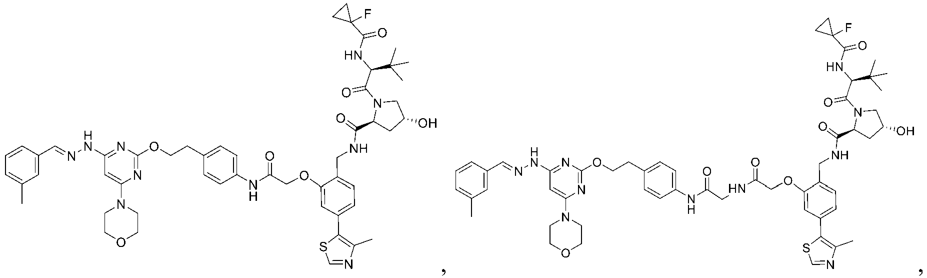

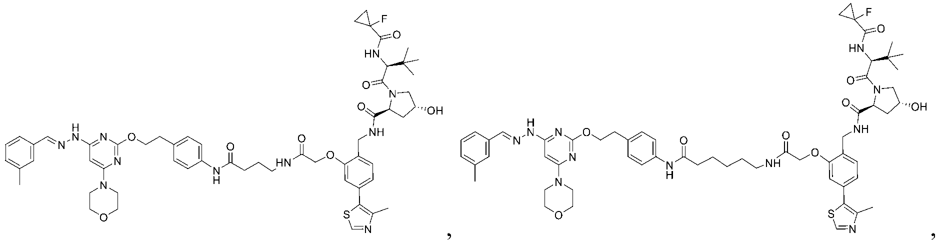

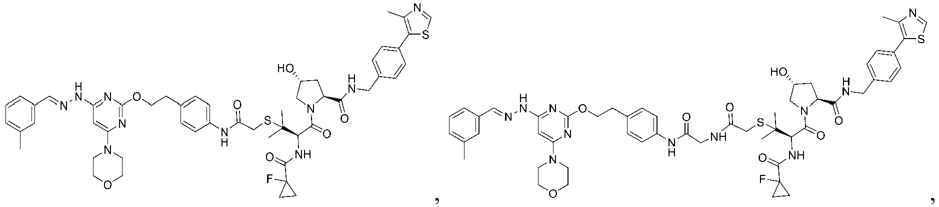

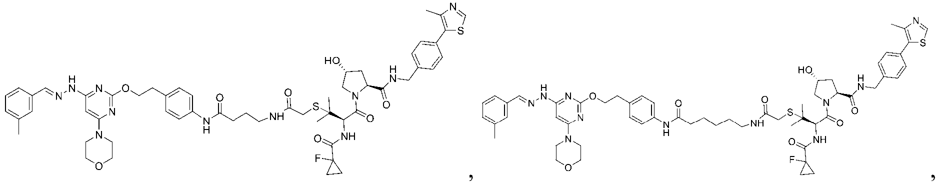

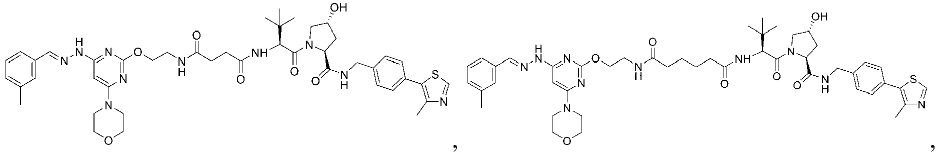

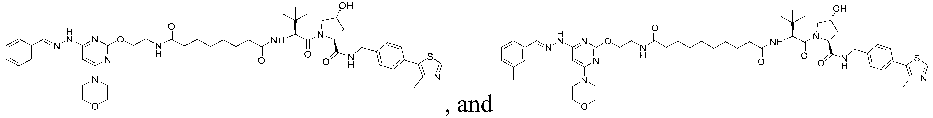

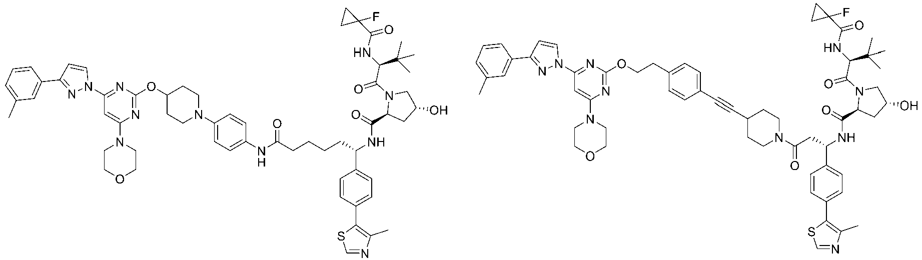

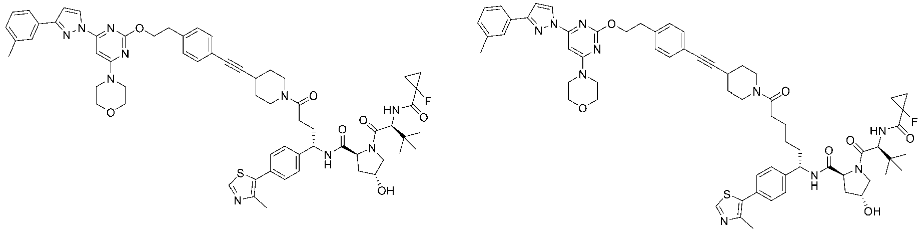

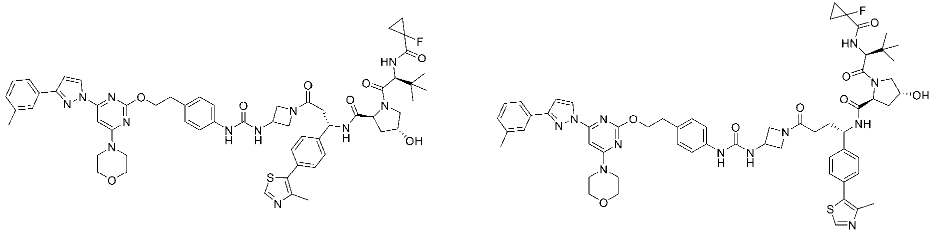

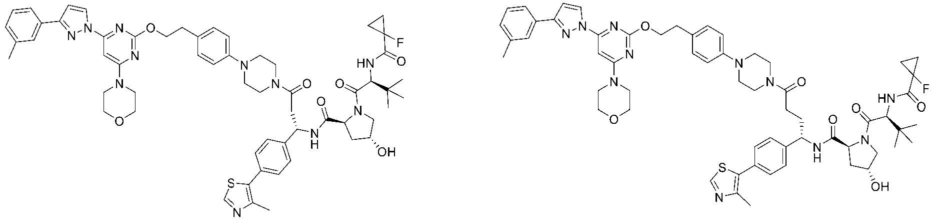

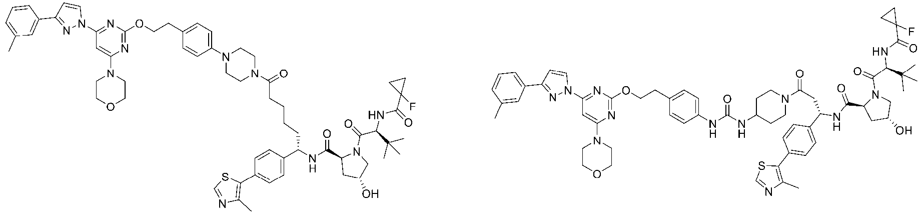

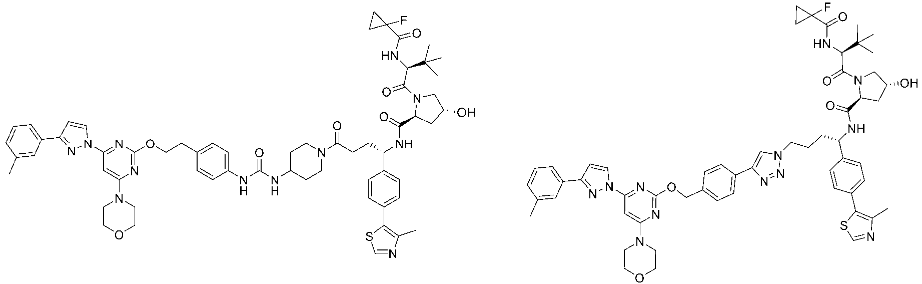

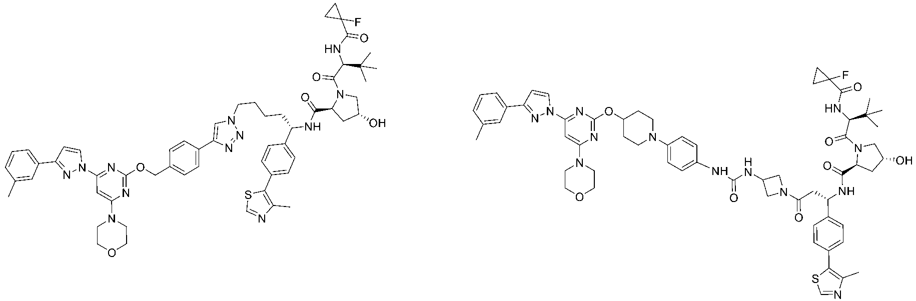

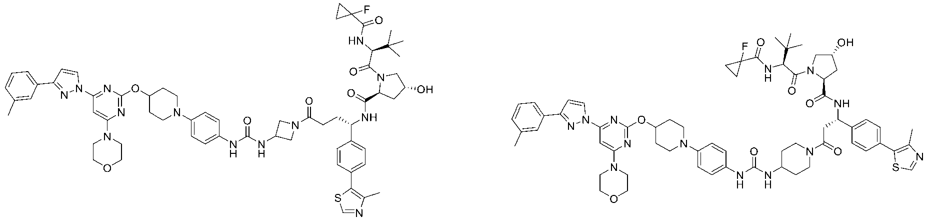

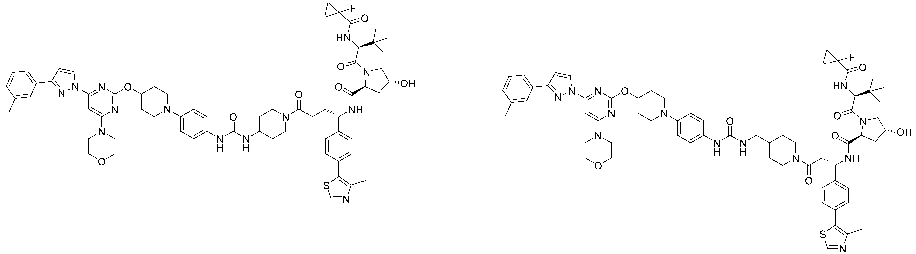

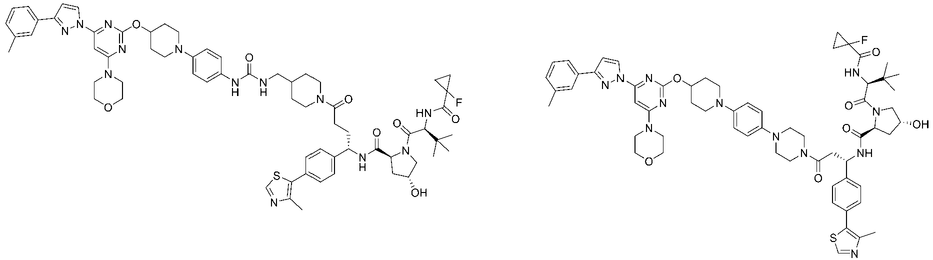

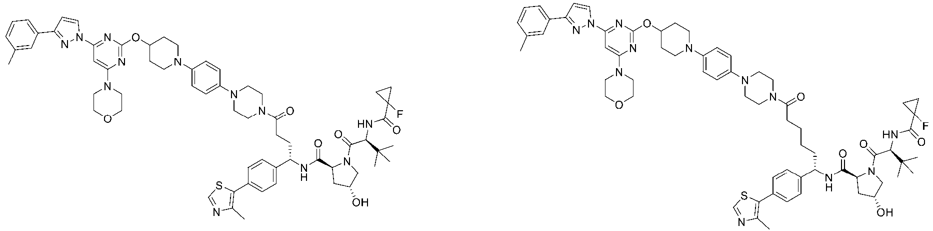

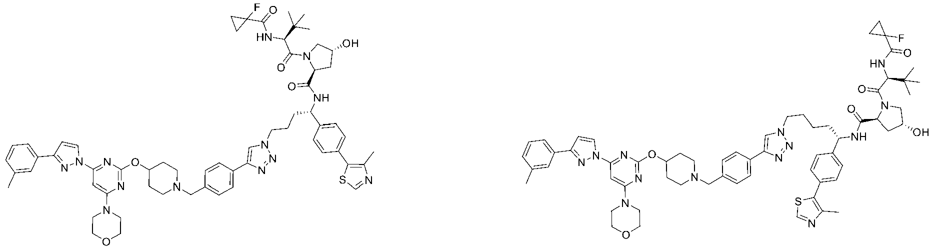

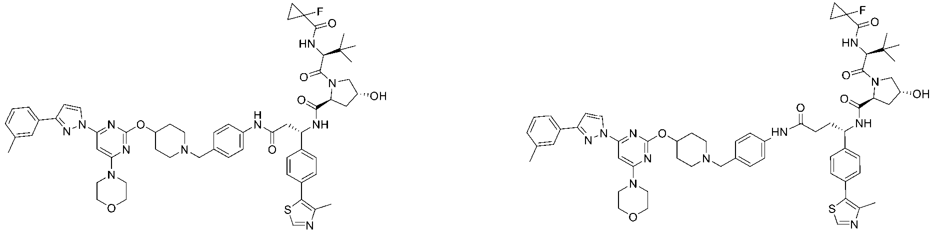

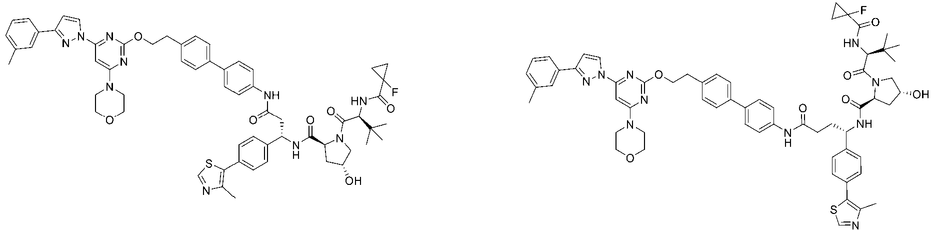

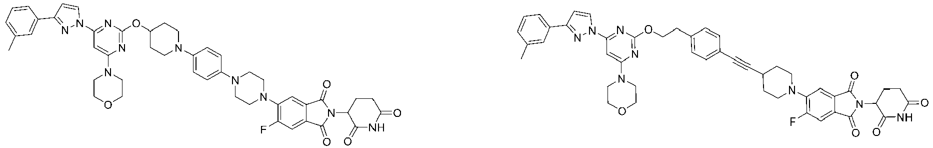

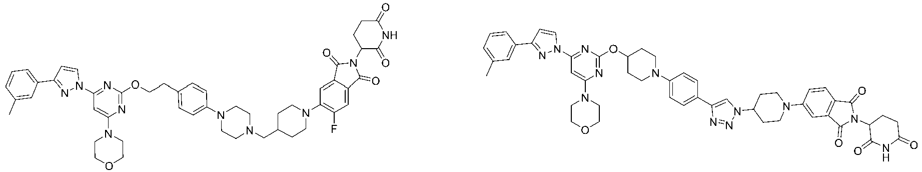

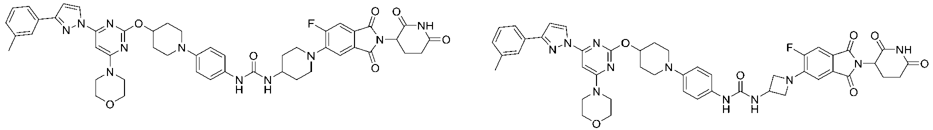

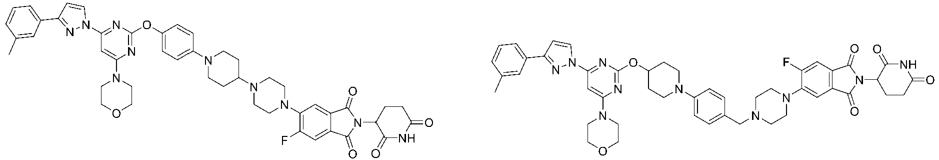

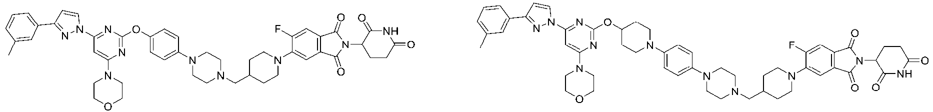

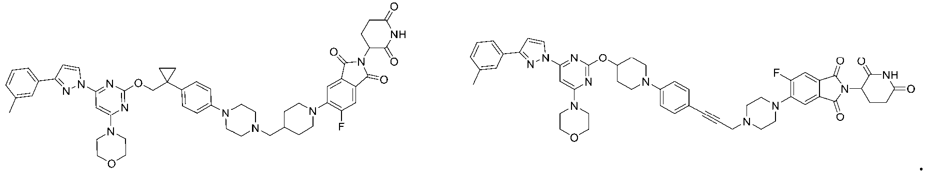

A second aspect of the present invention provides a pharmaceutical composition for treating and/or preventing a tumor, comprising:
(1) the compound or the pharmaceutically acceptable salt thereof or the stereoisomer thereof or the prodrug molecule thereof according to the first aspect of the present invention; and
   optionally (2) a pharmaceutically acceptable carrier.

A third aspect of the present invention provides use of the compound or the pharmaceutically acceptable salt thereof or the stereoisomer thereof or the prodrug molecule thereof according to the first aspect of the present invention or the pharmaceutical composition according to the second aspect of the present invention for treating and/or preventing diseases or disorders related to aberrant expression of PIKfyve.

In another preferred example, the diseases or disorders are selected from the group consisting of: tumor, autoimmune disease, virus infection, and neurodegenerative disease.

In some embodiments, the diseases or disorders are selected from the group consisting of: hematological tumor, gastrointestinal stromal tumor, histiocytic lymphoma, non-small cell lung cancer, small cell lung cancer, lung adenocarcinoma, lung squamous cell carcinoma, pancreatic cancer, breast cancer, prostate cancer, liver cancer, skin cancer, epithelial cell carcinoma, colorectal cancer, renal cancer, gastric cancer, head and neck cancer, and nasopharyngeal cancer.

A fourth aspect of the present invention provides a non-diagnostic and non-therapeutic method for degrading PIKfyve in a subject in need thereof, comprising administering to the subject the compound or the pharmaceutically acceptable salt thereof or the stereoisomer thereof or the prodrug molecule thereof according to the first aspect of the present invention or the pharmaceutical composition according to the second aspect of the present invention.

In another preferred example, the subject is a human.

It should be understood that within the scope of the present invention, the above-mentioned technical features and the technical features specifically described below (e.g., in the examples) in the present invention may be combined with each other, thus forming novel or preferred technical solutions, which are not repeated one by one here due to limited space.

### Description of Drawings

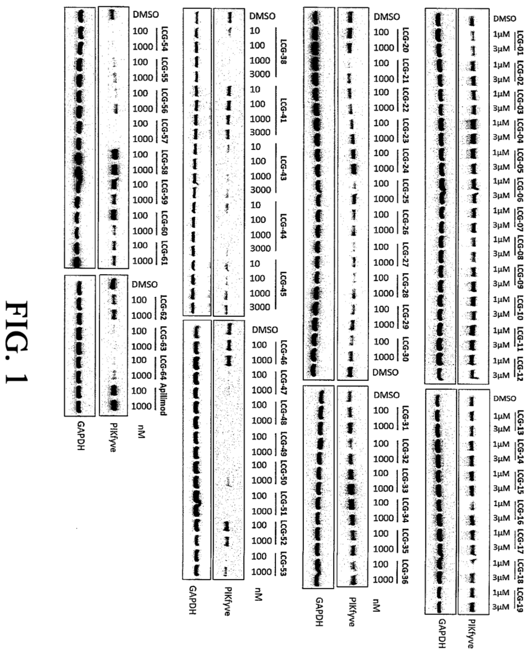

FIG. 1 shows a detection result of PIKfyve protein level in VCaP cells.

### Detailed Description

After long-term and in-depth research, the present inventors have prepared a class of compounds capable of targeting ubiquitination and degradation of PIKfyve proteins and a pharmaceutical composition thereof. The compounds can efficiently and highly selectively degrade PIKfyve proteins in cells, and therefore have potential use for treating indications related to aberrant expression of the PIKfyve proteins. Based on the above findings, the inventors have completed the present invention.

### Terms

In the compound of the present invention, when any variable (e.g., R₁ or R₂) occurs more than once in any component, the definition of the variable at each occurrence is independent of the definition of the variable at each of other occurrences. Likewise, a combination of substituents and variables is allowed, as long as such a combination makes a compound stable. A line drawn from a substituent into a ring system means that the indicated bond may be attached to any substitutable ring atom. If the ring system is polycyclic, it means that such a bond is only attached to any appropriate carbon atom on an adjacent ring. It should be understood that those of ordinary skills in the art may select a substituent and a substitution form for the compound of the present invention to provide a compound that is chemically stable and is readily synthesized from readily available starting materials in accordance with a technique in the art and a method set forth below. If a substituent itself is substituted with more than one group, it should be understood that these groups may be on the same carbon atom or on different carbon atoms, as long as the structure is stable.

Unless otherwise indicated, all technical terms and scientific terms used herein have the same meaning as commonly understood by those skilled in the art of the present invention. The terms used in the description are only for the purpose of describing specific embodiments, and are not intended to limit the present invention.

The following terms are used to describe the present invention. Where a term is not specifically defined herein, those with ordinary skills using the term in the context in which the term is used to describe the present invention provide the term with a meaning recognized in the art.

The term "Ubiquitin Ligase" as used herein refers to a family of proteins that promote the transfer of ubiquitin to a particular substrate protein, thereby targeting the substrate protein for degradation. For example, Von Hippel-Lindau E3 ubiquitin ligase or CRBN E3 ubiquitin ligase is such protein that results, alone or in combination with an E2 ubiquitin conjugase, in the attachment of ubiquitin to a lysine on a target protein and subsequent targeting of a particular protein substrate for degradation by proteasome. Therefore, E3 ubiquitin ligase, alone or complexed with the E2 ubiquitin conjugase, is responsible for the transfer of ubiquitin to the target protein. Generally, the ubiquitin ligase is involved in polyubiquitination, whereby a second ubiquitin is attached to a first ubiquitin; a third ubiquitin is attached to the second ubiquitin, and so on. Polyubiquitination labels proteins for degradation by proteasome. However, some ubiquitination events are limited to monoubiquitination, in which only a single ubiquitin is added to substrate molecules by ubiquitin ligase. Monoubiquitinated proteins are not targeted to proteasomes for degradation, but can, on the contrary, be altered in their cellular locations or functions, for example, by binding to other proteins with domains capable of binding to ubiquitins. Further complicating the situation, different lysines on the ubiquitins can be targeted by E3 for chain formation. A most common lysine is Lys48 on a ubiquitin chain. This is a lysine used to prepare polyubiquitin which is recognized by a proteasome.

As used herein, the term "alkyl" is intended to include a branched or linear saturated aliphatic hydrocarbon group having a particular number of carbon atoms. For example, the definition of "C₁-C₆" in the "C₁-C₆ alkyl" includes a group having 1, 2, 3, 4, 5, or 6 carbon atoms in a linear or branched arrangement. For example, the "C₁-₆ alkyl" specifically includes methyl, ethyl, n-propyl, isopropyl, n-butyl, sec-butyl, isobutyl, pentyl, or hexyl.

As used herein, the term "cycloalkyl" refers to a saturated or partially unsaturated monocyclic, bicyclic, or polycyclic hydrocarbon group with ring atoms being composed of carbon atoms. Bicyclic or polycyclic rings include spiro ring, fused ring, and bridged ring. For example, "cycloalkyl" includes, but is not limited to, the following groups: cyclopropyl, cyclobutyl, cyclopentyl, cyclohexyl, 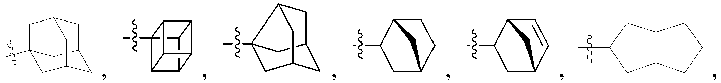 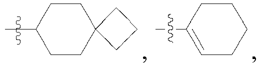 and the like.

As used herein, the term "alkoxy" refers to a group having -O-alkyl structure, such as -OCH₃, -OCH₂CH₃, -OCH₂CH₂CH₃, -O-CH₂CH(CH₃)₂, -OCH₂CH₂CH₂CH₃, -O-CH(CH₃)₂, and the like.

As used herein, the term "heterocycloalkyl" refers to a saturated or partially unsaturated monocyclic, bicyclic, or polycyclic substituent with one or more ring atoms being selected from heteroatoms of N, O, or S(O)m (wherein m is an integer of 0-2) and other ring atoms being carbon atoms. Bicyclic or polycyclic rings include spiro ring, fused ring, and bridged ring, for example: morpholinyl, piperidinyl, tetrahydropyrrolyl, pyrrolidinyl, dihydroimidazolyl, dihydroisoxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydrotetrazolyl, dihydrothiadiazolyl, dihydrothiazolyl, dihydrothiophenyl, dihydrotriazolyl, dihydroazetidinyl, tetrahydrofuranyl, tetrahydrothiophenyl, 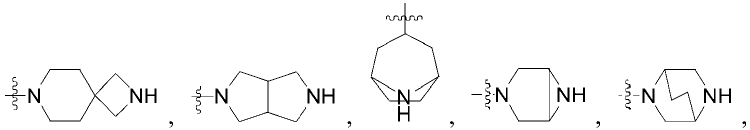 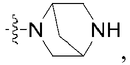 etc., and N-oxides thereof. The heterocyclic substituent can be attached through a carbon atom or through a heteroatom.

In the present application, unless otherwise specified, the term "ring" or "cyclic structure" is intended to include various forms of cyclic groups, including but not limited to: a saturated ring, a partially unsaturated but non-aromatic ring, or an aromatic ring (such as an aryl or a heteroaryl); and in particular, the ring may be a monocyclic ring or a polycyclic ring formed by a plurality of monocyclic rings, such as an aromatic or non-aromatic fused bicyclic ring, or bridged or spirocyclic structure. Unless otherwise stated, the "ring" is intended to include, but is not limited to, a cyclic structure selected from the group consisting of: an aromatic ring (including a monocyclic ring or a polycyclic ring such as a bicyclic ring), a heteroaromatic ring (including a monocyclic ring or a polycyclic ring such as a bicyclic ring), a saturated or partially unsaturated carbocyclic ring (including a monocyclic ring or a polycyclic ring such as a fused ring, a bridge ring, or a spiro ring structure), and a saturated or partially unsaturated heterocyclic ring (including a monocyclic ring or a polycyclic ring such as a fused ring, a bridged ring, or a spiro ring structure).

As used herein, the term "aryl" refers to an aromatic ring group having a particular number of carbon atoms. For example, a C₆-C₁₀ aryl represents an aromatic ring group having 6-10 carbon atoms, such as phenyl, or naphthyl, or the like.

As used herein, the term "heteroaryl" refers to an aromatic ring group having a particular number of atoms, and wherein 1-3 atoms are heteroatoms selected from N, S, and O. For example, a 5-12-membered heteroaryl represents an aromatic ring group having 5-12 carbon atoms. It may be in the form of a monocyclic ring or a fused ring. Specific examples may be pyridinyl, pyridazinyl, pyrimidinyl, pyrazinyl, triazinyl, pyrrolyl, pyrazolyl, imidazolyl, (1,2,3)-triazolyl and (1,2,4)-triazolyl, tetrazolyl, furanyl, thienyl, isoxazolyl, thiazolyl, oxazolyl, and the like.

Unless the groups in the present invention are particularly stated to be "substituted or unsubstituted," the groups in the present invention may each be substituted with a substituent selected from the group consisting of: halogen, cyano, nitro, hydroxyl, amino, a C₁-C₆ alkyl-amino, a C₁-C₆ alkyl, a C₂-C₆ alkenyl, a C₂-C₆ alkynyl, a C₁-C₆ alkoxy, a C₁-C₆ haloalkyl, a C₂-C₆ haloalkenyl, a C₂-C₆ haloalkynyl, a C₁-C₆ haloalkoxy, allyl, benzyl, a C₆-C₁₂ aryl, a C₁-C₆ alkoxy-C₁-C₆ alkyl, a C₁-C₆ alkoxy-carbonyl, phenoxycarbonyl, a C₂-C₆ alkynyl-carbonyl, a C₂-C₆ alkenyl-carbonyl, a C₃-C₆ carbocyclyl-carbonyl, a C₁-C₆ alkyl-sulfonyl, or the like.

As understood by those skilled in the art, the "halogen" or "halo" as used herein is intended to mean chlorine, fluorine, bromine, and iodine.

As used herein, the term "subject" refers to a mammal. For example, the mammal encompassed in the present invention includes humans, primates, and domesticated animals such as cattle, sheep, pigs, horses, mice, rats and humans.

Contents of the present disclosure further include isotopically labeled compounds that are the same as the compound represented by formula (I), except that one or more atoms thereof are substituted with an atom which has an atomic mass or mass number different from the atomic mass or mass number usually found in nature. Isotopes suitable for inclusion in the compounds of the present disclosure are hydrogen, carbon, nitrogen, oxygen, phosphorus, sulfur, fluorine, and chlorine, such as, but not limited to ²H, ³H, ¹³C, ¹⁴C, ¹⁵N, ¹⁸O, ³¹P, ³⁵S, ¹⁸F, and ³⁶Cl. Replacement with a heavier isotope such as deuterium (i.e., ²H) can bring some therapeutic advantages due to stronger metabolic stability, such as increasing in vivo half-life or reducing dose demand, and may therefore be more favored in some cases. Positron emission isotopes can be added to the compounds for studies on medical imaging and positron emission tomography (PET), to determine the distribution of receptors. Suitable positron emission isotopes that can be added to the compound of formula (I) include ¹¹C, ¹³N, ¹⁵O, and ¹⁸F. The isotopically labeled compound of formula (I) can generally be prepared by replacing a non-isotopically labeled reagent with a suitable isotopically labeled reagent according to a conventional technique known to those skilled in the art or a process similar to the process described herein.

The compound disclosed herein may exist in a soluble or unsoluble form in a pharmaceutically acceptable solvent, such as water or ethanol. In an embodiment, the compound is amorphous. In an embodiment, the compound is a single polymorph. In another embodiment, the compound is a mixture of polymorphs. In another embodiment, the compound is a crystalline form.

### Pharmaceutically acceptable salt

The present invention not only includes a free form of a compound of formula (I), but also includes a pharmaceutically acceptable salt thereof and a stereoisomer thereof. Some particular example compounds herein are protonated salts of amine compounds. The term "free form" refers to an amine compound in a non-salt form. The included pharmaceutically acceptable salt not only includes example salts of the particular compounds described herein, but also includes typical pharmaceutically acceptable salts in free forms of all compounds of formula I. The free forms of the particular salts of the compounds may be isolated using techniques known in the art. For example, the free form may be regenerated by treating the salt with an appropriate dilute aqueous base solution, such as a dilute aqueous NaOH solution, a dilute aqueous potassium carbonate solution, dilute aqueous ammonia, and a dilute aqueous sodium bicarbonate solution. The free forms differ somewhat from their respective salt forms in some physical properties such as solubility in a polar solvent, but for the purpose of the invention, such acidic and basic salts are otherwise pharmaceutically equivalent to their respective free forms.

The pharmaceutically acceptable salt of the present invention may be synthesized from the compound of the present invention comprising a basic moiety or an acidic moiety in accordance with a conventional chemical method. Generally, a salt of an alkali compound is prepared by ion exchange chromatography or by reacting a free base with a stoichiometric amount or excess amount of an inorganic or organic acid in a desired salt form in a suitable solvent or a combination of a plurality of solvents. Similarly, a salt of an acidic compound is formed by a reaction with a suitable inorganic or organic base.

Accordingly, the pharmaceutically acceptable salt of the compound of the present invention includes a conventional non-toxic salt of the compound of the present invention formed by reacting an alkali compound of the present invention with an inorganic or organic acid. For example, the conventional non-toxic salt not only includes a salt prepared from an inorganic acid, such as hydrochloric acid, hydrobromic acid, sulfuric acid, sulfamic acid, phosphoric acid, or nitric acid, but also includes a salt prepared from an organic acid, such as acetic acid, propionic acid, succinic acid, glycolic acid, stearic acid, lactic acid, malic acid, tartaric acid, citric acid, ascorbic acid, pamoic acid, maleic acid, hydroxymaleic acid, phenylacetic acid, glutamic acid, benzoic acid, salicylic acid, sulfanilic acid, 2-acetoxybenzoic acid, fumaric acid, toluenesulfonic acid, methylsulfonic acid, ethanedisulfonic acid, oxalic acid, isethionic acid, or trifluoroacetic acid.

If the compound of the present invention is acidic, an appropriate "pharmaceutically acceptable salt" refers to a salt prepared from a pharmaceutically acceptable non-toxic base including an inorganic base and an organic base. A salt derived from an inorganic base includes an aluminum salt, an ammonium salt, a calcium salt, a copper salt, a ferric salt, a ferrous salt, a lithium salt, a magnesium salt, a manganese salt, a manganous salt, a potassium salt, a sodium salt, a zinc salt, and the like, and is particularly preferably an ammonium salt, a calcium salt, a magnesium salt, a potassium salt, and a sodium salt. A salt derived from a pharmaceutically acceptable organic non-toxic base includes a salt of a primary amine, a secondary amine, and a tertiary amine, and a substituted amine includes a naturally occurring substituted amine, a cyclic amine, and an basic ion exchange resin, such as arginine, betaine, caffeine, choline, N,N'-dibenzylethylenediamine, diethylamine, 2-diethylaminoethanol, 2-dimethylaminoethanol, aminoethanol, ethanolamine, ethyldiamine, N-ethylmorpholine, N-ethylpiperidine, glucosamine, aminoglucose, histidine, hydroxocobalamin, isopropylamine, lysine, methylglucosamine, morpholine, piperazine, piperidine, acridine, polyamine resin, procaine, purine, theobromine, triethylamine, trimethylamine, tripropylamine, and tromethamine.

Berg et al., "Pharmaceutical Salts, "J. Pharm. Sci. '1977: 66: 1-19 describes in more detail the preparation of the pharmaceutically acceptable salt described above and other typical pharmaceutically acceptable salts.

### Drug metabolites and prodrug

The compound involved in the present invention, a metabolite of the pharmaceutically acceptable salt thereof, and a prodrug that can be converted in vivo into the structure of the compound involved in the present application and the pharmaceutically acceptable salt thereof are also encompassed in the claims of the present invention. As used herein, the term "protodrug" refers to a compound that, when metabolized (e.g., in vivo or in vitro), produces an active compound. In some embodiments, the protodrug may be inactive or less active than the free drug, but may provide advantageous treatment, administration, or metabolic properties. Example protodrug molecules of the present invention may be linked to a free drug via hydroxyl, amino, phosphate, or thiophosphate backbone of a nucleotide, and may comprise an ester, a carbamate, carbonyl, a thioester, an amide, an isocyanate, urea, thiourea, or other physiologically acceptable metabolically labile molecules. In some embodiments, the protodrug is activated by enzymatic hydrolysis.

### Pharmaceutical composition and use thereof

The present invention further provides a pharmaceutical composition, comprising an active ingredient in a range of a safe and effective amount and a pharmaceutically acceptable carrier or adjuvant. Because the active ingredient, the compound of formula I and the pharmaceutically acceptable salt thereof according to the present application have potent inhibitory effects against aberrant expression of PIKfyve proteins, the pharmaceutical composition can be used to treat, e.g., cancers, autoimmune diseases, virus infections, or neurodegenerative diseases related to aberrant expression of the PIKfyve proteins in humans or other mammals.

The present invention provides a protein kinase degradation agent and use thereof. Specifically, the present invention provides a compound having a structure represented by formula (I), or a pharmaceutically acceptable salt thereof, or a stereoisomer thereof, or a prodrug molecule thereof. The compound can degrade the PIKfyve proteins through a ubiquitination pathway, and can be used to treat indications related to aberrant expression of PIKfyve.

In an embodiment, the active ingredient of the present application or the pharmaceutical composition comprising the active ingredient can be used to prevent and/or treat tumors such as prostate cancer, non-small cell lung cancer, malignant melanoma, renal cancer, bladder cancer, ovarian cancer, colon cancer, rectal cancer, breast cancer, cervical cancer, lung cancer, laryngeal cancer, nasopharyngeal cancer, pancreatic cancer, multiple myeloma, B lymphoma, or leukemia, or prevent postoperative recurrence of a tumor.

The "active ingredient" according to the present invention refers to the compound of formula I or the pharmaceutically acceptable salt thereof or the stereoisomer thereof or the prodrug molecule thereof according to the present invention.

The "active ingredient" and the pharmaceutical composition according to the present invention can be used as a PIKfyve protein degradation agent to prepare a medicament for preventing and/or treating a cancer, an autoimmune disease, a virus infection, a neurodegenerative disease, or the like.

The "safe and effective amount" means that: the amount of the active ingredient is sufficient to obviously ameliorate the condition without causing severe side effects. Generally, the pharmaceutical composition comprises 1-2,000 mg of an active ingredient/agent, and more preferably, comprises 10-200 mg of the active ingredient/agent. Preferably, the "one dose" is one tablet.

The "pharmaceutically acceptable carrier or adjuvant" refers to: one or more compatible solid or liquid fillers or gel substances which are suitable for human use, and must be of sufficient purity and sufficiently low toxicity.

The "compatibility" herein means that the components of the composition can be admixed with the active ingredient of the present invention and with each other without obviously reducing the efficacy of the active ingredient.

Examples of pharmaceutically acceptable carrier or adjuvant moieties include cellulose and a derivative thereof (such as sodium carboxymethylcellulose, sodium ethylcellulose, and cellulose acetate), gelatin, talc, a solid lubricant (such as stearic acid and magnesium stearate), calcium sulfate, a vegetable oil (such as soybean oil, sesame oil, peanut oil, and olive oil), polyol (such as propylene glycol, glycerin, mannitol, and sorbitol), an emulsifier (such as Tween^{®}), a wetting agent (such as sodium lauryl sulfate), a colorant, a flavoring agent, a stabilizer, an antioxidant, a preservative, and pyrogen-free water.

In another preferred embodiment, the compound of formula I of the present invention can form a complex with a macromolecular compound or a polymer through non-bonding effects. In another preferred embodiment, the compound of formula I of the present invention, as a small molecule, can also be linked to a macromolecular compound or a polymer through a chemical bond. The macromolecular compound may be a biomacromolecule, such as a polysaccharide, a protein, a nucleic acid, or a polypeptide.

The administration mode of the active ingredient or the pharmaceutical composition of the present invention is not particularly limited, and the representative administration mode includes (but is not limited to): oral, intratumoral, rectal, or parenteral (intravenous, intramuscular or subcutaneous) administration.

Solid dosage forms for oral administration include capsules, tablets, pills, powders, and granules.

In these solid dosage forms, the active ingredient is mixed with at least one conventional inert excipient (or carrier), such as sodium citrate or dicalcium phosphate, or with an ingredient below:
(a) a filler or a bulking agent, such as starch, lactose, sucrose, glucose, mannitol, and silicic acid;
(b) a binder, such as hydroxymethyl cellulose, alginate, gelatin, polyvinylpyrrolidone, sucrose, and arabic gum;
(c) a humectant, such as glycerin;
(d) a disintegrant, such as agar, calcium carbonate, potato starch or tapioca starch, alginic acid, some complex silicates, and sodium carbonate;
(e) a solution retarding agent, such as paraffin;
(f) an assimilation accelerant, such as a quaternary amine compound;
(g) a wetting agent, such as cetyl alcohol and glyceryl monostearate;
(h) an adsorbent, such as kaolin; and
(i) a lubricant, such as talc, calcium stearate, magnesium stearate, solid polyethylene glycol, sodium lauryl sulfate, or a mixture thereof. In a capsule, a tablet, and a pill, the dosage form may further include a buffer agent.

The solid dosage forms may also be prepared from a coating and a shell material, such as an enteric coating and other materials well known in the art. They may comprise an opacifying agent, and an active ingredient in such a composition may be released in a portion of the digestive tract in a delayed manner. Examples of available embedding components are polymeric substances and waxes.

Liquid dosage forms for oral administration include a pharmaceutically acceptable emulsion, solution, suspension, syrup, or elixir. In addition to the active ingredient, the liquid dosage forms may comprise an inert diluent commonly used in the art, such as water or other solvents, a solubilizing agent and an emulsifier, such as ethanol, isopropanol, ethyl carbonate, ethyl acetate, propanediol, 1,3-butanediol, dimethylformamide, and an oil, in particular, cottonseed oil, peanut oil, maize germ oil, olive oil, castor oil, and sesame oil, or a mixture thereof. Besides these inert diluents, the composition may further comprise an auxiliary agent, such as a wetting agent, an emulsifier and a suspending agent, a sweetening agent, a corrigent, and a perfume.

Besides the active ingredient, the suspension may comprise a suspending agent, such as ethoxylated isooctadecanol, polyoxyethylene sorbitol and sorbitan ester, microcrystalline cellulose, aluminum methoxide, and agar, or a mixture thereof.

A composition for parenteral injection may comprise a physiologically acceptable sterile aqueous or anhydrous solution, dispersion, suspension or emulsion, and a sterile powder for redissolution into a sterile injectable solution or dispersion. An appropriate aqueous and nonaqueous carrier, diluent, solvent, or excipient comprises water, ethanol, polyol, and an appropriate mixture thereof.

The compound of the present invention may be administered alone or may be administered in combination with other therapeutic drugs.

When the pharmaceutical composition is used, a safe and effective amount of the compound of the present invention is suitable for a mammal (such as a human) in need of treatment, where the dose at the time of administration is considered a pharmaceutically effective dose, and the daily dose for a person with a body weight of 60 kg is generally 1-2,000 mg, preferably 20-500 mg. Of course, a specific dose should also be determined by considering factors, such as a route of administration and the health condition of the patient, which are all within the scope of skills of skilled physicians.

### Combined administration

The compound of formula I may be administered in combination with other drugs known to treat or ameliorate similar conditions. During combined administration, the original drug administration mode and dose remain unchanged, and the compound of formula I is administered concurrently or subsequently. When the compound of formula I is administered concurrently with one or more additional drugs, use of a pharmaceutical composition comprising one or more known drugs and the compound of formula I is preferred. Drug combination further includes administration of the compound of formula I with one or more known drugs in an overlapping time period. When the compound of formula I is administered in combination with one or more additional drugs, the compound of formula I or the known drugs may be administered at a dose lower than when they are administered alone.

The drug or the active ingredient that can be administered in combination with the compound of formula I includes, but is not limited to, an estrogen receptor modulator, an androgen receptor modulator, a retinoid receptor modulator, a cytotoxin/cytostatic, an antiproliferative agent, a protein transferase inhibitor, a HMG-CoA reductase inhibitor, a HIV protein kinase inhibitor, a reverse transcriptase inhibitor, an angiogenesis inhibitor, a cell proliferation and survival signal inhibitor, a drug interfering with cell cycle checkpoints and an apoptosis inducer, a cytotoxic drug, a tyrosine protein inhibitor, an EGFR inhibitor, a VEGFR inhibitor, a serine/threonine protein inhibitor, a Bcr-Abl inhibitor, a c-Kit inhibitor, a Met inhibitor, a Raf inhibitor, an MEK inhibitor, an MMP inhibitor, a topoisomerase inhibitor, a histidine deacetylase inhibitor, a proteasome inhibitor, a CDK inhibitor, a Bcl-2 family protein inhibitor, an MDM2 family protein inhibitor, an IAP family protein inhibitor, an STAT family protein inhibitor, a PI3K inhibitor, an AKT inhibitor, an integrin blocker, interferon-, interleukin-12, a COX-2 inhibitor, p53, a p53 activator, a VEGF antibody, an EGF antibody, a JAK inhibitor, and the like.

In an embodiment, the drug or the active ingredient that can be administered in combination with the compound of formula I includes, but is not limited to: aldesleukin, alendronic acid, interferon, alitretinoin, allopurinol, allopurinol sodium, palonosetron hydrochloride, hexamethylmelamine, aminoglutethimide, amifostine, amrubicin, amsacrine, anastrozole, dolasetron, aranesp, arglabin, arsenic trioxide, aromasin, 5-azacytidine, azathioprine, bcg vaccine or tice bcg vaccine, bestatin, betamethasone acetate, betamethasone sodium phosphate preparation, bexarotene, bleomycin sulfate, broxuridine, bortezomib, busulfan, calcitonin, alemtuzumab injection, capecitabine, carboplatin, casodex, cefesone, celmoleukin, daunorubicin, chlorambucil, cisplatin, cladribine, cladribine, clodronic acid, cyclophosphamide, cytarabine, dacarbazine, actinomycin D, daunorubicin liposome, dexamethasone, dexamethasone phosphate, estradiol valerate, denileukin-diftitox 2, methylprednisone, deslorelin, dexrazoxane, diethylstilbestrol, diflucan, docetaxel, doxifluridine, doxorubicin, dronabinol, holmium-166-chitosan complex, eligard, rasburicase, epirubicin hydrochloride, aprepitant, epirubicin, epoetin alfa, erythrogenin, eptaplatin, levomisole tablet, estradiol preparation, 17-β-estradiol, estramustine phosphate sodium, ethinyloestradiol, amifostine, hydroxyphosphoric acid, etopophos, etoposide, fadrozole, tamoxifen preparation, filgrastim, finasteride, filgrastim, floxuridine, fluconazole, fludarabine, 5-fluorodeoxyuridine monophosphate, 5-fluorouracil, halotestin, flutamide, formestan, 1-β-D-arabinofuranosylcytosine-5'-stearoyl phosphate, fotemustine, fulvestrant, gamma globulin, gemcitabine, gemtuzumab, imatinib mesylate, gliadel, goserelin, granisetron hydrochloride, histrelin, hycamtin, hydrocortisone, erythro-hydroxynonyladenine, hydroxyurea, ibritumomab tiuxetan, idarubicin, ifosfamide, interferon α, interferon-α2, interferon α-2A, interferon α-2B, interferon α-n1, interferon α-n3, interferon β, interferon γ-la, interleukin-2, intron A, gefitinib, irinotecan, granisetron hydrochloride injection, lentinan sulfate, letrozole, leucovorin, leuprolide, leuprolide acetate, levamisole, levofolinate calcium, levoid, levoid preparation, lomustine, lonidamine, dronabinol, mechlorethamine, mecobalamin, medroxyprogesterone acetate, megestrol acetate, melphalan, esterified estrogen, 6-mercaptopurine, mesna, methotrexate, methyl aminolevulinate, miltefosine, minocycline, mitomycin C, mitotane, mitoxantrone, trilostane, doxorubicin citrate liposome, nedaplatin, pegfilgrastim, oprelvekin, neupogen, nilutamide, tamoxifen, NSC-631570, recombinant human interleukinl-β, octreotide, ondansetron hydrochloride, oral solution of dehydrocortisone, oxaliplatin, paclitaxel, prednisone sodium phosphate preparation, pegaspargase, pegasys, pentostatin, picibanil, pilocarpine hydrochloride, pirarubicin, mithramycin, porfimer sodium, prednimustine, prednisolone steaglate, prednisone, premarin, procarbazine, recombinant human erythropoietin, raltitrexed, libby, rhenium-186 etidronate, rituximab, vitamin C effervescent tablet-A, romurtide, pilocarpine hydrochloride tablet, octreotide, sargramostim, semustine, sizofiran, sobuzoxane, solu-medrol, spar-fosic acid, stem cell treatment, streptozocin, strontium chloride-89, levoid, tamoxifen, tamsulosin, tasonermin, tastolactone, taxotere, teceleukin, temozolomide, teniposide, testosterone propionate, methyltestosterone, thioguanine, thiotepa, thyrotropin, tiludronic acid, topotecan, toremifene, tositumomab, trastuzumab, treosulfan, tretinoin, methotrexate tablet, trimethylmelamine, trimetrexate, triptorelin acetate, triptorelin pamoate, UFT, uridine, valrubicin, vesnarinone, vinblastine, vincristine, vindesine, vinorelbine, virulizin, dexrazoxine, zinostatin stimalamer, zofran, stable paclitaxel protein preparation, acolbifene, interferon r-lb, affinitak, aminopterin, arzoxifene, asoprisnil, atamestane, atrasentan, BAY43-9006, avastin, CCI-779, CDC-501, celebrex, cetuximab, crisnatol, cyproterone acetate, decitabine, DN-101, doxorubicin-MTC, dSLIM, dutasteride, edotecarin, eflornithine, exatecan, fenretinide, histamine dihydrochloride, histrelin hydrogel implant, holmium-166 DOTMP, ibandronic acid, interferon γ, intron-PEG, ixabepilone, keyhole limpet hemocyanin, L-651582, lanreotide, lasofoxifene, libra, lonafamib, miproxifene, minotauroate, MS-209, liposome MTP-PE, MX-6, nafarelin, nemorubicin, neovastat, nolatrexed, oblimersen, onco-TCS, osidem, paclitaxel polyglutamate, pamidronate disodium, PN-401, QS-21, quazepam, R-1549, raloxifene, onconase, 13-cis-retinoic acid, satraplatin, seocalcitol, T-138067, tarceva, docosahexaenoic acid-taxol, thymosin α1, tiazofurine, tipifarnib, tirapazamine, TLK-286, toremifene, trans-MID-lo7R, valspodar, vapreotide, vatalanib, verteporfin, vinflunine, Z-100, and zoledronic acid, or a combination thereof.

In some embodiments, the compound described herein is administered in combination with other therapeutic modes, including surgery, radiotherapy, transplant (e.g., stem cell transplant or bone marrow transplant), chemotherapy, immunotherapy, cryotherapy, and/or thermotherapy. This combined therapy can reduce the dosage of the agent and/or other agents, thereby avoiding possible toxicity or complications from various therapies.

### Therapeutic method

As described above, the compound of the present disclosure is a PIKfyve protein degradation agent, and thus the compound or the composition comprising the compound can be used to treat, prevent, and alleviate diseases related to the activity or aberrant expression of PIKfyve. In some embodiments, use of the compound disclosed herein for the manufacture of a medicament for preventing and/or treating diseases mediated by PIKfyve proteins is disclosed herein. In some embodiments, the compound disclosed herein is used to prevent and/or treat diseases mediated by PIKfyve proteins. In some embodiments, disclosed herein is a method for treating a disease mediated by a PIKfyve protein in a subject in need thereof, comprising administering to the subject an effective amount of the compound disclosed herein (e.g., the compound of formula (I)) or the pharmaceutical composition disclosed herein (e.g., a pharmaceutical composition comprising the compound of formula (I)). In some embodiments, the diseases mediated by PIKfyve proteins are tumor, autoimmune disease, virus infection, or neurodegenerative disease. In some embodiments, the diseases mediated by PIKfyve proteins are tumor selected from the group consisting of, e.g., hematological tumor, gastrointestinal stromal tumor, histocyclic lymphoma, non-small cell lung cancer, small cell lung cancer, lung adenocarcinoma, and lung cancer, lung adenocarcinoma, lung squamous cell carcinoma, pancreatic cancer, breast cancer, prostate cancer, liver cancer, skin cancer, epithelial cell carcinoma, colorectal cancer, renal cancer, gastric cancer, head and neck cancer, nasopharyngeal cancer, or the like.

### Advantages of the present invention mainly include:

A PROTAC compound with a novel structure is provided. This class of compounds can efficiently and highly selectively degrade PIKfyve proteins in cells, effectively inhibit the growth of a variety of tumor cells, and can be used to prepare an anti-tumor drug.

The present invention will be further described below with reference to specific examples. It should be understood that these examples are only used to illustrate the present invention, rather than limiting the scope of the present invention. Experimental methods without clear indication of specific conditions in the following examples are usually subject to conventional conditions such as those described in Sambrook et al., Molecular Cloning: A Laboratory Manual (New York: Cold Spring Harbor Laboratory Press, 1989), or conditions recommended by manufacturers. Unless otherwise specified, percentages and parts are calculated by weight.

Unless otherwise defined, all professional and scientific terms used herein have the same meaning familiar to those skilled in the art. In addition, any methods and materials similar or equivalent to the described content may be applied to the method of the present invention. The preferred embodiments and materials described herein are for illustrative purposes only.

The starting materials in the following examples can be obtained commercially, or prepared using methods known in the art, or prepared according to the methods described herein.

The structures of compounds are determined by nuclear magnetic resonance (1H-NMR) and/or mass spectrometry (MS). A nuclear magnetic resonance spectrometer (Bruker AV-400 or AV-500) is used for NMR measurement with a solvent of deuterated chloroform (CDCl3) or deuterated dimethyl sulfoxide (DMSO-D6), and with an internal standard of TMS. A mass spectrometer LCQAD-40000 is used for MS measurement. 200-300 mesh silica gel (produced by Qingdao Ocean Chemical Plant) is used for column chromatography.

### Examples

### Example 1: (E)-2-((2-(2,6-dioxopiperidin-3-yl)-1,3-dioxoisoindolin-4-yl)oxy)-N-(4-(2-((4-(2-(3-methylbenzylidene)hydrazino)-6-morpholinopyrimidin-2-yl)oxy)ethyl)phenyl)acetamide (LCG-1)

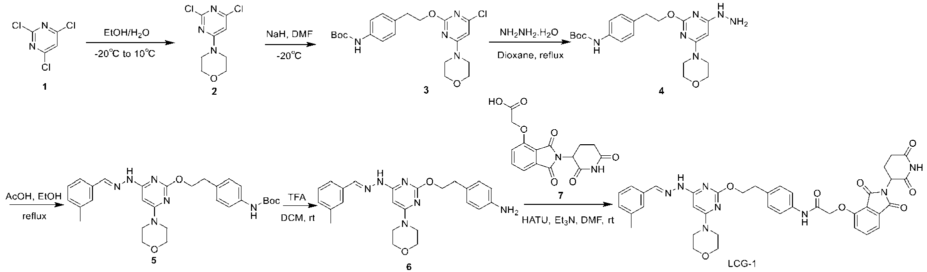

### Step 1: Preparation of 4-(2,6-dichloropyrimidin-4-yl)morpholine (2)

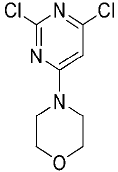

Morpholine (0.9 g, 10.9 mmol) was dissolved in 25 mL of 50% aqueous ethanol, and the mixture was stirred at a low temperature of -20°C. 2,4,6-trichloropyrimidine (1.0 g, 5.5 mmol) was dissolved in 10 mL of 50% aqueous ethanol, and the mixture was added dropwise to the aqueous morpholine-ethanol solution. The resulting mixture was kept at a low temperature of -20°C for 2h, and then adjusted to 10°C for reaction for 1 h. After completion of the reaction was monitored by TLC, 80 mL of water was added to precipitate a white solid, which was filtered, spin-dried, and subjected to column chromatography to provide 0.8 g of a white solid (62%). ¹H NMR (500 MHz, CDCl₃) δ 6.39 (s, 1H), 3.79 - 3.73 (m, 4H), 3.63 (brs, 4H). MS (ESI),m/z: 234.2 [M+H]⁺.

### Step 2: Preparation of tert-butyl (4-(2-((4-chloro-6-morpholinopyrimidin-2-yl)oxy)ethyl) phenyl)carbamate (3)

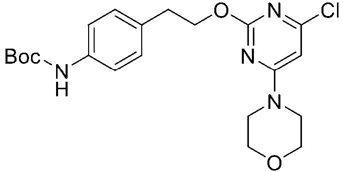

NaH (0.15 g, 6.4 mmol) was added to 40 mL of DMF, and the mixture was stirred at -20°C. Then, tert-butyl (4-(2-hydroxyethyl)phenyl)carbamate (1.0 g, 4.3 mmol) was dissolved in 5 mL of DMF solution, and the mixture was slowly added. 10 min later, a solution of 4-(2,6-dichloropyrimidin-4-yl)morpholine (2) (1.0 g, 4.3 mmol) in DMF was added, and the mixture was kept at this temperature for reaction for 12 h. After the completion of the reaction was monitored by TLC, 100 mL of water was added to precipitate a distinct white solid, which was filtered, washed with water, dried, and recrystallized with PE/EA=1:1 to provide an intermediate 3 (1.1 g, 60 %). ¹H NMR (500 MHz, CDCl₃) δ 7.28 (d, *J* = 8.2 Hz, 2H), 7.19 (d, *J* = 8.2 Hz, 2H), 6.43 (s, 1H), 6.15 (s, 1H), 4.42 (t, *J* = 7.4 Hz, 2H), 3.86 - 3.68 (m, 4H), 3.59 (brs, 4H), 3.03 (t, *J* = 7.4 Hz, 2H), 1.51 (s, 9H). MS (ESI), m/z: 435.3 [M+H]⁺.

### Step 3: Preparation of tert-butyl (4-(2-((4-hydrazino-6-morpholinopyrimidin-2-yl)oxy)ethyl) phenyl)carbamate (4)

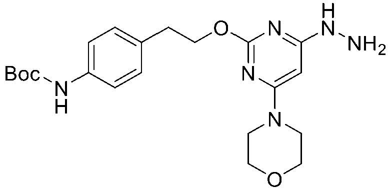

The intermediate 3 (2 g, 4.6 mmol) was dissolved in 50 mL of dioxane, 10 mL of 50% hydrazine hydrate solution was added, and the mixture was heated under reflux and stirred for 24 h. After the completion of the reaction was monitored by TLC, the mixture was rotarily evaporated under reduced pressure to provide a white solid, which was washed with 50 mL of water and dried to provide a white intermediate 4 (1.8 g, 93%). ¹H NMR (400 MHz, DMSO-*d*₆) δ 9.27 (s, 1H), 7.67 (s, 1H), 7.37 (d, *J* = 8.2 Hz, 2H), 7.14 (d, *J* = 8.2 Hz, 2H), 5.61 (s, 1H), 4.63 (brs, 3H), 4.25 (t, *J* = 7.1 Hz, 2H), 3.68 - 3.59 (m, 4H), 3.43 - 3.36 (m, 4H), 2.86 (t, *J* = 7.1 Hz, 2H), 1.46 (s, 9H). MS (ESI), m/z: 431.4 [M+H]⁺.

### Step 4: Preparation of tert-butyl (E)-(4-(2-((4-(-2-(3-methylbenzylidene)hydrazino)-6-morpholinopyrimidin-2-yl)oxy)ethyl)phenyl)carbamate (5)

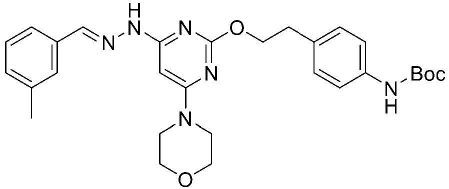

The intermediate 4 (1.7 g, 3.9 mmol) and 3-methylbenzaldehyde (0.7 g, 0.6 mmol) were dissolved in 30 mL of ethanol, 10 drops of acetic acid were added, and the mixture was refluxed for reaction for 4 h. The completion of the reaction was monitored by TLC. The mixture was rotarily evaporated under reduced pressure to provide a white solid, which was resuspended in 50 mL of a solution of PE/DCM=1:1 and filtered to provide a white intermediate 5 (1.7 g, 84 %). ¹H NMR (400 MHz, DMSO-*d*₆) δ 10.87 (s, 1H), 9.26 (s, 1H), 7.99 (s, 1H), 7.50 (d, *J* = 8.1 Hz, 2H), 7.37 (d, *J=* 8.1 Hz, 2H), 7.29 (t, *J* = 7.5 Hz, 1H), 7.20 - 7.10 (m, 3H), 6.06 (s, 1H), 4.32 (t, *J* = 7.0 Hz, 2H), 3.74 - 3.62 (m, 4H), 3.57 - 3.50 (m, 4H), 2.90 (t, *J=* 7.0 Hz, 2H), 2.34 (s, 3H), 1.47 (s, 9H). MS (ESI), m/z: 533.1 [M+H]⁺.

### Step 5: Preparation of (E)-4-(2-((4-(2-(3-methylbenzylidene)hydrazino)-6-morpholinopyrimidin-2-yl)oxy)ethyl)aniline (6)

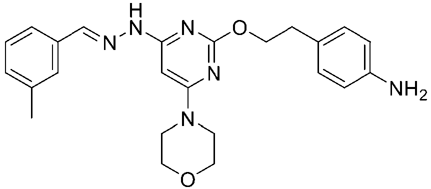

The intermediate 5 (100 mg, 0.2 mmol) was added to 10 mL of a DCM solution containing 25% TFA, and the mixture was stirred at room temperature for 4 h. The completion of the reaction was monitored by TLC. The mixture was rotarily evaporated under reduced pressure to provide an oil, which was dissolved in DCM, backwashed with a saturated NaHCO₃ solution twice, and spin-dried to provide an intermediate 6 (65 mg, 81%). ¹H NMR (500 MHz, DMSO-*d*₆) δ 10.83 (s, 1H), 7.97 (s, 1H), 7.48 (d, *J* = 7.8 Hz, 1H), 7.45 (s, 1H), 7.27 (t, *J* = 7.6 Hz, 1H), 7.15 (d, *J* = 7.6 Hz, 1H), 6.91 (d, *J* = 8.2 Hz, 2H), 6.48 (d, *J* = 8.2 Hz, 2H), 6.04 (s, 1H), 4.86 (s, 2H), 4.23 (t, *J* = 7.3 Hz, 2H), 3.70 - 3.62 (m, 4H), 3.55 - 3.48 (m, 4H), 2.77 (t, *J* = 7.3 Hz, 2H), 2.32 (s, 3H). MS (ESI), m/z: 433.3 [M+H]⁺.

### Step 6: Preparation of (E)-2-((2-(2,6-dioxopiperidin-3-yl)-1,3-dioxoisoindolin-5-yl)oxy)-N-(4-(2-((4-(2-(3-methylbenzylidene)hydrazino)-6-morpholinopyrimidin-2-yl)oxy)ethyl)phenyl)acetamide (LCG-1)

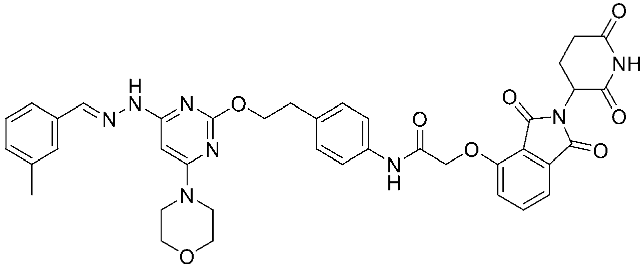

In a 25 mL eggplant-shaped flask, the intermediate 5 (50 mg, 0.1 mmol) and 2-((2-(2,6-dioxopiperidin-3-yl)-1,3-dioxoisoindolin-4-yl)oxy)acetic acid (7) (40 mg, 0.1 mmol) were dissolved in 10 mL of DMF, HATU (70 mg, 0.2 mmol) was added, finally triethylamine (40 mg, 0.3 mmol) was added, and the mixture was stirred at room temperature for 5 h. After the reaction was quenched with water, the mixture was extracted with EtOAc, backwashed with saturated brine, dried over Na₂SO₄, filtered, spin-dried, and then purified by flash column chromatography (DCM/MeOH) to provide 42 mg of a white solid product (42%). ¹H NMR (600 MHz, DMSO-*d*₆) δ 11.13 (s, 1H), 10.85 (s, 1H), 10.05 (s, 1H), 7.99 (s, 1H), 7.86 - 7.79 (m, 1H), 7.55 (d, *J* = 8.4 Hz, 2H), 7.53 - 7.45 (m, 4H), 7.32 - 7.25 (m, 3H), 7.17 (d, *J* = 7.5 Hz, 1H), 6.06 (s, 1H), 5.14 (dd, *J* = 12.8, 5.4 Hz, 1H), 5.00 (s, 2H), 4.35 (t, *J* = 7.0 Hz, 2H), 3.71 - 3.63 (m, 4H), 3.58 - 3.50 (m, 4H), 3.01 - 2.86 (m, 3H), 2.66 - 2.52 (m, 2H), 2.34 (s, 3H), 2.09 - 2.02 (m, 1H). ¹³C NMR (150 MHz, DMSO-*d*₆) δ 173.27, 170.39, 167.23, 166.06, 165.97, 164.84, 164.43, 163.99, 155.66, 141.69, 138.40, 137.45, 136.92, 135.23, 134.48, 133.54, 130.24, 129.81, 129.08, 127.49, 124.02, 120.97, 119.86, 117.24, 116.57, 76.14, 68.10, 66.94, 66.38, 49.32, 44.69, 34.75, 31.43, 22.47, 21.41. HRMS (ESI) calcd for C₃₉H₃₈N₈O₈ [M + H]⁺ 747.2891, found 747.2870.

### Example 2: (E)-4-((2-(2,6-dioxopiperidin-3-yl)-1,3-dioxoisoindolin-4-yl)oxy)-N-(4-(2-((4-(2-(3-methylbenzylidene)hydrazino)-6-morpholinopyrimidin-2-yl)oxy)ethyl)phenyl)butanamide (LCG-2)

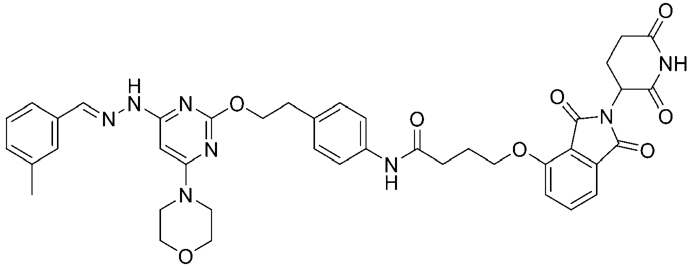

The synthesis method was as shown in Example 1.

¹H NMR (600 MHz, DMSO-*d*₆) δ 11.12 (s, 1H), 10.86 (s, 1H), 9.93 (s, 1H), 8.00 (s, 1H), 7.81 (dd, *J* = 8.3, 7.5 Hz, 1H), 7.54 (t, *J=* 8.1 Hz, 3H), 7.50 (d, *J =* 7.9 Hz, 1H), 7.48 (s, 1H), 7.46 (d, *J* = 7.2 Hz, 1H), 7.29 (t, *J* = 7.6 Hz, 1H), 7.21 (d, *J* = 8.5 Hz, 2H), 7.17 (d, *J* = 7.5 Hz, 1H), 6.07 (s, 1H), 5.09 (dd, *J* = 12.9, 5.5 Hz, 1H), 4.34 (t, *J* = 7.0 Hz, 2H), 4.28 (t, *J =* 6.2 Hz, 2H), 3.71 - 3.65 (m, 4H), 3.58 - 3.51 (m, 4H), 2.96 - 2.85 (m, 3H), 2.66 - 2.52 (m, 4H), 2.34 (s, 3H), 2.14 - 2.06 (m, 2H), 2.06 - 1.99 (m, 1H). ¹³C NMR (150 MHz, DMSO-*d*₆) δ 173.28, 170.82, 170.44, 167.32, 165.83, 164.85, 164.44, 163.99, 156.35, 141.69, 138.39, 137.98, 137.55, 135.24, 133.72, 133.52, 130.23, 129.56, 129.07, 127.48, 124.02, 120.28, 119.66, 116.76, 115.75, 76.13, 68.70, 67.01, 66.38, 49.22, 44.68, 34.74, 32.72, 31.43, 24.76, 22.49, 21.40. HRMS (ESI) calcd for C₄₁H₄₂N₈O₈ [M + H]⁺ 775.3204, found 775.3197.

### Example 3: (E)-6-((2-(2,6-dioxopiperidin-3-yl)-1,3-dioxoisoindolin-4-yl)oxy)-N-(4-(2-((4-(2-(3-methylbenzylidene)hydrazino)-6-morpholinopyrimidin-2-yl)oxy)ethyl)phenyl)hexanamide (LCG-3)

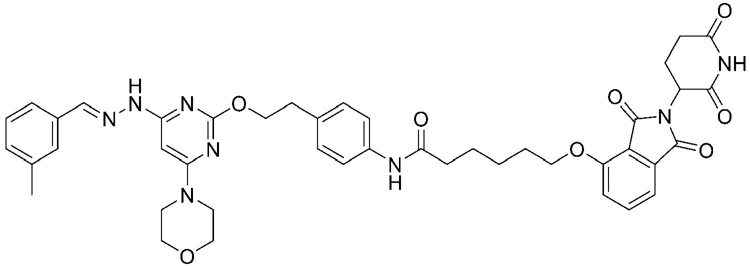

The synthesis method was as shown in Example 1.

¹H NMR (600 MHz, DMSO-*d*₆) δ 11.11 (s, 1H), 10.87 (brs, 1H), 9.84 (s, 1H), 8.00 (s, 1H), 7.80 (dd, *J* = 8.4, 7.3 Hz, 1H), 7.55 - 7.47 (m, 5H), 7.44 (d, *J* = 7.2 Hz, 1H), 7.29 (t, *J* = 7.5 Hz, 1H), 7.20 (d, *J* = 8.5 Hz, 2H), 7.17 (d, *J =* 7.5 Hz, 1H), 6.06 (s, 1H), 5.08 (dd, *J =* 12.9, 5.5 Hz, 1H), 4.35 (t, *J* = 7.0 Hz, 2H), 4.21 (t, *J* = 6.4 Hz, 2H), 3.72 - 3.63 (m, 4H), 3.59 - 3.49 (m, 4H), 2.97 - 2.81 (m, 3H), 2.63 - 2.51 (m, 2H), 2.36 - 2.30 (m, 5H), 2.05 - 1.99 (m, 1H), 1.83 - 1.77 (m, 2H), 1.70 - 1.64 (m, 2H), 1.54 - 1.47 (m, 2H). ¹³C NMR (150 MHz, DMSO-*d*₆) δ 173.27, 171.46, 170.44, 167.32, 165.79, 156.46, 138.39, 138.07, 137.49, 135.21, 133.71, 133.42, 130.26, 129.54, 129.07, 127.51, 124.05, 120.24, 119.61, 116.68, 115.61, 76.11, 69.15, 66.37, 49.21, 44.70, 36.82, 34.73, 31.44, 28.72, 25.51, 25.34, 22.48, 21.41. HRMS (ESI) calcd for C₄₃H₄₆N₈O₈ [M + H]⁺ 803.3517, found 803.3509.

### Example 4: (E)-8-((2-(2,6-dioxopiperidin-3-yl)-1,3-dioxoisoindolin-4-yl)oxy)-N-(4-(2-((4-(2-(3-methylbenzylidene)hydrazino)-6-morpholinopyrimidin-2-yl)oxy)ethyl)phenyl)octanamide (LCG-4)

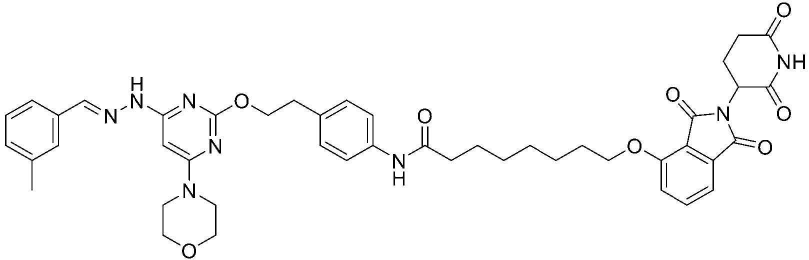

The synthesis method was as shown in Example 1.

¹H NMR (600 MHz, DMSO-*d*₆) δ 11.11 (s, 1H), 10.85 (s, 1H), 9.80 (s, 1H), 7.99 (s, 1H), 7.80 (dd, *J* = 8.4, 7.4 Hz, 1H), 7.53 - 7.47 (m, 5H), 7.43 (d, *J =* 7.2 Hz, 1H), 7.29 (t, *J =* 7.6 Hz, 1H), 7.20 (d, *J =* 8.5 Hz, 2H), 7.17 (d, *J =* 7.6 Hz, 1H), 6.07 (s, 1H), 5.08 (dd, *J =* 12.8, 5.5 Hz, 1H), 4.34 (t, *J =* 7.0 Hz, 2H), 4.20 (t, *J* = 6.4 Hz, 2H), 3.69 - 3.63 (m, 4H), 3.55 - 3.49 (m, 4H), 2.95 - 2.83 (m, 3H), 2.61 - 2.51 (m, 2H), 2.34 (s, 3H), 2.29 (t, *J* = 7.4 Hz, 2H), 2.06 - 1.99 (m, 1H), 1.80 - 1.72 (m, 2H), 1.63 - 1.56 (m, 2H), 1.50 - 1.43 (m, 2H), 1.41 - 1.30 (m, 4H). ¹³C NMR (150 MHz, DMSO-*d*₆) δ 170.95, 169.25, 168.13, 165.02, 163.48, 162.53, 162.12, 161.67, 154.17, 139.37, 136.08, 135.77, 135.17, 132.92, 131.41, 131.11, 127.92, 127.24, 126.76, 125.18, 121.71, 117.92, 117.27, 114.38, 113.28, 73.82, 66.92, 64.70, 64.07, 46.89, 42.37, 34.52, 32.43, 29.12, 26.79, 26.62, 26.55, 23.35, 23.26, 20.16, 19.10. HRMS (ESI) calcd for C₄₅H₅₀N₈O₈ [M + H]⁺ 831.3830, found 831.3828.

### Example 5: (E)-10-((2-(2,6-dioxopiperidin-3-yl)-1,3-dioxoisoindolin-4-yl)oxy)-N-(4-(2-((4-(2-(3-methylbenzylidene)hydrazino)-6-morpholinopyrimidin-2-yl)oxy)ethyl)phenyl)decanamide (LCG-5)

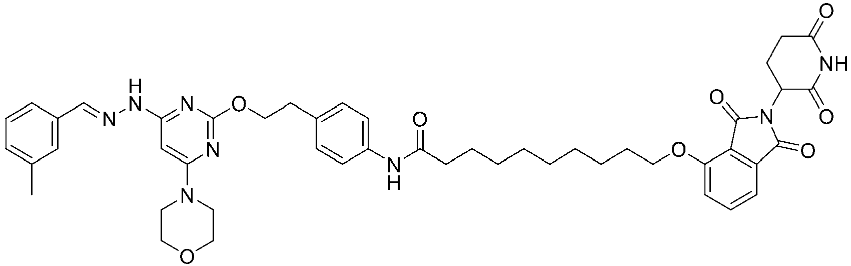

The synthesis method was as shown in Example 1.

¹H NMR (600 MHz, DMSO-*d*₆) δ 11.10 (brs, 1H), 10.86 (s, 1H), 9.80 (s, 1H), 7.99 (s, 1H), 7.79 (dd, *J* = 8.4, 7.4 Hz, 1H), 7.54 - 7.47 (m, 5H), 7.43 (d, *J* = 7.2 Hz, 1H), 7.29 (t, *J =* 7.6 Hz, 1H), 7.20 (d, *J =* 8.5 Hz, 2H), 7.17 (d, *J =* 7.5 Hz, 1H), 6.07 (s, 1H), 5.08 (dd, *J* = 12.9, 5.5 Hz, 1H), 4.34 (t, *J* = 7.0 Hz, 2H), 4.18 (t, *J* = 6.4 Hz, 2H), 3.70 - 3.64 (m, 4H), 3.56 - 3.51 (m, 4H), 2.93 (t, *J* = 7.1 Hz, 2H), 2.91 - 2.84 (m, 1H), 2.64 - 2.53 (m, 2H), 2.34 (s, 3H), 2.28 (t, *J* = 7.4 Hz, 2H), 2.06 - 2.00 (m, 1H), 1.78 - 1.71 (m, 2H), 1.62 - 1.55 (m, 2H), 1.48 - 1.42 (m, 2H), 1.35 - 1.28 (m, 8H). ¹³C NMR (150 MHz, DMSO-*d*₆) δ 173.26, 171.58, 170.44, 167.33, 165.78, 164.84, 164.44, 163.99, 156.49, 141.67, 138.39, 138.09, 137.48, 135.24, 133.72, 133.42, 130.22, 129.54, 129.07, 127.48, 124.01, 120.22, 119.57, 116.68, 115.58, 76.13, 69.25, 67.01, 66.38, 49.20, 44.68, 36.86, 34.75, 31.43, 29.35, 29.22, 29.14, 29.13, 28.90, 25.73, 25.63, 22.48, 21.40. HRMS (ESI) calcd for C₄₇H₅₄N₈O₈ [M + H]⁺ 859.4143, found 859.4142.

### Example 6: (E)-2-((2-(2,6-dioxopiperidin-3-yl)-1,3-dioxoisoindolin-5-yl)oxy)-N-(4-(2-((4-(2-(3-methylbenzylidene)hydrazino)-6-morpholinopyrimidin-2-yl)oxy)ethyl)phenyl)acetamide (LCG-6)

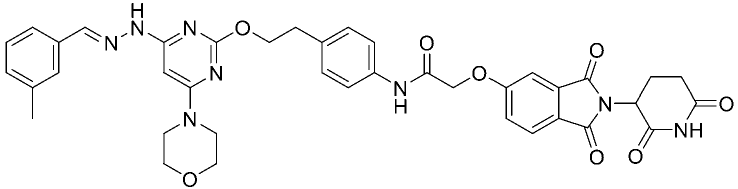

The synthesis method was as shown in Example 1.

¹H NMR (600 MHz, DMSO-*d*₆) δ 11.12 (s, 1H), 10.99 (brs, 1H), 10.14 (s, 1H), 8.02 (s, 1H), 7.88 (d, *J* = 8.3 Hz, 1H), 7.64 - 7.48 (m, 5H), 7.44 (d, *J* = 8.2 Hz, 1H), 7.35 - 7.23 (m, 3H), 7.19 (d, *J* = 7.3 Hz, 1H), 6.03 (s, 1H), 5.13 (dd, *J* = 12.9, 5.3 Hz, 1H), 4.96 (s, 2H), 4.41 (s, 2H), 3.67 (s, 4H), 3.56 (s, 4H), 2.98 (s, 2H), 2.93 - 2.84 (m, 1H), 2.65 - 2.52 (m, 2H), 2.34 (s, 3H), 2.12 - 1.99 (m, 1H). ¹³C NMR (150 MHz, DMSO-*d*₆) δ 173.25, 170.38, 167.30, 167.20, 166.02, 163.73, 151.62, 138.41, 137.02, 134.98, 134.23, 130.50, 129.74, 129.35, 129.07, 127.72, 125.81, 124.27, 124.04, 121.45, 121.22, 120.27, 109.78, 75.95, 67.88, 66.32, 49.47, 44.83, 34.61, 31.42, 22.52, 21.41. HRMS (ESI) calcd for C₃₉H₃₈N₈O₈ [M + H]⁺ 747.2891, found 747.2875.

### Example 7: (E)-4-((2-(2,6-dioxopiperidin-3-yl)-1,3-dioxoisoindolin-5-yl)oxy)-N-(4-(2-((4-(2-(3-methylbenzylidene)hydrazino)-6-morpholinopyrimidin-2-yl)oxy)ethyl)phenyl)butanamide (LCG-7)

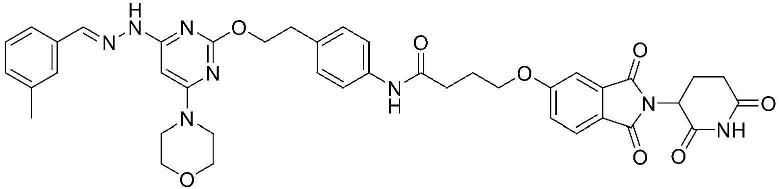

The synthesis method was as shown in Example 1.

¹H NMR (600 MHz, DMSO-*d*₆) δ 11.11 (s, 1H), 10.85 (s, 1H), 9.91 (s, 1H), 7.99 (s, 1H), 7.83 (d, *J* = 8.3 Hz, 1H), 7.52 (d, *J* = 8.5 Hz, 2H), 7.50 (d, *J* = 7.9 Hz, 1H), 7.47 (s, 1H), 7.44 (d, *J* = 2.2 Hz, 1H), 7.35 (dd, *J* = 8.3, 2.3 Hz, 1H), 7.29 (t, *J* = 7.6 Hz, 1H), 7.21 (d, *J* = 8.5 Hz, 2H), 7.17 (d, *J=* 7.5 Hz, 1H), 6.06 (s, 1H), 5.12 (dd, *J=* 12.9, 5.4 Hz, 1H), 4.34 (t, *J=* 7.1 Hz, 2H), 4.24 (t, *J=* 6.4 Hz, 2H), 3.71 - 3.62 (m, 4H), 3.57 - 3.49 (m, 4H), 2.93 (t, *J* = 7.0 Hz, 2H), 2.91 - 2.84 (m, 1H), 2.63 - 2.52 (m, 2H), 2.34 (s, 3H), 2.10 - 2.02 (m, 3H). ¹³C NMR (150 MHz, DMSO-*d*₆) δ 172.17, 169.74, 169.33, 166.27, 166.20, 163.77, 163.36, 162.91, 140.60, 137.32, 136.90, 134.16, 133.35, 132.46, 129.16, 128.50, 128.00, 126.42, 124.73, 122.94, 122.37, 120.10, 118.56, 108.34, 75.05, 67.69, 65.92, 65.30, 48.36, 43.61, 33.67, 31.92, 30.35, 23.74, 21.47, 20.33. HRMS (ESI) calcd for C₄₁H₄₂N₃O₈ [M + H]⁺ 775.3204, found 775.3204.

### Example 8: (E)-6-((2-(2,6-dioxopiperidin-3-yl)-1,3-dioxoisoindolin-5-yl)oxy)-N-(4-(2-((4-(2-(3-methylbenzylidene)hydrazino)-6-morpholinopyrimidin-2-yl)oxy)ethyl)phenyl)hexanamide (LCG-8)

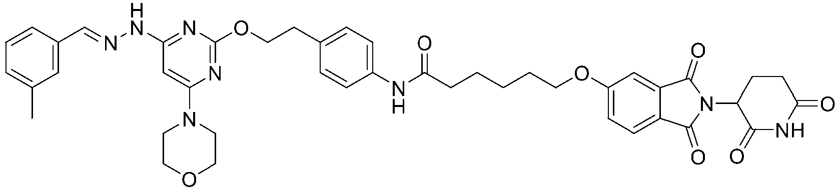

The synthesis method was as shown in Example 1.

¹H NMR (600 MHz, DMSO-*d*₆) δ 11.11 (s, 1H), 10.85 (s, 1H), 9.82 (s, 1H), 7.99 (s, 1H), 7.82 (d, *J* = 8.3 Hz, 1H), 7.54 - 7.48 (m, 3H), 7.47 (s, 1H), 7.42 (d, *J =* 2.2 Hz, 1H), 7.34 (dd, *J =* 8.3, 2.3 Hz, 1H), 7.29 (t, *J=* 7.6 Hz, 1H), 7.20 (d, *J=* 8.5 Hz, 2H), 7.17 (d, *J=* 7.5 Hz, 1H), 6.06 (s, 1H), 5.12 (dd, *J =* 12.9, 5.4 Hz, 1H), 4.33 (t, *J =* 7.0 Hz, 2H), 4.18 (t, *J =* 6.5 Hz, 2H), 3.71 - 3.62 (m, 4H), 3.57 - 3.48 (m, 4H), 2.97 - 2.91 (m, 2H), 2.90 - 2.83 (m, 1H), 2.64 - 2.52 (m, 2H), 2.38 - 2.29 (m, 5H), 2.09 - 2.00 (m, 1H), 1.83 - 1.74 (m, 2H), 1.70 - 1.63 (m, 2H), 1.51 - 1.41 (m, 2H). ¹³C NMR (150 MHz, DMSO-*d*₆) δ 172.16, 170.35, 169.32, 166.28, 166.21, 163.76, 163.47, 163.35, 162.90, 140.59, 137.31, 136.97, 134.15, 133.34, 132.37, 129.14, 128.47, 127.99, 126.40, 124.68, 122.93, 122.26, 120.12, 118.50, 108.22, 75.04, 68.09, 65.92, 65.29, 48.34, 43.60, 35.66, 33.66, 30.34, 27.56, 24.45, 24.23, 21.46, 20.32. HRMS (ESI) calcd for C₄₃H₄₆N₈O₈ [M + H]⁺ 803.3517, found 803.3517.

### Example 9: (E)-8-((2-(2,6-dioxopiperidin-3-yl)-1,3-dioxoisoindolin-5-yl)oxy)-N-(4-(2-((4-(2-(3-methylbenzylidene)hydrazino)-6-morpholinopyrimidin-2-yl)oxy)ethyl)phenyl)octanamide (LCG-9)

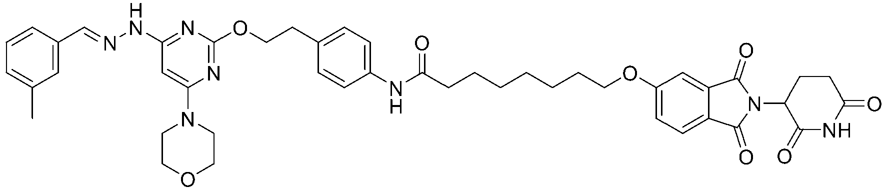

The synthesis method was as shown in Example 1.

¹H NMR (600 MHz, DMSO-*d*₆) δ 11.12 (s, 1H), 10.87 (s, 1H), 9.81 (s, 1H), 8.00 (s, 1H), 7.82 (d, *J* = 8.3 Hz, 1H), 7.54 - 7.47 (m, 4H), 7.42 (d, *J =* 2.2 Hz, 1H), 7.33 (dd, *J =* 8.3, 2.3 Hz, 1H), 7.29 (t, *J=* 7.6 Hz, 1H), 7.20 (d, *J* = 8.5 Hz, 2H), 7.17 (d, *J* = 7.5 Hz, 1H), 6.06 (s, 1H), 5.12 (dd, *J=* 12.9, 5.4 Hz, 1H), 4.34 (t, *J=* 7.0 Hz, 2H), 4.16 (t, *J* = 6.5 Hz, 2H), 3.68 - 3.63 (m, 4H), 3.55 - 3.50 (m, 4H), 2.93 (t, *J=* 7.1 Hz, 2H), 2.90 - 2.84 (m, 1H), 2.63 - 2.51 (m, 2H), 2.34 (s, 3H), 2.31 - 2.27 (m, 2H), 2.07 - 2.02 (m, 1H), 1.79 - 1.71 (m, 2H), 1.64 - 1.56 (m, 2H), 1.47 - 1.40 (m, 2H), 1.40 - 1.30 (m, 4H). ¹³C NMR (150 MHz, DMSO-*d*₆) δ 171.24, 169.55, 168.41, 165.37, 165.29, 162.56, 136.38, 136.08, 133.21, 132.41, 131.40, 128.24, 127.54, 127.06, 125.49, 123.75, 122.03, 121.31, 119.17, 117.58, 107.28, 74.11, 67.24, 64.37, 47.42, 44.19, 42.69, 34.81, 32.73, 29.43, 27.06, 26.93, 26.80, 23.71, 23.55, 20.55, 19.40. HRMS (ESI) calcd for C₄₅H₅₀N₈O₈ [M + H]⁺ 831.3830, found 831.3818.

### Example 10: (E)-10-((2-(2,6-dioxopiperidin-3-yl)-1,3-dioxoisoindolin-5-yl)oxy)-N-(4-(2-((4-(2-(3-methylbenzylidene)hydrazino)-6-morpholinopyrimidin-2-yl)oxy)ethyl)phenyl)decanamide (LCG-10)

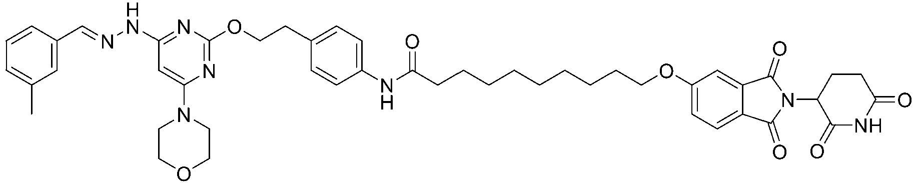

The synthesis method was as shown in Example 1.

¹H NMR (600 MHz, DMSO-*d*₆) δ 11.11 (s, 1H), 10.85 (s, 1H), 9.80 (s, 1H), 7.99 (s, 1H), 7.82 (d, *J* = 8.3 Hz, 1H), 7.55 - 7.45 (m, 4H), 7.41 (d, *J =* 2.0 Hz, 1H), 7.33 (dd, *J =* 8.3, 2.2 Hz, 1H), 7.29 (t, *J=* 7.6 Hz, 1H), 7.20 (d, *J* = 8.4 Hz, 2H), 7.17 (d, *J* = 7.5 Hz, 1H), 6.06 (s, 1H), 5.12 (dd, *J=* 12.9, 5.4 Hz, 1H), 4.33 (t, *J=* 7.0 Hz, 2H), 4.15 (t, *J* = 6.5 Hz, 2H), 3.68 - 3.63 (m, 4H), 3.56 - 3.49 (m, 4H), 2.95 - 2.84 (m, 3H), 2.63 - 2.51 (m, 2H), 2.34 (s, 3H), 2.28 (t, *J=* 7.4 Hz, 2H), 2.09 - 2.00 (m, 1H), 1.77 - 1.71 (m, 2H), 1.62 - 1.54 (m, 2H), 1.45 - 1.38 (m, 2H), 1.32 - 1.24 (m, 8H). ¹³C NMR (150 MHz, DMSO-*d*₆) δ 173.24, 171.56, 170.41, 167.37, 167.29, 164.84, 164.57, 164.43, 163.99, 141.67, 138.39, 138.08, 135.24, 134.42, 133.42, 130.22, 129.54, 129.07, 127.48, 125.76, 124.01, 123.32, 121.18, 119.57, 109.28, 76.13, 69.27, 67.01, 66.38, 49.43, 44.68, 36.84, 34.74, 31.43, 29.34, 29.21, 29.14, 29.13, 28.84, 25.81, 25.62, 22.55, 21.41. HRMS (ESI) calcd for C₄₇H₅₄N₈O₈ [M + H]⁺ 859.4143, found 859.4140.

### Example 11: (E)-2-((2-(2,6-dioxopiperidin-3-yl)-1,3-dioxoisoindolin-4-yl)amino)-N-(4-(2-((4-(2-(3-methylbenzylidene)hydrazino)-6-morpholinopyrimidin-2-yl)oxy)ethyl)phenyl)acetamide (LCG-11)

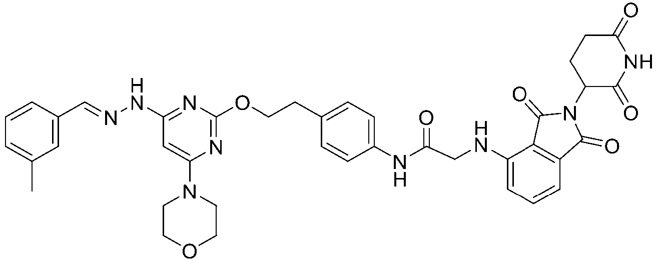

The synthesis method was as shown in Example 1.

¹H NMR (600 MHz, DMSO-*d*₆) δ 11.11 (s, 1H), 10.86 (s, 1H), 10.15 (s, 1H), 7.99 (s, 1H), 7.61 (dd, *J=* 8.4, 7.2 Hz, 1H), 7.53 (d, *J* = 8.5 Hz, 2H), 7.50 (d, *J=* 7.6 Hz, 1H), 7.48 (s, 1H), 7.29 (t, *J* = 7.6 Hz, 1H), 7.24 (d, *J* = 8.5 Hz, 2H), 7.17 (d, *J* = 7.5 Hz, 1H), 7.09 (d, *J =* 7.0 Hz, 1H), 7.02 (t, *J=* 5.7 Hz, 1H), 6.96 (d, *J* = 8.6 Hz, 1H), 6.06 (s, 1H), 5.09 (dd, *J* = 12.7, 5.5 Hz, 1H), 4.35 (t, *J =* 7.0 Hz, 2H), 4.18 (d, *J=* 5.6 Hz, 2H), 3.69 - 3.64 (m, 4H), 3.55 - 3.49 (m, 4H), 2.94 (t, *J* = 6.8 Hz, 2H), 2.93 - 2.86 (m, 1H), 2.63 - 2.51 (m, 2H), 2.34 (s, 3H), 2.08 - 2.01 (m, 1H). ¹³C NMR (150 MHz, DMSO-*d*₆) δ 172.20, 169.45, 168.14, 166.72, 166.70, 163.71, 163.29, 162.85, 145.34, 140.63, 137.30, 136.35, 135.61, 134.12, 132.92, 131.45, 129.15, 128.62, 127.98, 126.41, 122.93, 118.65, 117.00, 110.41, 109.20, 75.02, 65.89, 65.73, 65.28, 47.97, 44.99, 43.60, 33.63, 30.37, 21.54, 20.31. HRMS (ESI) calcd for C₃₉H₃₉N₉O₇ [M + H]⁺ 746.3050, found 746.3040.

### Example 12: (E)-4-((2-(2,6-dioxopiperidin-3-yl)-1,3-dioxoisoindolin-4-yl)amino)-N-(4-(2-((4-(2-(3-methylbenzylidene)hydrazino)-6-morpholinopyrimidin-2-yl)oxy)ethyl)phenyl)butanamide (LCG-12)

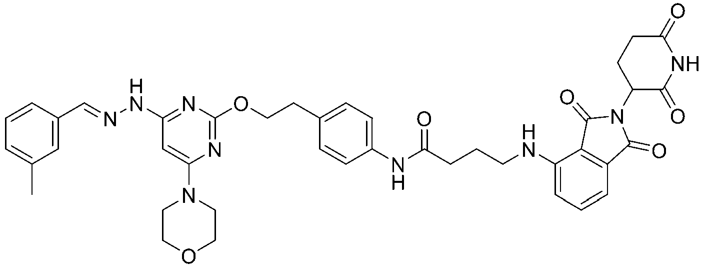

The synthesis method was as shown in Example 1.

¹H NMR (600 MHz, DMSO-*d*₆) δ 11.10 (s, 1H), 10.86 (s, 1H), 9.89 (s, 1H), 7.99 (s, 1H), 7.59 (dd, *J =* 8.5, 7.2 Hz, 1H), 7.53 - 7.46 (m, 4H), 7.29 (t, *J =* 7.6 Hz, 1H), 7.21 (d, *J =* 8.5 Hz, 2H), 7.17 (d, *J=* 7.5 Hz, 1H), 7.15 (d, *J =* 8.6 Hz, 1H), 7.03 (d, *J =* 7.0 Hz, 1H), 6.67 (t, *J=* 6.0 Hz, 1H), 6.06 (s, 1H), 5.05 (dd, *J =* 12.9, 5.5 Hz, 1H), 4.34 (t, *J =* 7.0 Hz, 2H), 3.70 - 3.64 (m, 4H), 3.56 - 3.51 (m, 4H), 3.39 - 3.36 (m, 2H), 2.93 (t, *J =* 6.9 Hz, 2H), 2.91 - 2.85 (m, 1H), 2.62 - 2.51 (m, 2H), 2.40 (t, *J* = 7.3 Hz, 2H), 2.34 (s, 3H), 2.05 - 1.99 (m, 1H), 1.93 - 1.85 (m, 2H). ¹³C NMR (150 MHz, DMSO-*d*₆) δ 172.20, 169.93, 169.49, 168.22, 166.68, 163.71, 163.30, 162.85, 145.73, 140.63, 137.30, 136.87, 135.65, 134.12, 132.41, 131.62, 129.15, 128.46, 127.98, 126.41, 122.93, 118.58, 116.56, 109.81, 108.50, 75.02, 65.93, 65.73, 65.28, 47.90, 43.60, 40.85, 33.64, 32.77, 30.36, 23.90, 21.54, 20.31. HRMS (ESI) calcd for C₄₁H₄₃N₉O₇ [M + H]⁺ 774.3363, found 774.3349.

### Example 13: (E)-6-((2-(2,6-dioxopiperidin-3-yl)-1,3-dioxoisoindolin-4-yl)amino)-N-(4-(2-((4-(2-(3-methylbenzylidene)hydrazino)-6-morpholinopyrimidin-2-yl)oxy)ethyl)phenyl)hexanamide (LCG-13)

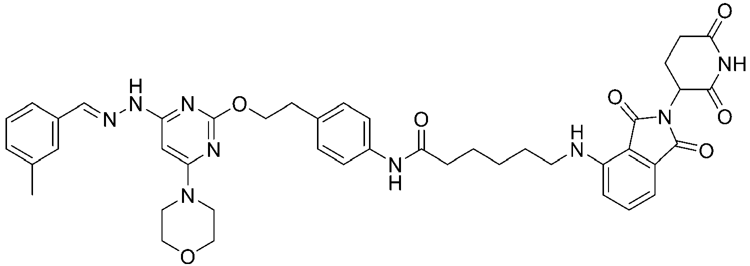

The synthesis method was as shown in Example 1.

¹H NMR (600 MHz, DMSO-*d*₆) δ 11.09 (s, 1H), 10.85 (s, 1H), 9.81 (s, 1H), 7.99 (s, 1H), 7.59 - 7.54 (m, 1H), 7.52 - 7.45 (m, 4H), 7.29 (t, *J* = 7.6 Hz, 1H), 7.20 (d, *J* = 8.5 Hz, 2H), 7.17 (d, *J* = 7.6 Hz, 1H), 7.10 (d, *J* = 8.6 Hz, 1H), 7.01 (d, *J* = 7.0 Hz, 1H), 6.55 (t, *J* = 5.8 Hz, 1H), 6.06 (s, 1H), 5.05 (dd, *J* = 12.9, 5.4 Hz, 1H), 4.33 (t, *J* = 7.0 Hz, 2H), 3.72 - 3.63 (m, 4H), 3.57 - 3.45 (m, 4H), 3.32 - 3.27 (m, 2H), 2.94 - 2.85 (m, 3H), 2.64 - 2.51 (m, 2H), 2.34 (s, 3H), 2.30 (t, *J* = 7.4 Hz, 2H), 2.05 - 1.99 (m, 1H), 1.66 - 1.57 (m, 4H), 1.42 - 1.35 (m, 2H). ¹³C NMR (150 MHz, DMSO-*d*₆) δ 172.19, 170.35, 169.49, 168.32, 166.68, 163.75, 163.34, 162.89, 161.68, 145.79, 140.58, 137.30, 136.96, 135.65, 134.14, 132.35, 131.57, 129.14, 128.45, 127.99, 126.40, 122.92, 118.49, 116.59, 109.76, 108.38, 75.03, 65.91, 65.28, 64.29, 47.91, 43.59, 41.11, 35.67, 33.64, 30.36, 27.91, 25.37, 24.28, 21.53, 20.32, 14.55. HRMS (ESI) calcd for C₄₃H₄₇N₉O₇ [M + H]⁺ 802.3676, found 802.3673.

### Example 14: (E)-8-((2-(2,6-dioxopiperidin-3-yl)-1,3-dioxoisoindolin-4-yl)amino)-N-(4-(2-((4-(2-(3-methylbenzylidene)hydrazino)-6-morpholinopyrimidin-2-yl)oxy)ethyl)phenyl)octanamide (LCG-14)

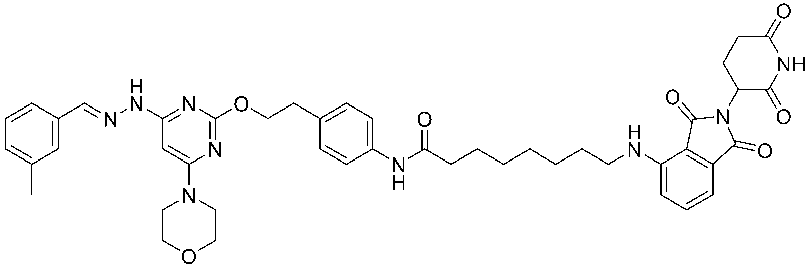

The synthesis method was as shown in Example 1.

¹H NMR (600 MHz, DMSO-*d*₆) δ 11.09 (s, 1H), 10.85 (s, 1H), 9.80 (s, 1H), 7.99 (s, 1H), 7.57 (t, *J* = 7.7 Hz, 1H), 7.54 - 7.44 (m, 4H), 7.29 (t, *J* = 7.5 Hz, 1H), 7.24 - 7.14 (m, 3H), 7.08 (d, *J =* 8.5 Hz, 1H), 7.01 (d, *J* = 6.9 Hz, 1H), 6.53 (s, 1H), 6.06 (s, 1H), 5.05 (dd, *J =* 12.7, 5.2 Hz, 1H), 4.33 (t, *J* = 6.7 Hz, 2H), 3.68 - 3.63 (m, 4H), 3.55 - 3.50 (m, 4H), 3.31 - 3.24 (m, 2H), 2.97 - 2.82 (m, 3H), 2.64 - 2.52 (m, 2H), 2.34 (s, 3H), 2.28 (t, *J=* 7.0 Hz, 2H), 2.06 - 1.99 (m, 1H), 1.59 - 1.56 (m, 4H), 1.42 - 1.26 (m, 6H). ¹³C NMR (150 MHz, DMSO-*d*₆) δ 173.28, 171.55, 170.58, 169.43, 167.78, 164.84, 164.44, 163.99, 146.90, 141.68, 138.40, 138.07, 136.75, 135.23, 133.43, 132.67, 130.23, 129.55, 129.08, 127.49, 124.01, 119.57, 117.65, 110.84, 109.48, 76.13, 67.01, 66.38, 49.01, 44.68, 42.28, 36.81, 34.74, 31.45, 29.13, 29.11, 29.00, 26.69, 25.56, 22.62, 21.41. HRMS (ESI) calcd for C₄₅H₅₁N₉O₇ [M + H]⁺ 830.3989, found 830.3979.

### Example 15: (E)-2-((2-(2,6-dioxopiperidin-3-yl)-1,3-dioxoisoindolin-5-yl)amino)-N-(4-(2-((4-(2-(3-methylbenzylidene)hydrazino)-6-morpholinopyrimidin-2-yl)oxy)ethyl)phenyl) acetamide (LCG-15)

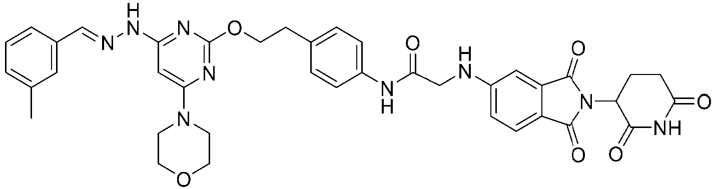

The synthesis method was as shown in Example 1.

¹H NMR (600 MHz, DMSO-*d*₆) δ 11.06 (s, 1H), 10.86 (s, 1H), 10.11 (s, 1H), 7.99 (s, 1H), 7.61 (d, *J* = 8.3 Hz, 1H), 7.53 (d, *J* = 8.5 Hz, 2H), 7.50 (d, *J* = 7.8 Hz, 1H), 7.48 (s, 1H), 7.43 (t, *J* = 5.6 Hz, 1H), 7.29 (t, *J =* 7.6 Hz, 1H), 7.24 (d, *J* = 8.5 Hz, 2H), 7.17 (d, *J =* 7.5 Hz, 1H), 7.02 (s, 1H), 6.92 (d, *J* = 7.9 Hz, 1H), 6.06 (s, 1H), 5.04 (dd, *J =* 12.8, 5.4 Hz, 1H), 4.34 (t, *J* = 7.0 Hz, 2H), 4.09 (d, *J* = 5.3 Hz, 2H), 3.70 - 3.64 (m, 4H), 3.55 - 3.50 (m, 4H), 2.94 (t, *J* = 7.0 Hz, 2H), 2.91 - 2.82 (m, 1H), 2.62 - 2.51 (m, 2H),- 2.34 (s, 3H), 2.03 - 1.97 (m, 1H). ¹³C NMR (150 MHz, DMSO-*d*₆) δ 172.20, 169.52, 167.04, 166.53, 163.74, 163.31, 162.89, 153.70, 140.64, 137.32, 136.39, 134.14, 133.41, 132.94, 129.17, 128.64, 128.00, 126.42, 124.38, 122.95, 118.69, 116.33, 75.04, 65.90, 65.74, 65.30, 48.05, 45.63, 43.61, 33.65, 30.37, 21.60, 20.33. HRMS (ESI) calcd for C₃₉H₃₉N₉O₇ [M + H]⁺ 746.3050, found 746.3032.

### Example 16: (E)-4-((2-(2,6-dioxopiperidin-3-yl)-1,3-dioxoisoindolin-5-yl)amino)-N-(4-(2-((4-(2-(3-methylbenzylidene)hydrazino)-6-morpholinopyrimidin-2-yl)oxy)ethyl)phenyl) butanamide (LCG-16)

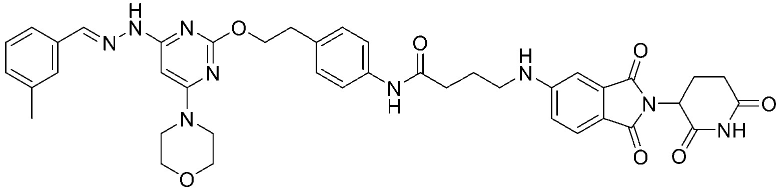

The synthesis method was as shown in Example 1.

¹H NMR (600 MHz, DMSO-*d*₆) δ 11.06 (s, 1H), 10.87 (s, 1H), 9.88 (s, 1H), 7.99 (s, 1H), 7.57 (d, *J =* 8.4 Hz, 1H), 7.54 - 7.46 (m, 4H), 7.29 (t, *J* = 7.6 Hz, 1H), 7.23 - 7.15 (m, 4H), 6.97 (d, *J =* 1.8 Hz, 1H), 6.87 (dd, *J* = 8.4, 2.0 Hz, 1H), 6.06 (s, 1H), 5.03 (dd, *J=* 12.8, 5.5 Hz, 1H), 4.34 (t, *J* = 6.9 Hz, 2H), 3.69 - 3.63 (m, 4H), 3.56 - 3.50 (m, 4H), 3.25 - 3.19 (m, 2H), 2.93 (t, *J* = 7.0 Hz, 2H), 2.91 - 2.83 (m, 1H), 2.64 - 2.51 (m, 2H), 2.43 (t, *J* = 7.4 Hz, 2H), 2.34 (s, 3H), 2.03 - 1.96 (m, 1H), 1.92 - 1.84 (m, 2H). ¹³C NMR (150 MHz, DMSO-*d*₆) δ 172.19, 169.96, 169.55, 167.06, 166.52, 163.67, 163.24, 162.81, 153.78, 140.68, 137.30, 136.89, 134.10, 133.61, 132.39, 129.16, 128.47, 127.98, 126.42, 124.50, 122.95, 118.56, 115.33, 75.00, 65.96, 65.72, 65.27, 47.99, 43.60, 41.39, 33.62, 32.90, 30.36, 23.55, 21.61, 20.31. HRMS (ESI) calcd for C₄₁H₄₃N₉O₇ [M + H]⁺ 774.3363, found 774.3354.

### Example 17: (E)-6-((2-(2,6-dioxopiperidin-3-yl)-1,3-dioxoisoindolin-5-yl)amino)-N-(4-(2-((4-(2-(3-methylbenzylidene)hydrazino)-6-morpholinopyrimidin-2-yl)oxy)ethyl)phenyl) hexanamide (LCG-17)

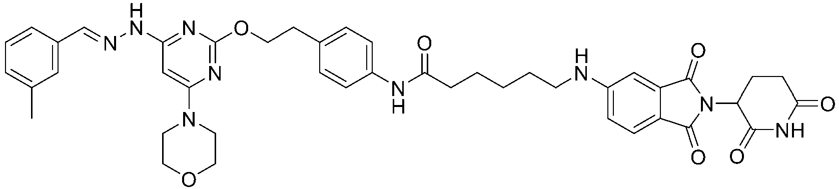

The synthesis method was as shown in Example 1.

¹H NMR (600 MHz, DMSO-*d*₆) δ 11.05 (s, 1H), 10.85 (s, 1H), 9.81 (s, 1H), 7.99 (s, 1H), 7.55 (d, *J=* 8.4 Hz, 1H), 7.53 - 7.45 (m, 4H), 7.29 (t, *J =* 7.6 Hz, 1H), 7.20 (d, *J =* 8.5 Hz, 2H), 7.17 (d, *J=* 7.5 Hz, 1H), 7.12 (t, *J =* 5.4 Hz, 1H), 6.94 (s, 1H), 6.84 (dd, *J =* 8.4, 1.9 Hz, 1H), 6.06 (s, 1H), 5.02 (dd, *J =* 12.8, 5.5 Hz, 1H), 4.33 (t, *J* = 7.1 Hz, 2H), 3.70 - 3.64 (m, 4H), 3.55 - 3.49 (m, 4H), 3.20 - 3.12 (m, 3H), 2.94 - 2.90 (m, 2H), 2.89 - 2.83 (m, 1H), 2.63 - 2.52 (m, 2H), 2.34 (s, 3H), 2.30 (t, *J* = 7.3 Hz, 2H), 2.02 - 1.96 (m, 1H), 1.66 - 1.57 (m, 4H), 1.44 - 1.37 (m, 2H). ¹³C NMR (150 MHz, DMSO-*d*₆) δ 172.19, 170.36, 169.56, 167.08, 166.53, 163.75, 163.34, 162.89, 153.83, 140.58, 137.30, 136.96, 134.13, 133.59, 132.36, 129.14, 128.45, 127.99, 126.40, 124.47, 122.92, 118.49, 115.16, 75.03, 65.91, 65.28, 64.29, 47.98, 43.59, 41.75, 35.70, 33.64, 30.36, 27.44, 25.61, 24.30, 21.61, 20.32. HRMS (ESI) calcd for C₄₃H₄₇N₉O₇ [M + H]⁺ 802.3676, found 802.3679.

### Example 18: (E)-8-((2-(2,6-dioxopiperidin-3-yl)-1,3-dioxoisoindolin-5-yl)amino)-N-(4-(2-((4-(2-(3-methylbenzylidene)hydrazino)-6-morpholinopyrimidin-2-yl)oxy)ethyl)phenyl) octanamide (LCG-18)

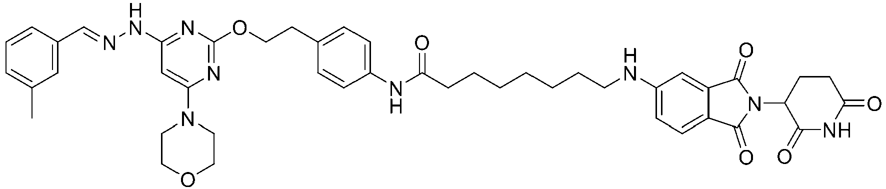

The synthesis method was as shown in Example 1.

¹H NMR (600 MHz, DMSO-*d*₆) δ 11.06 (s, 1H), 10.85 (s, 1H), 9.80 (s, 1H), 7.99 (s, 1H), 7.55 (d, *J=* 8.4 Hz, 1H), 7.53 - 7.46 (m, 4H), 7.29 (t, *J* = 7.6 Hz, 1H), 7.20 (d, *J =* 8.5 Hz, 2H), 7.17 (d, *J =* 7.5 Hz, 1H), 7.09 (t, *J =* 5.3 Hz, 1H), 6.94 (s, 1H), 6.84 (dd, *J =* 8.4, 1.9 Hz, 1H), 6.06 (s, 1H), 5.03 (dd, *J =* 12.8, 5.5 Hz, 1H), 4.33 (t, *J =* 7.0 Hz, 2H), 3.69 - 3.63 (m, 4H), 3.55 - 3.50 (m, 4H), 3.18 - 3.12 (m, 2H), 2.94 - 2.84 (m, 3H), 2.62 - 2.51 (m, 2H), 2.34 (s, 3H), 2.28 (t, *J* = 7.4 Hz, 2H), 2.01 - 1.96 (m, 1H), 1.62 - 1.54 (m, 4H), 1.39 - 1.29 (m, 6H). ¹³C NMR (150 MHz, DMSO-*d*₆) δ 173.29, 171.55, 170.66, 168.18, 167.63, 164.84, 164.44, 163.99, 154.94, 141.68, 138.40, 138.07, 135.23, 134.68, 133.43, 130.23, 129.55, 129.08, 127.49, 125.57, 124.01, 119.57, 116.24, 76.13, 67.00, 66.38, 65.39, 49.08, 44.68, 42.93, 36.82, 34.74, 31.46, 29.13, 29.05, 28.69, 26.91, 25.59, 22.71, 21.41. HRMS (ESI) calcd for C₄₅H₅₁N₉O₇ [M + H]⁺ 830.3989, found 830.3985.

### Example 19: (E)-10-((2-(2,6-dioxopiperidin-3-yl)-1,3-dioxoisoindolin-5-yl)amino)-N-(4-(2-((4-(2-(3-methylbenzylidene)hydrazino)-6-morpholinopyrimidin-2-yl)oxy)ethyl)phenyl) decanamide (LCG-19)

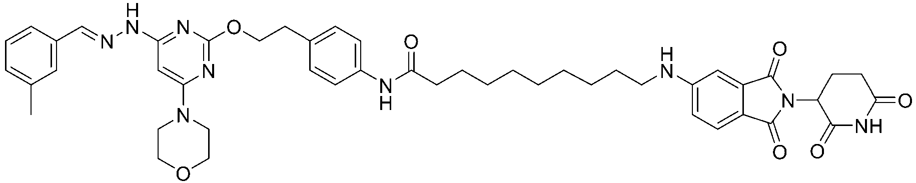

The synthesis method was as shown in Example 1.

¹H NMR (600 MHz, DMSO-*d*₆) δ 11.06 (s, 1H), 10.85 (s, 1H), 9.79 (s, 1H), 7.99 (s, 1H), 7.55 (d, *J* = 8.4 Hz, 1H), 7.53 - 7.46 (m, 4H), 7.29 (t, *J* = 7.6 Hz, 1H), 7.20 (d, *J=* 8.5 Hz, 2H), 7.17 (d, *J=* 7.5 Hz, 1H), 7.09 (t, *J* = 5.3 Hz, 1H), 6.93 (s, 1H), 6.83 (dd, *J =* 8.4, 1.8 Hz, 1H), 6.06 (s, 1H), 5.03 (dd, *J=* 12.8, 5.5 Hz, 1H), 4.33 (t, *J =* 7.0 Hz, 2H), 3.71 - 3.62 (m, 4H), 3.55 - 3.49 (m, 4H), 3.19 - 3.10 (m, 2H), 2.92 (t, *J* = 7.0 Hz, 2H), 2.90 - 2.83 (m, 1H), 2.62 - 2.51 (m, 2H), 2.34 (s, 3H), 2.27 (t, *J* = 7.4 Hz, 2H), 2.03 - 1.95 (m, 1H), 1.63 - 1.52 (m, 4H), 1.40 - 1.22 (m, 10H). ¹³C NMR (150 MHz, DMSO-*d*₆) δ 172.71, 170.98, 170.08, 167.60, 167.05, 164.25, 163.84, 163.40, 154.36, 141.12, 137.81, 137.50, 134.65, 134.10, 132.85, 129.73, 129.05, 128.89, 128.57, 128.41, 126.87, 124.99, 123.40, 118.97, 115.65, 75.55, 66.43, 66.25, 65.80, 48.50, 44.10, 42.37, 36.26, 34.16, 30.88, 28.81, 28.67, 28.56, 28.13, 26.42, 25.04, 22.13, 20.83. HRMS (ESI) calcd for: C₄₇H₅₅N₉O₇ [M + H]⁺ 858.4302, found 858.4284.

### Example 20: (E)-N¹-(2-(2,6-dioxopiperidin-3-yl)-1-oxoisoindolin-4-yl)-N⁶-(4-(2-((4-(2-(3-methylbenzylidene)hydrazino)-6-morpholinopyrimidin-2-yl)oxy)ethyl)phenyl)adipamide (LCG-20)

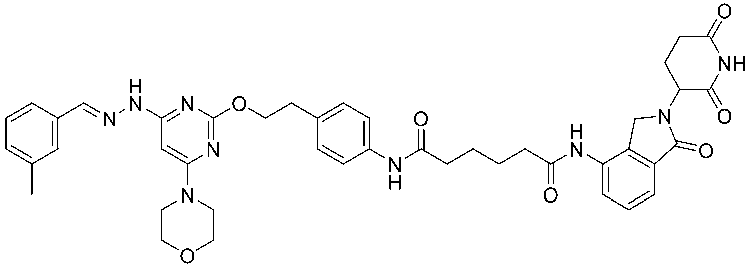

The synthesis method was as shown in Example 1.

¹H NMR (600 MHz, DMSO-*d*₆) δ 11.02 (s, 1H), 10.85 (s, 1H), 9.84 (s, 1H), 9.80 (s, 1H), 7.99 (s, 1H), 7.80 (d, *J* = 7.4 Hz, 1H), 7.54 - 7.46 (m, 6H), 7.29 (t, *J* = 7.6 Hz, 1H), 7.21 (d, *J=* 8.4 Hz, 2H), 7.17 (d, *J=* 7.5 Hz, 1H), 6.06 (s, 1H), 5.14 (dd, *J* = 13.3, 5.1 Hz, 1H), 4.44 - 4.30 (m, 4H), 3.72 - 3.64 (m, 4H), 3.52 (t, *J* = 8.7 Hz, 4H), 2.96 - 2.87 (m, 3H), 2.59 (d, *J* = 16.6 Hz, 1H), 2.42 - 2.31 (m, 8H), 2.06 - 1.97 (m, 1H), 1.71 - 1.59 (m, 4H). ¹³C NMR (150 MHz, DMSO-*d*₆) δ 173.33, 171.66, 171.55, 171.38, 168.31, 164.84, 164.44, 163.99, 141.69, 138.40, 138.04, 135.23, 134.30, 134.23, 133.47, 133.15, 130.24, 129.57, 129.09, 127.49, 125.82, 124.02, 119.58, 119.52, 76.13, 67.00, 66.38, 51.99, 46.95, 44.69, 36.67, 36.15, 34.74, 31.69, 25.32, 25.30, 23.09, 21.41. HRMS (ESI) calcd for C₄₃H₄₇N₉O₇ [M+ H]⁺ 802.3676, found 802.3676.

### Example 21: (E)-N¹-(2-(2,6-dioxopiperidin-3-yl)-1-oxoisoindolin-4-yl)-N⁸-(4-(2-((4-(2-(3-methylbenzylidene)hydrazino)-6-morpholinopyrimidin-2-yl)oxy)ethyl)phenyl)octanediamide (LCG-21)

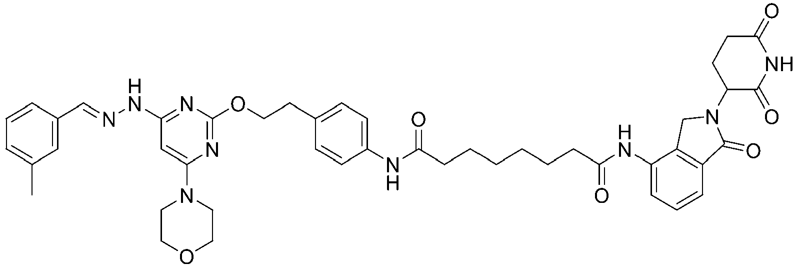

The synthesis method was as shown in Example 1.

¹H NMR (600 MHz, DMSO-*d*₆) δ 11.02 (s, 1H), 10.85 (s, 1H), 9.81 (s, 1H), 9.77 (s, 1H), 7.99 (s, 1H), 7.83 - 7.79 (m, 1H), 7.54 - 7.46 (m, 6H), 7.29 (t, *J* = 7.6 Hz, 1H), 7.20 (d, *J =* 8.5 Hz, 2H), 7.17 (d, *J=* 7.5 Hz, 1H), 6.06 (s, 1H), 5.14 (dd, *J =* 13.3, 5.1 Hz, 1H), 4.44 - 4.30 (m, 4H), 3.70 - 3.64 (m, 4H), 3.55 - 3.49 (m, 4H), 2.96 - 2.86 (m, 3H), 2.63 - 2.58 (m, 1H), 2.40 - 2.31 (m, 6H), 2.29 (t, *J* = 7.4 Hz, 2H), 2.07 - 1.98 (m, 1H), 1.65 - 1.57 (m, 4H), 1.40 - 1.30 (m, 4H). ¹³C NMR (150 MHz, DMSO-*d*₆) δ 172.03, 170.51, 170.25, 170.22, 167.00, 163.54, 163.13, 162.69, 140.38, 137.09, 136.76, 133.93, 132.97, 132.88, 132.14, 131.84, 128.93, 128.25, 127.78, 126.19, 124.43, 122.71, 118.28, 118.14, 74.83, 65.70, 65.07, 50.70, 45.66, 43.38, 35.50, 34.95, 33.43, 30.37, 27.67, 27.64, 24.24, 24.18, 21.80, 20.10. HRMS (ESI) calcd for C₄₅H₅₁N₉O₇ [M + H]⁺ 830.3989, found 830.3988.

### Example 22: (E)-N¹-(2-(2,6-dioxopiperidin-3-yl)-1-oxoisoindolin-4-yl)-N¹⁰-(4-(2-((4-(2-(3-methylbenzylidene)hydrazino)-6-morpholinopyrimidin-2-yl)oxy)ethyl)phenyl)decanediamide (LCG-22)

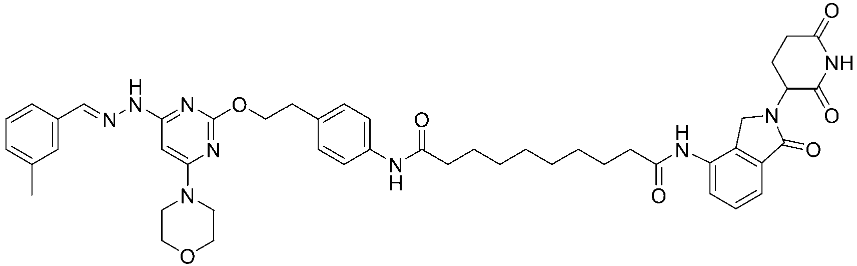

The synthesis method was as shown in Example 1.

¹H NMR (600 MHz, DMSO-*d*₆) δ 11.02 (s, 1H), 10.85 (s, 1H), 9.80 (s, 1H), 9.76 (s, 1H), 7.99 (s, 1H), 7.81 (d, *J* = 6.8 Hz, 1H), 7.53 - 7.46 (m, 6H), 7.29 (t, *J* = 7.6 Hz, 1H), 7.20 (d, *J* = 8.5 Hz, 2H), 7.17 (d, *J* = 7.5 Hz, 1H), 6.06 (s, 1H), 5.15 (dd, *J* = 13.3, 5.1 Hz, 1H), 4.41 - 4.31 (m, 4H), 3.69 - 3.64 (m, 4H), 3.55 - 3.50 (m, 4H), 2.95 - 2.87 (m, 3H), 2.64 - 2.57 (m, 1H), 2.38 - 2.31 (m, 6H), 2.28 (t, *J* = 7.4 Hz, 2H), 2.05 - 2.00 (m, 1H), 1.64 - 1.55 (m, 4H), 1.37 - 1.22 (m, 8H). ¹³C NMR (150 MHz, DMSO-*d*₆) δ 173.34, 171.85, 171.56, 168.31, 164.84, 164.44, 163.99, 141.68, 138.40, 138.07, 135.23, 134.28, 134.16, 133.44, 133.14, 130.23, 129.55, 129.08, 127.49, 125.71, 124.02, 119.58, 119.44, 76.13, 67.00, 66.38, 51.99, 46.95, 44.68, 36.84, 36.28, 34.74, 31.68, 29.21, 29.16, 29.14, 25.61, 25.55, 23.12, 21.41. HRMS (ESI) calcd for C₄₇H₅₅N₉O₇ [M + H]⁺ 858.4320, found 858.4296.

### Example 23: (E)-N¹-(2-(2,6-dioxopiperidin-3-yl)-1-oxoisoindolin-4-yl)-N¹²-(4-(2-((4-(2-(3-methylbenzylidene)hydrazino)-6-morpholinopyrimidin-2-yl)oxy)ethyl)phenyl)dodecanediamide (LCG-23)

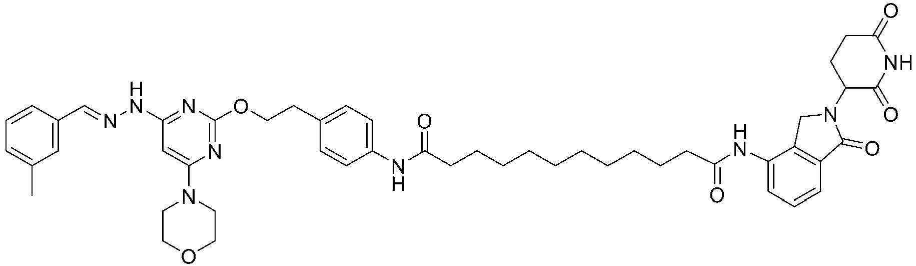

The synthesis method was as shown in Example 1.

¹H NMR (600 MHz, DMSO-*d*₆) δ 11.02 (s, 1H), 10.85 (s, 1H), 9.79 (s, 1H), 9.75 (s, 1H), 7.99 (s, 1H), 7.81 (dd, *J* = 7.4, 1.0 Hz, 1H), 7.52 - 7.46 (m, 6H), 7.29 (t, *J* = 7.6 Hz, 1H), 7.20 (d, *J* = 8.5 Hz, 2H), 7.17 (d, *J* = 7.5 Hz, 1H), 6.06 (s, 1H), 5.14 (dd, *J* = 13.3, 5.1 Hz, 1H), 4.40 - 4.30 (m, 4H), 3.73 - 3.64 (m, 4H), 3.56 - 3.49 (m, 4H), 2.95 - 2.88 (m, 3H), 2.64 - 2.58 (m, 1H), 2.38 - 2.31 (m, 6H), 2.27 (t, *J* = 7.4 Hz, 2H), 2.06 - 1.99 (m, 1H), 1.63 - 1.54 (m, 4H), 1.34 - 1.19 (m, 12H). ¹³C NMR (150 MHz, DMSO-*d*₆) δ 173.33, 171.85, 171.57, 171.55, 168.30, 164.84, 164.43, 163.99, 141.68, 138.40, 138.07, 135.23, 134.28, 134.16, 133.43, 133.13, 130.23, 129.55, 129.08, 127.49, 125.70, 124.01, 119.57, 119.44, 76.13, 67.00, 66.38, 51.99, 46.93, 44.68, 36.84, 36.28, 34.74, 31.68, 29.41, 29.39, 29.28, 29.25, 29.16, 25.63, 25.56, 23.12, 21.41. HRMS (ESI) calcd for C₄₉H₅₉N₉O₇ [M + H]⁺ 886.4615, found 886.4605.

### Example 24: (E)-N¹-(2-(2,6-dioxopiperidin-3-yl)-1-oxoisoindol-5-yl)-N⁴-(4-(2-((4-(2-(3-methylbenzylidene)hydrazino)-6-morpholinopyrimidin-2-yl)oxy)ethyl)phenyl)succinimide (LCG-24)

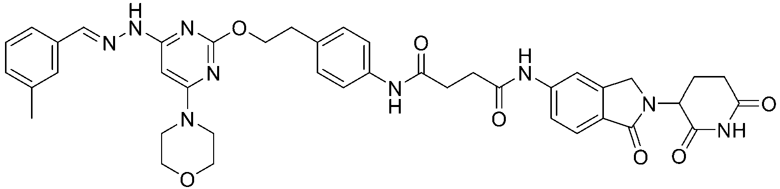

The synthesis method was as shown in Example 1.

¹H NMR (600 MHz, DMSO-*d*₆) δ 10.97 (s, 1H), 10.85 (s, 1H), 10.35 (s, 1H), 9.96 (s, 1H), 7.98 (d, *J=* 2.4 Hz, 2H), 7.65 (d, *J =* 8.3 Hz, 1H), 7.60 (d, *J* = 8.3 Hz, 1H), 7.55 - 7.45 (m, 4H), 7.29 (t, *J* = 7.6 Hz, 1H), 7.21 (d, *J* = 8.4 Hz, 2H), 7.17 (d, *J* = 7.5 Hz, 1H), 6.06 (s, 1H), 5.08 (dd, *J* = 13.3, 5.1 Hz, 1H), 4.41 (d, *J* = 17.2 Hz, 1H), 4.33 (t, *J* = 7.0 Hz, 2H), 4.28 (d, *J* = 17.2 Hz, 1H), 3.70 - 3.63 (m, 4H), 3.55 - 3.47 (m, 4H), 2.98 - 2.87 (m, 3H), 2.75 - 2.64 (m, 4H), 2.59 (d, *J* = 17.1 Hz, 1H), 2.41 - 2.35 (m, 1H), 2.34 (s, 3H), 2.02 - 1.95 (m, 1H). ¹³C NMR (150 MHz, DMSO-*d*₆) δ 173.37, 171.59, 171.45, 170.57, 168.32, 164.84, 164.43, 163.99, 143.79, 143.02, 141.68, 138.40, 138.05, 135.23, 133.42, 130.23, 129.59, 129.08, 127.49, 126.54, 124.12, 124.02, 119.47, 119.02, 113.45, 76.13, 67.00, 66.38, 51.99, 47.60, 44.69, 34.73, 31.84, 31.69, 31.49, 23.00, 21.41. HRMS (ESI) calcd for C₄₁H₄₃N₉O₇ [M + H]⁺ 774.3363, found 774.3342.

### Example 25: (E)-N¹-(2-(2,6-dioxopiperidin-3-yl)-1-oxoisoindolin-5-yl)-N⁶-(4-(2-((4-(2-(3-methylbenzylidene)hydrazino)-6-morpholinopyrimidin-2-yl)oxy)ethyl)phenyl)adipamide (LCG-25)

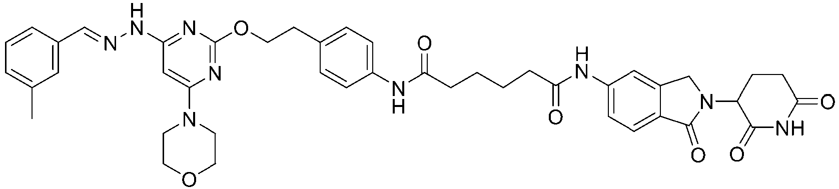

The synthesis method was as shown in Example 1.

¹H NMR (600 MHz, DMSO-*d*₆) δ 10.97 (s, 1H), 10.86 (s, 1H), 10.33 (d, *J* = 8.4 Hz, 1H), 9.91 (d, *J* = 5.6 Hz, 1H), 8.00 (d, *J* = 3.2 Hz, 2H), 7.66 - 7.59 (m, 2H), 7.53 (d, *J* = 8.4 Hz, 2H), 7.50 (d, *J* = 7.8 Hz, 1H), 7.47 (s, 1H), 7.29 (t, *J* = 7.6 Hz, 1H), 7.20 (d, *J* = 8.5 Hz, 2H), 7.17 (d, *J* = 7.5 Hz, 1H), 6.06 (s, 1H), 5.08 (dd, *J* = 13.3, 5.1 Hz, 1H), 4.42 (d, *J* = 17.3 Hz, 1H), 4.33 (t, *J* = 7.1 Hz, 2H), 4.28 (d, *J* = 17.3 Hz, 1H), 3.70 - 3.63 (m, 4H), 3.56 - 3.48 (m, 4H), 2.96 - 2.86 (m, 3H), 2.63 - 2.56 (m, 1H), 2.43 - 2.31 (m, 8H), 2.02 - 1.95 (m, 1H), 1.69 - 1.59 (m, 4H). ¹³C NMR (150 MHz, DMSO-*d*₆) δ 172.29, 171.07, 170.51, 170.31, 167.25, 163.76, 163.35, 162.91, 142.66, 141.97, 140.60, 137.32, 136.99, 134.16, 132.36, 129.15, 128.47, 128.00, 126.41, 125.49, 122.99, 122.94, 118.52, 118.05, 112.49, 75.05, 65.93, 65.30, 50.91, 46.52, 43.60, 35.73, 35.57, 33.66, 30.61, 24.25, 24.16, 21.92, 20.33. HRMS (ESI) calcd for C₄₃H₄₇N₉O₇ [M + H]⁺ 802.3676, found 802.3667.

### Example 26: (E)-N¹-(2-(2,6-dioxopiperidin-3-yl)-1-oxoisoindolin-5-yl)-N⁸-(4-(2-((4-(2-(3-methylbenzylidene)hydrazino)-6-morpholinopyrimidin-2-yl)oxy)ethyl)phenyl)octanediamide (LCG-26)

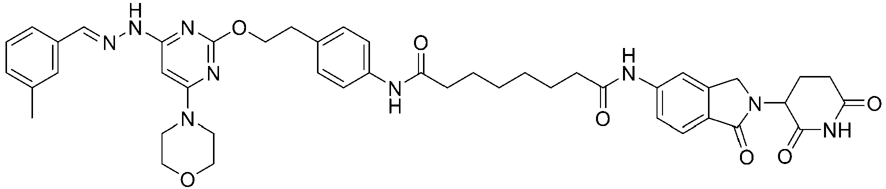

The synthesis method was as shown in Example 1.

¹H NMR (600 MHz, DMSO-*d*₆) δ 10.97 (s, 1H), 10.85 (s, 1H), 10.21 (s, 1H), 9.80 (s, 1H), 7.99 (s, 2H), 7.64 (d, *J =* 8.3 Hz, 1H), 7.58 (dd, *J* = 8.3, 1.5 Hz, 1H), 7.52 - 7.46 (m, 4H), 7.29 (t, *J =* 7.6 Hz, 1H), 7.20 (d, *J* = 8.5 Hz, 2H), 7.17 (d, *J* = 7.5 Hz, 1H), 6.06 (s, 1H), 5.08 (dd, *J=* 13.3, 5.1 Hz, 1H), 4.42 (d, *J* = 17.2 Hz, 1H), 4.33 (t, *J* = 7.0 Hz, 2H), 4.28 (d, *J=* 17.3 Hz, 1H), 3.69 - 3.63 (m, 4H), 3.55 - 3.49 (m, 4H), 2.95 - 2.86 (m, 3H), 2.63 - 2.56 (m, 1H), 2.39 - 2.32 (m, 6H), 2.29 (t, *J =* 7.4 Hz, 2H), 2.02 - 1.96 (m, 1H), 1.65 - 1.56 (m, 4H), 1.36 - 1.32 (m, 4H). ¹³C NMR (150 MHz, DMSO-*d*₆) δ 173.37, 172.25, 171.59, 171.53, 168.34, 164.84, 164.43, 163.99, 143.76, 143.03, 141.68, 138.40, 138.07, 135.23, 133.44, 130.24, 129.55, 129.08, 127.49, 126.57, 124.08, 124.02, 119.59, 119.11, 113.56, 76.13, 67.01, 66.38, 51.99, 47.60, 44.68, 36.95, 36.80, 34.74, 31.69, 28.96, 28.95, 25.52, 25.40, 23.00, 21.41. HRMS (ESI) calcd for C₄₅H₅₁N₉O₇ [M + H]⁺ 830.3989, found 830.3986.

### Example 27: (E)-N¹-(2-(2,6-dioxopiperidin-3-yl)-1-oxoisoindolin-5-yl)-N¹⁰-(4-(2-((4-(2-(3-methylbenzylidene)hydrazino)-6-morpholinopyrimidin-2-yl)oxy)ethyl)phenyl)decanediamide (LCG-27)

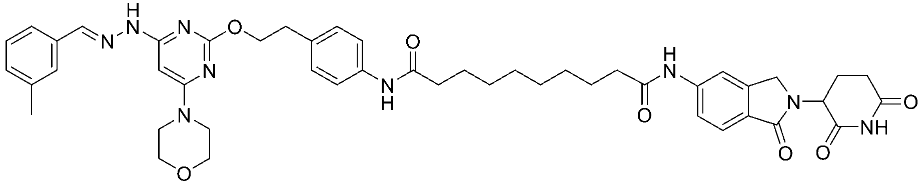

The synthesis method was as shown in Example 1.

¹H NMR (600 MHz, DMSO-*d*₆) δ 10.97 (s, 1H), 10.85 (s, 1H), 10.20 (s, 1H), 9.79 (s, 1H), 7.99 (s, 2H), 7.64 (d, *J* = 8.3 Hz, 1H), 7.58 (dd, *J* = 8.3, 1.5 Hz, 1H), 7.53 - 7.46 (m, 4H), 7.29 (t, *J* = 7.6 Hz, 1H), 7.20 (d, *J* = 8.5 Hz, 2H), 7.17 (d, *J* = 7.5 Hz, 1H), 6.06 (s, 1H), 5.08 (dd, *J* = 13.3, 5.1 Hz, 1H), 4.42 (d, *J* = 17.3 Hz, 1H), 4.33 (t, *J* = 7.0 Hz, 2H), 4.28 (d, *J* = 17.3 Hz, 1H), 3.69 - 3.61 (m, 4H), 3.56 - 3.48 (m, 4H), 2.96 - 2.87 (m, 3H), 2.59 (d, *J* = 17.1 Hz, 1H), 2.38 - 2.31 (m, 6H), 2.27 (t, *J* = 7.4 Hz, 2H), 2.02 - 1.95 (m, 1H), 1.63 - 1.54 (m, 4H), 1.35 - 1.23 (m, 8H). ¹³C NMR (150 MHz, DMSO-*d*₆) δ 172.29, 171.20, 170.51, 170.48, 167.25, 163.76, 163.35, 162.91, 142.68, 141.96, 140.60, 137.32, 137.00, 134.15, 132.35, 129.15, 128.47, 128.00, 126.41, 125.49, 123.00, 122.94, 118.50, 118.02, 112.47, 75.05, 65.93, 65.30, 50.91, 46.51, 43.61, 35.90, 35.76, 33.66, 30.61, 28.11, 28.06, 28.03, 24.53, 24.40, 21.93, 20.33. HRMS (ESI) calcd for C₄₇H₅₅N₉O₇ [M + H]⁺ 858.4302, found 858.4298.

### Example 28: (E)-N¹-(2-(2,6-dioxopiperidin-3-yl)-3-oxoisoindolin-5-yl)-N⁴-(4-(2-((4-(2-(3-methylbenzylidene)hydrazino)-6-morpholinopyrimidin-2-yl)oxy)ethyl)phenyl)succinimide (LCG-28)

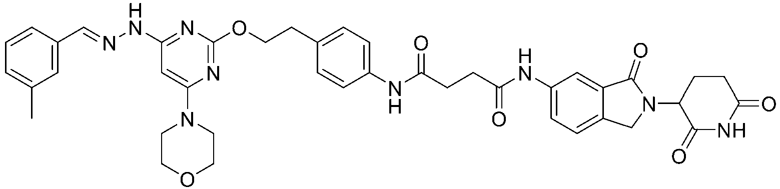

The synthesis method was as shown in Example 1.

¹H NMR (600 MHz, DMSO-*d*₆) δ 10.98 (s, 1H), 10.86 (s, 1H), 10.25 (s, 1H), 9.97 (s, 1H), 8.10 (d, *J* = 1.4 Hz, 1H), 7.99 (s, 1H), 7.72 (dd, *J* = 8.2, 1.8 Hz, 1H), 7.56 - 7.44 (m, 5H), 7.29 (t, *J* = 7.6 Hz, 1H), 7.21 (d, *J* = 8.5 Hz, 2H), 7.17 (d, *J* = 7.5 Hz, 1H), 6.06 (s, 1H), 5.09 (dd, *J* = 13.3, 5.1 Hz, 1H), 4.39 (d, *J* = 17.0 Hz, 1H), 4.33 (t, *J* = 7.1 Hz, 2H), 4.27 (d, *J* = 17.0 Hz, 1H), 3.70 - 3.61 (m, 4H), 3.56 - 3.48 (m, 4H), 2.96 - 2.86 (m, 3H), 2.75 - 2.63 (m, 4H), 2.60 (d, *J* = 18.0 Hz, 1H), 2.43 - 2.30 (m, 4H), 2.05 - 1.97 (m, 1H). ¹³C NMR (150 MHz, DMSO-*d*₆) δ 172.26, 170.41, 170.06, 169.52, 167.41, 163.75, 163.33, 162.89, 140.58, 138.64, 137.30, 136.95, 135.63, 134.14, 132.31, 131.52, 129.14, 128.49, 127.99, 126.40, 123.15, 122.92, 121.87, 118.40, 112.19, 75.03, 65.91, 65.28, 51.04, 46.32, 43.59, 33.64, 30.67, 30.59, 30.48, 21.85, 20.32. HRMS (ESI) calcd for C₄₁H₄₃N₉O₇ [M + H]⁺ 774.3363, found 774.3355.

### Example 29: (E)-N¹-(2-(2,6-dioxopiperidin-3-yl)-3-oxoisoindolin-5-yl)-N⁶-(4-(2-((4-(2-(3-methylbenzylidene)hydrazino)-6-morpholinopyrimidin-2-yl)oxy)ethyl)phenyl)adipamide (LCG-29)

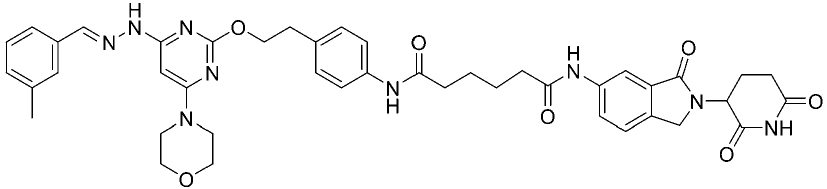

The synthesis method was as shown in Example 1.

¹H NMR (600 MHz, DMSO-*d*₆) δ 10.98 (brs, 1H), 10.85 (s, 1H), 10.14 (s, 1H), 9.84 (s, 1H), 8.10 (d, *J* = 1.7 Hz, 1H), 7.99 (s, 1H), 7.71 (dd, *J =* 8.2, 1.9 Hz, 1H), 7.54 - 7.48 (m, 4H), 7.47 (s, 1H), 7.29 (t, *J* = 7.6 Hz, 1H), 7.20 (d, *J* = 8.5 Hz, 2H), 7.17 (d, *J* = 7.5 Hz, 1H), 6.06 (s, 1H), 5.09 (dd, *J* = 13.3, 5.1 Hz, 1H), 4.39 (d, *J* = 17.1 Hz, 1H), 4.33 (t, *J* = 7.1 Hz, 2H), 4.27 (d, *J* = 17.1 Hz, 1H), 3.70 - 3.61 (m, 4H), 3.54 - 3.49 (m, 4H), 2.96 - 2.86 (m, 3H), 2.63 - 2.57 (m, 1H), 2.43 - 2.24 (m, 8H), 2.02 - 1.98 (m, 1H), 1.73 - 1.57 (m, 4H). ¹³C NMR (150 MHz, DMSO-*d*₆) δ 173.36, 171.83, 171.50, 171.38, 168.51, 164.84, 164.43, 163.99, 141.67, 139.71, 138.40, 138.04, 136.78, 135.23, 133.46, 132.59, 130.23, 129.56, 129.08, 127.49, 124.22, 124.02, 123.09, 119.61, 113.45, 76.12, 67.01, 66.38, 52.14, 47.42, 44.68, 36.78, 36.71, 34.74, 31.69, 25.38, 25.28, 22.95, 21.41. HRMS (ESI) calcd for C₄₃H₄₇N₉O₇ [M + H]⁺ 802.3676, found 802.3675.

### Example 30: (E)-N¹-(2-(2,6-dioxopiperidin-3-yl)-3-oxoisoindol-5-yl)-N³-(4-(2-((4-(2-(3-methylbenzylidene)hydrazino)-6-morpholinopyrimidin-2-yl)oxy)ethyl)phenyl)octanediamide (LCG-30)

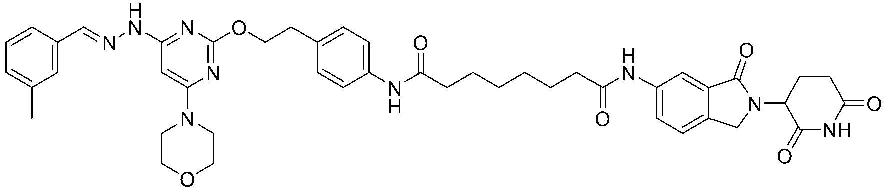

The synthesis method was as shown in Example 1.

¹H NMR (600 MHz, DMSO-*d*₆) δ 10.98 (s, 1H), 10.85 (s, 1H), 10.10 (s, 1H), 9.80 (s, 1H), 8.10 (d, *J* = 1.5 Hz, 1H), 7.99 (s, 1H), 7.70 (dd, *J* = 8.2, 1.8 Hz, 1H), 7.54 - 7.46 (m, 5H), 7.29 (t, *J* = 7.6 Hz, 1H), 7.20 (d, *J* = 8.5 Hz, 2H), 7.17 (d, *J* = 7.5 Hz, 1H), 6.06 (s, 1H), 5.10 (dd, *J* = 13.3, 5.1 Hz, 1H), 4.39 (d, *J* = 17.0 Hz, 1H), 4.33 (t, *J* = 7.0 Hz, 2H), 4.26 (d, *J* = 17.0 Hz, 1H), 3.70 - 3.62 (m, 4H), 3.56 - 3.48 (m, 4H), 2.95 - 2.86 (m, 3H), 2.60 (d, *J* = 17.5 Hz, 1H), 2.43 - 2.31 (m, 6H), 2.29 (t, *J* = 7.4 Hz, 2H), 2.04 - 1.97 (m, 1H), 1.66 - 1.56 (m, 4H), 1.39 - 1.30 (m, 4H). ¹³C NMR (150 MHz, DMSO-*d*₆) δ 172.27, 170.89, 170.44, 170.42, 167.44, 163.76, 163.35, 162.90, 140.60, 138.66, 137.32, 137.00, 135.66, 134.15, 132.34, 131.50, 129.15, 128.47, 128.00, 126.41, 123.12, 122.94, 121.99, 118.51, 112.34, 75.05, 65.93, 65.30, 51.06, 46.34, 43.60, 35.80, 35.73, 33.66, 30.61, 27.88, 24.45, 24.35, 21.87, 20.33. HRMS (ESI) calcd for C₄₃H₅₁N₉O₇ [M + H]⁺ 830.3989, found 830.3991.

### Example 31: (E)-N¹-(2-(2,6-dioxopiperidin-3-yl)-3-oxoisoindol-5-yl)-N¹⁰-(4-(2-((4-(2-(3-methylbenzylidene)hydrazino)-6-morpholinopyrimidin-2-yl)oxy)ethyl)phenyl)decanediamide (LCG-31)

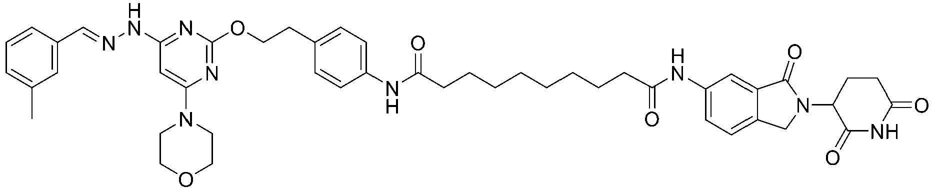

The synthesis method was as shown in Example 1.

¹H NMR (600 MHz, DMSO-*d*₆) δ 10.98 (brs, 1H), 10.85 (s, 1H), 10.11 (s, 1H), 9.80 (s, 1H), 8.10 (t, *J* = 3.4 Hz, 1H), 7.99 (s, 1H), 7.70 (dd, *J* = 8.2, 1.9 Hz, 1H), 7.53 - 7.46 (m, 5H), 7.29 (t, *J* = 7.6 Hz, 1H), 7.20 (d, *J* = 8.5 Hz, 2H), 7.17 (d, *J =* 7.5 Hz, 1H), 6.06 (s, 1H), 5.09 (dd, *J* = 13.3, 5.1 Hz, 1H), 4.39 (d, *J=* 17.0 Hz, 1H), 4.33 (t, *J=* 7.1 Hz, 2H), 4.26 (d, *J=* 17.0 Hz, 1H), 3.70 - 3.62 (m, 4H), 3.55 - 3.49 (m, 4H), 2.96 - 2.86 (m, 3H), 2.63 - 2.56 (m, 1H), 2.43 - 2.30 (m, 6H), 2.27 (t, *J=* 7.4 Hz, 2H), 2.03 - 1.97 (m, 1H), 1.64 - 1.53 (m, 4H), 1.34 - 1.26 (m, 8H). ¹³C NMR (150 MHz, DMSO-*d*₆) δ 172.28, 170.94, 170.50, 170.43, 167.46, 163.77, 163.36, 162.92, 140.61, 138.67, 137.32, 137.00, 135.67, 134.16, 132.35, 131.50, 129.16, 128.47, 128.01, 126.42, 123.13, 122.94, 122.00, 118.51, 112.35, 75.05, 65.93, 65.31, 51.07, 46.35, 43.61, 35.83, 35.77, 33.66, 30.62, 28.11, 28.06, 24.54, 24.43, 21.87, 20.34. HRMS (ESI) calcd for C₄₇H₅₅N₉O₇ [M + H]⁺ 858.4302, found 858.4296.

### Example 32: (E)-N¹-(2-(2,6-dioxopiperidin-3-yl)-3-oxoisoindolin-4-yl)-N⁴-(4-(2-((4-(2-(3-methylbenzylidene)hydrazino)-6-morpholinopyrimidin-2-yl)oxy)ethyl)phenyl)succinimide (LCG-32)

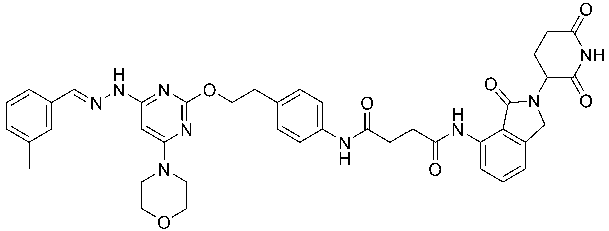

The synthesis method was as shown in Example 1.

¹H NMR (600 MHz, DMSO-*d*₆) δ 11.04 (s, 1H), 10.85 (s, 1H), 10.30 (s, 1H), 9.98 (s, 1H), 8.30 (d, *J =* 8.2 Hz, 1H), 7.98 (s, 1H), 7.56 (t, *J* = 7.9 Hz, 1H), 7.54 - 7.45 (m, 4H), 7.29 (t, *J =* 7.6 Hz, 1H), 7.25 (d, *J* = 7.5 Hz, 1H), 7.20 (d, *J =* 8.4 Hz, 2H), 7.17 (d, *J =* 7.5 Hz, 1H), 6.06 (s, 1H), 5.08 (dd, *J =* 13.3, 5.1 Hz, 1H), 4.48 (d, *J =* 17.6 Hz, 1H), 4.37 (d, *J =* 17.6 Hz, 1H), 4.33 (t, *J =* 7.0 Hz, 2H), 3.70 - 3.64 (m, 4H), 3.56 - 3.49 (m, 4H), 2.95 - 2.87 (m, 3H), 2.76 - 2.66 (m, 4H), 2.65 - 2.59 (m, 1H), 2.47 - 2.39 (m, 1H), 2.34 (s, 3H), 2.07 - 2.02 (m, 1H). ¹³C NMR (150 MHz, DMSO-*d*₆) δ 172.22, 170.17, 170.03, 169.19, 168.56, 163.74, 163.32, 162.88, 142.04, 140.59, 137.30, 136.90, 136.43, 134.14, 132.56, 132.33, 129.14, 128.49, 127.98, 126.40, 122.92, 118.39, 117.00, 116.35, 116.09, 75.02, 65.91, 65.28, 51.00, 46.90, 43.59, 33.63, 31.16, 30.55, 30.29, 21.72, 20.31. HRMS (ESI) calcd for C₄₁H₄₃N₉O₇ [M + H]⁺ 774.3363, found 774.3360.

### Example 33: (E)-N¹-(2-(2,6-dioxopiperidin-3-yl)-3-oxoisoindolin-4-yl)-N⁶-(4-(2-((4-(2-(3-methylbenzylidene)hydrazino)-6-morpholinopyrimidin-2-yl)oxy)ethyl)phenyl)adipamide (LCG-33)

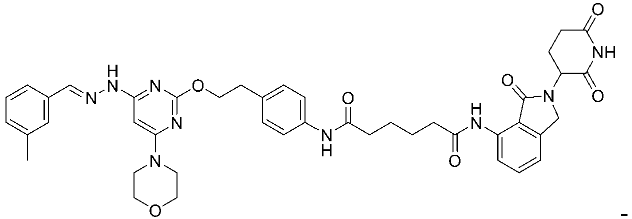

The synthesis method was as shown in Example 1.

¹H NMR (600 MHz, DMSO-*d*₆) δ 11.04 (s, 1H), 10.85 (s, 1H), 10.27 (s, 1H), 9.84 (s, 1H), 8.32 (d, *J* = 8.2 Hz, 1H), 7.99 (s, 1H), 7.57 (t, *J =* 7.9 Hz, 1H), 7.53 - 7.47 (m, 4H), 7.29 (t, *J =* 7.6 Hz, 1H), 7.25 (d, *J* = 7.5 Hz, 1H), 7.20 (d, *J =* 8.5 Hz, 2H), 7.17 (d, *J =* 7.5 Hz, 1H), 6.06 (s, 1H), 5.06 (dd, *J =* 13.3, 5.1 Hz, 1H), 4.47 (d, *J =* 17.6 Hz, 1H), 4.39 - 4.32 (m, 3H), 3.69 - 3.65 (m, 4H), 3.55 - 3.50 (m, 4H), 2.95 - 2.85 (m, 3H), 2.62 (d, *J=* 16.8 Hz, 1H), 2.48 - 2.40 (m, 3H), 2.36 - 2.32 (m, 5H), 2.07 - 2.01 (m, 1H), 1.70 - 1.63 (m, 4H). ¹³C NMR (150 MHz, DMSO-*d*₆) δ 172.21, 170.57, 170.20, 170.17, 168.58, 163.74, 163.33, 162.88, 142.01, 140.60, 137.30, 136.93, 136.43, 134.14, 132.57, 132.37, 129.14, 128.46, 127.99, 126.40, 122.93, 118.53, 117.03, 116.41, 116.14, 75.03, 65.92, 65.29, 51.00, 46.91, 43.59, 36.11, 35.43, 33.64, 30.55, 24.08, 23.82, 21.72, 20.32. HRMS (ESI) calcd for C₄₃H₄₇N₉O₇ [M + H]⁺ 802.3676, found 802.3670.

### Example 34: (E)-N¹-(2-(2,6-dioxopiperidin-3-yl)-3-oxoisoindolin-4-yl)-N⁸-(4-(2-((4-(2-(3-methylbenzylidene)hydrazino)-6-morpholinopyrimidin-2-yl)oxy)ethyl)phenyl)octanediamide (LCG-34)

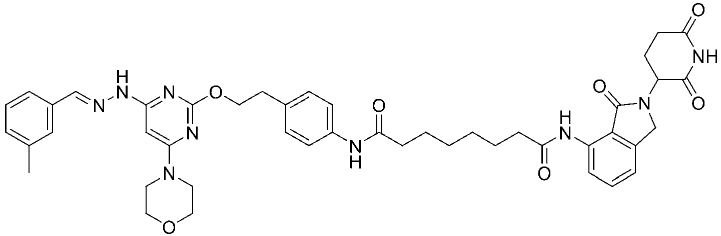

The synthesis method was as shown in Example 1.

¹H NMR (600 MHz, DMSO-*d*₆) δ 11.03 (s, 1H), 10.85 (s, 1H), 10.27 (s, 1H), 9.80 (s, 1H), 8.32 (d, *J =* 8.2 Hz, 1H), 7.99 (s, 1H), 7.56 (t, *J =* 7.9 Hz, 1H), 7.53 - 7.46 (m, 4H), 7.29 (t, *J =* 7.6 Hz, 1H), 7.25 (d, *J =* 7.5 Hz, 1H), 7.20 (d, *J =* 8.5 Hz, 2H), 7.17 (d, *J =* 7.5 Hz, 1H), 6.06 (s, 1H), 5.07 (dd, *J =* 13.3, 5.1 Hz, 1H), 4.47 (d, *J =* 17.6 Hz, 1H), 4.38 - 4.31 (m, 3H), 3.68 - 3.63 (m, 4H), 3.56 - 3.50 (m, 4H), 2.95 - 2.85 (m, 3H), 2.64 - 2.58 (m, 1H), 2.45 - 2.37 (m, 3H), 2.34 (s, 3H), 2.28 (t, *J* = 7.4 Hz, 2H), 2.06 - 2.00 (m, 1H), 1.67 - 1.62 (m, 2H), 1.62 - 1.56 (m, 2H), 1.39 - 1.30 (m, 4H). ¹³C NMR (150 MHz, DMSO-*d*₆) δ 173.30, 171.78, 171.52, 171.28, 169.69, 164.84, 164.43, 163.99, 143.10, 141.68, 138.40, 138.07, 137.55, 135.24, 133.66, 133.41, 130.23, 129.54, 129.08, 127.49, 124.02, 119.59, 118.09, 117.48, 117.21, 76.13, 67.01, 66.38, 52.06, 47.97, 44.68, 37.50, 36.78, 34.74, 31.64, 28.89, 28.80, 25.48, 25.19, 22.81, 21.41. HRMS (ESI) calcd for C₄₅H₅₁N₉O₇ [M + H]⁺ 830.3989, found 830.3983.

### Example 35: (E)-N¹-(2-(2,6-dioxopiperidin-3-yl)-3-oxoisoindolin-4-yl)-N¹⁰-(4-(2-((4-(2-(3-methylbenzylidene)hydrazino)-6-morpholinopyrimidin-2-yl)oxy)ethyl)phenyl)decanediamide (LCG-35)

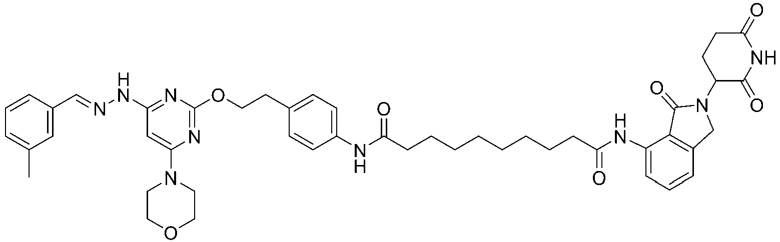

The synthesis method was as shown in Example 1.

¹H NMR (600 MHz, DMSO-*d*₆) δ 11.03 (s, 1H), 10.85 (s, 1H), 10.26 (s, 1H), 9.79 (s, 1H), 8.32 (d, *J =* 8.2 Hz, 1H), 7.99 (s, 1H), 7.56 (t, *J =* 7.9 Hz, 1H), 7.54 - 7.45 (m, 4H), 7.29 (t, *J =* 7.6 Hz, 1H), 7.24 (d, *J =* 7.4 Hz, 1H), 7.20 (d, *J =* 8.5 Hz, 2H), 7.17 (d, *J =* 7.6 Hz, 1H), 6.06 (s, 1H), 5.07 (dd, *J =* 13.3, 5.1 Hz, 1H), 4.47 (d, *J =* 17.6 Hz, 1H), 4.40 - 4.28 (m, 3H), 3.72 - 3.62 (m, 4H), 3.57 - 3.49 (m, 4H), 2.97 - 2.84 (m, 3H), 2.64 - 2.57 (m, 1H), 2.46 - 2.37 (m, 3H), 2.34 (s, 3H), 2.27 (t, *J =* 7.5 Hz, 2H), 2.07 - 2.00 (m, 1H), 1.68 - 1.60 (m, 2H), 1.60 - 1.54 (m, 2H), 1.38 - 1.21 (m, 8H). ¹³C NMR (150 MHz, DMSO-*d*₆) δ 171.97, 170.49, 170.25, 169.95, 168.37, 163.51, 163.10, 162.66, 141.77, 140.36, 137.07, 136.76, 136.23, 133.91, 132.34, 132.08, 128.91, 128.22, 127.75, 126.17, 122.70, 118.26, 116.76, 116.14, 115.86, 74.80, 65.69, 65.06, 50.73, 46.64, 43.36, 36.21, 35.52, 33.42, 30.32, 27.79, 27.66, 24.29, 23.96, 21.49, 20.09. HRMS (ESI) calcd for C₄₇H₅₅N₉O₇ [M + H]⁺ 858.4320, found 858.4298.

### Example 36: (E)-N¹-(2-(2,6-dioxopiperidin-3-yl)-3-oxoisoindolin-4-yl)-N¹²-(4-(2-((4-(2-(3-methylbenzylidene)hydrazino)-6-morpholinopyrimidin-2-yl)oxy)ethyl)phenyl)dodecanediamide (LCG-36)

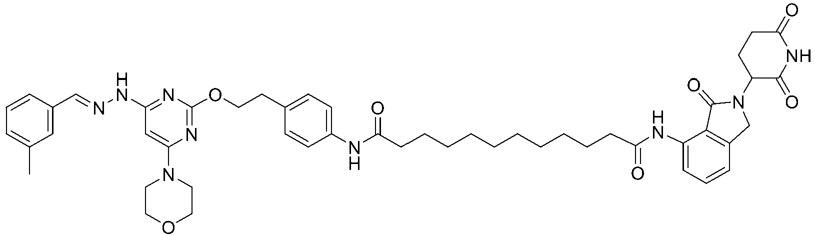

The synthesis method was as shown in Example 1.

¹H NMR (600 MHz, DMSO-*d*₆) δ 11.03 (s, 1H), 10.85 (s, 1H), 10.26 (s, 1H), 9.78 (s, 1H), 8.31 (d, *J* = 8.2 Hz, 1H), 7.99 (s, 1H), 7.56 (t, *J* = 7.9 Hz, 1H), 7.52 - 7.44 (m, 4H), 7.29 (t, *J =* 7.6 Hz, 1H), 7.24 (d, *J =* 7.5 Hz, 1H), 7.20 (d, *J =* 8.4 Hz, 2H), 7.17 (d, *J =* 7.6 Hz, 1H), 6.06 (s, 1H), 5.07 (dd, *J =* 13.3, 5.1 Hz, 1H), 4.47 (d, *J =* 17.6 Hz, 1H), 4.41 - 4.28 (m, 3H), 3.72 - 3.64 (m, 4H), 3.57 - 3.48 (m, 4H), 2.96 - 2.85 (m, 3H), 2.61 (d, *J =* 17.5 Hz, 1H), 2.45 - 2.37 (m, 3H), 2.34 (s, 3H), 2.26 (t, *J =* 7.4 Hz, 2H), 2.06 - 1.99 (m, 1H), 1.66 - 1.59 (m, 2H), 1.59 - 1.51 (m, 2H), 1.34 - 1.24 (m, 12H). ¹³C NMR (150 MHz, DMSO-*d*₆) δ 173.30, 171.82, 171.57, 171.26, 169.69, 164.84, 164.43, 163.99, 143.10, 141.67, 138.39, 138.08, 137.55, 135.24, 133.66, 133.41, 130.23, 129.54, 129.08, 127.49, 124.01, 119.57, 118.08, 117.46, 117.18, 76.13, 67.01, 66.38, 52.06, 47.96, 44.68, 37.53, 36.85, 34.74, 31.64, 29.35, 29.33, 29.24, 29.17, 29.15, 28.99, 25.62, 25.29, 22.81, 21.41. HRMS (ESI) calcd for C₄₉H₅₉N₉O₇ [M + H]⁺ 886.4615, found 886.4608.

### Example 37: N¹-((S)-1-((2S,4R)-4-hydroxy-2-((4-(4-methylthiazol-5-yl)benzyl)carbamoyl) pyrrolidine-1-yl)-3,3-dimethyl-1-oxobutan-2-yl)-N⁴-(4-(2-((4-(2-((E)-3-methylbenzylidene) hydrazino)-6-morpholinopyrimidin-2-yl)oxy)ethyl)phenyl)succinimide (LCG-37)

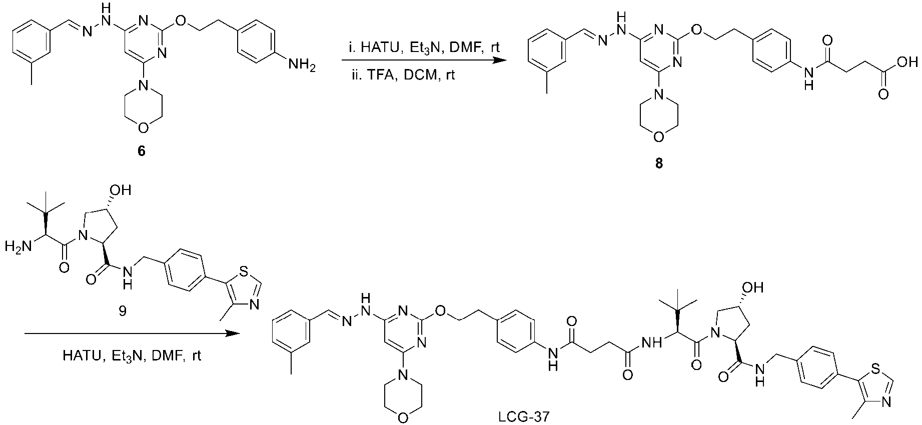

### Step 1: Preparation of (E)-4-((4-(2-((4-(2-(3-methylbenzylidene)hydrazino)-6-morpholinpyrimidin-2-yl)oxy)ethyl)phenyl)amino)-4-oxobutyric acid (8)

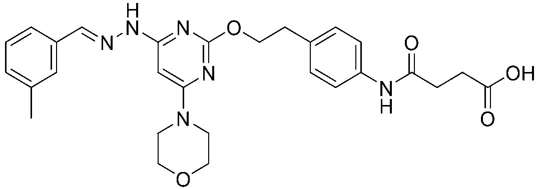

The intermediate 6 (300 mg, 0.7 mmol) and 4-(tert-butoxy)-4-oxobutyric acid (126 mg, 0.7 mmol) were dissolved in 15 mL of DMF, HATU (550 mg, 1.4 mmol) and triethylamine (220 mg, 2.1 mmol) were then added, and the mixture was kept at room temperature for reaction for 4 h. 50 mL of water was added, and the mixture was extracted with EA twice. The organic phases were combined, washed with saturated brine once, dried over anhydrous Na₂SO₄, filtered, and spin-dried to provide an oil, which was directly used in the next step without purification. The oil was dissolved in DCM containing 25% TFA, and the mixture was stirred at room temperature for 3 h. The completion of the reaction was monitored by TLC. The mixture was rotarily evaporated under reduced pressure, and then subjected to column chromatography to provide 250 mg of a pale white solid (67%). ¹H NMR (500 MHz, DMSO-*d*₆) δ 10.89 (brs, 1H), 9.88 (s, 1H), 7.98 (s, 1H), 7.56 - 7.43 (m, 4H), 7.27 (t, *J =* 7.5 Hz, 1H), 7.21 - 7.13 (m, 3H), 6.03 (s, 1H), 4.33 (t, *J =* 6.3 Hz, 2H), 3.69 - 3.62 (m, 6H), 3.56 - 3.50 (m, 4H), 2.91 (t, *J =* 6.9 Hz, 2H), 2.52 (t, *J =* 5.1 Hz, 2H), 2.32 (s, 3H). MS (ESI),m/z: 533.3 [M+H]⁺.

### Step 2: Preparation of N¹-((S)-1-((2S,4R)-4-hydroxy-2-((4-(4-methylthiazol-5-yl)benzyl) carbamoyl)pyrrolidine-1-yl)-3,3-dimethyl-1-oxobutan-2-yl)-N⁴-(4-(2-((4-(2-((E)-3-methylbenzylidene)hydrazino)-6-morpholinopyrimidin-2-yl)oxy)ethyl)phenyl)succinimide (LCG-37)

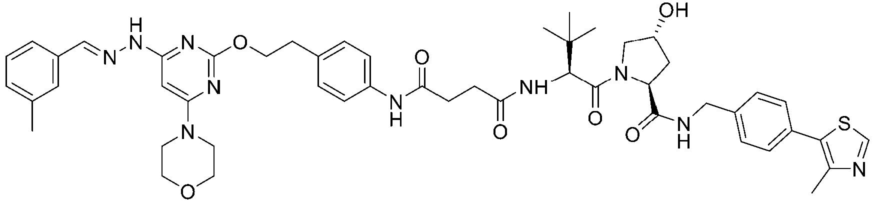

An intermediate 8 (40 mg, 0.07 mmol) and a reported intermediate 9 (30 mg, 0.7 mmol) of a VHL ligand of an E3 ubiquitin ligase were dissolved in 10 mL of DMF, HATU (70 mg, 0.2 mmol) and triethylamine (40 mg, 0.4 mmol) were then added, and the mixture was kept at room temperature for reaction for 4 h. 20 mL of water was added, and the mixture was extracted with EA twice. The organic phases were combined, washed with saturated brine once, dried over anhydrous Na₂SO₄, filtered, spin-dried, and then subjected to column chromatography to provide 29 mg of a white solid (44%). ¹H NMR (600 MHz, DMSO-*d*₆) δ 10.86 (s, 1H), 9.88 (s, 1H), 8.98 (s, 1H), 8.57 (t, *J =* 6.0 Hz, 1H), 7.99 (s, 1H), 7.96 (d, *J =* 9.3 Hz, 1H), 7.53 - 7.46 (m, 4H), 7.42 (d, *J =* 8.3 Hz, 2H), 7.39 (d, *J =* 8.3 Hz, 2H), 7.29 (t, *J =* 7.6 Hz, 1H), 7.20 (d, *J =* 8.5 Hz, 2H), 7.17 (d, *J =* 7.5 Hz, 1H), 6.06 (s, 1H), 5.12 (d, *J =* 3.5 Hz, 1H), 4.55 (d, *J =* 9.4 Hz, 1H), 4.47 - 4.40 (m, 2H), 4.33 (t, *J =* 7.1 Hz, 3H), 4.22 (dd, *J =* 15.8, 5.4 Hz, 1H), 3.72 - 3.60 (m, 6H), 3.55 - 3.49 (m, 4H), 2.92 (t, *J =* 7.0 Hz, 2H), 2.63 - 2.56 (m, 1H), 2.56 - 2.52 (m, 2H), 2.49 - 2.42 (m, 4H), 2.34 (s, 3H), 2.06 - 2.01 (m, 1H), 1.93 - 1.87 (m, 1H), 0.94 (s, 9H). ¹³C NMR (150 MHz, DMSO-*d*₆) δ 172.41, 171.65, 170.78, 170.05, 164.85, 164.43, 163.99, 151.93, 148.19, 141.67, 139.98, 138.40, 138.07, 135.23, 133.37, 131.64, 130.23, 130.11, 129.56, 129.12, 129.08, 127.90, 127.49, 124.01, 119.48, 76.13, 69.36, 67.01, 66.38, 59.19, 56.93, 56.82, 44.69, 42.12, 38.40, 35.86, 34.73, 32.32, 30.59, 26.84, 21.41, 16.42. HRMS (ESI) calcd for C₅₀H₆₀N₁₀O₇S [M + H]⁺ 945.4445, found 945.4438.

### Example 38: N¹-((S)-1-((2S,4R)-4-hydroxy-2-((4-(4-methylthiazol-5-yl)benzyl)carbamoyl) pyrrolidine-1-yl)-3,3-dimethyl-1-oxobutan-2-yl)-N⁶-(4-(2-((4-(2-((E)-3-methylbenzylidene) hydrazino)-6-morpholinopyrimidin-2-yl)oxy)ethyl)phenyl)adipamide (LCG-38)

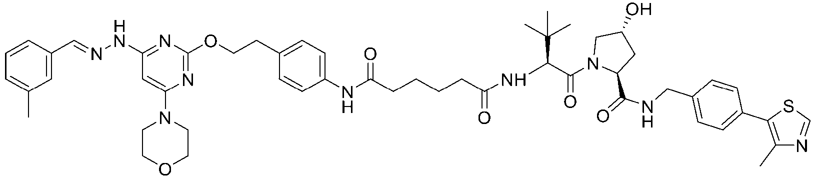

The synthesis method was as shown in Example 37.

¹H NMR (600 MHz, DMSO-*d*₆) δ 10.86 (s, 1H), 9.81 (s, 1H), 8.98 (s, 1H), 8.57 (t, *J=* 6.1 Hz, 1H), 7.99 (s, 1H), 7.88 (d, *J =* 9.3 Hz, 1H), 7.53 - 7.47 (m, 4H), 7.42 (d, *J =* 8.3 Hz, 2H), 7.40 - 7.36 (m, 2H), 7.29 (t, *J =* 7.6 Hz, 1H), 7.20 (d, *J =* 8.5 Hz, 2H), 7.17 (d, *J =* 7.5 Hz, 1H), 6.07 (s, 1H), 5.13 (d, *J =* 3.6 Hz, 1H), 4.55 (d, *J =* 9.4 Hz, 1H), 4.47 - 4.41 (m, 2H), 4.33 (t, *J =* 7.1 Hz, 3H), 4.22 (dd, *J =* 15.9, 5.5 Hz, 1H), 3.71 - 3.62 (m, 6H), 3.56 - 3.50 (m, 4H), 2.92 (t, *J =* 7.0 Hz, 2H), 2.45 (s, 3H), 2.34 (s, 3H), 2.32 - 2.24 (m, 3H), 2.20 - 2.13 (m, 1H), 2.06 - 2.00 (m, 1H), 1.94 - 1.87 (m, 1H), 1.61 - 1.48 (m, 4H), 0.94 (s, 9H). ¹³C NMR (150 MHz, DMSO-*d*₆) δ 172.42, 172.41, 171.45, 170.19, 164.85, 164.44, 163.99, 151.92, 148.19, 141.68, 139.98, 138.39, 138.04, 135.24, 133.46, 131.64, 130.23, 130.11, 129.55, 129.11, 129.08, 127.89, 127.49, 124.02, 119.60, 76.14, 69.35, 67.01, 66.38, 59.17, 56.85, 56.81, 44.69, 42.12, 38.42, 36.69, 35.69, 35.21, 34.74, 26.93, 26.87, 25.67, 25.44, 21.41, 16.42. HRMS (ESI) calcd for C₅₂H₆₄N₁₀O₇S [M + H]⁺ 973.4758, found 973.4757.

### Example 39: N¹-((S)-1-((2S,4R)-4-hydroxy-2-((4-(4-methylthiazol-5-yl)benzyl)carbamoyl) pyrrolidine-1-yl)-3,3-dimethyl-1-oxobutan-2-yl)-N⁸-(4-(2-((4-(2-((E)-3-methylbenzylidene) hydrazino)-6-morpholinopyrimidin-2-yl)oxy)ethyl)phenyl)octanediamide (LCG-39)

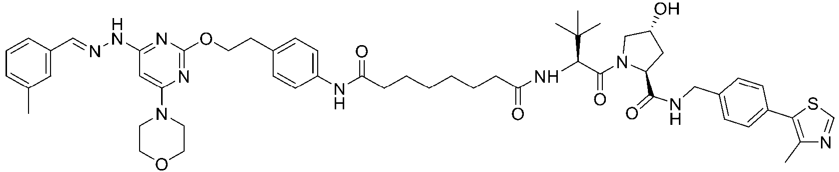

The synthesis method was as shown in Example 37.

¹H NMR (600 MHz, DMSO-*d*₆) δ 10.85 (s, 1H), 9.80 (s, 1H), 8.98 (s, 1H), 8.56 (t, *J =* 6.1 Hz, 1H), 7.99 (s, 1H), 7.85 (d, *J =* 9.4 Hz, 1H), 7.53 - 7.46 (m, 4H), 7.42 (d, *J =* 8.2 Hz, 2H), 7.40 - 7.36 (m, 2H), 7.29 (t, *J =* 7.6 Hz, 1H), 7.20 (d, *J =* 8.5 Hz, 2H), 7.17 (d, *J =* 7.5 Hz, 1H), 6.06 (s, 1H), 5.13 (d, *J =* 3.6 Hz, 1H), 4.55 (d, *J=* 9.4 Hz, 1H), 4.46 - 4.40 (m, 2H), 4.37 - 4.31 (m, 3H), 4.22 (dd, *J =* 15.9, 5.5 Hz, 1H), 3.70 - 3.63 (m, 6H), 3.55 - 3.50 (m, 4H), 2.92 (t, *J =* 7.0 Hz, 2H), 2.44 (s, 3H), 2.34 (s, 3H), 2.29 - 2.22 (m, 3H), 2.15 - 2.10 (m, 1H), 2.06 - 2.01 (m, 1H), 1.93 - 1.88 (m, 1H), 1.60 - 1.42 (m, 4H), 1.32 - 1.24 (m, 4H), 0.94 (s, 9H). ¹³C NMR (150 MHz, DMSO-*d*₆) δ 172.56, 172.43, 171.55, 170.20, 164.84, 164.43, 163.99, 151.92, 148.19, 141.68, 139.98, 138.39, 138.07, 135.23, 133.43, 131.64, 130.23, 130.11, 129.54, 129.11, 129.08, 127.89, 127.49, 124.02, 119.59, 76.13, 69.34, 67.01, 66.38, 59.16, 56.84, 56.76, 44.68, 42.12, 38.42, 36.84, 35.68, 35.34, 34.74, 28.98, 28.94, 26.86, 25.83, 25.56, 21.41, 16.42. HRMS (ESI) calcd for C₅₄H₆₈N₁₀O₇S [M + H]⁺ 1001.5071, found 1001.5063.

### Example 40: N¹-((S)-1-((2S,4R)-4-hydroxy-2-((4-(4-methylthiazol-5-yl)benzyl)carbamoyl) pyrrolidine-1-yl)-3,3-dimethyl-1-oxobutan-2-yl)-N¹⁰-(4-(2-((4-(2-((E)-3-methylbenzylidene) hydrazino)-6-morpholinopyrimidin-2-yl)oxy)ethyl)phenyl)decanediamide (LCG-40)

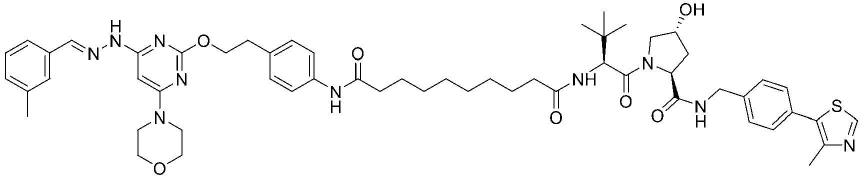

The synthesis method was as shown in Example 37.

¹H NMR (600 MHz, DMSO-*d*₆) δ 10.85 (s, 1H), 9.79 (s, 1H), 8.98 (s, 1H), 8.56 (t, *J =* 6.0 Hz, 1H), 7.99 (s, 1H), 7.84 (d, *J =* 9.4 Hz, 1H), 7.54 - 7.46 (m, 4H), 7.42 (d, *J =* 8.3 Hz, 2H), 7.38 (d, *J* = 8.3 Hz, 2H), 7.29 (t, *J* = 7.6 Hz, 1H), 7.20 (d, *J =* 8.5 Hz, 2H), 7.17 (d, *J =* 7.5 Hz, 1H), 6.06 (s, 1H), 5.12 (d, *J =* 3.6 Hz, 1H), 4.54 (d, *J* = 9.4 Hz, 1H), 4.46 - 4.40 (m, 2H), 4.37 - 4.31 (m, 3H), 4.22 (dd, *J =* 15.8, 5.4 Hz, 1H), 3.70 - 3.62 (m, 6H), 3.56 - 3.50 (m, 4H), 2.92 (t, *J =* 7.0 Hz, 2H), 2.44 (s, 3H), 2.34 (s, 3H), 2.29 - 2.23 (m, 3H), 2.14 - 2.08 (m, 1H), 2.05 - 1.99 (m, 1H), 1.94 - 1.87 (m, 1H), 1.60 - 1.42 (m, 4H), 1.32 - 1.17 (m, 8H), 0.93 (s, 9H). ¹³C NMR (150 MHz, DMSO-*d*₆) δ 172.56, 172.42, 171.56, 170.19, 164.84, 164.43, 163.99, 151.92, 148.19, 141.67, 139.98, 138.39, 138.08, 135.24, 133.41, 131.64, 130.23, 130.11, 129.54, 129.11, 129.08, 127.89, 127.49, 124.01, 119.57, 76.13, 69.33, 67.01, 66.38, 59.16, 56.82, 56.74, 44.68, 42.11, 38.43, 36.86, 35.68, 35.32, 34.74, 29.24, 29.15, 29.13, 26.85, 25.89, 25.63, 21.41, 16.42. HRMS (ESI) calcd for C₅₆H₇₂N₁₀O₇S [M + H]⁺ 1029.5384, found 1029.5378.

### Example 41: N¹-((S)-1-((2S,4R)-4-hydroxy-2-((4-(4-methylthiazol-5-yl)benzyl)carbamoyl) pyrrolidine-1-yl)-3,3-dimethyl-1-oxobutan-2-yl)-N⁵-(4-(2-((4-(2-((E)-3-methylbenzylidene) hydrazino)-6-morpholinopyrimidin-2-yl)oxy)ethyl)phenyl)glutaramide (LCG-41)

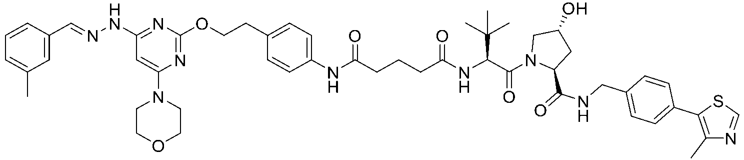

The synthesis method was as shown in Example 37.

¹H NMR (600 MHz, DMSO-*d*₆) δ 10.85 (s, 1H), 9.81 (s, 1H), 8.98 (s, 1H), 8.56 (t, *J =* 6.0 Hz, 1H), 7.99 (s, 1H), 7.92 (d, *J =* 9.2 Hz, 1H), 7.54 - 7.45 (m, 4H), 7.42 (d, *J =* 8.3 Hz, 2H), 7.40 - 7.36 (m, 2H), 7.29 (t, *J =* 7.6 Hz, 1H), 7.20 (d, *J =* 8.5 Hz, 2H), 7.17 (d, *J =* 7.5 Hz, 1H), 6.06 (s, 1H), 5.14 (d, *J =* 3.3 Hz, 1H), 4.55 (d, *J* = 9.3 Hz, 1H), 4.46 - 4.40 (m, 2H), 4.38 - 4.32 (m, 3H), 4.22 (dd, *J =* 15.8, 5.4 Hz, 1H), 3.70 - 3.62 (m, 6H), 3.55 - 3.50 (m, 4H), 2.92 (t, *J =* 6.9 Hz, 2H), 2.44 (s, 3H), 2.34 (s, 3H), 2.32 - 2.25 (m, 3H), 2.24 - 2.18 (m, 1H), 2.07 - 2.00 (m, 1H), 1.95 - 1.87 (m, 1H), 1.84 - 1.75 (m, 2H), 0.95 (s, 9H). ¹³C NMR (150 MHz, DMSO-*d*₆) δ 172.41, 172.18, 171.20, 170.20, 164.84, 164.43, 163.99, 151.92, 148.19, 141.68, 139.98, 138.39, 138.05, 135.23, 133.45, 131.64, 130.23, 130.11, 129.53, 129.11, 129.08, 127.89, 127.49, 124.02, 119.63, 76.13, 69.37, 67.01, 66.38, 59.18, 56.91, 56.84, 44.68, 42.12, 38.42, 36.32, 35.66, 34.74, 34.68, 26.88, 21.99, 21.41, 16.42. HRMS (ESI) calcd for C₅₁H₆₂N₁₀O₇S [M + H]⁺ 959.4602, found 959.4584.

### Example 42: N¹-((S)-1-((2S,4R)-4-hydroxy-2-((4-(4-methylthiazol-5-yl)benzyl)carbamoyl) pyrrolidine-1-yl)-3,3-dimethyl-1-oxobutan-2-yl)-N⁷-(4-(2-((4-(2-((E)-3-methylbenzylidene) hydrazino)-6-morpholinopyrimidin-2-yl)oxy)ethyl)phenyl)heptanediamide (LCG-42)

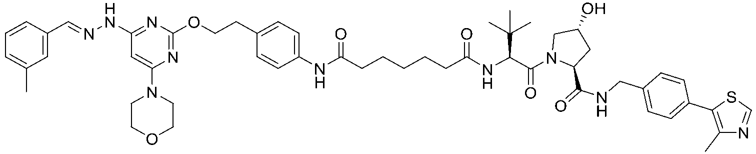

The synthesis method was as shown in Example 37.

¹H NMR (600 MHz, DMSO-*d*₆) δ 10.85 (s, 1H), 9.79 (s, 1H), 8.98 (d, *J =* 5.4 Hz, 1H), 8.56 (t, *J* = 6.1 Hz, 1H), 7.99 (s, 1H), 7.86 (d, *J = 9.3* Hz, 1H), 7.53 - 7.46 (m, 4H), 7.42 (d, *J =* 8.3 Hz, 2H), 7.38 (d, *J =* 8.3 Hz, 2H), 7.29 (t, *J =* 7.6 Hz, 1H), 7.20 (d, *J =* 8.5 Hz, 2H), 7.17 (d, *J* = 7.5 Hz, 1H), 6.06 (s, 1H), 5.13 (d, *J* = 3.6 Hz, 1H), 4.54 (d, *J* = 9.4 Hz, 1H), 4.45 - 4.40 (m, 2H), 4.37 - 4.30 (m, 3H), 4.22 (dd, *J =* 15.8, 5.5 Hz, 1H), 3.70 - 3.62 (m, 6H), 3.55 - 3.50 (m, 4H), 2.92 (t, *J* = 7.0 Hz, 2H), 2.44 (s, 3H), 2.34 (s, 3H), 2.30 - 2.22 (m, 3H), 2.16 - 2.10 (m, 1H), 2.07 - 2.00 (m, 1H), 1.95 - 1.86 (m, 1H), 1.62 - 1.43 (m, 4H), 1.32 - 1.23 (m, 2H), 0.93 (s, 9H). ¹³C NMR (150 MHz, DMSO-*d*₆) δ 172.51, 172.42, 171.51, 170.19, 164.84, 164.43, 163.99, 151.93, 148.19, 141.68, 139.98, 138.39, 138.08, 135.23, 133.41, 131.64, 130.23, 130.11, 129.54, 129.11, 129.08, 127.89, 127.49, 124.01, 119.58, 76.13, 69.34, 67.01, 66.38, 59.16, 56.84, 56.76, 44.68, 42.12, 38.43, 36.75, 35.68, 35.26, 34.74, 28.84, 26.86, 25.73, 25.41, 21.41, 16.42. HRMS (ESI) calcd for C₅₃H₆₆N₁₀O₇S [M + H]⁺ 987.4915, found 987.4912.

### Example 43: N¹-((S)-1-((2S,4R)-4-hydroxy-2-((4-(4-methylthiazol-5-yl)benzyl)carbamoyl) pyrrolidine-1-yl)-3,3-dimethyl-1-oxobutan-2-yl)-N⁹-(4-(2-((4-(2-((E)-3-methylbenzylidene) hydrazino)-6-morpholinopyrimidin-2-yl)oxy)ethyl)phenyl)nonediamide (LCG-43)

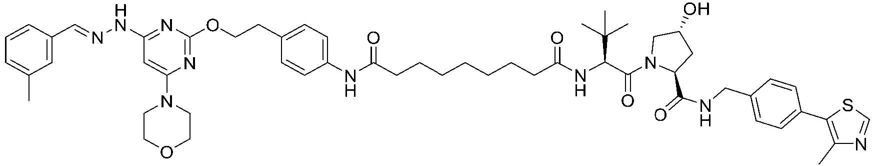

The synthesis method was as shown in Example 37.

¹H NMR (600 MHz, DMSO-*d*₆) δ 10.85 (s, 1H), 9.79 (s, 1H), 8.98 (s, 1H), 8.56 (t, *J =* 6.1 Hz, 1H), 7.99 (s, 1H), 7.84 (d, *J =* 9.4 Hz, 1H), 7.53 - 7.46 (m, 4H), 7.42 (d, *J =* 8.3 Hz, 2H), 7.40 - 7.36 (m, 2H), 7.29 (t, *J =* 7.6 Hz, 1H), 7.20 (d, *J =* 8.5 Hz, 2H), 7.17 (d, *J =* 7.5 Hz, 1H), 6.06 (s, 1H), 5.12 (d, *J =* 3.6 Hz, 1H), 4.54 (d, *J =* 9.4 Hz, 1H), 4.46 - 4.39 (m, 2H), 4.33 (t, *J=* 7.1 Hz, 3H), 4.21 (dd, *J =* 15.8, 5.5 Hz, 1H), 3.72 - 3.61 (m, 6H), 3.56 - 3.48 (m, 4H), 2.92 (t, *J =* 7.0 Hz, 2H), 2.44 (s, 3H), 2.34 (s, 3H), 2.30 - 2.20 (m, 3H), 2.15 - 2.08 (m, 1H), 2.05 - 1.98 (m, 1H), 1.95 - 1.85 (m, 1H), 1.62 - 1.40 (m, 4H), 1.30 - 1.20 (m, 6H), 0.93 (s, 9H). ¹³C NMR (150 MHz, DMSO-*d*₆) δ 170.70, 170.56, 169.71, 168.32, 162.98, 162.57, 162.13, 150.06, 146.33, 139.81, 138.12, 136.53, 136.21, 133.37, 131.56, 129.77, 128.37, 128.24, 127.68, 127.25, 127.22, 126.03, 125.63, 122.15, 117.72, 74.27, 67.47, 65.14, 64.52, 57.29, 54.96, 54.87, 42.82, 38.67, 36.56, 34.99, 33.81, 33.46, 32.87, 27.21, 27.16, 24.98, 24.03, 23.76, 19.55, 14.56. HRMS (ESI) calcd for C₅₅H₇₀N₁₀O₇S [M + H]⁺ 1015.5228, found 1015.5218.

### Example 44: N¹-((S)-1-((2S,4R)-4-hydroxy-2-((4-(4-methylthiazol-5-yl)benzyl)carbamoyl) pyrrolidine-1-yl)-3,3-dimethyl-1-oxobutan-2-yl)-N¹¹-(4-(2-((4-(2-((E)-3-methylbenzylidene) hydrazino)-6-morpholinopyrimidin-2-yl)oxy)ethyl)phenyl)undecanediamide (LCG-44)

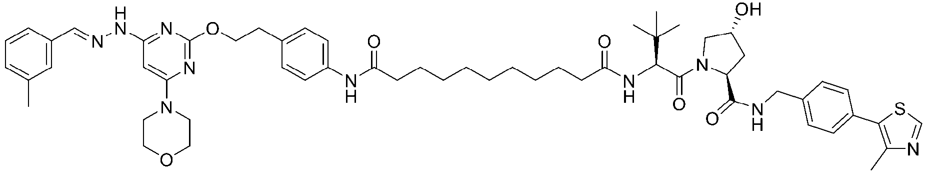

The synthesis method was as shown in Example 37.

¹H NMR (600 MHz, DMSO-*d*₆) δ 10.85 (s, 1H), 9.79 (s, 1H), 8.98 (s, 1H), 8.55 (t, *J =* 6.1 Hz, 1H), 7.99 (s, 1H), 7.84 (d, *J =* 9.4 Hz, 1H), 7.52 - 7.47 (m, 4H), 7.42 (d, *J =* 8.3 Hz, 2H), 7.39 - 7.37 (m, 2H), 7.29 (t, *J =* 7.6 Hz, 1H), 7.20 (d, *J =* 8.5 Hz, 2H), 7.17 (d, *J =* 7.5 Hz, 1H), 6.06 (s, 1H), 5.11 (d, *J =* 3.2 Hz, 1H), 4.54 (d, *J =* 9.4 Hz, 1H), 4.45 - 4.40 (m, 2H), 4.33 (t, *J=* 7.1 Hz, 3H), 4.21 (dd, *J =* 15.9, 5.4 Hz, 1H), 3.68 - 3.62 (m, 6H), 3.54 - 3.50 (m, 4H), 2.92 (t, *J =* 7.0 Hz, 2H), 2.44 (s, 3H), 2.34 (s, 3H), 2.29 - 2.22 (m, 3H), 2.14 - 2.07 (m, 1H), 2.05 - 1.97 (m, 1H), 1.93 - 1.86 (m, 1H), 1.62 - 1.52 (m, 2H), 1.53 - 1.42 (m, 2H), 1.30 - 1.20 (m, 12H), 0.93 (s, 9H). ¹³C NMR (150 MHz, DMSO-*d*₆) δ 172.56, 172.42, 171.56, 170.18, 164.84, 164.43, 163.99, 151.93, 148.19, 141.67, 139.98, 138.39, 138.08, 135.23, 133.42, 131.64, 130.23, 130.11, 129.54, 129.11, 129.08, 127.89, 127.49, 124.01, 119.58, 76.13, 69.33, 67.01, 66.38, 59.15, 56.82, 56.73, 44.68, 42.11, 38.43, 36.86, 35.68, 35.33, 34.74, 29.34, 29.27, 29.22, 29.16, 29.13, 26.85, 25.91, 25.64, 21.41, 16.42. HRMS (ESI) calcd for C₅₇H₇₄N₁₀O₇S [M + H]⁺ 1043.5541, found 1043.5527.

### Example 45: N¹-((S)-1-((2S,4R)-4-hydroxy-2-(((S)-1-4-(4-methylthiazol-5-yl)phenyl)ethyl) carbamoyl)pyrrolidine-1-yl)-3,3-dimethyl-1-oxobutan-2-yl)-N⁵-(4-(2-((4-(2-((E)-3-methylbenzylidene)hydrazino)-6-morpholinopyrimidin-2-yl)oxy)ethyl)phenyl)glutaramide (LCG-45)

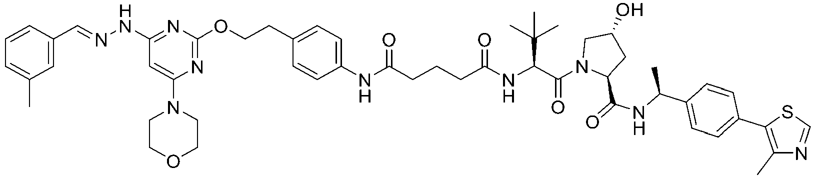

The synthesis method was as shown in Example 37.

¹H NMR (600 MHz, DMSO-*d*₆) δ 10.86 (s, 1H), 9.82 (s, 1H), 8.98 (s, 1H), 8.38 (d, *J =* 7.8 Hz, 1H), 7.99 (s, 1H), 7.86 (d, *J =* 9.2 Hz, 1H), 7.54 - 7.46 (m, 4H), 7.45 - 7.41 (m, 2H), 7.39 - 7.36 (m, 2H), 7.29 (t, *J =* 7.6 Hz, 1H), 7.21 (d, *J =* 8.5 Hz, 2H), 7.17 (d, *J =* 7.4 Hz, 1H), 6.06 (s, 1H), 5.12 (s, 1H), 4.95 - 4.88 (m, 1H), 4.52 (d, *J =* 9.3 Hz, 1H), 4.43 (t, *J =* 8.0 Hz, 1H), 4.33 (t, *J =* 7.0 Hz, 2H), 4.29 (s, 1H), 3.69 - 3.64 (m, 4H), 3.65 - 3.60 (m, 2H), 3.56 - 3.50 (m, 4H), 2.93 (t, *J =* 7.0 Hz, 2H), 2.45 (s, 3H), 2.34 (s, 3H), 2.32 - 2.25 (m, 3H), 2.24 - 2.18 (m, 1H), 2.04 - 1.99 (m, 1H), 1.83 - 1.75 (m, 3H), 1.37 (d, *J* = 7.0 Hz, 3H), 0.94 (d, *J* = 9.5 Hz, 9H). ¹³C NMR (150 MHz, DMSO-*d*₆) δ 171.06, 170.12, 170.01, 169.02, 163.76, 163.35, 162.90, 150.87, 147.14, 144.05, 140.60, 137.31, 136.98, 134.15, 132.38, 130.50, 129.15, 129.08, 128.45, 128.21, 128.00, 126.41, 125.77, 122.93, 118.54, 75.05, 68.18, 65.94, 65.30, 57.96, 55.91, 55.65, 47.08, 43.60, 37.11, 35.23, 34.55, 33.66, 33.61, 25.86, 21.83, 20.91, 20.33, 15.38. HRMS (ESI) calcd for C₅₂H₆₄N₁₀O₇S [M + Na]⁺995.4578, found 995.4565.

### Example 46: N¹-((S)-1-((2S,4R)-4-hydroxy-2-(((S)-1-4-(4-methylthiazol-5-yl)phenyl)ethyl) carbamoyl)pyrrolidine-1-yl)-3,3-dimethyl-1-oxobutan-2-yl)-N⁶-(4-(2-((4-(2-((E)-3-methylbenzylidene)hydrazino)-6-morpholinopyrimidin-2-yl)oxy)ethyl)phenyl)adipamide (LCG-46)

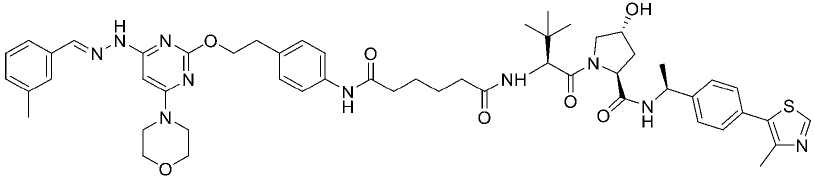

The synthesis method was as shown in Example 37.

¹H NMR (600 MHz, DMSO-*d*₆) δ 10.86 (s, 1H), 9.81 (s, 1H), 8.98 (s, 1H), 8.37 (d, *J* = 7.8 Hz, 1H), 7.99 (s, 1H), 7.81 (d, *J* = 9.2 Hz, 1H), 7.53 - 7.46 (m, 4H), 7.43 (d, *J =* 8.2 Hz, 2H), 7.37 (d, *J =* 8.2 Hz, 2H), 7.29 (t, *J =* 7.6 Hz, 1H), 7.20 (d, *J =* 8.5 Hz, 2H), 7.17 (d, *J =* 8.0 Hz, 1H), 6.06 (s, 1H), 5.10 (s, 1H), 4.96 - 4.88 (m, 1H), 4.51 (d, *J =* 9.3 Hz, 1H), 4.43 (t, *J =* 8.0 Hz, 1H), 4.33 (t, *J =* 7.0 Hz, 2H), 4.28 (s, 1H), 3.69 - 3.64 (m, 4H), 3.64 - 3.58 (m, 2H), 3.56 - 3.49 (m, 4H), 2.93 (t, *J =* 7.0 Hz, 2H), 2.45 (s, 3H), 2.34 (s, 3H), 2.31 - 2.25 (m, 3H), 2.19 - 2.12 (m, 1H), 2.04 - 1.97 (m, 1H), 1.83 - 1.75 (m, 1H), 1.60 - 1.48 (m, 4H), 1.37 (d, *J =* 7.0 Hz, 3H), 0.94 (s, 9H). ¹³C NMR (150 MHz, DMSO-*d*₆) δ 171.26, 170.35, 169.99, 168.98, 163.73, 163.31, 162.87, 150.85, 147.12, 144.03, 140.59, 137.29, 136.94, 134.13, 132.35, 130.48, 129.13, 129.06, 128.46, 128.19, 127.98, 126.40, 125.74, 125.61, 122.92, 118.50, 75.02, 68.14, 65.92, 65.28, 57.92, 55.78, 55.64, 47.06, 43.59, 37.08, 35.59, 34.56, 34.13, 33.64, 25.83, 24.55, 24.32, 21.81, 20.31, 15.36. HRMS (ESI) calcd for C₅₃H₆₆N₁₀O₇S [M + Na]⁺ 1009.4735, found 1009.4711.

### Example 47: N¹-((S)-1-((2S,4R)-4-hydroxy-2-(((S)-1-4-(4-methylthiazol-5-yl)phenyl)ethyl) carbamoyl)pyrrolidine-1-yl)-3,3-dimethyl-1-oxobutan-2-yl)-N⁷-(4-(2-((4-(2-((E)-3-methylbenzylidene)hydrazino)-6-morpholinopyrimidin-2-yl)oxy)ethyl)phenyl)heptanediamide (LCG-47)

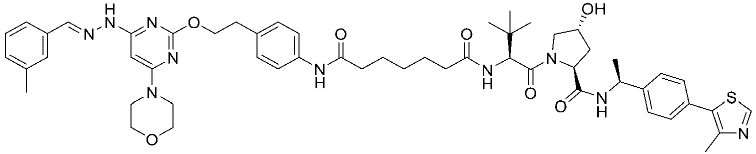

The synthesis method was as shown in Example 37.

¹H NMR (600 MHz, DMSO-*d*₆) δ 10.85 (s, 1H), 9.80 (s, 1H), 8.98 (s, 1H), 8.37 (d, *J =* 7.8 Hz, 1H), 7.99 (s, 1H), 7.80 (d, *J =* 9.3 Hz, 1H), 7.53 - 7.46 (m, 4H), 7.45 - 7.41 (m, 2H), 7.37 (d, *J =* 8.2 Hz, 2H), 7.29 (t, *J =* 7.6 Hz, 1H), 7.20 (d, *J =* 8.5 Hz, 2H), 7.17 (d, *J =* 7.5 Hz, 1H), 6.06 (s, 1H), 5.10 (d, *J =* 3.4 Hz, 1H), 4.96 - 4.88 (m, 1H), 4.51 (d, *J =* 9.3 Hz, 1H), 4.42 (t, *J =* 8.0 Hz, 1H), 4.33 (t, *J =* 7.0 Hz, 2H), 4.28 (s, 1H), 3.70 - 3.64 (m, 4H), 3.63 - 3.57 (m, 2H), 3.55 - 3.49 (m, 4H), 2.92 (t, *J =* 7.0 Hz, 2H), 2.45 (s, 3H), 2.34 (s, 3H), 2.30 - 2.22 (m, 3H), 2.17 - 2.10 (m, 1H), 2.05 - 1.98 (m, 1H), 1.82 - 1.76 (m, 1H), 1.62 - 1.45 (m, 4H), 1.37 (d, *J =* 7.0 Hz, 3H), 1.31 - 1.23 (m, 2H), 0.93 (s, 9H). ¹³C NMR (150 MHz, DMSO-*d*₆) δ 171.31, 170.36, 169.94, 168.93, 163.70, 163.29, 162.84, 150.81, 147.08, 143.99, 140.53, 137.25, 136.94, 134.09, 132.27, 130.44, 129.08, 129.01, 128.39, 128.15, 127.93, 126.35, 125.70, 122.87, 118.42, 74.98, 68.09, 65.87, 65.23, 57.87, 55.68, 55.59, 47.01, 43.54, 37.05, 35.62, 34.50, 34.12, 33.59, 27.68, 25.77, 24.57, 24.26, 21.75, 20.26, 15.31. HRMS (ESI) calcd for C₅₄H₆₈N₁₀O₇S [M + H]⁺ 1001.5071, found 1001.5070.

### Example 48: N¹-((S)-1-((2S,4R)-4-hydroxy-2-(((S)-1-4-(4-methylthiazol-5-yl)phenyl)ethyl) carbamoyl)pyrrolidine-1-yl)-3,3-dimethyl-1-oxobutan-2-yl)-N¹⁰-(4-(2-((4-(2-((E)-3-methylbenzylidene)hydrazino)-6-morpholinopyrimidin-2-yl)oxy)ethyl)phenyl)decanediamide (LCG-48)

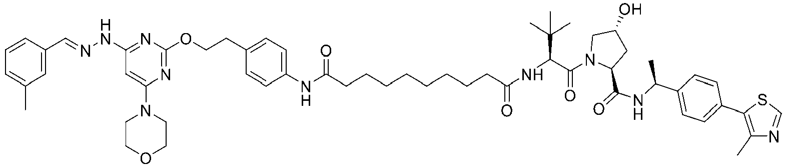

The synthesis method was as shown in Example 37.

¹H NMR (600 MHz, DMSO-*d*₆) δ 10.85 (s, 1H), 9.79 (s, 1H), 8.98 (s, 1H), 8.37 (d, *J =* 7.8 Hz, 1H), 7.99 (s, 1H), 7.78 (d, *J =* 9.3 Hz, 1H), 7.53 - 7.46 (m, 4H), 7.44 - 7.42 (m, 2H), 7.37 (d, *J =* 8.2 Hz, 2H), 7.29 (t, *J =* 7.6 Hz, 1H), 7.20 (d, *J =* 8.5 Hz, 2H), 7.17 (d, *J =* 7.5 Hz, 1H), 6.06 (s, 1H), 5.09 (d, *J =* 2.2 Hz, 1H), 4.94 - 4.89 (m, 1H), 4.51 (d, *J =* 9.4 Hz, 1H), 4.42 (t, *J =* 8.0 Hz, 1H), 4.33 (t, *J =* 7.1 Hz, 2H), 4.28 (s, 1H), 3.69 - 3.64 (m, 4H), 3.63 - 3.57 (m, 2H), 3.55 - 3.50 (m, 4H), 2.92 (t, *J =* 7.0 Hz, 2H), 2.45 (d, *J =* 2.5 Hz, 3H), 2.34 (s, 3H), 2.29 - 2.20 (m, 3H), 2.14 - 2.07 (m, 1H), 2.04 - 1.96 (m, 1H), 1.82 - 1.76 (m, 1H), 1.62 - 1.54 (m, 2H), 1.54 - 1.41 (m, 2H), 1.37 (d, *J =* 7.0 Hz, 3H), 1.32 - 1.16 (m, 8H), 0.93 (s, 9H). ¹³C NMR (150 MHz, DMSO-*d*₆) δ 171.41, 170.46, 169.99, 168.98, 163.74, 163.33, 162.88, 150.85, 147.13, 144.04, 140.58, 137.29, 136.98, 134.13, 132.31, 130.48, 129.13, 129.06, 128.45, 128.19, 127.98, 126.40, 125.75, 122.92, 118.48, 75.03, 68.12, 65.91, 65.28, 57.90, 55.69, 55.62, 47.06, 43.59, 37.10, 35.76, 34.56, 34.24, 33.64, 28.14, 28.05, 28.02, 25.81, 24.78, 24.54, 21.81, 20.32, 15.36. HRMS (ESI) calcd for C₅₇H₇₄N₁₀O₇S [M + Na]⁺ 1065.5361, found 1065.5359.

### Example 49: (2S,4R)-1-((S)-2-(1-fluorocyclopropane-1-carboxamido)-3,3-dimethylbutanoyl) -4-hydroxy-N-((S)-4-((4-(2-((4-(2-((E)-3-methylbenzylidene)hydrazino)-6-morpholinopyrimidin-2-yl)oxy)ethyl)phenyl)amino)-1-(4-(4-methylthiazol-5-yl)phenyl)4-oxobutyl)pyrrolidine-2-carboxamide (LCG-49)

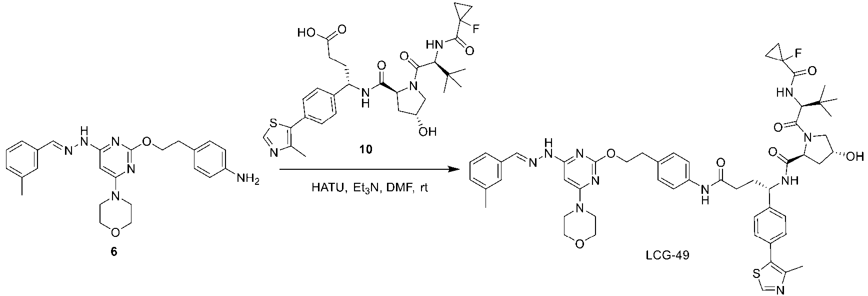

The intermediate 6 (40 mg, 0.09 mmol) and a reported intermediate 10 (55 mg, 0.09 mmol) of a VHL ligand of an E3 ubiquitin ligase were dissolved in 10 mL of DMF, HATU (70 mg, 0.18 mmol) and triethylamine (30 mg, 0.3 mmol) were then added, and the mixture was kept at room temperature for reaction for 4 h. 20 mL of water was added, and the mixture was extracted with EA twice. The organic phases were combined, washed with saturated brine once, dried over anhydrous Na₂SO₄, filtered, spin-dried, and then subjected to column chromatography to provide 45 mg of a white solid (49%). ¹H NMR (600 MHz, DMSO-*d*₆) δ 10.85 (s, 1H), 9.79 (s, 1H), 8.99 (s, 1H), 8.59 (d, *J* = 8.2 Hz, 1H), 7.99 (s, 1H), 7.50 (dd, *J =* 8.0, 3.0 Hz, 3H), 7.48 - 7.44 (m, 3H), 7.40 (d, *J =* 8.3 Hz, 2H), 7.31 - 7.25 (m, 2H), 7.19 (d, *J =* 8.5 Hz, 2H), 7.17 (d, *J =* 7.5 Hz, 1H), 6.06 (s, 1H), 5.16 (d, *J =* 3.6 Hz, 1H), 4.90 - 4.83 (m, 1H), 4.58 (d, *J =* 9.3 Hz, 1H), 4.50 (t, *J =* 8.3 Hz, 1H), 4.33 (t, *J =* 7.0 Hz, 2H), 4.28 (s, 1H), 3.72 - 3.63 (m, 4H), 3.64 - 3.57 (m, 2H), 3.56 - 3.48 (m, 4H), 2.92 (t, *J =* 7.0 Hz, 2H), 2.46 (s, 3H), 2.45 - 2.41 (m, 1H), 2.40 - 2.35 (m, 1H), 2.34 (s, 3H), 2.11 - 1.93 (m, 3H), 1.78 - 1.72 (m, 1H), 1.42 - 1.30 (m, 2H), 1.22 (dd, *J =* 8.2, 2.1 Hz, 2H), 0.96 (s, 9H). ¹³C NMR (150 MHz, DMSO-*d*₆) δ 170.22, 169.99, 168.22, 167.48, 167.35, 163.75, 163.34, 162.89, 150.91, 147.19, 142.59, 140.57, 137.30, 136.89, 134.14, 132.37, 130.45, 129.31, 129.13, 128.43, 128.25, 127.98, 126.39, 126.27, 122.92, 118.55, 78.27, 76.73, 75.03, 68.18, 65.91, 65.28, 58.16, 56.06, 55.98, 51.35, 43.59, 37.14, 35.42, 33.64, 32.61, 31.47, 25.68, 25.60, 20.31, 15.41, 12.38, 12.32, 12.11, 12.04. HRMS (ESI) calcd for C₅₃H₆₃FN₁₀O₇S [M + H]⁺ 1003.4664, found 1003.4646.

### Example 50: (2S,4R)-1-((S)-2-(1-fluorocyclopropane-1-carboxamido)-3,3-dimethylbutanoyl) -4-hydroxy-N-((S)-4-((2-((4-(2-((4-(2-((E)-3-methylbenzylidene)hydrazino)-6-morpholinopyrimidin-2-yl)oxy)ethyl)phenyl)amino)-2-oxoethyl)amino)-1-(4-(4-methylthiazol-5-yl)phenyl)4-oxobutyl)pyrrolidine-2-carboxamide (LCG-50)

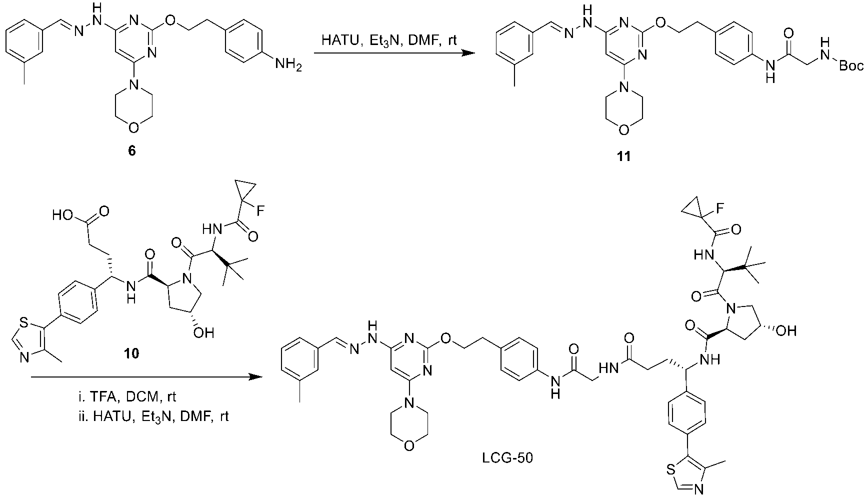

### Step 1: Preparation of tert-butyl (E)-(2-((4-(2-((4-(2-(3-methylbenzylidene)hydrazino)-6-morpholinpyrimidin-2-yl)oxy)ethyl)phenyl)amino)-2-oxoethyl)carbamate (11)

The intermediate 6 (300 mg, 0.7 mmol) and (tert-butoxycarbonyl)glycine (122 mg, 0.7 mmol) were dissolved in 15 mL of DMF, HATU (550 mg, 1.4 mmol) and triethylamine (220 mg, 2.1 mmol) were then added, and the mixture was kept at room temperature for reaction for 4 h. 50 mL of water was added, and the mixture was extracted with EA twice. The organic phases were combined, washed with saturated brine once, dried over anhydrous Na₂SO₄, filtered, spin-dried, and then subjected to column chromatography to provide 288 mg of a solid (70%). ¹H NMR (500 MHz, DMSO-*d*₆) δ 10.84 (s, 1H), 9.84 (s, 1H), 7.96 (s, 1H), 7.51 - 7.43 (m, 4H), 7.27 (t, *J =* 7.6 Hz, 1H), 7.20 (d, *J =* 8.4 Hz, 2H), 7.15 (d, *J =* 7.6 Hz, 1H), 7.01 (t, *J =* 6.0 Hz, 1H), 6.04 (s, 1H), 4.32 (t, *J =* 7.0 Hz, 2H), 3.68 (d, *J =* 6.0 Hz, 2H), 3.66 - 3.61 (m, 4H), 3.53 - 3.48 (m, 4H), 2.91 (t, *J =* 7.0 Hz, 2H), 2.32 (s, 3H), 1.37 (s, 9H). MS (ESI),m/z: 560.4 [M+H]⁺.

### Step 2: Preparation of (25,4R)-1-((S)-2-(1-fluorocyclopropane-1-carboxamido)-3,3-dimethylbutanoyl)-4-hydroxy-N-((S)-4-((2-((4-(2-((4-(2-((E)-3-methylbenzylidene)hydrazino)-6-morpholinopyrimidin-2-yl)oxy)ethyl)phenyl)amino)-2-oxoethyl)amino)-1-(4-(4-methylthiazol-5-yl)phenyl)4-oxobutyl)pyrrolidine-2-carboxamide (LCG-50)

An intermediate 11 (40 mg, 0.07 mmol) was dissolved in 10 mL of DCM containing 25% TFA, the mixture was stirred at room temperature for 3 h, and then the completion of the reaction was monitored by TLC. The reaction mixture was spin-dried to provide an oil, which was used in the reaction in the next step without purification. The oil was dissolved in 10 mL of DMF, the reported intermediate 10 (50 mg, 0.08 mmol) of the VHL ligand of the E3 ubiquitin ligase, HATU (65 mg, 0.14 mmol), and triethylamine (30 mg, 0.3 mmol) were added, and the mixture was kept at room temperature for reaction for 4 h. The completion of the reaction was monitored by TLC, 20 mL of water was added, and the mixture was extracted with EA twice. The organic phases were combined, washed with saturated brine once, dried over anhydrous Na₂SO₄, filtered, spin-dried, and then subjected to column chromatography to provide 30 mg of a white solid (40%). ¹H NMR (600 MHz, DMSO-*d*₆) δ 10.86 (s, 1H), 9.89 (s, 1H), 8.99 (s, 1H), 8.58 (d, *J =* 8.3 Hz, 1H), 8.03 (t, *J =* 5.8 Hz, 1H), 7.99 (s, 1H), 7.54 - 7.48 (m, 3H), 7.48 - 7.44 (m, 3H), 7.39 (d, *J=* 8.3 Hz, 2H), 7.35 (dd, *J =* 9.4, 2.5 Hz, 1H), 7.29 (t, *J =* 7.6 Hz, 1H), 7.22 (d, *J =* 8.5 Hz, 2H), 7.17 (d, *J =* 7.5 Hz, 1H), 6.06 (s, 1H), 5.16 (d, *J =* 3.6 Hz, 1H), 4.84 (dd, *J* = 15.2, 7.7 Hz, 1H), 4.61 (d, *J* = 9.3 Hz, 1H), 4.50 (t, *J =* 8.4 Hz, 1H), 4.34 (t, *J =* 7.1 Hz, 2H), 4.29 (s, 1H), 3.86 (d, *J =* 5.8 Hz, 2H), 3.71 - 3.64 (m, 4H), 3.64 - 3.56 (m, 2H), 3.56 - 3.49 (m, 4H), 2.93 (t, *J =* 7.0 Hz, 2H), 2.47 (s, 3H), 2.37 - 2.31 (m, 4H), 2.28 - 2.22 (m, 1H), 2.10 - 2.04 (m, 1H), 2.00 - 1.92 (m, 2H), 1.79 - 1.72 (m, 1H), 1.41 - 1.31 (m, 2H), 1.26 - 1.18 (m, 2H), 0.97 (s, 9H). ¹³C NMR (150 MHz, DMSO-*d*₆) δ 171.72, 170.20, 168.28, 167.55, 167.41, 167.00, 163.76, 163.34, 162.90, 150.90, 147.20, 142.66, 140.59, 137.31, 136.51, 134.15, 132.67, 130.47, 129.29, 129.14, 128.54, 128.25, 127.99, 126.40, 126.22, 122.93, 118.60, 78.18, 76.64, 75.05, 68.18, 65.88, 65.29, 58.21, 56.12, 55.99, 51.26, 43.60, 42.12, 37.16, 35.43, 33.64, 31.88, 31.55, 25.64, 20.32, 15.42, 12.41, 12.34, 12.12, 12.06. HRMS (ESI) calcd for C₅₅H₆₆FN₁₁O₈S [M + H]⁺ 1060.4879, found 1060.4879.

### Example 51: (2S,4R)-1-((S)-2-(1-fluorocyclopropane-1-carboxamido)-3,3-dimethylbutanoyl) -4-hydroxy-N-((S)-4-((4-((4-(2-((4-(2-((E)-3-methylbenzylidene)hydrazino)-6-morpholinopyrimidin-2-yl)oxy)ethyl)phenyl)amino)-4-oxobutyl)amino)-1-(4-(4-methylthiazol-5-yl)phenyl)4-oxobutyl)pyrrolidine-2-carboxamide (LCG-51)

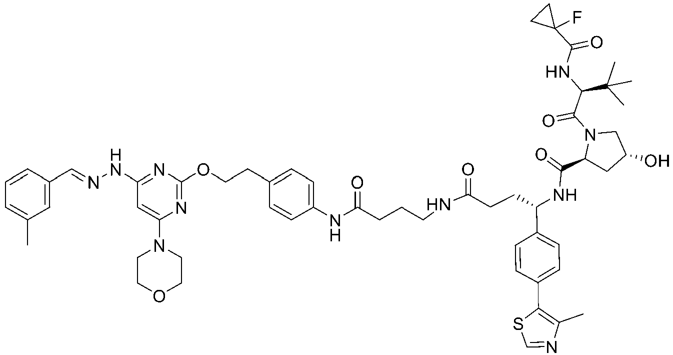

The synthesis method was as shown in Example 50.

¹H NMR (600 MHz, DMSO-*d*₆) δ 10.85 (s, 1H), 9.83 (s, 1H), 8.98 (s, 1H), 8.56 (d, *J =* 8.2 Hz, 1H), 7.99 (s, 1H), 7.73 (t, *J* = 5.6 Hz, 1H), 7.51 - 7.47 (m, 4H), 7.44 (d, *J =* 8.2 Hz, 2H), 7.37 (d, *J* = 8.2 Hz, 2H), 7.31 - 7.27 (m, 2H), 7.20 (d, *J =* 8.5 Hz, 2H), 7.17 (d, *J =* 7.5 Hz, 1H), 6.06 (s, 1H), 5.15 (d, *J =* 3.3 Hz, 1H), 4.82 - 4.76 (m, 1H), 4.59 (d, *J =* 9.3 Hz, 1H), 4.48 (t, *J =* 8.3 Hz, 1H), 4.33 (t, *J =* 7.1 Hz, 2H), 4.28 (s, 1H), 3.68 - 3.65 (m, 4H), 3.64 - 3.56 (m, 2H), 3.55 - 3.51 (m, 4H), 3.12 - 3.05 (m, 2H), 2.92 (t, *J =* 7.0 Hz, 2H), 2.46 (s, 3H), 2.34 (s, 3H), 2.28 (t, *J =* 7.4 Hz, 2H), 2.26 - 2.18 (m, 1H), 2.17 - 2.10 (m, 1H), 2.08 - 2.02 (m, 1H), 1.98 - 1.87 (m, 2H), 1.79 - 1.66 (m, 3H), 1.42 - 1.29 (m, 2H), 1.25 - 1.17 (m, 2H), 0.97 (s, 9H). ¹³C NMR (150 MHz, DMSO-*d*₆) δ 171.09, 170.17, 170.06, 168.23, 167.50, 167.37, 163.76, 163.35, 162.91, 150.90, 147.18, 142.67, 140.59, 137.31, 136.95, 134.15, 132.36, 130.47, 129.27, 129.15, 128.46, 128.23, 128.00, 126.41, 126.24, 122.93, 118.49, 78.27, 76.73, 75.05, 68.18, 65.93, 65.30, 58.17, 56.08, 55.97, 54.31, 51.35, 43.60, 37.52, 37.16, 35.42, 33.66, 33.24, 31.81, 31.75, 25.68, 25.61, 24.71, 20.33, 15.42, 12.40, 12.33, 12.12, 12.06. HRMS (ESI) calcd for C₅₇H₇₀FN₁₁O₈S [M+H]⁺ 1088.5192, found 1088.5190.

### Example 52: (2S,4R)-1-((S)-2-(1-fluorocyclopropane-1-carboxamido)-3,3-dimethylbutanoyl) -4-hydroxy-N-((S)-4-((6-((4-(2-((4-(2-((E)-3-methylbenzylidene)hydrazino)-6-morpholinopyrimidin-2-yl)oxy)ethyl)phenyl)amino)-6-oxohexyl)amino)-1-(4-(4-methylthiazol-5-yl)phenyl)4-oxobutyl)pyrrolidine-2-carboxamide (LCG-52)

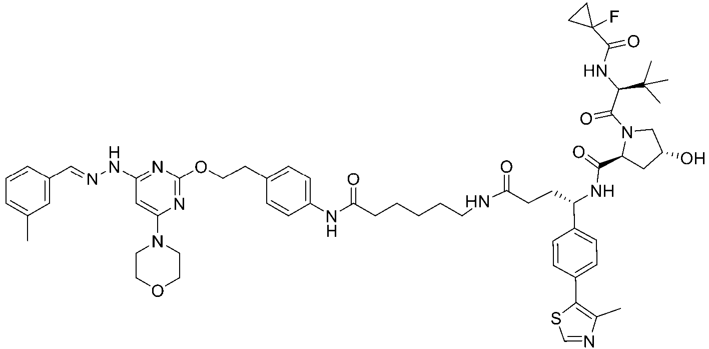

The synthesis method was as shown in Example 50.

¹H NMR (600 MHz, DMSO-*d*₆) δ 10.85 (s, 1H), 9.79 (s, 1H), 8.98 (s, 1H), 8.55 (d, *J =* 8.2 Hz, 1H), 7.99 (s, 1H), 7.65 (t, *J* = 5.6 Hz, 1H), 7.53 - 7.46 (m, 4H), 7.44 (d, *J =* 8.2 Hz, 2H), 7.36 (d, *J* = 8.2 Hz, 2H), 7.31 - 7.25 (m, 2H), 7.19 (d, *J =* 8.5 Hz, 2H), 7.17 (d, *J =* 7.5 Hz, 1H), 6.06 (s, 1H), 5.15 (s, 1H), 4.82 - 4.75 (m, 1H), 4.59 (d, *J =* 9.2 Hz, 1H), 4.48 (t, *J* = 8.3 Hz, 1H), 4.33 (t, *J =* 7.1 Hz, 2H), 4.28 (s, 1H), 3.72 - 3.64 (m, 4H), 3.63 - 3.56 (m, 2H), 3.56 - 3.49 (m, 4H), 3.07 - 2.99 (m, 2H), 2.92 (t, *J =* 7.0 Hz, 2H), 2.46 (s, 3H), 2.34 (s, 3H), 2.27 (t, *J =* 7.4 Hz, 2H), 2.23 - 2.16 (m, 1H), 2.14 - 2.08 (m, 1H), 2.05 (dd, *J* = 12.7, 7.8 Hz, 1H), 1.98 - 1.87 (m, 2H), 1.78 - 1.69 (m, 1H), 1.61 - 1.53 (m, 2H), 1.44 - 1.32 (m, 4H), 1.32 - 1.25 (m, 2H), 1.25 - 1.18 (m, 2H), 0.97 (s, 9H). ¹³C NMR (150 MHz, DMSO-*d*₆) δ 170.90, 170.38, 170.13, 168.22, 167.49, 167.36, 163.76, 163.34, 162.90, 150.90, 147.18, 142.69, 140.60, 137.31, 136.99, 134.15, 132.33, 130.47, 129.26, 129.15, 128.46, 128.23, 128.00, 126.41, 126.22, 122.94, 118.50, 78.28, 76.74, 75.05, 68.18, 65.93, 65.30, 58.16, 56.09, 55.96, 51.35, 43.60, 37.81, 37.15, 35.70, 35.44, 33.66, 31.86, 31.75, 28.41, 25.61, 25.53, 24.28, 20.33, 15.42, 12.40, 12.33, 12.12, 12.06. HRMS (ESI) calcd for C₅₉H₇₄FN₁₁O₈S [M + H]⁺ 1116.5505, found 1116.5507.

### Example 53: (2S,4R)-1-((S)-2-(1-fluorocyclopropane-1-carboxamido)-3,3-dimethylbutanoyl) -4-hydroxy-N-(2-(2-((4-(2-((4-(2-((E)-3-methylbenzylidene)hydrazino)-6-morpholinopyrimidin-2-yl)oxy)ethyl)phenyl)amino)-2-oxoethoxy)-4-(4-methylthiazol-5-yl)benzyl)pyrrolidine-2-carboxamide (LCG-53)

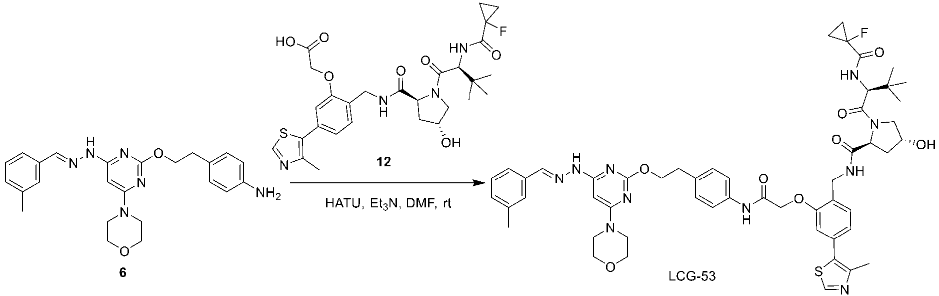

The intermediate 6 (40 mg, 0.09 mmol) and a reported intermediate 12 (53 mg, 0.09 mmol) of the VHL ligand of the E3 ubiquitin ligase were dissolved in 10 mL of DMF, HATU (70 mg, 0.18 mmol) and triethylamine (30 mg, 0.3 mmol) were then added, and the mixture was kept at room temperature for reaction for 4 h. 20 mL of water was added, and the mixture was extracted with EA twice. The organic phases were combined, washed with saturated brine once, dried over anhydrous Na₂SO₄, filtered, spin-dried, and then subjected to column chromatography to provide 35 mg of a white solid (39%). ¹H NMR (600 MHz, DMSO-*d*₆) δ 10.85 (s, 1H), 10.03 (s, 1H), 8.98 (s, 1H), 8.58 (t, *J =* 6.0 Hz, 1H), 7.99 (s, 1H), 7.61 (d, *J =* 8.5 Hz, 2H), 7.50 (d, *J =* 7.8 Hz, 1H), 7.47 (s, 1H), 7.45 (d, *J =* 8.1 Hz, 1H), 7.31 - 7.26 (m, 2H), 7.25 (d, *J =* 8.5 Hz, 2H), 7.17 (d, *J =* 7.5 Hz, 1H), 7.03 (dd, *J* = 3.9, 2.5 Hz, 2H), 6.06 (s, 1H), 5.16 (brs, 1H), 4.83 - 4.76 (m, 2H), 4.58 (d, *J* = 9.4 Hz, 1H), 4.50 (t, *J =* 8.2 Hz, 1H), 4.48 - 4.30 (m, 5H), 3.69 - 3.64 (m, 4H), 3.64 - 3.56 (m, 2H), 3.56 - 3.51 (m, 4H), 2.95 (t, *J =* 6.9 Hz, 2H), 2.42 (s, 3H), 2.34 (s, 3H), 2.10 - 2.03 (m, 1H), 1.94 - 1.88 (m, 1H), 1.41 - 1.30 (m, 2H), 1.21 (dd, *J =* 8.4, 2.7 Hz, 2H), 0.94 (s, 9H). ¹³C NMR (150 MHz, DMSO-*d*₆) δ 171.16, 168.27, 167.49, 167.36, 165.63, 163.73, 163.30, 162.86, 154.60, 151.01, 147.35, 140.60, 137.30, 135.90, 134.13, 133.28, 130.55, 130.45, 129.14, 128.52, 128.28, 127.98, 126.55, 126.41, 122.93, 120.94, 119.34, 111.58, 78.25, 76.71, 75.04, 68.25, 66.82, 65.86, 65.28, 58.27, 56.04, 55.92, 43.60, 37.25, 36.80, 35.38, 33.65, 25.58, 25.52, 20.32, 15.37, 12.38, 12.32, 12.12, 12.05. HRMS (ESI) calcd for C₅₂H₆₁FN₁₀O₈S [M + Na]⁺ 1027.4277, found 1027.4269.

### Example 54: (2S,4R)-1-((S)-2-(1-fluorocyclopropane-1-carboxamido)-3,3-dimethylbutanoyl) -4-hydroxy-N-(2-(2-((2-((4-(2-((4-(2-((E)-3-methylbenzylidene)hydrazino)-6-morpholinopyrimidin-2-yl)oxy)ethyl)phenyl)amino)-2-oxoethyl)amino)-2-oxoethoxy)-4-(4-methylthiazol-5-yl)benzyl)pyrrolidine-2-carboxamide (LCG-54)

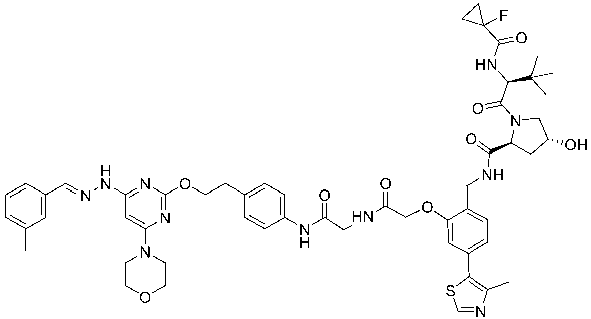

The synthesis method was as shown in Example 53.

¹H NMR (600 MHz, DMSO-*d*₆) δ 10.86 (s, 1H), 9.95 (s, 1H), 8.99 (s, 1H), 8.58 (t, *J =* 6.0 Hz, 1H), 8.39 (t, *J =* 5.9 Hz, 1H), 7.99 (s, 1H), 7.49 (t, *J =* 9.8 Hz, 4H), 7.45 (d, *J =* 7.7 Hz, 1H), 7.32 - 7.26 (m, 2H), 7.23 (d, *J =* 8.5 Hz, 2H), 7.17 (d, *J =* 7.5 Hz, 1H), 7.05 - 7.00 (m, 2H), 6.06 (s, 1H), 5.15 (brs, 1H), 4.68 (dd, *J =* 28.1, 14.9 Hz, 2H), 4.58 (d, *J =* 9.3 Hz, 1H), 4.50 (t, *J=* 8.3 Hz, 1H), 4.45 (dd, *J =* 16.0, 6.0 Hz, 1H), 4.39 - 4.31 (m, 4H), 4.03 - 3.92 (m, 2H), 3.68 - 3.65 (m, 4H), 3.65 - 3.57 (m, 2H), 3.56 - 3.50 (m, 4H), 2.94 (t, *J =* 7.0 Hz, 2H), 2.46 (s, 3H), 2.34 (s, 3H), 2.10 - 2.04 (m, 1H), 1.97 - 1.90 (m, 1H), 1.40 - 1.30 (m, 2H), 1.21 (dd, *J* = 8.4, 3.1 Hz, 2H), 0.95 (s, 9H). ¹³C NMR (150 MHz, DMSO-*d*₆) δ 171.25, 168.26, 167.50, 167.42, 167.36, 166.47, 163.73, 163.30, 162.88, 154.39, 150.94, 147.42, 140.63, 137.30, 136.47, 134.13, 132.70, 130.55, 130.46, 129.15, 128.57, 127.98, 126.53, 126.41, 122.93, 120.98, 118.58, 111.69, 78.25, 76.71, 75.03, 68.32, 66.54, 65.89, 65.28, 58.23, 56.09, 55.92, 43.60, 41.70, 37.21, 36.85, 35.42, 33.63, 25.53, 20.31, 15.37, 12.39, 12.32, 12.12, 12.05. HRMS (ESI) calcd for C₅₄H₆₄FN₁₁O₉S [M + Na]⁺ 1084.4491, found 1084.4491.

### Example 55: (2S,4R)-1-((S)-2-(1-fluorocyclopropane-1-carboxamido)-3,3-dimethylbutanoyl) -4-hydroxy-N-(2-(2-((4-((4-(2-((4-(2-((E)-3-methylbenzylidene)hydrazino)-6-morpholinopyrimidin-2-yl)oxy)ethyl)phenyl)amino)-4-oxobutyl)amino)-2-oxoethoxy)-4-(4-methylthiazol-5-yl)benzyl)pyrrolidine-2-carboxamide (LCG-55)

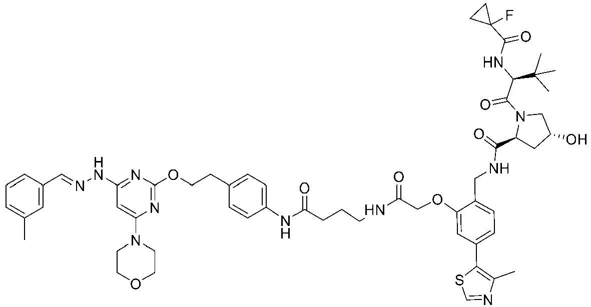

The synthesis method was as shown in Example 53.

¹H NMR (600 MHz, DMSO-*d*₆) δ 10.86 (s, 1H), 9.84 (s, 1H), 8.97 (s, 1H), 8.56 (t, *J =* 6.0 Hz, 1H), 8.18 (t, *J =* 5.8 Hz, 1H), 7.99 (s, 1H), 7.53 - 7.46 (m, 4H), 7.43 (d, *J =* 7.8 Hz, 1H), 7.32 - 7.25 (m, 2H), 7.21 - 7.16 (m, 3H), 7.02 (dd, *J =* 7.7, 1.3 Hz, 1H), 6.96 (d, *J =* 1.2 Hz, 1H), 6.06 (s, 1H), 5.16 (brs, 1H), 4.61 - 4.52 (m, 3H), 4.52 - 4.42 (m, 2H), 4.37 - 4.29 (m, 4H), 3.68 - 3.65 (m, 4H), 3.65 - 3.57 (m, 2H), 3.55 - 3.51 (m, 4H), 3.21 (dd, *J =* 13.2, 6.7 Hz, 2H), 2.92 (t, *J =* 7.0 Hz, 2H), 2.45 (s, 3H), 2.34 (s, 3H), 2.31 (t, *J* = 7.5 Hz, 2H), 2.09 - 2.02 (m, 1H), 1.92 - 1.86 (m, 1H), 1.82 - 1.75 (m, 2H), 1.41 - 1.30 (m, 2H), 1.25 - 1.19 (m, 2H), 0.95 (s, 9H). ¹³C NMR (150 MHz, DMSO-*d*₆) δ 171.13, 170.01, 168.26, 167.50, 167.36, 166.78, 163.70, 163.26, 162.84, 154.47, 150.95, 147.36, 140.66, 137.30, 136.92, 134.12, 132.32, 130.58, 130.46, 129.16, 128.45, 128.29, 127.98, 126.51, 126.42, 122.94, 120.89, 118.50, 111.54, 78.26, 76.72, 75.01, 68.26, 66.58, 65.95, 65.28, 58.24, 56.09, 55.92, 43.60, 37.45, 37.23, 36.83, 35.39, 33.63, 33.23, 25.58, 25.50, 24.62, 20.31, 15.35, 12.39, 12.32, 12.12, 12.05. HRMS (ESI) calcd for C₅₆H₆₈FN₁₁O₉S [M + H]⁺ 1090.4984, found 1090.4983.

### Example 56: (2S,4R)-1-((S)-2-(1-fluorocyclopropane-1-carboxamido)-3,3-dimethylbutanoyl) -4-hydroxy-N-(2-(2-((6-((4-(2-((4-(2-((E)-3-methylbenzylidene)hydrazino)-6-morpholinopyrimidin-2-yl)oxy)ethyl)phenyl)amino)-6-oxohexyl)amino)-2-oxoethoxy)-4-(4-methylthiazol-5-yl)benzyl)pyrrolidine-2-carboxamide (LCG-56)

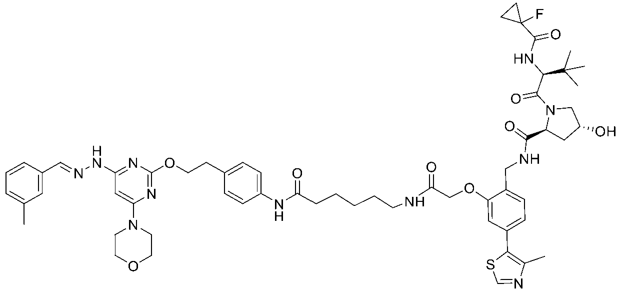

The synthesis method was as shown in Example 53.

¹H NMR (600 MHz, DMSO-*d*₆) δ 10.86 (s, 1H), 9.79 (s, 1H), 8.98 (s, 1H), 8.55 (t, *J =* 6.0 Hz, 1H), 8.12 (t, *J =* 5.8 Hz, 1H), 7.99 (s, 1H), 7.54 - 7.46 (m, 4H), 7.42 (d, *J =* 7.8 Hz, 1H), 7.32 - 7.25 (m, 2H), 7.22 - 7.15 (m, 3H), 7.02 (dd, *J =* 7.7, 1.4 Hz, 1H), 6.95 (d, *J =* 1.3 Hz, 1H), 6.06 (s, 1H), 5.17 (brs, 1H), 4.62 - 4.51 (m, 3H), 4.49 (t, *J =* 8.2 Hz, 1H), 4.45 (dd, *J =* 15.7, 6.3 Hz, 1H), 4.37 - 4.28 (m, 4H), 3.70 - 3.65 (m, 4H), 3.65 - 3.58 (m, 2H), 3.56 - 3.49 (m, 4H), 3.17 - 3.12 (m, 2H), 2.92 (t, *J =* 7.0 Hz, 2H), 2.45 (s, 3H), 2.34 (s, 3H), 2.26 (t, *J =* 7.4 Hz, 2H), 2.09 - 2.00 (m, 1H), 1.92 - 1.82 (m, 1H), 1.61 - 1.53 (m, 2H), 1.51 - 1.44 (m, 2H), 1.42 - 1.33 (m, 2H), 1.32 - 1.25 (m, 2H), 1.25 - 1.19 (m, 2H), 0.95 (s, 9H). ¹³C NMR (150 MHz, DMSO-*d*₆) δ 171.09, 170.34, 168.26, 167.49, 167.35, 166.59, 163.70, 163.29, 162.87, 154.49, 150.96, 147.35, 140.62, 137.29, 136.97, 134.12, 132.30, 130.58, 130.45, 129.14, 128.44, 128.30, 127.98, 126.48, 126.41, 122.93, 120.85, 118.49, 111.54, 78.26, 76.72, 75.02, 68.25, 66.59, 65.94, 65.28, 58.23, 56.08, 55.91, 43.59, 37.66, 37.22, 36.79, 35.63, 35.40, 33.63, 28.36, 25.57, 25.50, 25.44, 24.24, 20.31, 15.36, 12.38, 12.31, 12.12, 12.05. HRMS (ESI) calcd for C₅₈H₇₂FN₁₁O₉S [M + Na]⁺ 1140.5117, found 1140.5113.

### Example 57: (2S,4R)-1-((R)-2-(1-fluorocyclopropane-1-carboxamido)-3-methyl-3-((2-((4-(2-((4-(2-((E)-3-methylbenzylidene)hydrazino)-6-morpholinopyrimidin-2-yl)oxy)ethyl)phenyl) amino)-2-oxoethyl)thio)butanoyl)-4-hydroxy-N-(4-(4-methylthiazol-5-yl)benzyl)pyrrolidine-2-carboxamide (LCG-57)

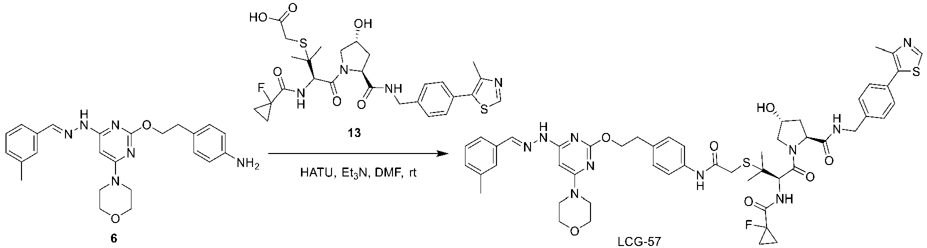

The intermediate 6 (40 mg, 0.09 mmol) and a reported intermediate 13 (54 mg, 0.09 mmol) of the VHL ligand of the E3 ubiquitin ligase were dissolved in 10 mL of DMF, HATU (70 mg, 0.18 mmol) and triethylamine (30 mg, 0.3 mmol) were then added, and the mixture was kept at room temperature for reaction for 4 h. 20 mL of water was added, and the mixture was extracted with EA twice. The organic phases were combined, washed with saturated brine once, dried over anhydrous Na₂SO₄, filtered, spin-dried, and then subjected to column chromatography to provide 38 mg of a white solid (42%). ¹H NMR (600 MHz, DMSO-*d*₆) δ 10.86 (s, 1H), 9.95 (s, 1H), 8.96 (s, 1H), 8.74 (t, *J =* 6.0 Hz, 1H), 7.99 (s, 1H), 7.56 (dd, *J =* 8.9, 2.3 Hz, 1H), 7.51 - 7.44 (m, 4H), 7.40 - 7.35 (m, 4H), 7.29 (t, *J =* 7.6 Hz, 1H), 7.21 - 7.15 (m, 3H), 6.05 (s, 1H), 5.22 (brs, 1H), 4.85 (d, *J* = 8.9 Hz, 1H), 4.50 (t, *J =* 8.3 Hz, 1H), 4.41 (dd, *J =* 15.7, 6.4 Hz, 1H), 4.37 (s, 1H), 4.33 (t, *J =* 7.0 Hz, 2H), 4.26 (dd, *J =* 15.7, 5.6 Hz, 1H), 3.77 - 3.68 (m, 2H), 3.68 - 3.64 (m, 4H), 3.55 - 3.50 (m, 4H), 3.40 - 3.38 (m, 2H), 2.92 (t, *J =* 6.9 Hz, 2H), 2.42 (s, 3H), 2.34 (s, 3H), 2.14 - 2.08 (m, 1H), 1.97 - 1.89 (m, 1H), 1.41 (d, *J =* 3.1 Hz, 6H), 1.39 - 1.34 (m, 2H), 1.24 - 1.19 (m, 2H). ¹³C NMR (150 MHz, DMSO-*d*₆) δ 171.24, 167.76, 167.62, 167.28, 167.22, 163.70, 163.26, 162.84, 150.81, 147.14, 140.63, 138.51, 137.30, 136.61, 134.12, 132.78, 130.47, 129.19, 129.15, 128.54, 128.24, 128.15, 127.98, 127.43, 126.81, 126.41, 122.94, 118.46, 78.21, 76.68, 75.02, 68.21, 65.90, 65.28, 58.49, 56.00, 54.38, 49.28, 45.13, 43.60, 41.18, 37.40, 33.63, 33.13, 26.00, 23.68, 20.31, 15.29, 12.47, 12.40, 12.32, 12.25. HRMS (ESI) calcd for C₅₁H₅₉FN₁₀O₇S₂ [M + Na]⁺ 1029.3892, found 1029.3884.

### Example 58: (2S,4R)-1-((R)-2-(1-fluorocyclopropane-1-carboxamido)-3-methyl-3-((2-((2-((4-(2-((4-(2-((E)-3-methylbenzylidene)hydrazino)-6-morpholinopyrimidin-2-yl)oxy)ethyl) phenyl)amino)-2-oxoethyl)amino)-2-oxoethyl)thio)butanoyl)-4-hydroxy-N-(4-(4-methylthiazol-5-yl)benzyl)pyrrolidine-2-carboxamide (LCG-58)

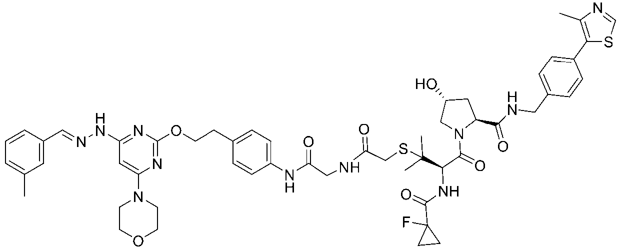

The synthesis method was as shown in Example 57.

¹H NMR (600 MHz, DMSO-*d*₆) δ 10.86 (s, 1H), 9.91 (s, 1H), 8.97 (s, 1H), 8.65 (t, *J =* 6.0 Hz, 1H), 8.25 (t, *J =* 5.6 Hz, 1H), 7.99 (s, 1H), 7.54 (dd, *J* = 9.0, 2.1 Hz, 1H), 7.51 - 7.46 (m, 4H), 7.42 - 7.37 (m, 4H), 7.29 (t, *J =* 7.6 Hz, 1H), 7.22 (d, *J =* 8.5 Hz, 2H), 7.17 (d, *J =* 7.5 Hz, 1H), 6.06 (s, 1H), 5.20 (brs, 1H), 4.84 (d, *J =* 9.0 Hz, 1H), 4.47 (t, *J=* 8.2 Hz, 1H), 4.42 - 4.26 (m, 5H), 3.89 (t, *J* = 7.2 Hz, 2H), 3.76 (dd, *J =* 10.7, 3.9 Hz, 1H), 3.69 - 3.62 (m, 5H), 3.55 - 3.49 (m, 4H), 3.40 - 3.34 (m, 2H), 2.93 (t, *J =* 7.0 Hz, 2H), 2.44 (s, 3H), 2.34 (s, 3H), 2.12 - 2.05 (m, 1H), 1.95 - 1.87 (m, 1H), 1.43 (s, 3H), 1.40 - 1.33 (m, 5H), 1.28 - 1.19 (m, 2H). ¹³C NMR (150 MHz, DMSO-*d*₆) δ 171.02, 168.80, 167.77, 167.64, 167.12, 166.60, 163.74, 163.32, 162.88, 150.82, 147.15, 140.59, 138.67, 137.30, 136.43, 134.13, 132.70, 130.50, 129.16, 128.56, 128.27, 128.13, 127.98, 126.92, 126.40, 122.92, 118.56, 78.18, 76.64, 75.03, 68.22, 65.87, 65.28, 58.43, 55.93, 49.08, 43.59, 42.29, 41.10, 37.37, 33.63, 31.72, 25.49, 23.55, 20.31, 15.32, 12.49, 12.42, 12.32, 12.25. HRMS (ESI) calcd for C₅₃H₆₂FN₁₁O₈S₂ [M + H]⁺ 1064.4286, found 1064.4274.

### Example 59: (2S,4R)-1-((R)-2-(1-fluorocyclopropane-1-carboxamido)-3-methyl-3-((2-((4-((4-(2-((4-(2-((E)-3-methylbenzylidene)hydrazino)-6-morpholinopyrimidin-2-yl)oxy)ethyl) phenyl)amino)-4-oxobutyl)amino)-2-oxoethyl)thio)butanoyl)-4-hydroxy-N-(4-(4-methylthiazol-5-yl)benzyl)pyrrolidine-2-carboxamide (LCG-59)

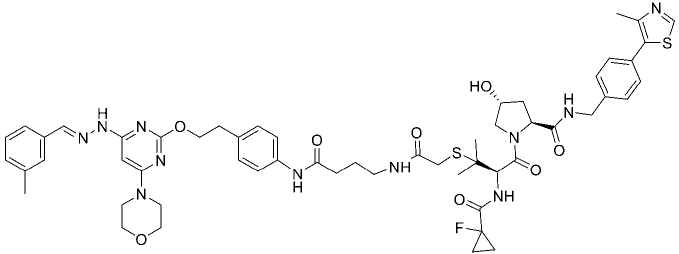

The synthesis method was as shown in Example 57.

¹H NMR (600 MHz, DMSO-*d*₆) δ 10.86 (s, 1H), 9.84 (s, 1H), 8.97 (d, *J =* 2.4 Hz, 1H), 8.68 (t, *J =* 6.0 Hz, 1H), 7.99 (s, 1H), 7.97 (d, *J =* 5.6 Hz, 1H), 7.53 (dd, *J* = 9.0, 2.2 Hz, 1H), 7.52 - 7.46 (m, 4H), 7.43 - 7.36 (m, 4H), 7.29 (t, *J =* 7.6 Hz, 1H), 7.20 (d, *J* = 8.5 Hz, 2H), 7.17 (d, *J =* 7.6 Hz, 1H), 6.06 (s, 1H), 5.20 (s, 1H), 4.82 (d, *J =* 9.0 Hz, 1H), 4.47 (t, *J =* 8.2 Hz, 1H), 4.43 - 4.35 (m, 2H), 4.33 (t, *J =* 7.0 Hz, 2H), 4.27 (dd, *J =* 15.7, 5.7 Hz, 1H), 3.74 (dd, *J =* 10.7, 3.9 Hz, 1H), 3.69 - 3.61 (m, 5H), 3.55 - 3.50 (m, 4H), 3.23 - 3.18 (m, 2H), 3.07 - 3.03 (m, 2H), 2.92 (t, *J =* 7.0 Hz, 2H), 2.43 (s, 3H), 2.34 (s, 3H), 2.27 (t, *J =* 7.5 Hz, 2H), 2.12 - 2.06 (m, 1H), 1.96 - 1.86 (m, 1H), 1.73 - 1.62 (m, 2H), 1.42 - 1.31 (m, 8H), 1.24 - 1.20 (m, 2H). ¹³C NMR (150 MHz, DMSO-*d*₆) δ 171.11, 169.97, 168.36, 167.73, 167.60, 167.14, 163.70, 163.28, 162.82, 150.82, 147.15, 140.63, 138.60, 137.30, 136.91, 134.12, 132.34, 130.50, 129.15, 128.45, 128.14, 127.98, 126.84, 126.41, 122.93, 118.50, 78.18, 76.64, 75.02, 68.20, 65.94, 65.28, 58.43, 55.92, 54.31, 49.01, 43.60, 41.12, 37.87, 37.37, 33.63, 33.11, 31.98, 25.60, 24.45, 23.62, 20.32, 15.31, 12.46, 12.39, 12.32, 12.25. HRMS (ESI) calcd for C₅₅H₆₆FN₁₁O₈S₂ [M + H]⁺ 1114.4419, found 1114.4379.

### Example 60: (2S,4R)-1-((R)-2-(1-fluorocyclopropane-1-carboxamido)-3-methyl-3-((2-((6-((4-(2-((4-(2-((E)-3-methylbenzylidene)hydrazino)-6-morpholinopyrimidin-2-yl)oxy)ethyl) phenyl)amino)-6-oxohexyl)amino)-2-oxoethyl)thio)butanoyl)-4-hydroxy-N-(4-(4-methylthiazol-5-yl)benzyl)pyrrolidine-2-carboxamide (LCG-60)

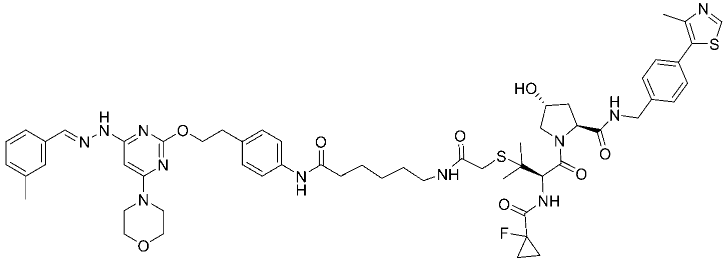

The synthesis method was as shown in Example 57.

¹H NMR (600 MHz, DMSO-*d*₆) δ 10.85 (s, 1H), 9.79 (s, 1H), 8.97 (s, 1H), 8.68 (t, *J=* 6.0 Hz, 1H), 7.99 (s, 1H), 7.88 (t, *J =* 5.6 Hz, 1H), 7.53 (dd, *J* = 9.0, 2.3 Hz, 1H), 7.52 - 7.46 (m, 4H), 7.42 - 7.36 (m, 4H), 7.29 (t, *J=* 7.6 Hz, 1H), 7.19 (d, *J =* 8.5 Hz, 2H), 7.17 (d, *J =* 7.5 Hz, 1H), 6.06 (s, 1H), 5.20 (s, 1H), 4.81 (d, *J* = 9.0 Hz, 1H), 4.47 (t, *J =* 8.2 Hz, 1H), 4.40 (dd, *J* = 15.7, 6.3 Hz, 1H), 4.37 (s, 1H), 4.33 (t, *J =* 7.1 Hz, 2H), 4.26 (dd, *J =* 15.8, 5.7 Hz, 1H), 3.73 (dd, *J =* 10.7, 4.0 Hz, 1H), 3.69 - 3.61 (m, 5H), 3.56 - 3.50 (m, 4H), 3.20 - 3.15 (m, 2H), 3.04 - 2.96 (m, 2H), 2.92 (t, *J* = 7.0 Hz, 2H), 2.44 (s, 3H), 2.34 (s, 3H), 2.25 (t, *J =* 7.5 Hz, 2H), 2.13 - 2.06 (m, 1H), 1.95 - 1.87 (m, 1H), 1.58 - 1.51 (m, 2H), 1.42 - 1.31 (m, 10H), 1.28 - 1.20 (m, 4H). ¹³C NMR (150 MHz, DMSO-*d*₆) δ 171.13, 170.34, 168.16, 167.73, 167.59, 167.16, 163.74, 163.32, 162.88, 150.82, 147.16, 140.60, 138.59, 137.30, 136.96, 134.13, 132.32, 130.49, 129.16, 128.44, 128.25, 128.15, 127.99, 127.37, 126.82, 126.40, 122.92, 118.50, 78.18, 76.64, 75.03, 68.20, 65.92, 65.28, 58.44, 55.92, 54.33, 48.99, 43.59, 41.11, 38.11, 37.38, 35.65, 33.64, 31.98, 28.12, 25.64, 25.45, 24.21, 23.66, 20.31, 15.31, 12.46, 12.39, 12.31, 12.25. HRMS (ESI) calcd for C₅₇H₇₀FN₁₁O₈S₂ [M + H]⁺ 1142.4732, found 1142.4719.

### Example 61: N¹-((S)-1-((2S,4R)-4-hydroxy-2-((4-(4-methylthiazol-5-yl)benzyl)carbamoyl) pyrrolidine-1-yl)-3,3-dimethyl-1-oxobutan-2-yl)-N⁴-(2-((4-(2-((E)-3-methylbenzylidene) hydrazino)-6-morpholinopyrimidin-2-yl)oxy)ethyl)succinimide (LCG-61)

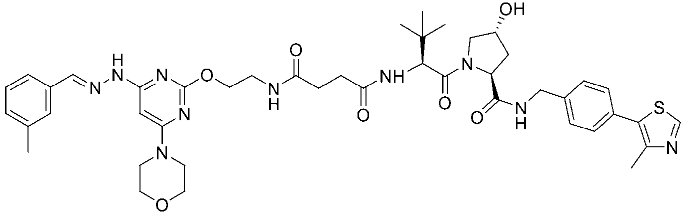

The synthesis method was as shown in Examples 1 and 37.

¹H NMR (600 MHz, DMSO-*d*₆) δ 10.84 (s, 1H), 8.98 (s, 1H), 8.57 (t, *J =* 6.0 Hz, 1H), 8.07 (t, *J=* 5.5 Hz, 1H), 8.02 (s, 1H), 7.91 (d, *J* = 9.3 Hz, 1H), 7.50 (d, *J =* 7.8 Hz, 1H), 7.48 (s, 1H), 7.42 (d, *J =* 8.4 Hz, 2H), 7.38 (d, *J =* 8.3 Hz, 2H), 7.29 (t, *J* = 7.6 Hz, 1H), 7.17 (d, *J* = 7.5 Hz, 1H), 6.07 (s, 1H), 5.13 (brs, 1H), 4.52 (d, *J =* 9.4 Hz, 1H), 4.45 - 4.39 (m, 2H), 4.35 (s, 1H), 4.22 (dd, *J =* 15.9, 5.5 Hz, 1H), 4.17 (t, *J =* 5.8 Hz, 2H), 3.69 - 3.60 (m, 6H), 3.57 - 3.50 (m, 4H), 3.41 - 3.35 (m, 3H), 2.44 (s, 3H), 2.40 - 2.28 (m, 6H), 2.07 - 2.01 (m, 1H), 1.93 - 1.86 (m, 1H), 0.93 (s, 9H). ¹³C NMR (150 MHz, DMSO-*d*₆) δ 171.31, 171.04, 170.64, 168.96, 163.71, 163.31, 162.82, 150.83, 147.09, 140.72, 138.88, 137.30, 134.13, 130.54, 129.16, 129.01, 128.02, 127.99, 126.80, 126.42, 122.94, 75.11, 68.26, 65.30, 63.93, 58.08, 55.80, 55.69, 43.60, 41.02, 37.60, 37.31, 34.71, 30.24, 29.87, 25.73, 20.31, 15.32. HRMS (ESI) calcd for C₄₄H₅₆N₁₀O₇S [M + Na]⁺ 891.3952, found 891.3949.

### Example 62: N¹-((S)-1-((2S,4R)-4-hydroxy-2-((4-(4-methylthiazol-5-yl)benzyl)carbamoyl) pyrrolidine-1-yl)-3,3-dimethyl-1-oxobutan-2-yl)-N⁶-(2-((4-(2-((E)-3-methylbenzylidene) hydrazino)-6-morpholinopyrimidin-2-yl)oxy)ethyl)adipamide (LCG-62)

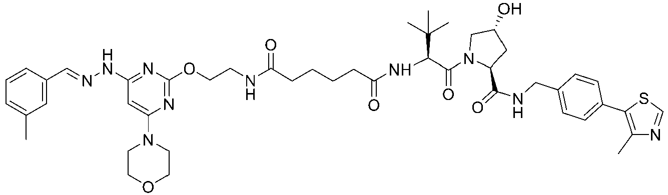

The synthesis method was as shown in Examples 1 and 37.

¹H NMR (600 MHz, DMSO-*d*₆) δ 10.85 (s, 1H), 8.98 (s, 1H), 8.57 (t, *J =* 6.1 Hz, 1H), 8.04 - 7.98 (m, 2H), 7.88 (d, *J =* 9.3 Hz, 1H), 7.51 (d, *J =* 7.8 Hz, 1H), 7.48 (s, 1H), 7.41 (d, *J =* 8.3 Hz, 2H), 7.38 (d, *J =* 8.3 Hz, 2H), 7.29 (t, *J =* 7.6 Hz, 1H), 7.17 (d, *J =* 7.5 Hz, 1H), 6.07 (s, 1H), 5.14 (brs, 1H), 4.54 (d, *J =* 9.4 Hz, 1H), 4.46 - 4.39 (m, 2H), 4.35 (s, 1H), 4.24 - 4.13 (m, 3H), 3.70 - 3.63 (m, 6H), 3.57 - 3.51 (m, 4H), 3.44 - 3.32 (m, 2H), 2.44 (s, 3H), 2.34 (s, 3H), 2.29 - 2.23 (m, 1H), 2.13 - 2.01 (m, 4H), 1.94 - 1.87 (m, 1H), 1.45 (dt, *J =* 11.7, 5.3 Hz, 4H), 0.93 (s, 9H). ¹³C NMR (150 MHz, DMSO-*d*₆) δ 171.67, 171.33, 169.13, 163.66, 163.23, 162.76, 150.83, 147.09, 140.79, 138.87, 137.29, 134.11, 130.54, 129.17, 129.01, 128.01, 127.98, 126.79, 126.44, 122.96, 75.09, 68.25, 65.30, 64.03, 58.08, 55.75, 55.71, 43.61, 41.02, 37.46, 37.32, 34.57, 34.45, 34.02, 25.76, 24.53, 24.30, 20.31, 15.32. HRMS (ESI) calcd for C₄₆H₆₀N₁₀O₇S [M + Na]⁺ 919.4265, found 919.4255.

### Example 63: N¹-((S)-1-((2S,4R)-4-hydroxy-2-((4-(4-methylthiazol-5-yl)benzyl)carbamoyl) pyrrolidine-1-yl)-3,3-dimethyl-1-oxobutan-2-yl)-N⁸-(2-((4-(2-((E)-3-methylbenzylidene) hydrazino)-6-morpholinopyrimidin-2-yl)oxy)ethyl)octanediamide (LCG-63)

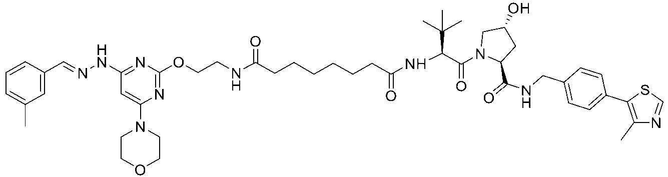

The synthesis method was as shown in Examples 1 and 37.

¹H NMR (600 MHz, DMSO-*d*₆) δ 10.82 (s, 1H), 8.98 (s, 1H), 8.56 (t, *J =* 6.1 Hz, 1H), 8.01 (s, 1H), 7.99 (t, *J* = 5.5 Hz, 1H), 7.85 (d, *J =* 9.4 Hz, 1H), 7.53 - 7.45 (m, 2H), 7.42 (d, *J =* 8.3 Hz, 2H), 7.39 - 7.36 (m, 2H), 7.29 (t, *J =* 7.6 Hz, 1H), 7.17 (d, *J =* 7.5 Hz, 1H), 6.07 (s, 1H), 5.12 (brs, 1H), 4.54 (d, *J =* 9.4 Hz, 1H), 4.46 - 4.39 (m, 2H), 4.35 (s, 1H), 4.22 (dd, *J =* 15.9, 5.5 Hz, 1H), 4.18 (t, *J =* 5.7 Hz, 2H), 3.70 - 3.62 (m, 6H), 3.56 - 3.49 (m, 4H), 3.38 - 3.35 (m, 2H), 2.44 (s, 3H), 2.34 (s, 3H), 2.27 - 2.20 (m, 1H), 2.13 - 2.08 (m, 1H), 2.08 - 2.05 (m, 2H), 2.05 - 2.00 (m, 1H), 1.94 - 1.86 (m, 1H), 1.51 - 1.41 (m, 4H), 1.25 - 1.20 (m, 4H), 0.93 (s, 9H). ¹³C NMR (150 MHz, DMSO-*d*₆) δ 171.77, 171.47, 171.33, 169.11, 163.71, 163.33, 162.82, 150.83, 147.09, 140.71, 138.88, 137.30, 134.13, 130.54, 129.15, 129.01, 128.01, 127.98, 126.79, 126.42, 122.93, 75.11, 68.24, 65.30, 64.29, 63.94, 58.07, 55.73, 55.66, 43.59, 41.02, 37.51, 37.32, 34.67, 34.57, 34.23, 27.86, 27.84, 25.76, 24.71, 24.57, 20.31, 15.32. HRMS (ESI) calcd for C₄₈H₆₄N₁₀O₇S [M + Na]⁺ 947.4578, found 947.4577.

### Example 64: N¹-((S)-1-((2S,4R)-4-hydroxy-2-((4-(4-methylthiazol-5-yl)benzyl)carbamoyl) pyrrolidine-1-yl)-3,3-dimethyl-1-oxobutan-2-yl)-N¹⁰-(2-((4-(2-((E)-3-methylbenzylidene) hydrazino)-6-morpholinopyrimidin-2-yl)oxy)ethyl)decanediamide (LCG-64)

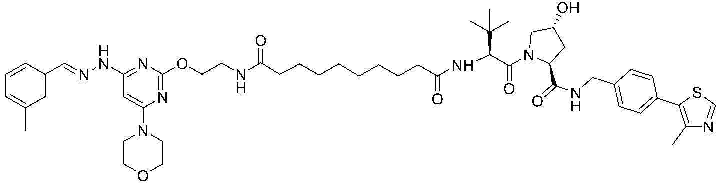

The synthesis method was as shown in Examples 1 and 37.

¹H NMR (600 MHz, DMSO-*d*₆) δ 10.82 (s, 1H), 8.98 (s, 1H), 8.56 (t, *J =* 6.1 Hz, 1H), 8.01 (s, 1H), 8.00 (t, *J* = 5.6 Hz, 1H), 7.83 (d, *J* = 9.4 Hz, 1H), 7.50 (d, *J =* 7.7 Hz, 1H), 7.48 (s, 1H), 7.42 (d, *J =* 8.2 Hz, 2H), 7.38 (d, *J =* 8.3 Hz, 2H), 7.29 (t, *J =* 7.6 Hz, 1H), 7.17 (d, *J =* 7.5 Hz, 1H), 6.07 (s, 1H), 5.12 (brs, 1H), 4.54 (d, *J =* 9.4 Hz, 1H), 4.46 - 4.40 (m, 2H), 4.35 (s, 1H), 4.21 (dd, *J=* 15.9, 5.5 Hz, 1H), 4.18 (t, *J =* 5.7 Hz, 2H), 3.69 - 3.62 (m, 6H), 3.56 - 3.50 (m, 4H), 3.37 - 3.34 (m, 2H), 2.44 (s, 3H), 2.34 (s, 3H), 2.28 - 2.20 (m, 1H), 2.13 - 2.00 (m, 4H), 1.93 - 1.86 (m, 1H), 1.52 - 1.40 (m, 4H), 1.27 - 1.17 (m, 8H), 0.93 (s, 9H). ¹³C NMR (150 MHz, DMSO-*d*₆) δ 171.77, 171.45, 171.32, 169.08, 163.70, 163.34, 162.80, 150.82, 147.08, 140.69, 138.88, 137.29, 134.12, 130.53, 129.15, 129.00, 128.00, 127.98, 126.79, 126.41, 122.92, 75.10, 68.23, 65.29, 64.29, 63.93, 58.05, 55.71, 55.63, 43.59, 41.01, 37.52, 37.32, 34.68, 34.57, 34.23, 28.13, 28.04, 25.74, 24.79, 24.64, 20.31, 15.31. HRMS (ESI) calcd for C₅₀H₆₈N₁₀O₇S [M + Na]⁺ 975.4891, found 975.4878.

### Example 65: (2S,4R)-1-((S)-2-(1-fluorocyclopropane-1-carboxamido)-3,3-dimethylbutanoyl)-4-hydroxy-N-((S)-1-(4-(4-methylthiazol-5-yl)phenyl)-4-((2-((4-(2-((4-morpholino-6-(3-(m-tolyl)-1H-pyrazol-1-yl)pyrimidin-2-yl)oxy)ethyl)phenyl)amino)-2-oxoethyl)amino)-4-oxobutyl)pyrrolidine-2-carboxamide (LCG-II-1)

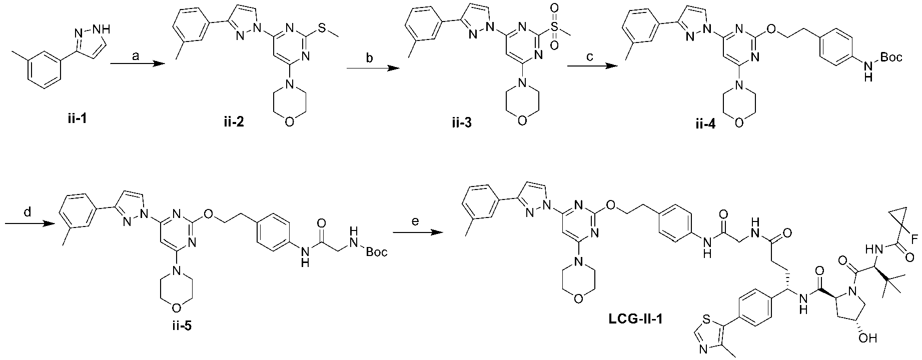

### Step 1: Preparation of intermediate ii-2

The ii-1 (150 mg, 0.95 mmol) was dissolved in 10 mL of ultra-dry DMF, NaH (80 mg, 2.85 mmol) was added under ice bath conditions, the mixture was stirred at room temperature for 2 h, a solution of 4,6-dichloro-2-methylsulfanylpyrimidine (276 mg, 1.42 mmol) in DMF was added dropwise, and the mixture was kept at room temperature for reaction for 3 h. Morpholine (330 mg, 3.8 mmol) was then added, and the mixture was continued to be kept at room temperature for reaction for 3 h. 50 mL of water was added, and the mixture was extracted with EA twice. The organic phases were combined, washed with saturated brine once, dried over anhydrous Na_{2SO}4, filtered, spin-dried, and separated by column chromatography to provide 167 mg of a white solid (48%). ¹H NMR (500 MHz, Chloroform-*d*) δ 8.56 (d, *J =* 2.7 Hz, 1H), 7.75 (s, 1H), 7.69 (d, *J =* 7.7 Hz, 1H), 7.33 (t, *J =* 7.6 Hz, 1H), 7.19 (d, *J =* 7.5 Hz, 1H), 6.91 (s, 1H), 6.74 (d, *J =* 2.7 Hz, 1H), 3.84 - 3.78 (m, 4H), 3.78 - 3.72 (m, 4H), 2.56 (s, 3H), 2.43 (s, 3H). LC-MS: calcd for C₁₉H₂₁N₅OS [M + H]⁺ 368.1, found 368.2.

### Step 2: Preparation of intermediate ii-3

The intermediate ii-2 (500 mg, 1.36 mmol) was dissolved in 30 mL of dichloromethane, and m-chloroperbenzoic acid (582 mg, 3.4 mmol) was added under ice bath conditions. The mixture was stirred at room temperature for 12 h. After the completion of the reaction was monitored by TLC, 10 mL of a saturated sodium thiosulfate solution was added, and the mixture was stirred at room temperature for 2 h. 50 mL of water was added, and the mixture was extracted with dichloromethane twice. The organic phases were combined, washed with saturated brine once, dried over anhydrous Na₂SO₄, filtered, spin-dried, and then slurried with methanol to provide 455 mg of a white solid (83%). ¹H NMR (600 MHz, DMSO-*d*₆) δ 8.69 (d, *J =* 2.7 Hz, 1H), 7.86 (s, 1H), 7.82 (d, *J =* 7.9 Hz, 1H), 7.37 (t, *J =* 7.6 Hz, 1H), 7.30 (s, 1H), 7.23 (d, *J=* 7.5 Hz, 1H), 7.15 (d, *J =* 2.7 Hz, 1H), 3.83 - 3.70 (m, 8H), 3.43 (s, 3H), 2.39 (s, 3H). LC-MS: calcd for C₁₉H₂₁N₅O₃S [M + H]⁺ 400.1, found 400.0.

### Step 3: Preparation of intermediate ii-4

*N*-BOC-2-(4-aminophenyl)ethanol (355 mg, 1.5 mmol) was dissolved in 10 mL of ultra-dry DMF, NaH (54 mg, 2.25 mmol) was added under ice bath conditions, the mixture was stirred at room temperature for 2 h, a solution of the intermediate ii-3 (300 mg, 0.75 mmol) in DMF was then added, and the mixture was kept at room temperature for reaction for 3 h. After the completion of the reaction was monitored by TLC, 50 mL of water was added, and the mixture was extracted with EA twice. The organic phases were combined, washed with saturated brine once, dried over anhydrous Na₂SO₄, filtered, spin-dried, and then separated by column chromatography to provide 312 mg of a white solid (75%). ¹H NMR (600 MHz, DMSO-*d*₆) δ 9.26 (s, 1H), 8.57 (d, *J= 2.7* Hz, 1H), 7.81 (s, 1H), 7.77 (d, *J =* 7.7 Hz, 1H), 7.39 (d, *J =* 8.1 Hz, 2H), 7.35 (t, *J =* 7.6 Hz, 1H), 7.23 - 7.17 (m, 3H), 7.05 (d, *J* = 2.7 Hz, 1H), 6.89 (s, 1H), 4.47 (t, *J =* 7.1 Hz, 2H), 3.76 - 3.64 (m, 8H), 2.97 (t, *J =* 7.0 Hz, 2H), 2.38 (s, 3H), 1.47 (s, 9H). LC-MS: calcd for C₃₁H₃₆N₆O₄ [M + H]⁺ 557.3, found 557.3.

### Step 4: Preparation of intermediate ii-5

The method for preparing the intermediate 11 in Example 50 was referred to for preparation of the intermediate ii-5. ¹H NMR (600 MHz, DMSO-*d*₆) δ 9.87 (s, 1H), 8.58 (d, *J =* 2.7 Hz, 1H), 7.95 (s, 1H), 7.81 (s, 1H), 7.77 (d, *J =* 7.7 Hz, 1H), 7.52 (d, *J =* 8.5 Hz, 2H), 7.35 (t, *J =* 7.6 Hz, 1H), 7.26 (d, *J =* 8.5 Hz, 2H), 7.21 (d, *J* = 7.5 Hz, 1H), 7.05 (d, *J* = 2.7 Hz, 1H), 7.02 (t, *J =* 6.1 Hz, 1H), 6.89 (s, 1H), 4.49 (t, *J =* 7.0 Hz, 2H), 3.76 - 3.64 (m, 10H), 3.00 (t, *J =* 7.0 Hz, 2H), 2.38 (s, 3H), 1.39 (s, 9H). LC-MS: calcd for C₃₃H₃₉N₇O₅ [M + H]⁺ 614.3, found 614.3.

### Step 5: Preparation of LCG-II-1

Based on the intermediate ii-5, the method for preparing the LCG-50 in Example 50 was referred to for the synthesis. ¹H NMR (600 MHz, DMSO-*d*₆) δ 9.90 (s, 1H), 8.98 (s, 1H), 8.57 (q, *J =* 2.8 Hz, 2H), 8.03 (t, *J =* 5.8 Hz, 1H), 7.81 (d, *J =* 1.8 Hz, 1H), 7.77 (d, *J =* 7.8 Hz, 1H), 7.53 (d, *J =* 8.5 Hz, 2H), 7.48 - 7.43 (m, 2H), 7.41 - 7.36 (m, 2H), 7.38 - 7.32 (m, 2H), 7.25 (d, *J* = 8.5 Hz, 2H), 7.21 (m, 1H), 7.04 (d, *J =* 2.7 Hz, 1H), 6.89 (s, 1H), 5.16 (d, *J =* 3.6 Hz, 1H), 4.84 (q, *J =* 7.7 Hz, 1H), 4.64 - 4.57 (m, 1H), 4.49 (m, 3H), 4.29 (s, 1H), 3.86 (d, *J =* 5.8 Hz, 2H), 3.74 - 3.58 (m, 8H), 3.65 - 3.53 (m, 2H), 3.00 (t, *J=* 7.0 Hz, 2H), 2.46 (s, 3H), 2.38 (s, 3H), 2.33 (m, 1H), 2.24 (m, 1H), 2.06 (dd, *J=* 12.7, 7.7 Hz, 1H), 1.95 (q, *J =* 7.7 Hz, 2H), 1.75 (m, 1H), 1.35 (m, 2H), 1.26 - 1.18 (m, 2H), 0.97 (s, 9H). ¹³C NMR (150 MHz, DMSO-*d*₆) δ 171.70, 170.19, 168.26, 167.53, 167.40, 167.00, 164.08, 163.43, 157.50, 152.96, 150.89, 147.18, 142.64, 137.33, 136.58, 132.37, 131.32, 130.45, 129.27, 128.72, 128.70, 128.52, 128.23, 128.03, 126.21, 125.68, 122.47, 118.63, 105.31, 81.54, 78.16, 76.62, 68.16, 66.54, 65.18, 58.19, 56.11, 55.97, 51.25, 43.66, 42.11, 37.15, 35.42, 33.46, 31.87, 31.53, 25.68, 25.62, 20.43, 15.40, 12.39, 12.32, 12.11, 12.04. HRMS (ESI) calcd for C₅₇H₆₆FN₁₁O₈S [M + H]⁺ 1084.4879, found 1084.4877.

### Example 66: (2S,4R)-1-((S)-2-(1-fluorocyclopropane-1-carboxamido)-3,3-dimethylbutanoyl) -4-hydroxy-N-(4-(4-methylthiazol-5-yl)-2-(2-((6-((4-(2-((4-morpholino-6-(3-(m-tolyl)-1H-pyrazol-1-yl)pyrimidin-2-yl)oxy)ethyl)phenyl)amino)-6-oxohexyl)amino)-2-oxoethoxy)benzyl) pyrrolidine-2-carboxamide (LCG-II-2)

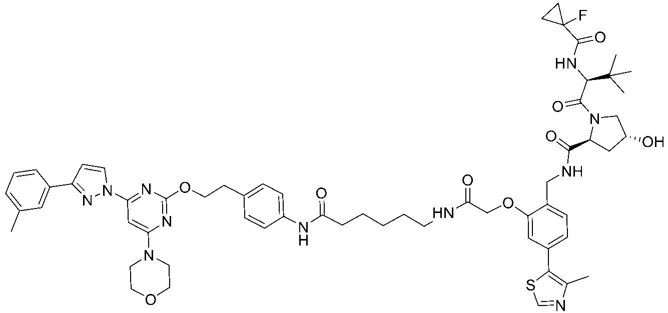

Based on the intermediate ii-4, the method for preparing the LCG-53 in Example 53 was referred to for the synthesis. ¹H NMR (600 MHz, DMSO-*d*₆) δ 9.80 (s, 1H), 8.98 (s, 1H), 8.57 (d, *J =* 2.7 Hz, 1H), 8.56 (t, *J =* 6.2 Hz, 1H), 8.13 (t, *J =* 5.9 Hz, 1H), 7.81 (d, *J =* 1.8 Hz, 1H), 7.77 (d, *J =* 7.7 Hz, 1H), 7.53 (d, *J =* 8.4 Hz, 2H), 7.43 (d, *J =* 7.8 Hz, 1H), 7.35 (t, *J=* 7.6 Hz, 1H), 7.27 (dd, *J =* 9.2, 2.7 Hz, 1H), 7.22 (m, 3H), 7.04 (d, *J =* 2.7 Hz, 1H), 7.02 (dd, *J =* 7.7, 1.6 Hz, 1H), 6.96 (d, *J =* 1.7 Hz, 1H), 6.89 (s, 1H), 5.17 (d, *J =* 3.6 Hz, 1H), 4.61 - 4.42 (m, 7H), 4.35 (s, 1H), 4.30 (dd, *J =* 15.8, 5.7 Hz, 1H), 3.73 - 3.58 (m, 10H), 3.15 (m, 2H), 2.99 (t, *J =* 7.1 Hz, 2H), 2.45 (s, 3H), 2.38 (s, 3H), 2.26 (t, *J =* 7.4 Hz, 2H), 2.09 - 2.02 (m, 1H), 1.91 - 1.85 (m, 1H), 1.57 (p, *J* = 7.5 Hz, 2H), 1.48 (p, *J =* 7.2 Hz, 2H), 1.40 - 1.32 (m, 2H), 1.32 - 1.25 (m, 2H), 1.21 (dd, *J =* 8.5, 3.2 Hz, 2H), 0.95 (s, 9H). ¹³C NMR (150 MHz, DMSO-*d*₆) δ 171.10, 170.37, 168.27, 167.50, 167.36, 166.60, 164.09, 163.44, 157.51, 154.51, 152.97, 150.96, 147.37, 137.34, 137.06, 132.02, 131.33, 130.60, 130.46, 128.72, 128.69, 128.45, 128.32, 128.03, 126.49, 125.69, 122.48, 120.86, 118.55, 111.55, 105.31, 81.54, 78.27, 76.73, 68.27, 66.60, 65.19, 64.30, 58.24, 56.10, 55.93, 43.67, 37.68, 37.23, 36.81, 35.65, 35.63, 35.41, 33.48, 28.37, 25.59, 25.51, 25.45, 24.26, 20.44, 15.40, 15.37, 14.55, 12.39, 12.32, 12.13, 12.06. HRMS (ESI) calcd for C₆₀H₇₂FN₁₁O₉S [M + H]⁺ 1142.5297, found 1142.5284.

### Example 67: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-3-((4-(4-((4-morpholino-6-(3-(m-tolyl)-1H-pyrazol-1-yl)pyrimidin-2-yl)oxy)piperidin-1-yl)phenyl)amino)-3-oxopropyl)pyrrolidine-2-carboxamide (LCG-II-3)

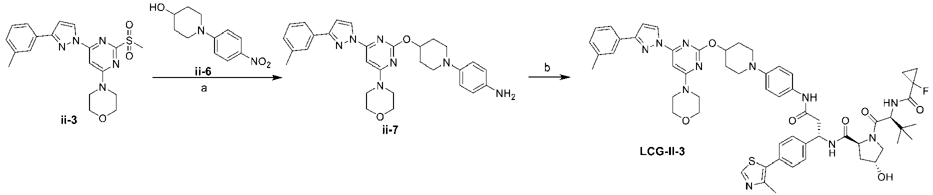

### Step 1: Preparation of intermediate ii-6

4-hydroxypiperidine (500 mg, 5.0 mmol) was dissolved in 10 mL of acetonitrile. K₂CO₃ (2 g, 15.0 mmol) and 4-fluoronitrobenzene (700 mg, 5.0 mmol) were added. The mixture was stirred at room temperature for 5 h, filtered, and spin-dried to provide a yellow solid, which was not further purified. LC-MS: calcd for C₁₁H₁₄N₂O₃ [M + H]⁺ 222.1, found 222.3.

### Step 2: Preparation of intermediate ii-7

Based on the intermediates ii-3 and ii-6, a nitro-containing intermediate was obtained by referring to the above synthesis method of ii-4, and then the intermediate ii-7 was obtained through a conventional nitro hydrogenation reduction reaction. ¹H NMR (600 MHz, DMSO-*d*₆) δ 8.59 (d, *J =* 2.7 Hz, 1H), 7.82 (s, 1H), 7.78 (d, *J =* 8.0 Hz, 1H), 7.35 (t, *J =* 7.6 Hz, 1H), 7.21 (dt, *J =* 7.5, 0.9 Hz, 1H), 7.06 (d, *J =* 2.7 Hz, 1H), 6.89 (s, 1H), 6.74 (d, *J =* 8.8 Hz, 2H), 6.50 (d, *J =* 8.7 Hz, 2H), 5.12 - 5.04 (m, 1H), 4.59 (s, 2H), 3.74 - 3.63 (m, 8H), 3.29 - 3.22 (m, 2H), 2.87 - 2.80 (m, 2H), 2.39 (s, 3H), 2.15 - 2.08 (m, 2H), 1.87 - 1.75 (m, 2H). LC-MS: calcd for C₂₉H₃₃N₇O₂ [M + H]⁺ 512.3, found 512.3.

### Step 3: Preparation of LCG-II-3

Based on the intermediate ii-7, the method for preparing the LCG-50 in Example 50 was referred to for the synthesis. ¹H NMR (600 MHz, DMSO-*d*₆) δ 9.69 (s, 1H), 8.98 (s, 1H), 8.61 (d, *J* = 7.9 Hz, 1H), 8.60 (d, *J =* 2.7 Hz, 1H), 7.82 (s, 1H), 7.78 (d, *J =* 7.7 Hz, 1H), 7.46 - 7.40 (m, 4H), 7.40 - 7.32 (m, 3H), 7.26 (dd, *J* = 9.2, 2.9 Hz, 1H), 7.24 - 7.17 (m, 1H), 7.06 (d, *J =* 2.7 Hz, 1H), 6.91 (s, 1H), 6.90 (s, 2H), 5.27 (q, *J =* 7.7 Hz, 1H), 5.18 - 5.10 (m, 2H), 4.58 (d, *J* = 9.2 Hz, 1H), 4.48 (t, *J =* 8.3 Hz, 1H), 4.28 (s, 1H), 3.74 - 3.65 (m, 8H), 3.64 - 3.54 (m, 2H), 3.50 - 3.42 (m, 2H), 3.00 (t, *J =* 9.2 Hz, 2H), 2.87 - 2.77 (m, 2H), 2.44 (s, 3H), 2.39 (s, 3H), 2.11 (d, *J =* 11.7 Hz, 2H), 2.05 (dd, *J =* 12.8, 7.9 Hz, 1H), 1.85 - 1.72 (m, 3H), 1.43 - 1.29 (m, 2H), 1.22 (dd, *J =* 8.6, 3.0 Hz, 2H), 0.98 (s, 9H). ¹³C NMR (150 MHz, DMSO-*d*₆) δ 169.92, 168.24, 167.48, 167.34, 166.83, 164.21, 162.88, 157.55, 152.99, 150.93, 147.21, 141.63, 137.36, 131.35, 130.43, 129.41, 128.75, 128.10, 128.05, 126.51, 125.71, 122.50, 119.94, 115.66, 105.34, 81.48, 78.31, 76.77, 71.38, 68.19, 65.23, 64.32, 58.21, 56.01, 55.98, 49.28, 46.25, 45.14, 43.70, 41.98, 37.07, 35.48, 29.69, 25.69, 25.65, 20.46, 15.42, 14.57, 12.41, 12.34, 12.13, 12.06, 8.03. HRMS (ESI) calcd for C₅₇H₆₆FN₁₁O₇S [M + H]⁺ 1068.4929, found 1068.4927.

### Example 68: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-4-((4-(4-((4-morpholino-6-(3-(m-tolyl)-1H-pyrazol-1-yl)pyrimidin-2-yl)oxy)piperidin-1-yl)phenyl)amino)-4-oxobutyl)pyrrolidine-2-carboxamide (LCG-II-4)

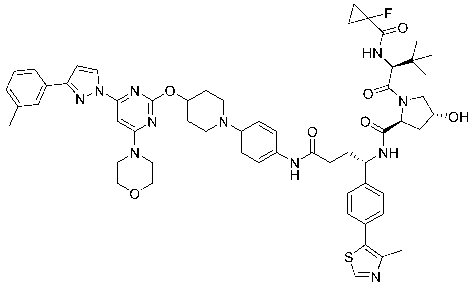

The method for preparing the LCG-II-3 in Example 67 was referred to for the synthesis. ¹H NMR (600 MHz, DMSO-*d*₆) δ 9.60 (s, 1H), 8.99 (s, 1H), 8.60 (d, *J =* 2.7 Hz, 1H), 8.57 (d, *J =* 8.2 Hz, 1H), 7.82 (s, 1H), 7.78 (d, *J =* 7.7 Hz, 1H), 7.46 (d, *J =* 8.3 Hz, 2H), 7.41 (dd, *J =* 13.7, 8.4 Hz, 4H), 7.35 (t, *J =* 7.6 Hz, 1H), 7.28 (dd, *J =* 9.2, 2.8 Hz, 1H), 7.21 (d, *J =* 7.6 Hz, 1H), 7.06 (d, *J =* 2.7 Hz, 1H), 6.92 (d, *J =* 8.5 Hz, 2H), 6.90 (s, 1H), 5.17 - 5.10 (m, 2H), 4.86 (q, *J =* 8.1 Hz, 1H), 4.59 (d, *J =* 9.2 Hz, 1H), 4.50 (t, *J =* 8.3 Hz, 1H), 4.28 (s, 1H), 3.75 - 3.66 (m, 8H), 3.65 - 3.55 (m, 2H), 3.52 - 3.43 (m, 2H), 3.00 (t, *J=* 10.1 Hz, 2H), 2.47 (s, 3H), 2.39 (s, 3H), 2.37 - 2.30 (m, 1H), 2.16 - 1.95 (m, 5H), 1.86 - 1.71 (m, 3H), 1.45 - 1.29 (m, 2H), 1.22 (dd, *J =* 8.4, 2.5 Hz, 2H), 0.97 (s, 9H). ¹³C NMR (150 MHz, DMSO-*d*₆) δ 170.23, 169.51, 168.24, 167.50, 167.37, 164.21, 162.88, 157.55, 152.99, 150.92, 147.20, 146.37, 142.62, 137.35, 131.34, 130.72, 130.47, 129.31, 128.75, 128.26, 128.05, 126.29, 126.20, 125.70, 122.49, 119.63, 115.73, 105.34, 81.48, 78.29, 76.75, 71.40, 68.20, 65.23, 64.31, 58.19, 56.09, 56.00, 51.41, 46.33, 45.14, 43.70, 37.16, 35.45, 32.56, 31.59, 29.73, 25.70, 25.63, 20.45, 15.43, 14.57, 12.41, 12.34, 12.14, 12.07, 8.03. HRMS (ESI) calcd for C₅₈H₆₈FN₁₁O₇S [M + H]⁺ 1082.5086, found 1082.5076.

### Example 69: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-6-((4-(4-((4-morpholino-6-(3-(m-tolyl)-1H-pyrazol-1-yl)pyrimidin-2-yl)oxy)piperidin-1-yl)phenyl)amino)-6-oxohexyl)pyrrolidine-2-carboxamide (LCG-II-5)

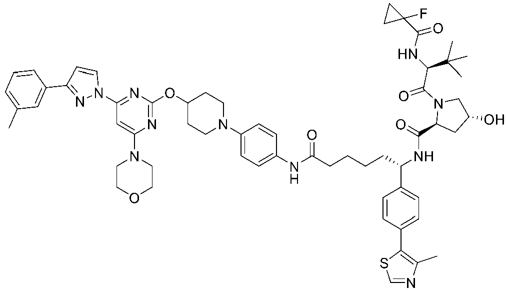

The method for preparing the LCG-II-3 in Example 67 was referred to for the synthesis. ¹H NMR (600 MHz, DMSO-*d*₆) δ 9.61 (s, 1H), 8.99 (s, 1H), 8.60 (d, *J* = 2.7 Hz, 1H), 8.46 (d, *J* = 8.2 Hz, 1H), 7.82 (s, 1H), 7.78 (d, *J=* 7.7 Hz, 1H), 7.42 (d, *J =* 8.3 Hz, 4H), 7.36 (m, 3H), 7.27 (dd, *J* = 9.3, 2.8 Hz, 1H), 7.21 (d, *J* = 7.5 Hz, 1H), 7.06 (d, *J= 2.7* Hz, 1H), 6.91 (d, *J* = 8.3 Hz, 3H), 5.17 - 5.10 (m, 2H), 4.80 (q, *J* = 7.7 Hz, 1H), 4.58 (d, *J =* 9.2 Hz, 1H), 4.50 (t, *J =* 8.2 Hz, 1H), 4.28 (s, 1H), 3.75 - 3.64 (m, 8H), 3.63 - 3.53 (m, 2H), 3.50 - 3.43 (m, 2H), 2.99 (t, *J =* 9.4 Hz, 2H), 2.45 (s, 3H), 2.39 (s, 3H), 2.24 (t, *J =* 7.4 Hz, 2H), 2.16 - 2.09 (m, 2H), 2.04 (dd, *J* = 12.9, 7.9 Hz, 1H), 1.84 - 1.77 (m, 2H), 1.77 - 1.67 (m, 3H), 1.65 - 1.55 (m, 2H), 1.49 - 1.40 (m, 1H), 1.40 - 1.28 (m, 3H), 1.22 (dd, *J =* 8.4, 2.9 Hz, 2H), 0.97 (s, 9H). ¹³C NMR (150 MHz, DMSO-*d*₆) δ 170.05, 169.91, 168.11, 167.47, 167.34, 164.21, 162.89, 157.55, 152.99, 150.86, 147.14, 146.33, 143.23, 137.35, 131.34, 130.81, 130.51, 129.10, 128.75, 128.16, 128.05, 126.22, 125.70, 122.49, 119.61, 115.73, 105.34, 81.48, 78.30, 76.75, 71.40, 68.21, 65.23, 64.31, 58.04, 56.03, 55.96, 51.44, 46.32, 45.13, 43.69, 37.12, 35.59, 35.48, 35.46, 29.73, 25.73, 25.62, 24.78, 24.35, 20.45, 15.43, 14.57, 12.41, 12.34, 12.12, 12.06, 8.03. HRMS (ESI) calcd for C₆₀H₇₂FN₁₁O₇S [M + H]⁺ 1110.5399, found 1110.5399.

### Example 70: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-3-(4-((4-(2-((4-morpholino-6-(3-(m-tolyl)-1H-pyrazol-1-yl)pyrimidin-2-yl)oxy)ethyl)phenyl)ethynyl)piperidin-1-yl)-3-oxopropyl) pyrrolidine-2-carboxamide (LCG-II-6)

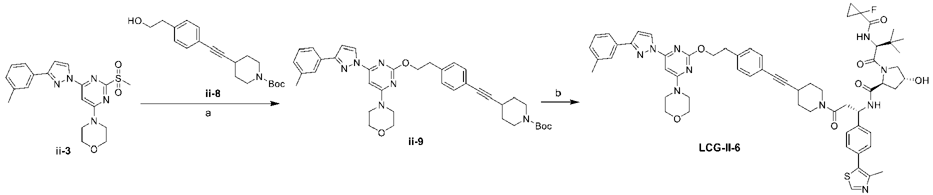

### Step 1: Preparation of intermediate ii-8

4-bromophenethyl alcohol (200 mg, 1.0 mmol), 1-Boc-4-ethynylpiperidine (630 mg, 3.0 mmol), cuprous iodide (400 mg, 0.2 mmol), bis(triphenylphosphine)palladium dichloride (70 mg, 0.1 mmol), and triethylamine (304 mg, 3.0 mmol) were dissolved in 10 mL of ultra-dry DMF. After nitrogen replacement twice, the mixture was kept at 60°C for reaction for 8 h. After the completion of the reaction was monitored by TLC, 50 mL of water was added, and the mixture was extracted with EA twice. The organic phases were combined, washed with saturated brine once, dried over anhydrous Na₂SO₄, filtered, spin-dried, and then separated by column chromatography to provide 200 mg of a yellow oil (61%). ¹H NMR (600 MHz, DMSO-*d*₆) δ 7.29 (d, *J =* 8.2 Hz, 2H), 7.19 (d, *J =* 8.2 Hz, 2H), 4.64 (s, 1H), 3.69 - 3.61 (m, 2H), 3.59 (t, *J =* 7.0 Hz, 2H), 3.12 (brs, 2H), 2.89 - 2.80 (m, 1H), 2.71 (t, *J =* 6.9 Hz, 2H), 1.83 - 1.76 (m, 2H), 1.55 - 1.45 (m, 2H), 1.40 (s, 9H). LC-MS: calcd for C₂₀H₂₇NO₃ [M + H]⁺ 330.2, found 330.2.

### Step 2: Preparation of intermediate ii-9

Based on the intermediate ii-8, the intermediate ii-9 was obtained by referring to the above synthesis method of ii-4. ¹H NMR (500 MHz, Chloroform-d) δ 8.52 (t, *J =* 2.4 Hz, 1H), 7.74 (s, 1H), 7.69 (d, *J =* 7.4 Hz, 1H), 7.37 - 7.30 (m, 3H), 7.23 (d, *J =* 8.0 Hz, 2H), 7.20 - 7.16 (m, 1H), 6.89 (d, *J* = 1.9 Hz, 1H), 6.74 (t, *J =* 2.4 Hz, 1H), 4.52 (t, *J =* 7.5 Hz, 2H), 3.83 - 3.64 (m, 10H), 3.33 - 3.17 (m, 2H), 3.13 (t, *J =* 7.4 Hz, 2H), 2.78 (d, *J =* 7.5 Hz, 1H), 2.43 (s, 3H), 1.84 (brs, 2H), 1.66 (d, *J =* 10.5 Hz, 2H), 1.47 (s, 9H). LC-MS: calcd for C₃₈H₄₄N₆O₄ [M + H]⁺ 649.3, found 649.4.

### Step 3: Preparation of LCG-II-6

Based on the intermediate ii-9, the method for preparing the LCG-50 in Example 50 was referred to for the synthesis. ¹H NMR (600 MHz, DMSO-*d*₆) δ 8.95 (d, *J =* 24.6 Hz, 1H), 8.57 (t, *J =* 2.8 Hz, 1H), 8.51 (dd, *J =* 11.6, 8.0 Hz, 1H), 7.81 (s, 1H), 7.77 (d, *J =* 7.8 Hz, 1H), 7.46 - 7.37 (m, 4H), 7.35 (t, *J* = 7.6 Hz, 1H), 7.33 - 7.24 (m, 4H), 7.22 (dd, *J =* 12.2, 7.9 Hz, 2H), 7.05 (d, *J =* 2.7 Hz, 1H), 6.89 (s, 1H), 5.24 - 5.10 (m, 2H), 4.57 (d, *J =* 9.2 Hz, 1H), 4.52 (t, *J =* 6.8 Hz, 2H), 4.45 (t, *J =* 8.2 Hz, 1H), 4.28 (s, 1H), 3.92 - 3.50 (m, 13H), 3.27 - 3.16 (m, 1H), 3.05 (t, *J* = 6.7 Hz, 2H), 2.94 - 2.78 (m, 3H), 2.44 (d, *J =* 7.2 Hz, 3H), 2.38 (s, 3H), 2.09 - 2.00 (m, 1H), 1.82 - 1.70 (m, 3H), 1.52 - 1.29 (m, 4H), 1.25 - 1.19 (m, 2H), 0.97 (s, 9H). ¹³C NMR (150 MHz, DMSO-*d*₆) δ 169.92, 169.87, 168.26, 168.22, 167.48, 167.35, 167.05, 166.96, 164.09, 163.41, 157.51, 153.00, 150.92, 150.84, 147.17, 141.76, 137.96, 137.87, 137.35, 131.33, 130.72, 130.66, 130.47, 130.45, 129.48, 129.38, 128.75, 128.72, 128.54, 128.49, 128.11, 128.05, 126.66, 125.70, 122.49, 120.21, 105.34, 81.60, 78.29, 76.75, 68.19, 66.19, 65.21, 64.32, 58.18, 55.95, 49.61, 49.21, 45.14, 37.01, 35.46, 33.84, 31.01, 30.47, 26.32, 26.17, 25.66, 25.63, 20.46, 15.41, 15.37, 14.57, 12.40, 12.33, 12.12, 12.05, 8.04. HRMS (ESI) calcd for C₆₁H₆₉FN₁₀O₇S [M + H]⁺ 1105.5133, found 1105.5138.

### Example 71: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-4-(4-((4-(2-((4-morpholino-6-(3-(m-tolyl)-1H-pyrazol-1-yl)pyrimidin-2-yl)oxy)ethyl)phenyl)ethynyl)piperidin-1-yl)-4-oxobutyl) pyrrolidine-2-carboxamide (LCG-II-7)

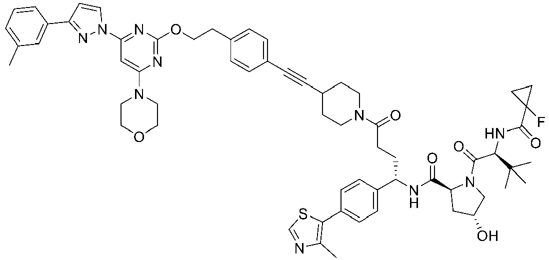

The method for preparing the LCG-II-6 in Example 70 was referred to for the synthesis. ¹H NMR (600 MHz, DMSO-*d*₆) δ 8.99 (s, 1H), 8.57 (d, *J =* 2.7 Hz, 1H), 8.53 (dd, *J =* 12.7, 8.7 Hz, 1H), 7.81 (s, 1H), 7.77 (d, *J* = 7.7 Hz, 1H), 7.46 (d, *J =* 8.3 Hz, 2H), 7.40 (d, *J =* 8.2 Hz, 2H), 7.38 - 7.28 (m, 5H), 7.21 (d, *J =* 7.6 Hz, 1H), 7.18 (d, *J* = 9.0 Hz, 1H), 7.05 (d, *J =* 2.7 Hz, 1H), 6.89 (s, 1H), 5.18 - 5.11 (m, 1H), 4.93 (m, 2H), 4.59 - 4.53 (m, 1H), 4.51 (q, *J =* 6.4 Hz, 2H), 4.49 - 4.44 (m, 1H), 4.31 (s, 1H), 3.83 - 3.54 (m, 12H), 3.06 (t, *J =* 6.9 Hz, 2H), 2.96 - 2.84 (m, 1H), 2.77 - 2.60 (m, 1H), 2.46 (s, 3H), 2.45 - 2.39 (m, 1H), 2.38 (s, 3H), 2.36 - 2.28 (m, 1H), 2.03 (dd, *J =* 12.9, 7.6 Hz, 1H), 1.92 - 1.72 (m, 5H), 1.69 - 1.54 (m, 1H), 1.50 - 1.41 (m, 1H), 1.37 - 1.28 (m, 1H), 1.27 - 1.15 (m, 3H), 0.97 (d, *J =* 18.9 Hz, 9H). ¹³C NMR (150 MHz, DMSO-*d*₆) δ 170.26, 170.22, 169.54, 169.45, 168.18, 168.14, 167.41, 167.38, 167.28, 167.26, 164.08, 163.40, 157.50, 152.99, 150.90, 147.21, 143.13, 143.08, 137.92, 137.35, 131.32, 130.71, 130.68, 130.47, 129.22, 129.21, 128.74, 128.69, 128.51, 128.48, 128.29, 128.04, 126.10, 126.06, 125.70, 122.49, 120.28, 105.33, 81.58, 68.28, 66.21, 65.20, 64.31, 58.38, 56.16, 55.98, 43.68, 42.61, 37.15, 37.12, 35.62, 33.83, 31.82, 31.66, 31.12, 28.83, 26.45, 26.24, 25.64, 25.54, 20.45, 15.41, 14.56, 12.31, 12.03. HRMS (ESI) calcd for C₆₂H₇₁FN₁₀O₇S [M + H]⁺ 1119.5290, found 1119.5293.

### Example 72: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-6-(4-((4-(2-((4-morpholino-6-(3-(m-tolyl)-1H-pyrazol-1-yl)pyrimidin-2-yl)oxy)ethyl)phenyl)ethynyl)piperidin-1-yl)-6-oxohexyl) pyrrolidine-2-carboxamide (LCG-II-8)

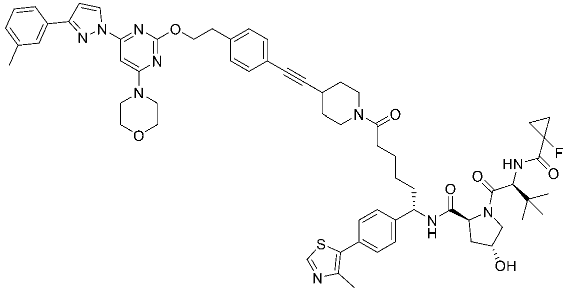

The method for preparing the LCG-II-6 in Example 70 was referred to for the synthesis. ¹H NMR (600 MHz, DMSO-*d*₆) δ 8.98 (s, 1H), 8.56 (d, *J =* 2.7 Hz, 1H), 8.45 (d, *J=* 6.9 Hz, 1H), 7.81 (s, 1H), 7.77 (d, *J =* 7.7 Hz, 1H), 7.43 (d, *J =* 8.3 Hz, 2H), 7.38 (d, *J =* 8.2 Hz, 2H), 7.38 - 7.31 (m, 3H), 7.30 (d, *J =* 7.8 Hz, 2H), 7.28 - 7.22 (m, 1H), 7.21 (d, *J =* 7.6 Hz, 1H), 7.04 (d, *J =* 2.2 Hz, 1H), 6.89 (s, 1H), 5.13 (brs, 1H), 4.82 (q, *J =* 5.7, 5.2 Hz, 1H), 4.57 (d, *J =* 9.2 Hz, 1H), 4.54 - 4.46 (m, 3H), 4.28 (s, 1H), 3.89 - 3.80 (m, 1H), 3.71 - 3.65 (m, 10H), 3.62 - 3.52 (m, 2H), 3.31 - 3.24 (m, 1H), 3.20 - 3.11 (m, 1H), 3.05 (t, *J =* 6.8 Hz, 2H), 2.94 - 2.84 (m, 1H), 2.45 (s, 3H), 2.38 (s, 3H), 2.33 - 2.25 (m, 2H), 2.03 (dd, *J =* 12.9, 7.8 Hz, 1H), 1.88 - 1.65 (m, 5H), 1.59 - 1.40 (m, 5H), 1.39 - 1.29 (m, 3H), 1.23 - 1.16 (m, 2H), 0.96 (d, *J =* 4.3 Hz, 9H). ¹³C NMR (150 MHz, DMSO-*d*₆) δ 170.09, 169.72, 168.09, 167.45, 167.31, 164.09, 163.41, 157.50, 153.00, 150.88, 147.15, 143.27, 143.22, 137.96, 137.35, 131.33, 130.72, 130.51, 129.12, 128.75, 128.71, 128.55, 128.20, 128.05, 126.25, 126.22, 126.19, 125.70, 122.49, 120.25, 105.33, 81.59, 80.81, 78.31, 76.77, 68.23, 66.19, 65.20, 64.32, 58.08, 56.04, 55.94, 51.32, 51.22, 43.68, 43.02, 37.13, 35.52, 33.85, 31.73, 31.67, 31.28, 30.53, 26.34, 25.72, 25.60, 24.87, 23.95, 20.45, 15.41, 14.57, 12.39, 12.32, 12.11, 12.05. HRMS (ESI) calcd for C₆₄H₇₅FN₁₀O₇S [M + H]⁺ 1147.5603, found 1147.5604.

### Example 73: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-3-(3-(3-(4-(2-((4-morpholino-6-(3-(m-tolyl)-1H-pyrazol-1-yl)pyrimidin-2-yl)oxy)ethyl)phenyl)ureido)azetidin-1-yl)-3-oxopropyl) pyrrolidine-2-carboxamide (LCG-II-9)

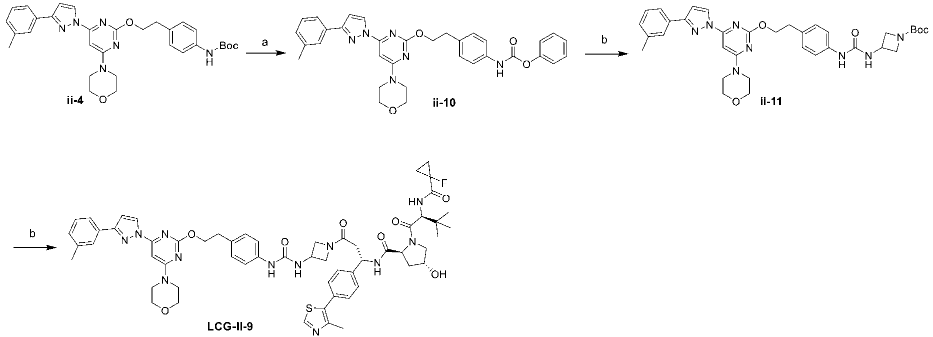

### Step 1: Preparation of intermediate ii-10

The intermediate ii-4 (300 mg, 0.44 mmol) was dissolved in 10 mL of dichloromethane, and 4 mL of trifluoroacetic acid was added. The mixture was stirred at room temperature for 3 h, spin-dried, and then redissolved in isopropanol/water (10 mL/8 mL). Phenyl chloroformate (86 mg, 0.6 mmol) and Na₂HPO₄ (93 mg, 0.7 mmol) were added. The mixture was kept at room temperature for reaction for 12 h. After the completion of the reaction was monitored by TLC, 10 mL of water was added, and the mixture was extracted with EA twice. The organic phases were combined, washed with saturated brine once, dried over anhydrous Na₂SO₄, filtered, spin-dried, and then slurried with PE/EA to provide 200 mg of a white solid (79%). ¹H NMR (600 MHz, DMSO-*d*₆) δ 10.18 (s, 1H), 8.58 (d, *J=* 2.7 Hz, 1H), 7.82 (s, 1H), 7.77 (d, *J =* 7.7 Hz, 1H), 7.48 - 7.40 (m, 4H), 7.35 (t, *J =* 7.6 Hz, 1H), 7.31 - 7.23 (m, 3H), 7.24 - 7.19 (m, 3H), 7.05 (d, *J* = 2.7 Hz, 1H), 6.89 (s, 1H), 4.50 (t, *J =* 7.1 Hz, 2H), 3.74 - 3.59 (m, 8H), 3.01 (t, *J =* 7.0 Hz, 2H), 2.38 (s, 3H). LC-MS: calcd for C₃₃H₃₂N₆O₄ [M + H]⁺ 577.3, found 577.2.

### Step 2: Preparation of intermediate ii-11

An intermediate ii-11 (200 mg, 0.34 mmol) was dissolved in 10 mL of toluene, 1-Boc-3-aminocyclobutylamine (120 mg, 0.70 mmol) and DMAP (4.2 mg, 0.03 mmol) were added, and then the mixture was heated to 90°C for reaction for 24 h. After completion of the reaction was monitored by TLC, the mixture was cooled to precipitate a white solid, and filtered to provide 139 mg of the target product (61%). LC-MS: calcd for C₃₅H₄₂N₈O₅ [M + H]⁺ 655.3, found 655.3.

### Step 3: Preparation of LCG-II-9

Based on the intermediate ii-11, the method for preparing the LCG-50 in Example 50 was referred to for the synthesis. ¹H NMR (600 MHz, DMSO-*d*₆) δ 8.98 (d, *J =* 10.7 Hz, 1H), 8.57 (d, *J= 2.7* Hz, 1H), 8.56 - 8.45 (m, 2H), 7.81 (s, 1H), 7.77 (d, *J =* 7.7 Hz, 1H), 7.48 - 7.42 (m, 2H), 7.40 (d, *J =* 8.2 Hz, 2H), 7.38 - 7.25 (m, 4H), 7.21 (d, *J =* 7.5 Hz, 1H), 7.18 (dd, *J =* 8.7, 2.3 Hz, 2H), 7.05 (d, *J =* 2.7 Hz, 1H), 6.89 (s, 1H), 6.73 (dd, *J =* 17.3, 6.6 Hz, 1H), 5.19 - 5.11 (m, 1H), 4.58 (d, *J =* 9.2 Hz, 1H), 4.51 - 4.42 (m, 3H), 4.42 - 4.25 (m, 3H), 4.19 - 4.07 (m, 1H), 4.06 - 3.91 (m, 1H), 3.82 - 3.52 (m, 12H), 2.97 (t, *J =* 6.9 Hz, 2H), 2.70 - 2.60 (m, 1H), 2.59 - 2.52 (m, 1H), 2.46 (d, *J =* 8.4 Hz, 3H), 2.38 (s, 3H), 2.09 - 1.99 (m, 1H), 1.81 - 1.74 (m, 1H), 1.42 - 1.30 (m, 2H), 1.25 - 1.19 (m, 2H), 0.98 (s, 9H). ¹³C NMR (150 MHz, DMSO-*d*₆) δ 169.88, 168.68, 168.63, 168.24, 167.54, 167.50, 167.40, 167.37, 164.10, 163.47, 157.52, 154.03, 152.98, 150.96, 150.91, 147.25, 147.22, 141.84, 141.70, 137.80, 137.35, 131.34, 130.44, 129.48, 129.43, 128.74, 128.70, 128.48, 128.15, 128.05, 126.49, 126.44, 125.70, 122.49, 117.56, 117.52, 105.34, 81.54, 78.28, 78.25, 76.74, 76.71, 68.18, 66.70, 66.32, 65.21, 64.32, 58.18, 58.16, 56.93, 56.76, 55.97, 54.10, 53.96, 48.80, 48.77, 45.09, 43.69, 37.09, 37.07, 37.02, 35.47, 35.44, 33.40, 25.64, 20.46, 15.40, 14.57, 12.40, 12.33, 12.13, 12.06, 8.01. HRMS (ESI) calcd for C₅₈H₆₇FN₁₂O₈S [M + H]⁺ 1111.4988, found 1111.4989.

### Example 74: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-4-(3-(3-(4-(2-((4-morpholino-6-(3-(m-tolyl)-1H-pyrazol-1-yl)pyrimidin-2-yl)oxy)ethyl)phenyl)ureido)azetidin-1-yl)-4-oxobutyl) pyrrolidine-2-carboxamide (LCG-II-10)

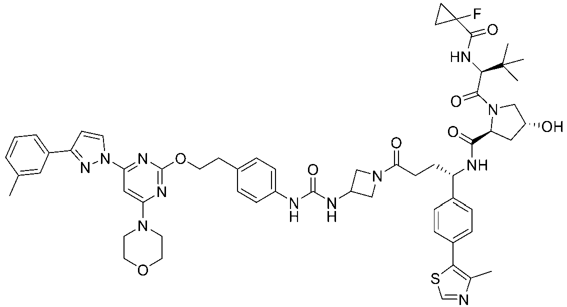

The method for preparing the LCG-II-9 in Example 73 was referred to for the synthesis. ¹H NMR (600 MHz, DMSO-*d*₆) δ 8.98 (d, *J =* 1.9 Hz, 1H), 8.57 (d, *J =* 2.7 Hz, 1H), 8.52 (d, *J =* 8.5 Hz, 1H), 8.50 (d, *J =* 5.3 Hz, 1H), 7.82 (s, 1H), 7.77 (d, *J =* 7.8 Hz, 1H), 7.46 (d, *J =* 8.1 Hz, 2H), 7.41 - 7.36 (m, 2H), 7.37 - 7.32 (m, 3H), 7.31 - 7.23 (m, 1H), 7.21 (d, *J =* 7.6 Hz, 1H), 7.18 (d, *J* = 8.4 Hz, 2H), 7.05 (d, *J =* 2.7 Hz, 1H), 6.89 (s, 1H), 6.77 (dd, *J =* 10.0, 7.0 Hz, 2H), 5.15 (brs, 1H), 4.90 - 4.79 (m, 1H), 4.62 - 4.54 (m, 1H), 4.51 - 4.32 (m, 5H), 4.31 - 4.27 (s, 1H), 4.13 - 4.03 (m, 1H), 3.97 - 3.87 (m, 1H), 3.74 - 3.63 (m, 9H), 3.64 - 3.53 (m, 2H), 2.97 (t, *J =* 7.1 Hz, 2H), 2.46 (d, *J =* 2.1 Hz, 3H), 2.38 (s, 3H), 2.35 - 2.01 (m, 3H), 1.97 - 1.82 (m, 2H), 1.80 - 1.71 (m, 1H), 1.40 - 1.25 (m, 2H), 1.25 - 1.17 (m, 2H), 0.97 (d, *J =* 7.5 Hz, 9H). ¹³C NMR (150 MHz, DMSO-*d*₆) δ 171.31, 171.18, 170.23, 168.19, 168.16, 167.51, 167.47, 167.38, 167.34, 164.09, 163.45, 157.51, 154.02, 152.97, 150.90, 147.19, 142.87, 142.83, 137.85, 137.34, 131.33, 130.46, 130.43, 130.37, 129.25, 128.73, 128.68, 128.48, 128.45, 128.25, 128.04, 126.14, 126.12, 125.69, 122.48, 117.55, 117.52, 105.32, 81.53, 78.20, 76.66, 68.21, 66.71, 65.19, 64.30, 58.23, 58.18, 56.81, 56.73, 56.05, 55.97, 55.93, 54.10, 51.00, 50.80, 43.67, 37.17, 35.50, 35.44, 33.39, 30.97, 30.84, 25.59, 25.56, 20.44, 15.40, 14.56, 12.36, 12.30, 12.08, 12.01, 7.99. HRMS (ESI) calcd for C₅₉H₆₉FN₁₂O₈S [M + H]⁺ 1125.5144, found 1125.5143.

### Example 75: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-3-(4-(4-(2-((4-morpholino-6-(3-(m-tolyl)-1H-pyrazol-1-yl)pyrimidin-2-yl)oxy)ethyl)phenyl)piperazin-1-yl)-3-oxopropyl)pyrrolidine-2-carboxamide (LCG-II-11)

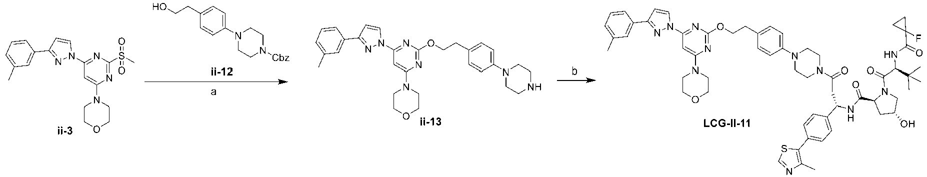

### Step 1: Preparation of intermediate ii-12

4-bromophenethyl alcohol (500 mg, 2.5 mmol), 1-Cbz-piperazine (700 mg, 3.8 mmol), tris(dibenzylideneacetone)dipalladium (230 mg, 0.25 mmol), K₃PO₄ (1.1 g, 5.0 mmol) , and BrettPhos (270 mg, 0.50 mmol) were dissolved in 20 mL of ultra-dry 1,4-dioxane. After nitrogen replacement twice, the mixture was kept at 90°C for reaction for 12 h. After completion of the reaction was monitored by TLC, 20 mL of water was added, and the mixture was extracted with EA twice. The organic phases were combined, washed with saturated brine once, dried over anhydrous Na₂SO₄, filtered, spin-dried, and then separated by column chromatography to provide 540 mg of a yellow oil (64%). ¹H NMR (600 MHz, DMSO-*d*₆) δ 7.38 (d, *J =* 3.9 Hz, 4H), 7.35 - 7.30 (m, 1H), 7.07 (d, *J =* 8.6 Hz, 2H), 6.86 (d, *J =* 8.6 Hz, 2H), 5.10 (s, 2H), 4.57 (t, *J =* 5.2 Hz, 1H), 3.63 - 3.46 (m, 6H), 3.09 - 2.99 (m, 3H), 2.62 (t, *J* = 7.2 Hz, 2H). LC-MS: calcd for C₂₀H₂₄N₂O₃ [M + H]⁺ 341.2, found 341.2.

### Step 2: Preparation of intermediate ii-13

Based on the intermediates ii-3 and ii-12, an intermediate containing a Cbz protecting group was obtained by referring to the above synthesis method of ii-4, and then the intermediate ii-13 was obtained through a conventional hydrogenation reduction reaction. ¹H NMR (600 MHz, DMSO-*d*₆) δ 8.57 (d, *J =* 2.7 Hz, 1H), 7.81 (s, 1H), 7.77 (d, *J* = 7.7 Hz, 1H), 7.35 (t, *J =* 7.6 Hz, 1H), 7.25 - 7.18 (m, 3H), 7.05 (d, *J =* 2.7 Hz, 1H), 6.94 (d, *J =* 8.7 Hz, 1H), 6.89 (s, 1H), 4.47 (t, *J* = 7.0 Hz, 2H), 3.75 - 3.63 (m, 8H), 3.41 - 3.26 (m, 8H), 3.22 - 3.13 (m, 4H), 2.97 (t, *J =* 7.0 Hz, 2H), 2.38 (s, 3H). LC-MS: calcd for C₃₀H₃₅N₇O₂ [M + H]⁺ 526.3, found 526.2.

### Step 3: Preparation of LCG-II-11

Based on the intermediate ii-13, the method for preparing the LCG-50 in Example 50 was referred to for the synthesis. ¹H NMR (600 MHz, DMSO-*d*₆) δ 8.96 (s, 1H), 8.57 (d, *J =* 2.7 Hz, 1H), 8.53 (d, *J =* 7.9 Hz, 1H), 7.81 (s, 1H), 7.77 (d, *J =* 7.7 Hz, 1H), 7.44 - 7.39 (m, 4H), 7.35 (t, *J* = 7.6 Hz, 1H), 7.26 (dd, *J =* 9.2, 2.8 Hz, 1H), 7.21 (d, *J =* 7.1 Hz, 1H), 7.15 (d, *J =* 8.5 Hz, 2H), 7.05 (d, *J* = 2.7 Hz, 1H), 6.89 (s, 1H), 6.85 (d, *J =* 8.7 Hz, 2H), 5.21 (q, *J =* 7.2 Hz, 1H), 5.14 (s, 1H), 4.57 (d, *J =* 9.8 Hz, 1H), 4.46 (q, *J =* 7.7, 6.9 Hz, 3H), 4.28 (s, 1H), 3.72 - 3.44 (m, 15H), 3.08 - 3.00 (m, 2H), 3.00 - 2.85 (m, 5H), 2.82 - 2.72 (m, 1H), 2.39 (s, 3H), 2.38 (s, 3H), 2.10 - 2.01 (m, 1H), 1.82 - 1.71 (m, 1H), 1.42 - 1.30 (m, 2H), 1.21 (dd, *J =* 8.5, 3.3 Hz, 2H), 0.96 (s, 9H). ¹³C NMR (150 MHz, DMSO-*d*₆) δ 169.89, 168.22, 167.47, 167.34, 167.17, 164.10, 163.46, 157.52, 152.98, 150.91, 150.88, 148.73, 147.19, 147.18, 141.65, 137.34, 131.33, 130.40, 129.43, 128.82, 128.79, 128.73, 128.68, 128.34, 128.12, 128.04, 126.92, 126.66, 125.69, 122.48, 115.47, 115.44, 105.32, 81.52, 78.27, 76.73, 68.16, 66.79, 65.19, 64.30, 58.16, 55.94, 55.89, 49.39, 48.26, 47.90, 45.14, 44.47, 43.68, 40.33, 37.91, 37.02, 35.43, 33.18, 25.65, 25.61, 20.44, 15.32, 14.56, 12.39, 12.32, 12.11, 12.05, 8.02. HRMS (ESI) calcd for C₅₈H₆₈FN₁₁O₇S [M + H]⁺ 1082.5086, found 1082.5083.

### Example 76: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-4-(4-(4-(2-((4-morpholino-6-(3-(m-tolyl)-1H-pyrazol-1-yl)pyrimidin-2-yl)oxy)ethyl)phenyl)piperazin-1-yl)-4-oxobutyl)pyrrolidine-2-carboxamide (LCG-II-12)

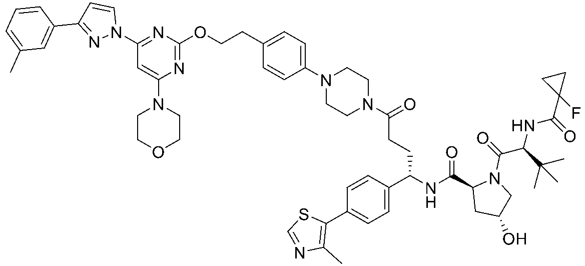

The method for preparing the LCG-II-11 in Example 75 was referred to for the synthesis. ¹H NMR (600 MHz, DMSO-*d*₆) δ 8.99 (s, 1H), 8.57 (d, *J* = 2.7 Hz, 1H), 8.55 (d, *J* = 8.8 Hz, 1H), 7.82 (s, 1H), 7.77 (d, *J =* 7.8 Hz, 1H), 7.46 (d, *J =* 8.3 Hz, 2H), 7.40 (d, *J =* 8.3 Hz, 2H), 7.35 (t, *J =* 7.6 Hz, 1H), 7.25 - 7.13 (m, 4H), 7.05 (d, *J =* 2.7 Hz, 1H), 6.90 (d, *J =* 8.1 Hz, 3H), 5.16 (brs, 1H), 4.98 - 4.90 (m, 1H), 4.57 (d, *J =* 9.2 Hz, 1H), 4.51 - 4.43 (m, 3H), 4.31 (s, 1H), 3.75 - 3.49 (m, 14H), 3.20 - 2.98 (m, 4H), 2.96 (t, *J =* 7.0 Hz, 2H), 2.75 - 2.70 (m, 1H), 2.46 (s, 3H), 2.40 - 2.35 (m, 4H), 2.06 - 1.96 (m, 2H), 1.94 - 1.84 (m, 1H), 1.81 - 2.35 (m, 1H), 1.41 - 1.28 (m, 2H), 1.24 - 1.19 (m, 2H), 0.98 (s, 9H). ¹³C NMR (150 MHz, DMSO-*d*₆) δ 170.25, 169.79, 168.23, 167.43, 167.30, 164.11, 163.48, 157.53, 152.99, 150.91, 148.79, 147.22, 142.99, 137.36, 131.34, 130.48, 129.26, 128.84, 128.75, 128.67, 128.45, 128.30, 128.05, 126.12, 125.70, 122.49, 115.50, 105.33, 81.53, 78.41, 76.87, 68.27, 66.81, 65.21, 58.35, 56.15, 56.04, 50.76, 48.37, 48.17, 43.91, 43.69, 40.30, 37.17, 35.59, 33.19, 31.67, 28.76, 25.66, 25.59, 20.46, 15.42, 12.41, 12.34, 12.09, 12.02, 10.66. HRMS (ESI) calcd for C₅₉H₇₀FN₁₁O₇S [M + H]⁺ 1096.5242, found 1096.5243.

### Example 77: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-6-(4-(4-(2-((4-morpholino-6-(3-(m-tolyl)-1H-pyrazol-1-yl)pyrimidin-2-yl)oxy)ethyl)phenyl)piperazin-1-yl)-6-oxohexyl)pyrrolidine-2-carboxamide (LCG-II-13)

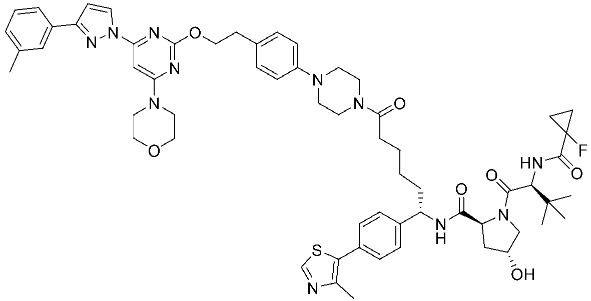

The method for preparing the LCG-II-11 in Example 75 was referred to for the synthesis. ¹H NMR (600 MHz, DMSO-*d*₆) δ 8.98 (s, 1H), 8.57 (d, *J =* 2.7 Hz, 1H), 8.46 (d, *J =* 8.4 Hz, 1H), 7.81 (s, 1H), 7.77 (d, *J =* 7.9 Hz, 1H), 7.43 (d, *J =* 8.2 Hz, 2H), 7.38 (d, *J =* 8.3 Hz, 2H), 7.35 (t, *J* = 7.6 Hz, 1H), 7.26 (dd, *J* = 9.2, 2.8 Hz, 1H), 7.21 (d, *J =* 7.5 Hz, 1H), 7.18 (d, *J =* 8.5 Hz, 2H), 7.04 (d, *J =* 2.7 Hz, 1H), 6.92 - 6.86 (m, 3H), 5.13 (brs, 1H), 4.81 (td, *J=* 8.4, 6.1 Hz, 1H), 4.58 (dd, *J =* 9.3, 1.2 Hz, 1H), 4.50 (t, *J =* 8.2 Hz, 1H), 4.46 (t, *J =* 7.1 Hz, 2H), 4.29 (s, 1H), 3.72 - 3.64 (m, 8H), 3.63 - 3.51 (m, 6H), 3.12 - 2.98 (m, 4H), 2.95 (t, *J =* 7.1 Hz, 2H), 2.45 (s, 3H), 2.38 (s, 3H), 2.33 (t, *J =* 7.5 Hz, 2H), 2.07 - 2.00 (m, 1H), 1.77 - 1.65 (m, 3H), 1.57 - 1.48 (m, 2H), 1.49 - 1.41 (m, 1H), 1.40 - 1.28 (m, 3H), 1.21 (dd, *J =* 8.5, 3.1 Hz, 2H), 0.96 (s, 9H). ¹³C NMR (150 MHz, DMSO-*d*₆) δ 170.08, 169.96, 168.10, 167.45, 167.32, 164.10, 163.48, 157.52, 152.99, 150.87, 148.81, 147.15, 143.24, 137.35, 131.34, 130.51, 129.12, 128.85, 128.74, 128.67, 128.42, 128.19, 128.05, 126.24, 125.70, 122.49, 115.51, 105.32, 81.53, 78.33, 76.79, 68.23, 66.80, 65.20, 64.32, 58.09, 56.05, 55.96, 51.34, 48.49, 48.08, 45.13, 44.15, 43.68, 40.20, 37.13, 35.61, 35.53, 33.21, 31.62, 25.73, 25.62, 24.88, 23.91, 20.46, 15.42, 14.57, 12.40, 12.33, 12.11, 12.04, 8.03. HRMS (ESI) calcd for C₆₁H₇₄FN₁₁O₇S [M + H]⁺ 1124.5555, found 1124.5555.

### Example 78: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-3-(4-(3-(4-(2-((4-morpholino-6-(3-(m-tolyl)-1H-pyrazol-1-yl)pyrimidin-2-yl)oxy)ethyl)phenyl)ureido)piperidin-1-yl)-3-oxopropyl) pyrrolidine-2-carboxamide (LCG-II-14)

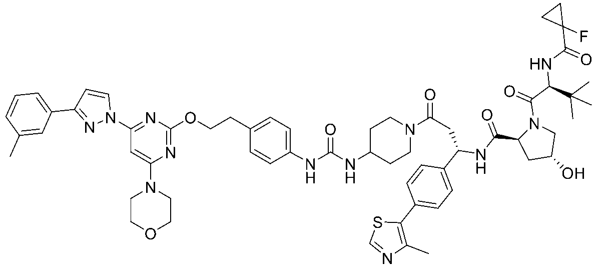

The method for preparing the LCG-II-9 in Example 73 was referred to for the synthesis. ¹H NMR (600 MHz, DMSO-*d*₆) δ 8.98 (d, *J =* 8.5 Hz, 1H), 8.57 (d, *J =* 2.7 Hz, 1H), 8.48 (dd, *J =* 14.9, 8.0 Hz, 1H), 8.27 (d, *J =* 20.7 Hz, 1H), 7.81 (s, 1H), 7.77 (d, *J =* 7.7 Hz, 1H), 7.46 - 7.37 (m, 4H), 7.35 (t, *J =* 7.6 Hz, 1H), 7.34 - 7.25 (m, 3H), 7.21 (d, *J =* 7.5 Hz, 1H), 7.17 (d, *J =* 8.5 Hz, 2H), 7.05 (d, *J =* 2.7 Hz, 1H), 6.89 (s, 1H), 6.21 - 6.04 (m, 1H), 5.25 - 5.16 (m, 1H), 5.15 (s, 1H), 4.58 (d, *J =* 9.2 Hz, 1H), 4.46 (q, *J* = 8.7, 7.9 Hz, 3H), 4.29 (s, 1H), 4.18 - 4.05 (m, 1H), 3.80 - 3.52 (m, 12H), 3.22 - 3.04 (m, 1H), 2.96 (t, *J =* 7.1 Hz, 2H), 2.93 - 2.75 (m, 3H), 2.46 (d, *J =* 3.3 Hz, 3H), 2.38 (s, 3H), 2.04 (dd, *J =* 12.9, 7.9 Hz, 1H), 1.85 - 1.67 (m, 3H), 1.47 - 1.15 (m, 6H), 0.98 (s, 9H). ¹³C NMR (150 MHz, DMSO-*d*₆) δ 169.92, 169.90, 168.23, 167.51, 167.38, 166.97, 164.09, 163.46, 157.51, 153.90, 153.88, 152.97, 150.93, 150.90, 147.19, 141.79, 141.74, 138.10, 137.34, 131.33, 130.46, 130.03, 129.41, 129.38, 128.73, 128.69, 128.49, 128.12, 128.08, 128.04, 126.91, 126.73, 126.67, 125.69, 122.48, 117.17, 105.32, 81.53, 78.26, 76.72, 68.18, 68.16, 66.72, 66.30, 65.19, 64.30, 58.18, 55.94, 49.37, 49.16, 45.48, 45.38, 43.67, 43.22, 43.13, 37.96, 37.02, 35.71, 35.42, 33.39, 31.87, 31.02, 25.62, 20.44, 15.37, 15.34, 14.55, 12.39, 12.32, 12.13, 12.06. HRMS (ESI) calcd for C₆₀H₇₁FN₁₂O₈S [M + H]⁺ 1139.5301, found 1139.5303.

### Example 79: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-4-(4-(3-(4-(2-((4-morpholino-6-(3-(m-tolyl)-1H-pyrazol-1-yl)pyrimidin-2-yl)oxy)ethyl)phenyl)ureido)piperidin-1-yl)-4-oxobutyl) pyrrolidine-2-carboxamide (LCG-II-15)

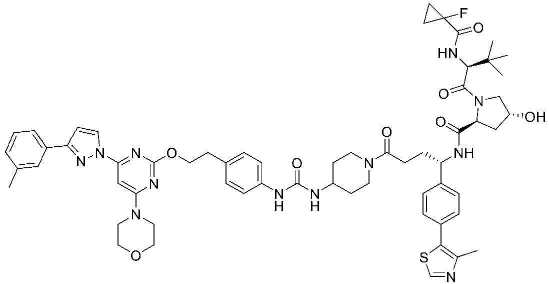

The method for preparing the LCG-II-9 in Example 73 was referred to for the synthesis. ¹H NMR (600 MHz, DMSO-*d*₆) δ 8.99 (s, 1H), 8.57 (d, *J =* 2.7 Hz, 1H), 8.55 (dd, *J =* 8.9, 4.9 Hz, 1H), 8.28 (d, *J =* 13.3 Hz, 1H), 7.82 (s, 1H), 7.77 (d, *J =* 7.7 Hz, 1H), 7.47 (d, *J = 8.2* Hz, 2H), 7.41 (d, *J* = 7.9 Hz, 2H), 7.35 (t, *J =* 7.6 Hz, 1H), 7.32 (d, *J =* 8.3 Hz, 2H), 7.19 (dd, *J =* 20.2, 7.8 Hz, 4H), 7.05 (d, *J =* 2.7 Hz, 1H), 6.89 (s, 1H), 6.14 (dd, *J =* 35.1, 7.6 Hz, 1H), 5.17 (s, 1H), 4.93 (td, *J =* 10.1, 4.4 Hz, 2H), 4.58 (dd, *J =* 9.5, 3.2 Hz, 1H), 4.52 - 4.44 (m, 3H), 4.32 (s, 1H), 4.19 (dd, *J* = 58.7, 12.8 Hz, 1H), 3.88 (s, 1H), 3.74 - 3.55 (m, 11H), 3.23 - 3.07 (m, 1H), 2.97 (t, *J* = 7.1 Hz, 2H), 2.91 - 2.66 (m, 2H), 2.47 (s, 3H), 2.38 (s, 3H), 2.37 - 2.27 (m, 1H), 2.04 (dd, *J =* 12.8, 7.6 Hz, 1H), 1.99 - 1.73 (m, 5H), 1.43 - 1.12 (m, 6H), 0.98 (s, 9H). ¹³C NMR (150 MHz, DMSO-*d*₆) δ 170.26, 170.22, 169.61, 169.53, 168.22, 168.19, 167.49, 167.46, 167.36, 167.32, 164.10, 163.47, 157.53, 153.91, 153.89, 152.99, 150.91, 147.22, 143.09, 143.02, 138.20, 138.15, 137.36, 131.35, 130.48, 130.04, 130.01, 129.25, 128.74, 128.69, 128.49, 128.30, 128.05, 126.12, 125.71, 122.49, 117.21, 117.17, 117.12, 105.33, 81.54, 78.36, 78.23, 76.82, 76.69, 68.28, 66.75, 65.21, 64.32, 58.40, 58.35, 56.17, 56.13, 55.96, 50.87, 50.69, 45.59, 45.45, 43.68, 42.76, 37.16, 35.66, 35.58, 33.40, 32.22, 32.00, 31.79, 31.26, 31.19, 29.00, 28.77, 25.64, 25.58, 25.55, 20.46, 15.44, 15.41, 14.57, 12.38, 12.31, 12.23, 12.11, 12.04, 11.97. HRMS (ESI) calcd for C₆₁H₇₃FN₁₂O₈S [M + H]⁺ 1153.5458, found 1153.5457.

### Example 80: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-3-(3-(3-(4-(4-((4-morpholino-6-(3-(m-tolyl)-1H-pyrazol-1-yl)pyrimidin-2-yl)oxy)piperidin-1-yl)phenyl)ureido)azetidin-1-yl)-3-oxopropyl)pyrrolidine-2-carboxamide (LCG-II-16)

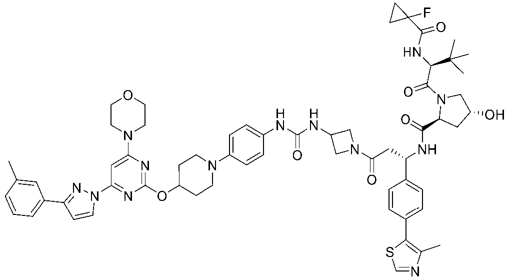

The method for preparing the LCG-II-3 in Example 67 and the method for preparing the LCG-II-9 in Example 73 were referred to for the synthesis. ¹H NMR (600 MHz, DMSO-*d*₆) δ 8.99 (d, *J* = 7.2 Hz, 1H), 8.60 (d, *J =* 2.6 Hz, 1H), 8.53 (dd, *J =* 11.8, 8.1 Hz, 1H), 8.27 (d, *J =* 23.1 Hz, 1H), 7.82 (s, 1H), 7.78 (d, *J =* 7.7 Hz, 1H), 7.48 - 7.42 (m, 2H), 7.40 (dd, *J =* 8.3, 1.7 Hz, 2H), 7.35 (t, *J =* 7.7 Hz, 1H), 7.30 (ddd, *J =* 24.0, 9.3, 2.8 Hz, 1H), 7.25 - 7.19 (m, 3H), 7.06 (d, *J =* 2.7 Hz, 1H), 6.90 (s, 3H), 6.67 - 6.58 (m, 1H), 5.20 - 5.06 (m, 3H), 4.59 (d, *J =* 9.2 Hz, 1H), 4.49 - 4.43 (m, 1H), 4.41 - 4.35 (m, 1H), 4.34 - 3.73 (m, 4H), 3.72 - 3.66 (m, 8H), 3.65 - 3.54 (m, 3H), 3.48 - 3.41 (m, 2H), 2.97 (brs, 2H), 2.70 - 2.59 (m, 1H), 2.57 - 2.52 (m, 1H), 2.46 (d, *J =* 7.1 Hz, 3H), 2.39 (s, 3H), 2.12 (brs, 2H), 2.09 - 2.01 (m, 1H), 1.92 - 1.73 (m, 3H), 1.41 - 1.29 (m, 2H), 1.24 - 1.19 (m, 2H), 0.98 (s, 9H). ¹³C NMR (150 MHz, DMSO-*d*₆) δ 170.37, 169.18, 169.13, 168.74, 168.73, 168.04, 168.00, 167.90, 167.87, 164.71, 163.38, 158.05, 154.70, 153.49, 151.46, 151.42, 147.74, 147.73, 142.34, 142.20, 137.85, 131.84, 130.94, 129.97, 129.92, 129.24, 128.65, 128.55, 126.98, 126.94, 126.20, 122.99, 119.31, 116.71, 105.84, 81.98, 78.78, 78.75, 77.24, 77.20, 71.90, 68.68, 66.82, 65.73, 64.81, 58.67, 58.66, 57.47, 57.31, 56.47, 54.65, 54.51, 49.29, 49.27, 47.17, 44.19, 37.59, 37.57, 37.49, 35.96, 35.94, 30.31, 26.14, 20.95, 15.90, 15.07, 12.90, 12.83, 12.63, 12.57. HRMS (ESI) calcd for C₆₁H₇₂FN₁₃O₈S [M + H]⁺ 1166.5410, found 1166.5414.

### Example 81: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-4-(3-(3-(4-(4-((4-morpholino-6-(3-(m-tolyl)-1H-pyrazol-1-yl)pyrimidin-2-yl)oxy)piperidin-1-yl)phenyl)ureido)azetidin-1-yl)-4-oxobutyl)pyrrolidine-2-carboxamide (LCG-II-17)

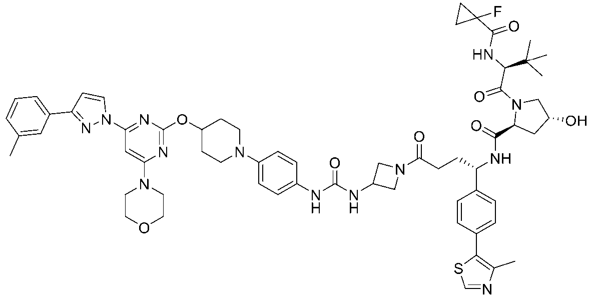

The method for preparing the LCG-II-3 in Example 67 and the method for preparing the LCG-II-9 in Example 73 were referred to for the synthesis. ¹H NMR (600 MHz, DMSO-*d*₆) δ 8.99 (d, *J* = 1.0 Hz, 1H), 8.60 (d, *J =* 2.6 Hz, 1H), 8.52 (d, *J =* 8.5 Hz, 1H), 8.26 (brs, 1H), 7.82 (s, 1H), 7.78 (d, *J =* 7.8 Hz, 1H), 7.46 (d, *J =* 8.1 Hz, 2H), 7.38 (dd, *J =* 8.2, 3.4 Hz, 2H), 7.35 (t, *J =* 7.6 Hz, 1H), 7.30 - 7.19 (m, 4H), 7.06 (d, *J =* 2.7 Hz, 1H), 6.90 (s, 3H), 6.67 (brs, 1H), 5.14 (d, *J =* 12.7 Hz, 2H), 4.88 - 4.79 (m, 1H), 4.59 (d, *J =* 9.2 Hz, 1H), 4.50 - 4.44 (m, 1H), 4.44 - 4.31 (m, 2H), 4.29 (s, 1H), 4.13 - 4.00 (m, 1H), 3.95 - 3.84 (m, 1H), 3.69 (d, *J =* 9.9 Hz, 9H), 3.62 (dd, *J =* 10.8, 3.7 Hz, 1H), 3.57 (d, *J =* 10.9 Hz, 1H), 3.48 - 3.40 (m, 2H), 2.97 (brs, 2H), 2.46 (d, *J =* 2.2 Hz, 3H), 2.39 (s, 3H), 2.33 - 2.00 (m, 5H), 1.95 - 1.73 (m, 5H), 1.45 - 1.28 (m, 2H), 1.26 - 1.16 (m, 2H), 0.98 (d, *J =* 6.0 Hz, 9H). ¹³C NMR (150 MHz, DMSO-*d*₆) δ 171.81, 171.68, 170.73, 168.70, 168.66, 168.02, 167.98, 167.89, 167.85, 164.71, 163.38, 158.05, 154.69, 153.49, 151.40, 147.71, 143.37, 143.34, 137.85, 131.84, 130.96, 129.76, 129.24, 128.76, 128.54, 126.64, 126.20, 122.99, 119.32, 116.70, 105.84, 81.98, 78.73, 78.71, 77.19, 71.91, 68.71, 65.72, 64.81, 58.72, 58.68, 57.32, 57.25, 56.56, 56.47, 56.44, 54.70, 51.51, 51.33, 47.18, 44.19, 37.68, 36.00, 35.95, 31.48, 31.34, 30.31, 27.67, 27.42, 26.21, 26.19, 26.11, 26.08, 20.95, 15.92, 15.06, 12.88, 12.81, 12.60, 12.53. HRMS (ESI) calcd for C₆₂H₇₄FN₁₃O₈S [M + H]⁺ 1180.5566, found 1180.5566.

### Example 82: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-3-(4-(3-(4-(4-((4-morpholino-6-(3-(m-tolyl)-1H-pyrazol-1-yl)pyrimidin-2-yl)oxy)piperidin-1-yl)phenyl)ureido)piperidin-1-yl)-3-oxopropyl)pyrrolidine-2-carboxamide (LCG-II-18)

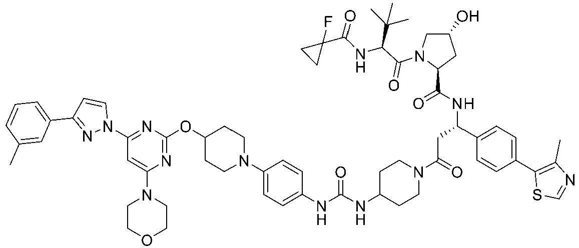

The method for preparing the LCG-II-3 in Example 67 and the method for preparing the LCG-II-9 in Example 73 were referred to for the synthesis. ¹H NMR (600 MHz, DMSO-*d*₆) δ 8.98 (d, *J* = 6.4 Hz, 1H), 8.60 (d, *J* = 2.7 Hz, 1H), 8.48 (dd, *J =* 15.2, 8.0 Hz, 1H), 8.05 (d, *J =* 18.0 Hz, 1H), 7.82 (s, 1H), 7.78 (d, *J=* 7.7 Hz, 1H), 7.47 - 7.40 (m, 4H), 7.35 (t, *J =* 7.6 Hz, 1H), 7.33 - 7.28 (m, 1H), 7.22 (t, *J =* 7.8 Hz, 3H), 7.06 (d, *J = 2.7* Hz, 1H), 6.89 (d, *J* = 12.7 Hz, 3H), 6.01 (dd, *J* = 46.4, 6.7 Hz, 1H), 5.27 - 5.09 (m, 3H), 4.59 (d, *J* = 9.2 Hz, 1H), 4.46 (t, *J =* 8.2 Hz, 1H), 4.29 (s, 1H), 4.11 (dd, *J =* 25.5, 12.9 Hz, 1H), 3.81 - 3.53 (m, 12H), 3.47 - 3.40 (m, 2H), 3.22 - 3.06 (m, 1H), 3.02 - 2.74 (m, 5H), 2.46 (d, *J =* 2.0 Hz, 3H), 2.39 (s, 3H), 2.12 (brs, 2H), 2.05 (dd, *J =* 12.9, 8.0 Hz, 1H), 1.88 - 1.67 (m, 5H), 1.44 - 1.30 (m, 2H), 1.26 - 1.17 (m, 2H), 0.98 (s, 9H). ¹³C NMR (150 MHz, DMSO-*d*₆) δ 170.43, 170.40, 168.74, 168.02, 167.89, 167.47, 164.71, 163.39, 158.05, 154.59, 153.49, 151.43, 147.70, 145.80, 142.30, 142.24, 137.85, 132.55, 131.84, 130.97, 129.88, 129.24, 128.63, 128.59, 128.55, 127.42, 127.24, 127.18, 126.20, 122.99, 118.88, 116.83, 105.84, 81.97, 78.77, 77.22, 71.93, 68.69, 68.67, 66.81, 65.73, 64.81, 58.69, 56.45, 56.41, 49.88, 49.67, 47.25, 45.99, 45.89, 44.19, 43.76, 43.66, 38.47, 37.53, 36.23, 35.93, 32.46, 31.61, 30.35, 26.13, 20.95, 15.88, 15.85, 15.07, 12.90, 12.83, 12.64, 12.57. HRMS (ESI) calcd for C₆₃H₇₆FN₁₃O₈S [M + H]⁺ 1194.5723, found 1194.5726.

### Example 83: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-4-(4-(3-(4-(4-((4-morpholino-6-(3-(m-tolyl)-1H-pyrazol-1-yl)pyrimidin-2-yl)oxy)piperidin-1-yl)phenyl)ureido)piperidin-1-yl)-4-oxobutyl)pyrrolidine-2-carboxamide (LCG-II-19)

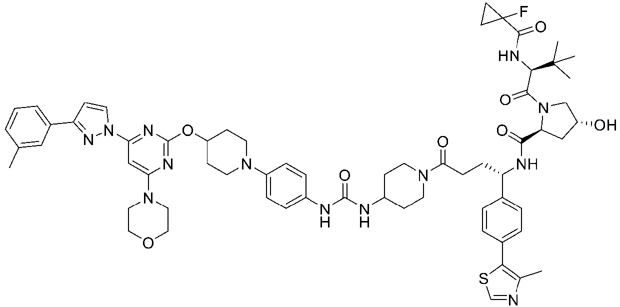

The method for preparing the LCG-II-3 in Example 67 and the method for preparing the LCG-II-9 in Example 73 were referred to for the synthesis. ¹H NMR (600 MHz, DMSO-*d*₆) δ 8.99 (s, 1H), 8.60 (d, *J* = 2.7 Hz, 1H), 8.57 - 8.51 (m, 1H), 8.05 (d, *J =* 12.6 Hz, 1H), 7.82 (s, 1H), 7.78 (d, *J =* 7.7 Hz, 1H), 7.47 (d, *J =* 8.3 Hz, 2H), 7.41 (d, *J =* 7.9 Hz, 2H), 7.35 (t, *J =* 7.6 Hz, 1H), 7.26 - 7.16 (m, 4H), 7.06 (d, *J =* 2.7 Hz, 1H), 6.89 (d, *J =* 10.8 Hz, 3H), 6.02 (dd, *J =* 36.2, 7.7 Hz, 1H), 5.16 (d, *J =* 3.4 Hz, 1H), 5.13 - 5.08 (m, 1H), 4.97 - 4.88 (m, 1H), 4.58 (d, *J =* 8.9 Hz, 1H), 4.49 - 4.44 (m, 1H), 4.31 (s, 1H), 4.19 (dd, *J =* 57.6, 12.9 Hz, 1H), 3.87 (brs, 1H), 3.75 - 3.55 (m, 12H), 3.51 - 3.40 (m, 2H), 3.22 - 3.07 (m, 1H), 2.96 (t, *J =* 9.4 Hz, 2H), 2.89 - 2.64 (m, 2H), 2.47 (s, 3H), 2.39 (s, 3H), 2.37 - 2.26 (m, 1H), 2.12 (brs, 2H), 2.04 (dd, *J =* 12.8, 7.6 Hz, 1H), 1.99 - 1.72 (m, 7H), 1.41 - 1.14 (m, 6H), 0.99 (s, 9H). ¹³C NMR (150 MHz, DMSO-*d*₆) δ 170.75, 170.71, 170.09, 170.01, 168.70, 168.68, 167.98, 167.83, 164.71, 163.39, 158.05, 154.58, 153.49, 151.41, 147.72, 145.79, 143.58, 143.52, 137.85, 132.64, 132.58, 131.85, 130.97, 129.75, 129.24, 128.79, 128.75, 128.55, 126.61, 126.20, 122.99, 118.86, 118.79, 116.83, 105.84, 81.97, 78.87, 78.74, 77.32, 77.20, 71.93, 68.77, 65.73, 64.81, 58.88, 58.83, 56.66, 56.46, 51.36, 51.20, 47.26, 46.09, 45.94, 44.19, 43.28, 37.65, 36.14, 36.07, 32.77, 32.56, 32.49, 32.29, 31.85, 31.77, 30.35, 29.49, 29.25, 26.16, 26.07, 26.05, 20.95, 15.93, 15.91, 15.07, 12.89, 12.82, 12.55. HRMS (ESI) calcd for C₆₄H₇₈FN₁₃O₈S [M + H]⁺ 1208.5879, found 1208.5874.

### Example 84: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-3-(4-(4-(4-((4-morpholino-6-(3-(m-tolyl)-1H-pyrazol-1-yl)pyrimidin-2-yl)oxy)piperidin-1-yl)phenyl)piperazin-1-yl)-3-oxopropyl) pyrrolidine-2-carboxamide (LCG-II-20)

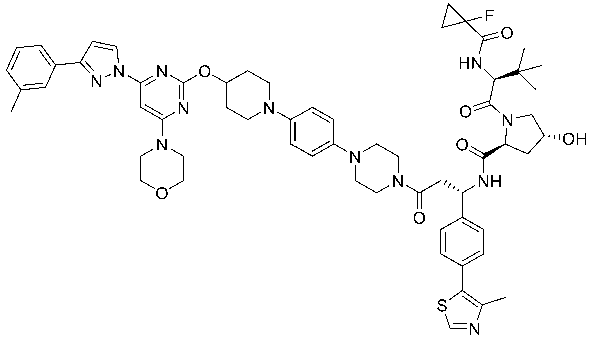

The method for preparing the LCG-II-3 in Example 67 was referred to for the synthesis. ¹H NMR (600 MHz, DMSO-*d*₆) δ 8.98 (s, 1H), 8.60 (d, *J= 2.7* Hz, 1H), 8.53 (d, *J* = 7.9 Hz, 1H), 7.82 (s, 1H), 7.78 (d, *J =* 7.8 Hz, 1H), 7.45 - 7.38 (m, 4H), 7.35 (t, *J =* 7.6 Hz, 1H), 7.27 (dd, *J =* 9.3, 2.8 Hz, 1H), 7.21 (dt, *J =* 7.5, 1.0 Hz, 1H), 7.06 (d, *J =* 2.7 Hz, 1H), 6.92 - 6.75 (m, 5H), 5.21 (q, *J=* 7.2 Hz, 1H), 5.17 - 5.09 (m, 2H), 4.58 (d, *J =* 9.3 Hz, 1H), 4.46 (t, *J =* 8.2 Hz, 1H), 4.28 (brs, 1H), 3.73 - 3.36 (m, 17H), 3.01 - 2.62 (m, 8H), 2.40 (s, 3H), 2.39 (s, 3H), 2.12 (brs, 2H), 2.05 (dd, *J=* 12.3, 8.4 Hz, 1H), 1.86 - 1.73 (m, 2H), 1.41 - 1.31 (m, 2H), 1.22 (dd, *J* = 8.4, 3.3 Hz, 2H), 0.97 (s, 9H). ¹³C NMR (150 MHz, DMSO-*d*₆) δ 170.40, 168.73, 167.98, 167.85, 164.71, 163.39, 158.05, 153.49, 151.41, 147.70, 142.15, 137.85, 131.84, 130.91, 129.94, 129.24, 128.63, 128.55, 127.18, 126.20, 122.99, 117.35, 105.84, 81.98, 78.79, 77.25, 68.67, 65.72, 58.67, 56.44, 49.92, 47.39, 45.62, 44.19, 38.40, 37.53, 36.24, 35.95, 26.16, 26.12, 20.95, 15.88, 15.85, 12.90, 12.83, 12.62, 12.55, 8.52. HRMS (ESI) calcd for C₆₁H₇₃FN₁₂O₇S [M + H]⁺ 1137.5508, found 1137.5508.

### Example 85: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-4-(4-(4-(4-((4-morpholino-6-(3-(m-tolyl)-1H-pyrazol-1-yl)pyrimidin-2-yl)oxy)piperidin-1-yl)phenyl)piperazin-1-yl)-4-oxobutyl) pyrrolidine-2-carboxamide (LCG-II-21)

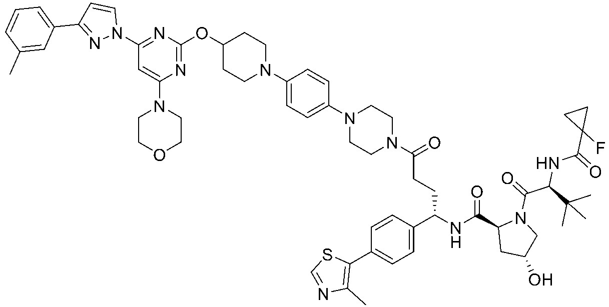

The method for preparing the LCG-II-3 in Example 67 was referred to for the synthesis. ¹H NMR (600 MHz, DMSO-*d*₆) δ 8.99 (s, 1H), 8.60 (d, *J =* 2.7 Hz, 1H), 8.55 (d, *J =* 8.7 Hz, 1H), 7.82 (s, 1H), 7.78 (d, *J* = 7.8 Hz, 1H), 7.47 (d, *J =* 8.2 Hz, 2H), 7.41 (d, *J =* 8.3 Hz, 2H), 7.35 (t, *J =* 7.6 Hz, 1H), 7.26 - 7.18 (m, 2H), 7.06 (d, *J =* 2.7 Hz, 1H), 6.93 - 6.79 (m, 5H), 5.16 (d, *J =* 3.2 Hz, 1H), 5.12 (brs, 1H), 4.96 - 4.91 (m, 1H), 4.58 (d, *J* = 9.2 Hz, 1H), 4.51 - 4.45 (m, 1H), 4.31 (brs, 1H), 3.76 - 3.49 (m, 15H), 3.40 (brs, 2H), 3.05 - 2.87 (m, 5H), 2.78 - 2.66 (m, 1H), 2.47 (s, 3H), 2.40 - 2.35 (m, 4H), 2.12 (brs, 2H), 2.07 - 1.73 (m, 6H), 1.42 - 1.29 (m, 2H), 1.26 - 1.21 (m, 2H), 0.98 (s, 9H). ¹³C NMR (150 MHz, DMSO-*d*₆) δ 170.74, 170.26, 168.71, 167.94, 167.80, 164.71, 163.40, 158.05, 153.49, 151.41, 147.72, 143.50, 137.85, 131.84, 130.97, 129.75, 129.23, 128.79, 128.55, 126.62, 126.20, 122.99, 117.37, 105.84, 81.98, 78.92, 77.38, 71.92, 68.77, 65.72, 64.81, 58.84, 56.65, 56.52, 51.27, 47.41, 45.60, 44.19, 37.67, 36.08, 32.18, 30.39, 29.26, 26.16, 26.09, 20.95, 15.92, 15.07, 12.93, 12.86, 12.61, 12.55, 8.51. HRMS (ESI) calcd for C₆₂H₇₅FN₁₂O₇S [M + H]⁺ 1151.5664, found 1151.5664.

### Example 86: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-6-(4-(4-(4-((4-morpholino-6-(3-(m-tolyl)-1H-pyrazol-1-yl)pyrimidin-2-yl)oxy)piperidin-1-yl)phenyl)piperazin-1-yl)-6-oxohexyl) pyrrolidine-2-carboxamide (LCG-II-22)

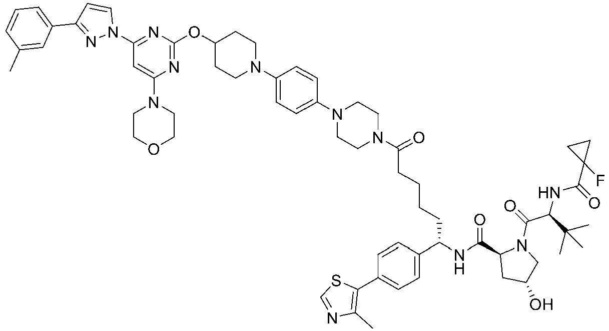

The method for preparing the LCG-II-3 in Example 67 was referred to for the synthesis. ¹H NMR (600 MHz, DMSO-*d*₆) δ 8.99 (s, 1H), 8.60 (d, *J* = 2.6 Hz, 1H), 8.46 (d, *J=* 8.3 Hz, 1H), 7.82 (s, 1H), 7.78 (d, *J =* 7.8 Hz, 1H), 7.43 (d, *J =* 8.2 Hz, 2H), 7.39 - 7.34 (m, 3H), 7.26 (dd, *J= 9.2,* 2.5 Hz, 1H), 7.21 (d, *J =* 7.5 Hz, 1H), 7.06 (d, *J =* 2.7 Hz, 1H), 6.88 (d, *J =* 23.2 Hz, 5H), 5.22 - 5.08 (m, 2H), 4.81 (q, *J =* 8.2 Hz, 1H), 4.58 (d, *J =* 9.3 Hz, 1H), 4.50 (t, *J =* 8.2 Hz, 1H), 4.29 (s, 1H), 3.78 - 3.62 (m, 8H), 3.58 (dd, *J =* 17.4, 6.8 Hz, 6H), 3.44 - 3.37 (m, 2H), 2.99 - 2.85 (m, 5H), 2.46 (s, 3H), 2.39 (s, 3H), 2.33 (t, *J =* 7.5 Hz, 2H), 2.12 (s, 2H), 2.09 - 2.00 (m, 1H), 1.87 - 1.64 (m, 5H), 1.57 - 1.42 (m, 3H), 1.41 - 1.31 (m, 3H), 1.22 (dd, *J =* 8.3, 2.5 Hz, 2H), 0.97 (s, 9H). ¹³C NMR (150 MHz, DMSO-*d*₆) δ 170.58, 170.43, 168.60, 167.95, 167.82, 164.71, 163.39, 158.05, 153.49, 151.37, 147.65, 143.73, 137.85, 131.84, 131.00, 129.62, 129.24, 128.69, 128.55, 126.74, 126.20, 122.99, 117.37, 105.84, 81.98, 78.83, 77.28, 71.92, 68.72, 65.72, 64.81, 58.58, 56.54, 56.45, 51.83, 49.99, 49.54, 47.41, 45.64, 44.19, 40.85, 37.62, 36.11, 36.02, 32.13, 30.39, 26.22, 26.12, 25.37, 24.42, 20.95, 15.95, 15.92, 15.07, 12.90, 12.84, 12.62, 12.55, 8.53. HRMS (ESI) calcd for C₆₄H₇₉FN₁₂O₇S [M + H]⁺ 1179.5977, found 1179.5983.

### Example 87: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-3-((4'-(2-((4-morpholino-6-(3-(m-tolyl)-1H-pyrazol-1-yl)pyrimidin-2-yl)oxy)ethyl)-[1,1'-biphenyl]-4-yl)amino)-3-oxopropyl)pyrrolidine-2-carboxamide (LCG-II-23)

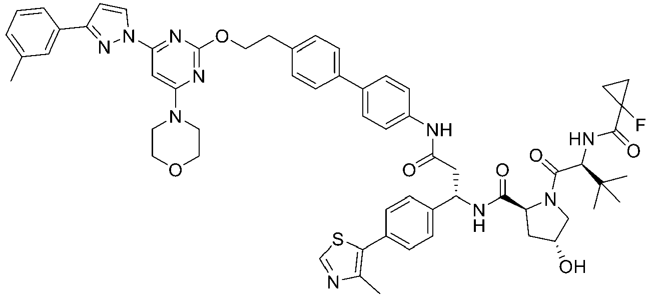

The method for preparing the LCG-II-6 in Example 70 was referred to for the synthesis. ¹H NMR (600 MHz, DMSO-*d*₆) δ 10.01 (s, 1H), 8.98 (s, 1H), 8.64 (d, *J* = 7.9 Hz, 1H), 8.58 (d, *J =* 2.7 Hz, 1H), 7.82 (s, 1H), 7.77 (d, *J =* 7.7 Hz, 1H), 7.62 (d, *J* = 8.7 Hz, 2H), 7.57 (d, *J =* 8.3 Hz, 4H), 7.44 (s, 4H), 7.39 (d, *J =* 8.1 Hz, 2H), 7.35 (t, *J =* 7.6 Hz, 1H), 7.26 (dd, *J* = 9.3, 2.8 Hz, 1H), 7.21 (d, *J* = 7.5 Hz, 1H), 7.05 (d, *J =* 2.7 Hz, 1H), 6.90 (s, 1H), 5.36 - 5.23 (m, 1H), 5.15 (d, *J =* 3.6 Hz, 1H), 4.59 (d, *J* = 9.3 Hz, 1H), 4.55 (t, *J =* 6.9 Hz, 2H), 4.49 (t, *J =* 8.3 Hz, 1H), 4.29 (brs, 1H), 3.73 - 3.65 (m, 8H), 3.64 - 3.54 (m, 2H), 3.08 (t, *J =* 6.8 Hz, 2H), 2.98 - 2.77 (m, 2H), 2.44 (s, 3H), 2.38 (s, 3H), 2.05 (dd, *J =* 12.2, 7.9 Hz, 1H), 1.81 - 1.73 (m, 1H), 1.42 - 1.30 (m, 2H), 1.25 - 1.18 (m, 2H), 0.98 (s, 9H). ¹³C NMR (150 MHz, DMSO-*d*₆) δ 170.47, 168.74, 168.01, 167.98, 167.84, 164.60, 163.96, 158.02, 153.49, 151.43, 147.72, 142.01, 138.17, 137.85, 137.72, 137.01, 134.67, 131.84, 130.91, 129.95, 129.36, 129.23, 128.63, 128.54, 127.02, 126.56, 126.20, 126.14, 122.99, 119.58, 105.84, 82.07, 78.80, 77.26, 68.69, 66.98, 65.70, 58.70, 56.51, 56.47, 49.73, 44.19, 42.64, 37.56, 35.97, 34.16, 26.18, 26.13, 20.95, 15.91, 12.91, 12.84, 12.62, 12.55. HRMS (ESI) calcd for C₆₀H₆₅FN₁₀O₇S [M + H]⁺ 1089.4820, found 1089.4816.

### Example 88: (2S,4R)-1-((S)-2-(1-fluorocyclopropane-1-carboxamido)-3,3-dimethylbutanoyl) -4-hydroxy-N-((S)-1-(4-(4-methylthiazol-5-yl)phenyl)-4-((4'-(2-((4-morpholino-6-(3-(m-tolyl)-1H-pyrazol-1-yl)pyrimidin-2-yl)oxy)ethyl)-[1,1'-biphenyl]-4-yl)amino)-4-oxobutyl)pyrrolidine-2-carboxamide (LCG-II-24)

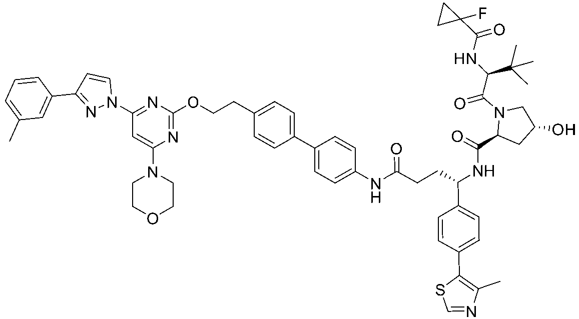

The method for preparing the LCG-II-6 in Example 70 was referred to for the synthesis. ¹H NMR (600 MHz, DMSO-*d*₆) δ 9.93 (s, 1H), 8.99 (s, 1H), 8.63 - 8.57 (m, 2H), 7.82 (s, 1H), 7.77 (d, *J* = 7.7 Hz, 1H), 7.67 (d, *J =* 8.5 Hz, 2H), 7.58 (d, *J =* 8.2 Hz, 4H), 7.47 (d, *J =* 8.3 Hz, 2H), 7.44 - 7.37 (m, 4H), 7.35 (t, *J* = 7.6 Hz, 1H), 7.28 (dd, *J* = 9.2*,* 2.8 Hz, 1H), 7.21 (d, *J =* 7.5 Hz, 1H), 7.05 (d, *J* = 2.7 Hz, 1H), 6.90 (s, 1H), 5.16 (d, *J* = 3.5 Hz, 1H), 4.89 (q, *J =* 8.1 Hz, 1H), 4.59 (d, *J* = 9.2 Hz, 1H), 4.55 (t, *J =* 6.9 Hz, 2H), 4.51 (t, *J =* 8.3 Hz, 1H), 4.29 (brs, 1H), 3.74 - 3.66 (m, 8H), 3.64 - 3.56 (m, 2H), 3.09 (t, *J =* 6.8 Hz, 2H), 2.47 (s, 3H), 2.45 - 2.40 (m, 1H), 2.38 (s, 3H), 2.09 - 2.01 (m, 3H), 1.82 - 1.73 (m, 1H), 1.41 - 1.32 (m, 2H), 1.27 - 1.18 (m, 2H), 0.97 (s, 9H). ¹³C NMR (150 MHz, DMSO-*d*₆) δ 170.76, 170.71, 168.75, 168.01, 167.87, 164.60, 163.96, 158.03, 153.50, 151.42, 147.71, 143.09, 138.49, 137.85, 137.76, 136.96, 134.38, 131.84, 130.97, 129.85, 129.36, 129.24, 129.22, 128.78, 128.54, 126.81, 126.55, 126.20, 126.12, 122.99, 119.31, 119.27, 105.84, 82.08, 78.80, 77.26, 68.71, 67.00, 65.70, 64.81, 58.69, 56.59, 56.57, 56.52, 51.87, 45.64, 44.19, 37.66, 35.95, 34.16, 33.19, 31.93, 26.21, 26.12, 20.95, 15.92, 15.07, 12.91, 12.84, 12.64, 12.57. HRMS (ESI) calcd for C₆₁H₆₇FN₁₀O₇S [M + H]⁺ 1103.4977, found 1103.4977.

### Example 89: 2-(2,6-Dioxopiperidin-3-yl)-5-fluoro-6-(4-(4-(4-((4-morpholino-6-(3-(m-tolyl)-1H-pyrazol-1-yl)pyrimidin-2-yl)oxy)piperidin-1-yl)phenyl)piperazin-1-yl)isoindoline-1,3-dione (LCG-II-25)

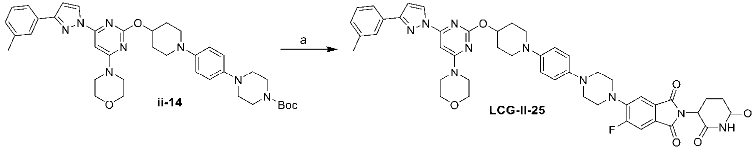

The intermediate ii-14 was obtained by referring to the above method for preparing the ii-4. The ii-14 (68 mg, 0.1 mmol) was dissolved in 10 mL of dichloromethane, and 4 mL of trifluoroacetic acid was added. The mixture was stirred at room temperature for 2 h, spin-dried, and then redissolved in 10 mL of DMF. 2-(2,6-dioxo-piperidin-3-yl)-5,6-difluoro-isoindol-1,3-dione (60 mg, 0.2 mmol) and DIPEA (129 mg, 1.0 mmol) were added. The mixture was kept at 100°C for reaction for 4 h. After the completion of the reaction was monitored by TLC, 20 mL of water was added, and the mixture was extracted with EA twice. The organic phases were combined, washed with saturated brine once, dried over anhydrous Na₂SO₄, filtered, spin-dried, and then separated by column chromatography to provide 30 mg of a yellow solid (35%). ¹H NMR (600 MHz, DMSO-*d*₆) δ 11.12 (s, 1H), 8.60 (d, *J =* 2.7 Hz, 1H), 7.82 (s, 1H), 7.77 (t, *J =* 10.0 Hz, 2H), 7.52 (d, *J =* 7.4 Hz, 1H), 7.35 (t, *J* = 7.6 Hz, 1H), 7.21 (d, *J =* 7.5 Hz, 2H), 7.06 (d, *J =* 2.7 Hz, 1H), 6.94 - 6.91 (m, 4H), 6.90 (s, 1H), 5.16 - 5.07 (m, 2H), 3.74 - 3.64 (m, 8H), 3.45 - 3.36 (m, 6H), 3.22 - 3.13 (m, 3H), 2.96 (t, *J =* 9.5 Hz, 2H), 2.91 - 2.85 (m, 1H), 2.64 - 2.57 (m, 1H), 2.58 - 2.51 (m, 1H), 2.39 (s, 3H), 2.14 - 2.12 (m, 2H), 2.08 - 2.01 (m, 1H), 1.88 - 1.78 (m, 2H). ¹³C NMR (150 MHz, DMSO-*d*₆) δ 172.14, 169.28, 166.02, 165.56, 164.20, 162.89, 161.68, 157.66, 157.54, 155.98, 152.97, 144.72, 144.66, 144.44, 143.65, 137.34, 131.33, 128.72, 128.15, 128.13, 128.03, 125.68, 123.06, 122.99, 122.47, 116.95, 116.61, 113.24, 111.45, 111.28, 105.33, 81.46, 71.43, 65.21, 64.29, 49.01, 48.99, 48.79, 48.45, 46.94, 43.68, 35.16, 30.33, 29.90, 21.45, 20.44, 14.55. HRMS (ESI) calcd for C₄₆H₄₇FN₁₀O₆ [M + H]⁺ 855.3742, found 855.3737.

### Example 90: 2-(2,6-Dioxopiperidin-3-yl)-5-fluoro-6-(4-((4-(2-((4-morpholino-6-(3-(m-tolyl)-1H-pyrazol-1-yl)pyrimidin-2-yl)oxy)ethyl)phenyl)ethynyl)piperidin-1-yl)isoindoline-1,3-dione (LCG-II-26)

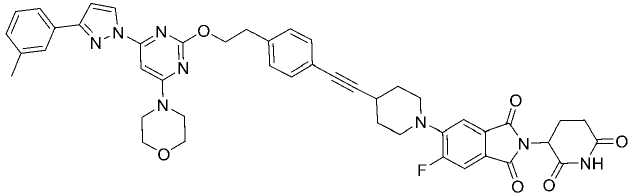

The method for preparing the LCG-II-25 in Example 89 was referred to for the synthesis. ¹H NMR (600 MHz, DMSO-*d*₆) δ 11.11 (s, 1H), 8.57 (d, *J =* 2.7 Hz, 1H), 7.81 (s, 1H), 7.77 (d, *J =* 7.7 Hz, 1H), 7.72 (d, *J =* 11.3 Hz, 1H), 7.49 (d, *J* = 7.4 Hz, 1H), 7.37 (d, *J =* 8.2 Hz, 2H), 7.34 (d, *J* = 7.6 Hz, 1H), 7.31 (d, *J* = 8.3 Hz, 2H), 7.21 (d, *J =* 7.5 Hz, 1H), 7.05 (d, *J =* 2.7 Hz, 1H), 6.89 (s, 1H), 5.11 (dd, *J =* 12.9, 5.4 Hz, 1H), 4.52 (t, *J =* 6.8 Hz, 2H), 3.72 - 3.63 (m, 8H), 3.52 - 3.46 (m, 2H), 3.15 (t, *J =* 9.4 Hz, 2H), 3.06 (t, *J =* 6.8 Hz, 2H), 2.95 - 2.84 (m, 2H), 2.63 - 2.57 (m, 1H), 2.56 - 2.51 (m, 1H), 2.38 (s, 3H), 2.09 - 1.93 (m, 3H), 1.82 - 1.70 (m, 2H). ¹³C NMR (150 MHz, DMSO-*d*₆) δ 172.13, 169.28, 166.03, 165.58, 164.06, 163.39, 157.53, 157.48, 155.86, 152.97, 145.07, 145.01, 137.95, 137.33, 131.31, 130.75, 128.72, 128.69, 128.54, 128.16, 128.15, 128.03, 125.68, 122.61, 122.54, 122.47, 120.26, 113.28, 111.41, 111.24, 105.32, 91.56, 81.57, 80.95, 66.17, 65.18, 64.29, 48.42, 47.90, 47.88, 43.66, 33.84, 30.59, 30.33, 25.84, 21.45, 20.43, 14.55. HRMS (ESI) calcd for C₄₆H₄₃FN₈O₆ [M + H]⁺ 823.3368, found 823.3368.

### Example 91: 2-(2,6-Dioxopiperidin-3-yl)-5-fluoro-6-(4-((4-(4-(2-((4-morpholino-6-(3-(m-tolyl)-1H-pyrazol-1-yl)pyrimidin-2-yl)oxy)ethyl)phenyl)piperazin-1-yl)methyl)piperidin-1-yl)isoindoline-1,3-dione (LCG-II-27)

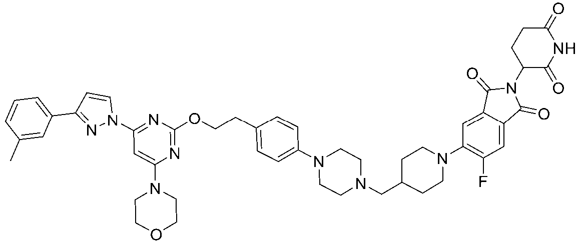

The method for preparing the LCG-II-25 in Example 89 was referred to for the synthesis. ¹H NMR (600 MHz, DMSO-*d*₆) δ 11.11 (s, 1H), 8.57 (d, *J =* 2.7 Hz, 1H), 7.81 (s, 1H), 7.77 (d, *J =* 7.7 Hz, 1H), 7.70 (d, *J =* 11.4 Hz, 1H), 7.43 (d, *J =* 7.3 Hz, 1H), 7.35 (t, *J =* 7.6 Hz, 1H), 7.21 (d, *J =* 7.5 Hz, 1H), 7.16 (d, *J =* 8.3 Hz, 2H), 7.05 (d, *J =* 2.7 Hz, 1H), 6.89 (t, *J* = 4.3 Hz, 3H), 5.10 (dd, *J =* 12.9, 5.4 Hz, 1H), 4.45 (t, *J =* 7.1 Hz, 2H), 3.74 - 3.64 (m, 8H), 3.61 (d, *J =* 12.1 Hz, 2H), 3.09 (s, 4H), 2.95 (t, *J =* 7.1 Hz, 2H), 2.92 - 2.85 (m, 3H), 2.62 - 2.52 (m, 3H), 2.48 - 2.45 (m, 1H), 2.38 (s, 3H), 2.23 (d, *J =* 7.2 Hz, 2H), 2.05 - 2.01 (m, 1H), 1.84 (d, *J =* 12.0 Hz, 2H), 1.79 - 1.72 (m, 1H), 1.27 (q, *J =* 11.3 Hz, 2H). ¹³C NMR (150 MHz, DMSO-*d*₆) δ 172.14, 169.30, 166.09, 165.60, 164.08, 163.46, 161.68, 157.50, 157.46, 155.79, 152.96, 149.07, 145.30, 145.24, 137.33, 131.32, 128.77, 128.72, 128.66, 128.18, 128.03, 127.69, 125.68, 122.47, 122.20, 122.14, 114.92, 113.06, 113.03, 111.35, 111.18, 105.31, 81.50, 66.83, 65.19, 64.29, 63.17, 52.58, 49.34, 49.31, 48.41, 47.92, 43.67, 35.16, 33.18, 31.63, 30.33, 29.65, 21.46, 20.44, 14.55. HRMS (ESI) calcd for C₄₉H₅₃FN₁₀O₆ [M + H]⁺ 897.4212, found 897.4210.

### Example 92: 1-(1-(2-(2,6-Dioxopiperidin-3-yl)-6-fluoro-1,3-dioxoisoindolin-5-yl)piperidin-4-yl)-3-(4-(4-((4-morpholino-6-(3-(m-tolyl)-1H-pyrazol-1-yl)pyrimidin-2-yl)oxy)piperidin-1-yl)phenyl)urea (LCG-II-28)

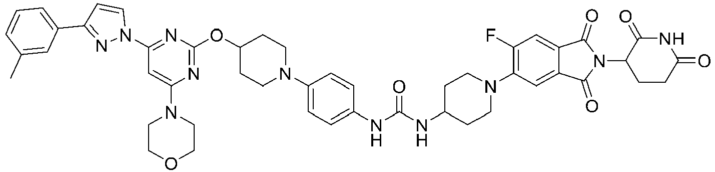

The method for preparing the LCG-II-25 in Example 89 was referred to for the synthesis. ¹H NMR (600 MHz, DMSO-*d*₆) δ 11.11 (s, 1H), 8.60 (d, *J =* 2.7 Hz, 1H), 8.10 (s, 1H), 7.82 (s, 1H), 7.78 (d, *J* = 7.7 Hz, 1H), 7.72 (d, *J =* 11.3 Hz, 1H), 7.48 (d, *J =* 7.4 Hz, 1H), 7.35 (t, *J =* 7.6 Hz, 1H), 7.25 (d, *J* = 8.5 Hz, 2H), 7.21 (d, *J* = 7.6 Hz, 1H), 7.06 (d, *J =* 2.7 Hz, 1H), 6.90 (s, 2H), 6.88 (s, 1H), 6.11 (d, *J =* 7.6 Hz, 1H), 5.16 - 5.05 (m, 2H), 3.75 - 3.67 (m, 9H), 3.55 (d, *J =* 12.9 Hz, 2H), 3.48 - 3.41 (m, 2H), 3.07 - 3.00 (m, 2H), 2.97 (t, *J =* 10.3 Hz, 2H), 2.89 (ddd, *J =* 17.0, 13.9, 5.5 Hz, 1H), 2.63 - 2.58 (m, 1H), 2.57 - 2.51 (m, 1H), 2.39 (s, 3H), 2.16 - 2.09 (m, 2H), 2.07 - 2.00 (m, 1H), 2.01 - 1.93 (m, 2H), 1.87 - 1.78 (m, 2H), 1.61 - 1.50 (m, 2H). ¹³C NMR (150 MHz, DMSO-*d*₆) δ 172.14, 169.30, 166.06, 165.59, 165.57, 164.19, 162.88, 157.53, 155.85, 154.16, 152.97, 145.30, 145.02, 144.97, 137.34, 132.08, 131.33, 128.72, 128.16, 128.14, 128.03, 125.69, 122.54, 122.48, 118.41, 116.32, 113.29, 113.26, 111.39, 111.22, 105.32, 81.46, 71.43, 65.21, 64.30, 48.43, 48.19, 48.16, 46.74, 45.13, 43.68, 31.40, 30.34, 29.85, 21.46, 20.44, 14.55. HRMS (ESI) calcd for C₄₈H₅₀FN₁₁O₇ [M + H]⁺ 912.3957, found 912.3955.

### Example 93: 1-(1-(2-(2,6-Dioxopiperidin-3-yl)-6-fluoro-1,3-dioxoisoindolin-5-yl)azetidin-3-yl)-3-(4-(4-((4-morpholino-6-(3-(m-tolyl)-1H-pyrazol-1-yl)pyrimidin-2-yl)oxy)piperidin-1-yl)phenyl)urea (LCG-II-29)

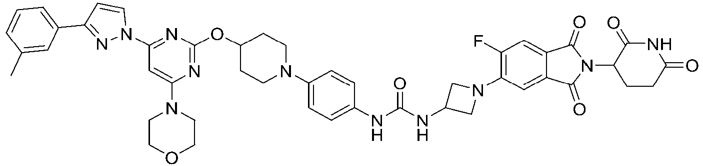

The method for preparing the LCG-II-25 in Example 89 was referred to for the synthesis. ¹H NMR (600 MHz, DMSO-*d*₆) δ 11.09 (s, 1H), 8.60 (d, *J =* 2.7 Hz, 1H), 8.32 (s, 1H), 7.82 (s, 1H), 7.78 (d, *J* = 7.7 Hz, 1H), 7.62 (d, *J =* 11.1 Hz, 1H), 7.35 (t, *J =* 7.6 Hz, 1H), 7.25 (d, *J =* 8.5 Hz, 2H), 7.21 (d, *J =* 7.5 Hz, 1H), 7.06 (d, *J =* 2.7 Hz, 1H), 6.97 (d, *J =* 7.6 Hz, 1H), 6.92 - 6.86 (m, 3H), 6.74 (d, *J* = 7.4 Hz, 1H), 5.16 - 5.09 (m, 1H), 5.08 (dd, *J =* 12.9, 5.4 Hz, 1H), 4.68 - 4.57 (m, 1H), 4.44 (t, *J* = 7.1 Hz, 2H), 4.01 (t, *J =* 7.1 Hz, 2H), 3.78 - 3.64 (m, 8H), 3.49 - 3.41 (m, 2H), 2.97 (t, *J =* 9.7 Hz, 2H), 2.88 (ddd, *J =* 17.1, 13.9, 5.5 Hz, 1H), 2.65 - 2.51 (m, 2H), 2.39 (s, 3H), 2.19 - 2.08 (m, 2H), 2.07 - 1.97 (m, 1H), 1.89 - 1.75 (m, 2H). ¹³C NMR (150 MHz, DMSO-*d*₆) δ 172.15, 169.37, 166.19, 165.79, 165.78, 164.19, 162.87, 157.53, 154.18, 153.92, 152.97, 152.28, 145.57, 143.38, 143.29, 137.33, 131.58, 131.33, 128.72, 128.69, 128.03, 125.69, 122.47, 118.91, 118.15, 118.09, 116.20, 110.74, 110.60, 107.83, 107.78, 105.32, 81.45, 71.41, 65.21, 60.19, 48.32, 46.64, 43.67, 30.34, 29.82, 21.53, 20.44. HRMS (ESI) calcd for C₄₆H₄₆FN₁₁O₇ [M + H]⁺ 884.3644, found 884.3643.

### Example 94: 2-(2,6-Dioxopiperidin-3-yl)-5-fluoro-6-(4-(1-(4-((4-morpholino-6-(3-(m-tolyl)-1H-pyrazol-1-yl)pyrimidin-2-yl)oxy)phenyl)piperidin-4-yl)piperazin-1-yl)isoindoline-1,3-dione (LCG-II-30)

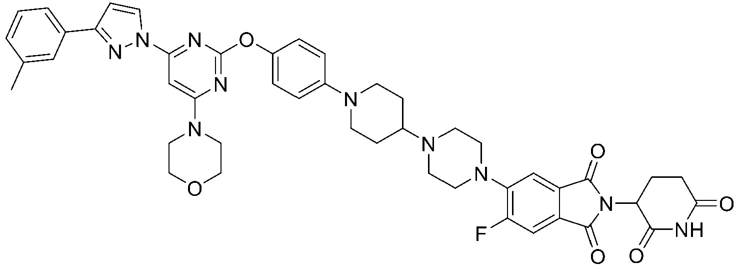

The method for preparing the LCG-II-25 in Example 89 was referred to for the synthesis. ¹H NMR (600 MHz, DMSO-*d*₆) δ 11.11 (s, 1H), 8.33 (d, *J* = 2.7 Hz, 1H), 7.81 (s, 1H), 7.77 (s, 1H), 7.73 (d, *J =* 11.3 Hz, 1H), 7.45 (d, *J =* 7.3 Hz, 1H), 7.35 (t, *J =* 7.6 Hz, 1H), 7.21 (d, *J =* 7.5 Hz, 1H), 7.12 - 7.06 (m, 2H), 7.04 (d, *J =* 2.7 Hz, 1H), 6.98 (d, *J* = 9.2 Hz, 2H), 6.95 (s, 1H), 5.11 (dd, *J =* 12.9, 5.4 Hz, 1H), 3.72 (d, *J =* 11.6 Hz, 2H), 3.66 (t, *J =* 4.8 Hz, 4H), 3.59 (t, *J =* 4.8 Hz, 4H), 3.26 (s, 4H), 2.93 - 2.83 (m, 1H), 2.74 - 2.52 (m, 8H), 2.49 - 2.39 (m, 1H), 2.38 (s, 3H), 2.12 - 2.01 (m, 1H), 1.90 (d, *J =* 11.9 Hz, 2H), 1.65 - 1.51 (m, 2H). ¹³C NMR (150 MHz, DMSO-*d*₆) δ 172.14, 169.28, 166.04, 165.58, 164.11, 163.69, 157.55, 157.50, 155.87, 153.11, 147.68, 144.79, 144.74, 144.34, 137.35, 131.22, 128.79, 128.44, 128.16, 128.04, 125.68, 122.73, 122.67, 122.49, 121.26, 115.75, 112.97, 111.39, 111.22, 105.45, 82.31, 65.10, 60.11, 49.30, 48.44, 48.00, 47.90, 43.64, 30.33, 27.09, 21.45, 20.43. HRMS (ESI) calcd for C₄₆H₄₇FN₁₀O₆ [M + H]⁺ 885.3722, found 885.3736.

### Example 95: 2-(2,6-Dioxopiperidin-3-yl)-5-fluoro-6-(4-(4-(4-((4-morpholino-6-(3-(m-tolyl)-1H-pyrazol-1-yl)pyrimidin-2-yl)oxy)piperidin-1-yl)benzyl)piperazin-1-yl)isoindoline-1,3-dione (LCG-II-31)

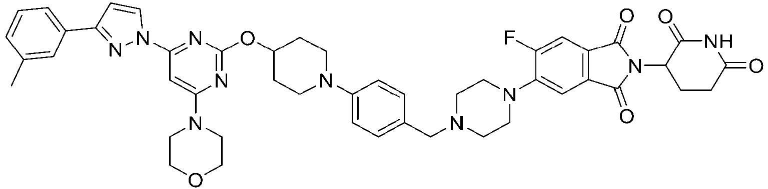

The method for preparing the LCG-II-25 in Example 89 was referred to for the synthesis. ¹H NMR (600 MHz, DMSO-*d*₆) δ 11.11 (s, 1H), 8.60 (d, *J* = 2.7 Hz, 1H), 7.82 (d, *J =* 1.8 Hz, 1H), 7.78 (d, *J* = 7.8 Hz, 1H), 7.71 (d, *J =* 11.3 Hz, 1H), 7.44 (d, *J =* 7.3 Hz, 1H), 7.35 (t, *J =* 7.6 Hz, 1H), 7.21 (d, *J* = 7.6 Hz, 1H), 7.17 (d, *J =* 8.2 Hz, 2H), 7.06 (d, *J =* 2.7 Hz, 1H), 6.95 (d, *J =* 8.2 Hz, 2H), 6.90 (s, 1H), 5.17 - 5.13 (m, 1H), 5.11 (dd, *J =* 12.9, 5.4 Hz, 1H), 3.74 - 3.62 (m, 8H), 3.57 - 3.49 (m, 2H), 3.43 (s, 2H), 3.23 (s, 4H), 3.10 - 2.99 (m, 2H), 2.94 - 2.83 (m, 1H), 2.65 - 2.53 (m, 4H), 2.39 (s, 3H), 2.24 - 2.08 (m, 2H), 2.08 - 1.98 (m, 1H), 1.86 - 1.75 (m, 2H). ¹³C NMR (150 MHz, DMSO-*d*₆) δ 172.14, 169.28, 166.04, 165.57, 164.20, 162.87, 161.68, 157.54, 155.85, 152.98, 149.39, 144.79, 144.74, 137.34, 131.33, 129.22, 128.73, 128.71, 128.13, 128.03, 127.08, 125.69, 122.74, 122.68, 122.48, 115.06, 113.06, 113.03, 111.38, 111.21, 105.32, 81.48, 71.42, 65.22, 64.30, 60.89, 51.54, 48.96, 48.92, 48.44, 45.79, 43.68, 30.34, 29.66, 27.87, 21.87, 21.45, 20.44, 14.56, 10.64. HRMS (ESI) calcd for C₄₇H₄₉FN₁₀O₆ [M + H]⁺ 869.3899, found 869.3886.

### Example 96: 2-(2,6-Dioxopiperidin-3-yl)-5-fluoro-6-(4-((4-(4-((4-morpholino-6-(3-(m-tolyl)-1H-pyrazol-1-yl)pyrimidin-2-yl)oxy)phenyl)piperazin-1-yl)methyl)piperidin-1-yl)isoindoline-1,3-dione (LCG-II-32)

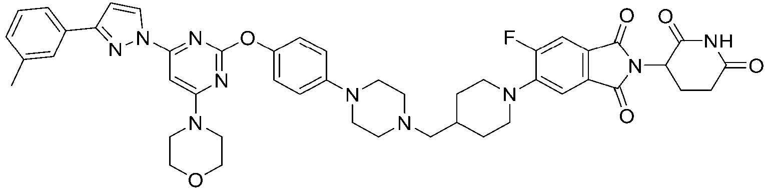

The method for preparing the LCG-II-25 in Example 89 was referred to for the synthesis. ¹H NMR (600 MHz, DMSO-*d*₆) δ 11.11 (s, 1H), 8.33 (d, *J =* 2.7 Hz, 1H), 7.81 (s, 1H), 7.77 (d, *J =* 7.7 Hz, 1H), 7.70 (d, *J =* 11.3 Hz, 1H), 7.44 (d, *J =* 7.3 Hz, 1H), 7.35 (t, *J =* 7.6 Hz, 1H), 7.24 - 7.19 (m, 1H), 7.09 (d, *J =* 8.9 Hz, 2H), 7.04 (d, *J* = 2.7 Hz, 1H), 6.97 (d, *J =* 9.1 Hz, 2H), 6.95 (s, 1H), 5.11 (dd, *J =* 12.9, 5.4 Hz, 1H), 3.72 - 3.52 (m, 10H), 3.14 (s, 4H), 2.97 - 2.82 (m, 3H), 2.65 - 2.52 (m, 5H), 2.38 (s, 3H), 2.25 (d, *J =* 7.1 Hz, 2H), 2.08 - 1.99 (m, 1H), 1.85 (d, *J =* 11.8 Hz, 2H), 1.79 (d, *J =* 3.8 Hz, 1H), 1.29 (q, *J =* 11.0 Hz, 2H). ¹³C NMR (150 MHz, DMSO-*d*₆) δ 172.14, 169.30, 166.09, 165.60, 164.11, 163.67, 157.50, 157.47, 155.79, 153.11, 147.68, 145.30, 145.24, 144.51, 137.35, 131.22, 128.79, 128.45, 128.19, 128.17, 128.04, 125.69, 122.49, 122.21, 122.15, 121.27, 115.25, 113.08, 113.05, 111.35, 111.19, 105.45, 82.31, 65.10, 64.29, 63.16, 52.58, 49.35, 49.31, 48.41, 48.09, 43.64, 31.65, 30.33, 29.65, 21.46, 20.43, 14.55. HRMS (ESI) calcd for C₄₇H₄₉FN₁₀O₆ [M + H]⁺ 869.3899, found 869.3887. **Example 97: Degradation of PIKfyve proteins in VCaP and DU145 tumor cells by compounds**

Method: 200 µL of cells at a density of 5×10⁶ cells/ml were inoculated into a 6-well plate, and then different concentrations of drugs were added. The cells were treated at 37°C for 24 h. The supernatant was collected and washed with PBS twice. Then, 100-200 µL of 1×SDS lysate was added, and the cell lysate was collected into a centrifuge tube. The cells were disrupted ultrasonically for 10-20 sec, and boiled for 10 min. Protein samples were subjected to SDS-PAGE electrophoresis at a voltage of 90 V, transferred to PVDF membranes at 110 V, blocked with 5% BSA for 4 h, incubated with primary antibodies (PIKfyve, GAPDH) at 4°C overnight, and incubated with secondary antibodies (anti-Rabbit IgG or anti-Mouse IgG) at room temperature. The membranes were washed with TBST buffer, developed with an ECL developing solution, and then imaged to obtain protein band signals, with single-concentration WB results as shown in FIG. 1. Compared with the DMSO control group, the concentration that achieves 50% degradation activity is defined as DC₅₀.

**Table 1 Degradation activity of compounds on PIKfyve proteins in VCaP and DU145 cells**

| **Examples** | **Compound structure** | **VCaP DC₅₀(µM)** |
|---|---|---|
| **1** | 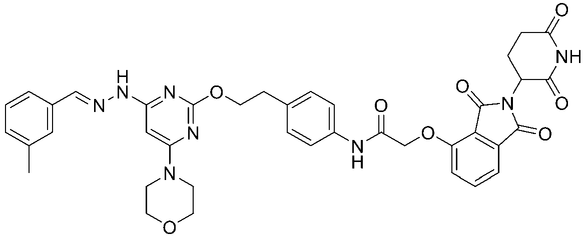 | 0.1-10 |
| **8** | 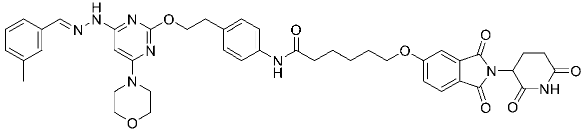 | 0.1-10 |
| **13** | 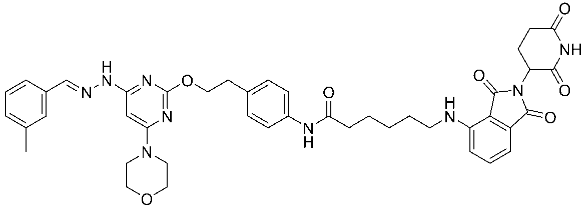 | 0.1-10 |
| **16** | 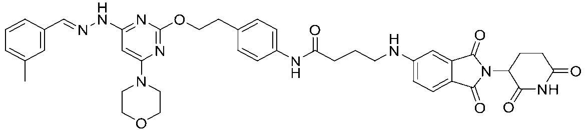 | 0.1-10 |
| **17** | 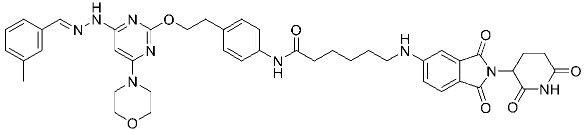 | 0.1-10 |
| **18** | 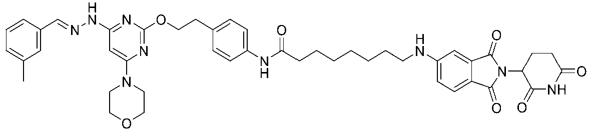 | 0.1-10 |
| **19** | 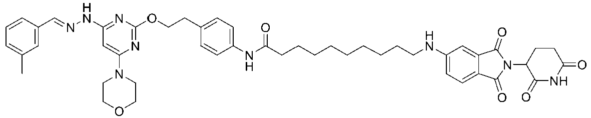 | 0.1-10 |
| **21** | 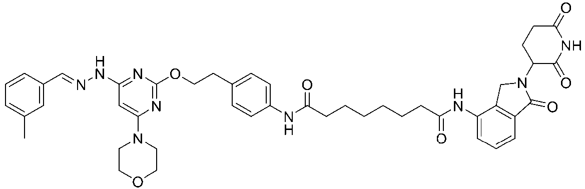 | 0.001-0.1 |
| **22** | 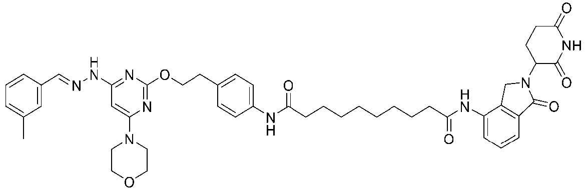 | 0.1-10 |
| **23** | 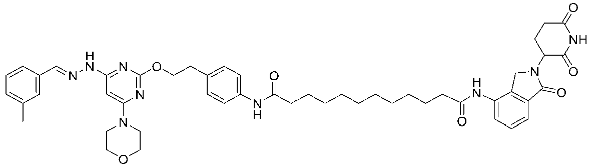 | 0.1-10 |
| **25** | 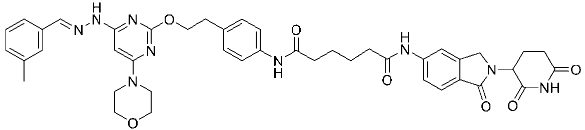 | 0.1-10 |
| **26** | 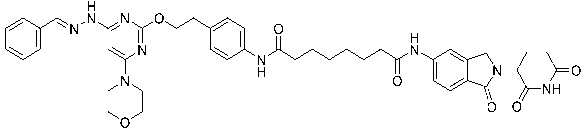 | 0.1-10 |
| **27** | 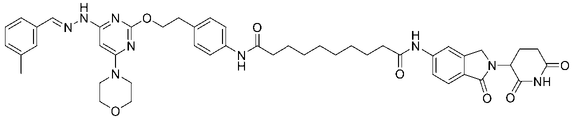 | 0.001-0.1 |
| **28** | 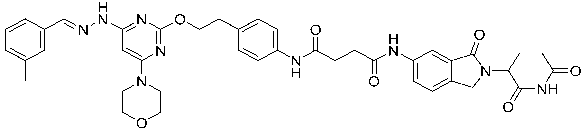 | 0.001-0.1 |
| **29** | 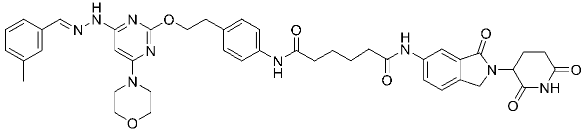 | 0.001-0.1 |
| **30** | 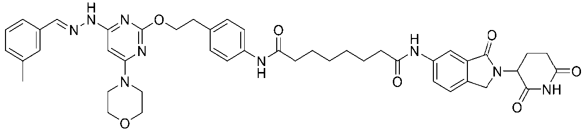 | 0.001-0.1 |
| **31** | 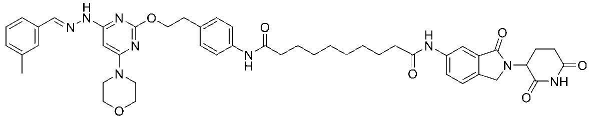 | 0.1-10 |
| **32** | 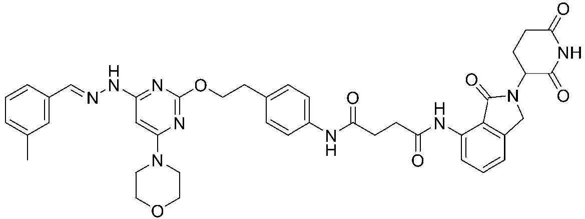 | 0.001-0.1 |
| **35** | 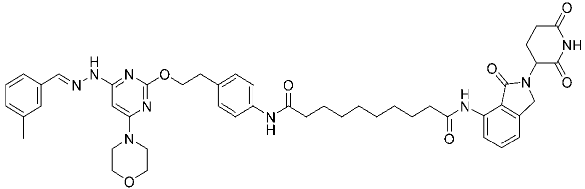 | 0.1-10 |
| **36** | 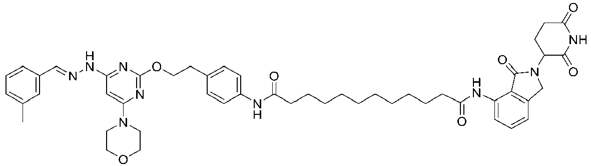 | 0.1-10 |
| **37** | 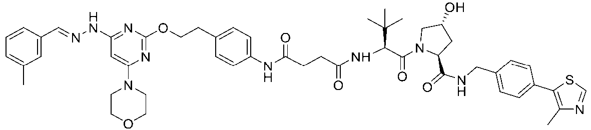 | 0.1-10 |
| **38** | 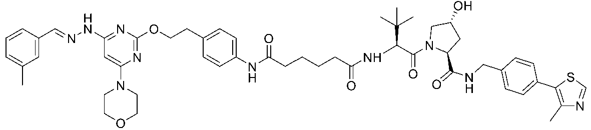 | 0.001-0.1 |
| **39** | 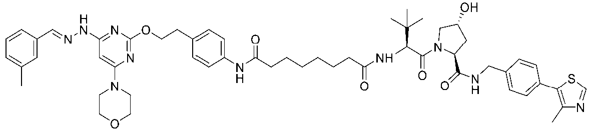 | 0.001-0.1 |
| **40** | 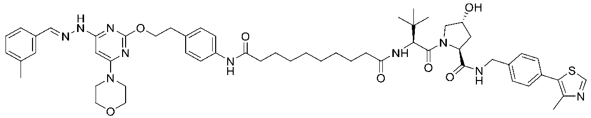 | 0.001-0.1 |
| **41** | 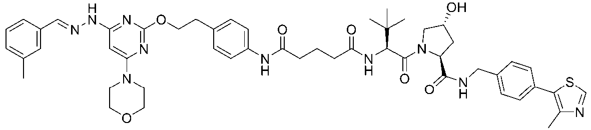 | 0.001-0.1 |
| **42** | 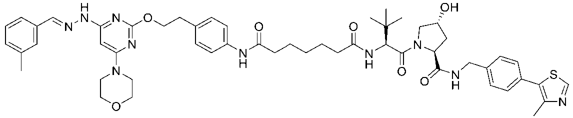 | 0.001-0.1 |
| **43** | 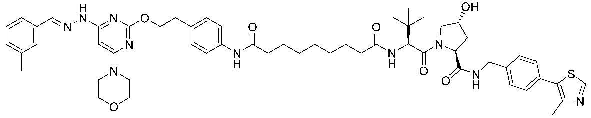 | 0.001-0.1 |
| **44** | 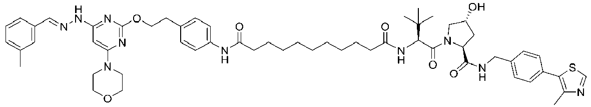 | 0.001-0.1 |
| **45** | 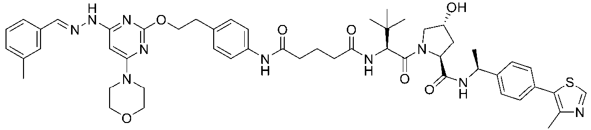 | 0.001-0.1 |
| **46** | 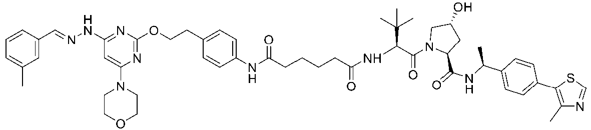 | 0.001-0.1 |
| **47** | 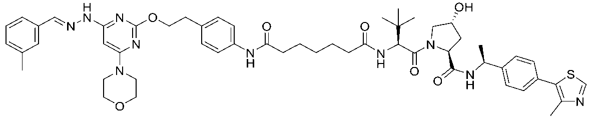 | 0.001-0.1 |
| **48** | 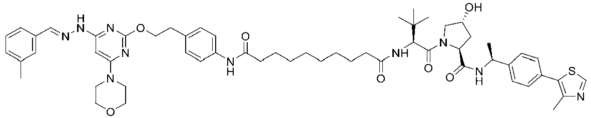 | 0.001-0.1 |
| **49** | 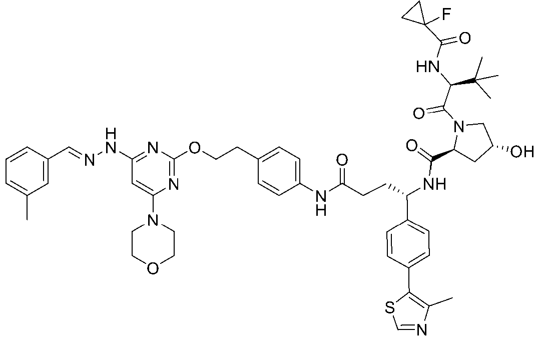 | 0.001-0.1 |
| **50** | 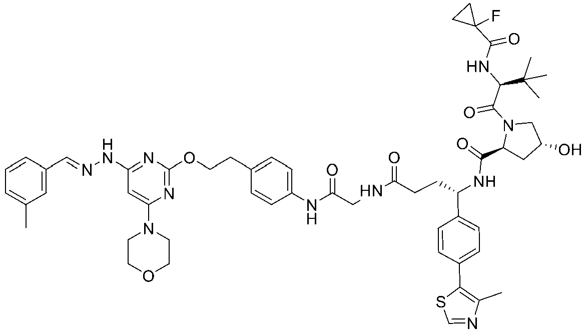 | 0.001-0.1 |
| **51** | 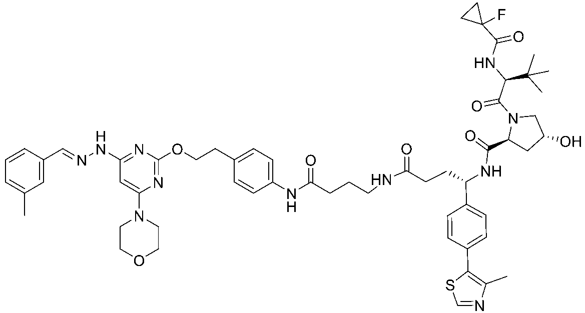 | 0.001-0.1 |
| **52** | 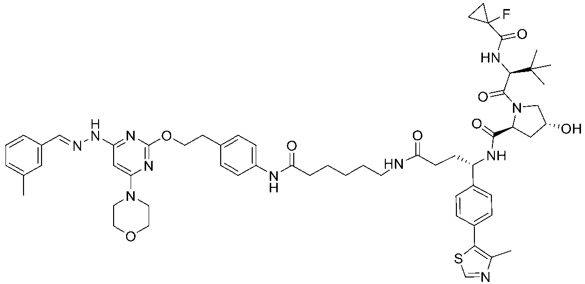 | 0.001-0.1 |
| **53** | 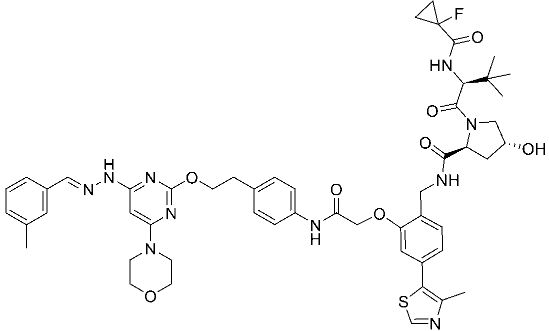 | 0.001-0.1 |
| **54** | 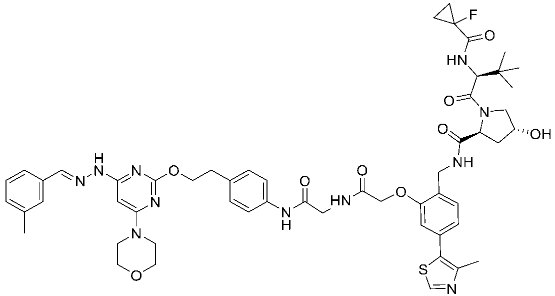 | 0.001-0.1 |
| **55** | 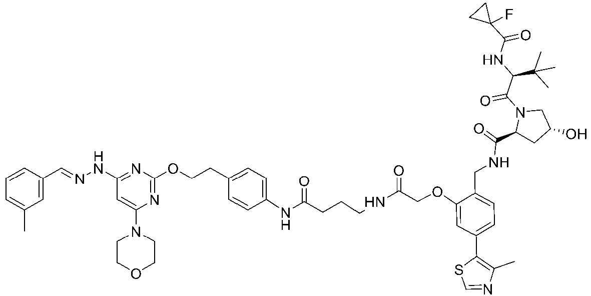 | 0.001-0.1 |
| **56** | 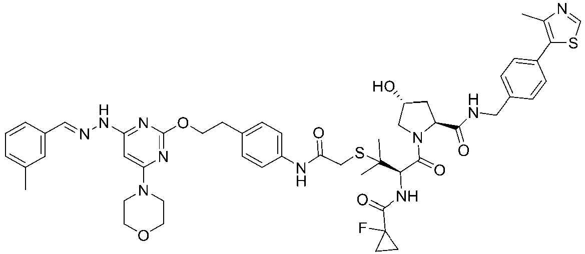 | 0.001-0.1 |
| **57** | 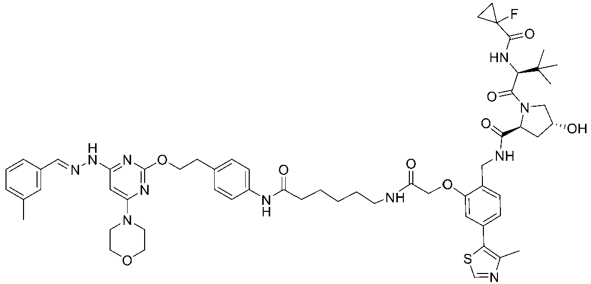 | 0.001-0.1 |
| **58** | 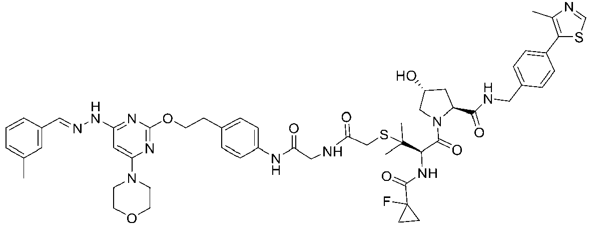 | 0.1-10 |
| **59** | 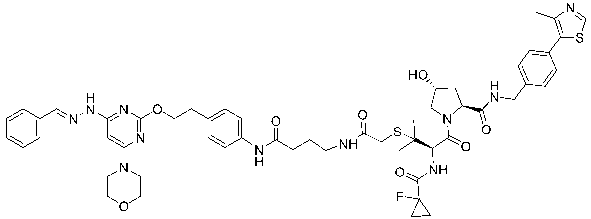 | 0.1-10 |
| **60** | 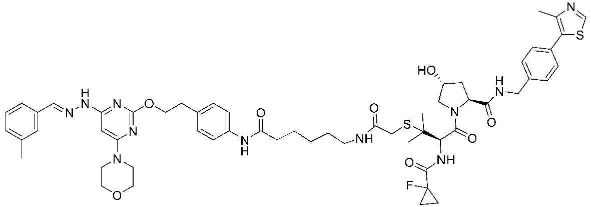 | 0.1-10 |
| **61** | 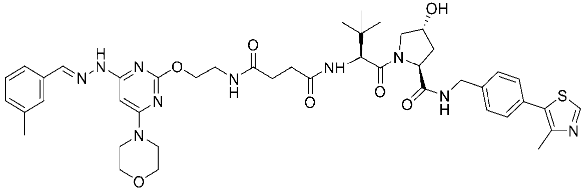 | 0.1-10 |
| **62** | 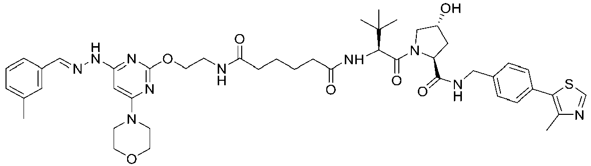 | 0.1-10 |
| **63** | 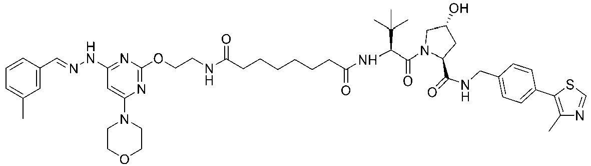 | 0.1-10 |
| **64** | 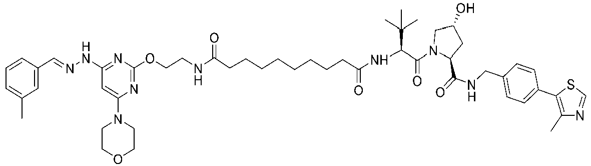 | 0.1-10 |
| **65** | 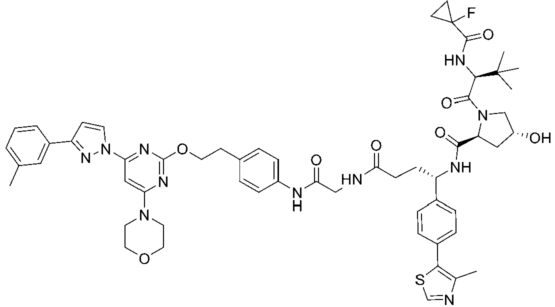 | 0.001-0.1 |
| **66** | 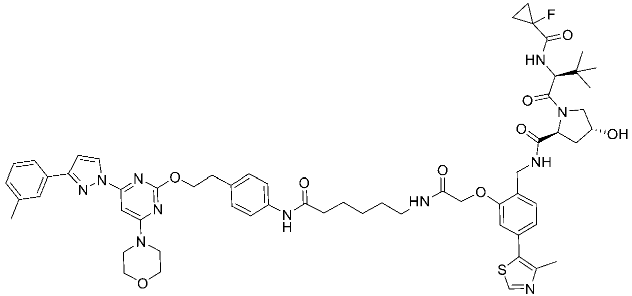 | 0.001-0.1 |
| **67** | 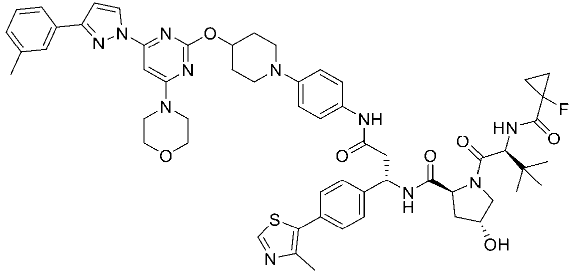 | 0.001-0.1 |
| **68** | 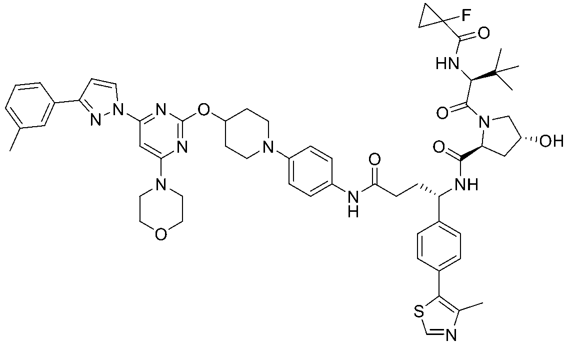 | 0.001-0.1 |
| **69** | 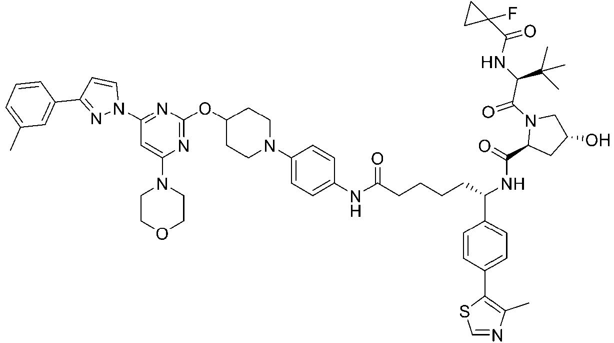 | 0.001-0.1 |
| **70** | 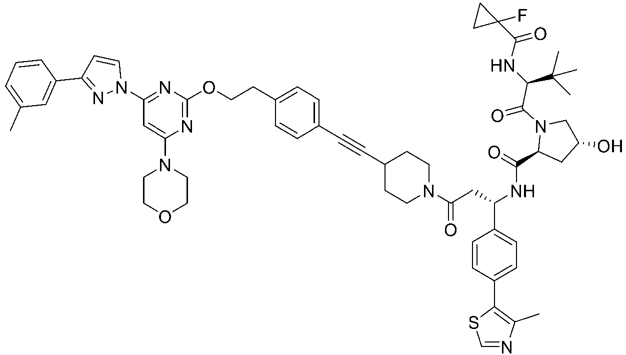 | 0.001-0.1 |
| **71** | 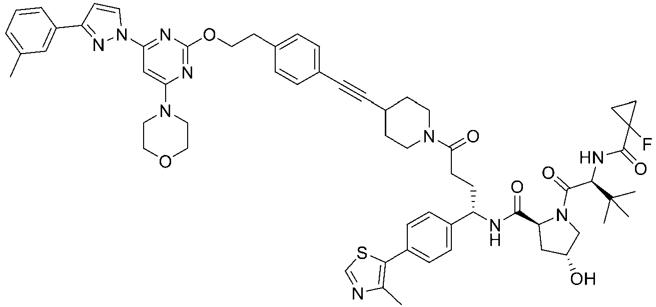 | 0.001-0.1 |
| **72** | 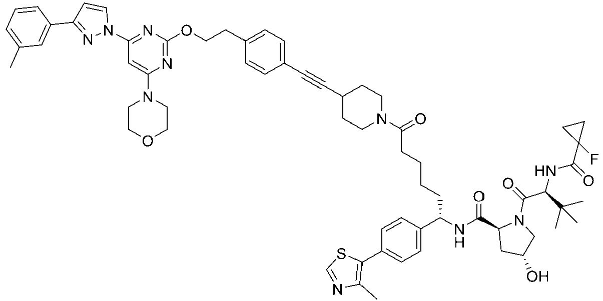 | 0.001-0.1 |
| **73** | 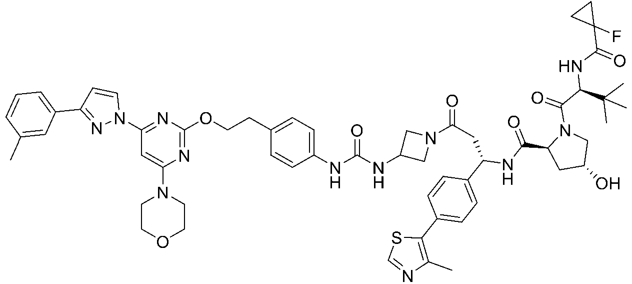 | 0.001-0.1 |
| **74** | 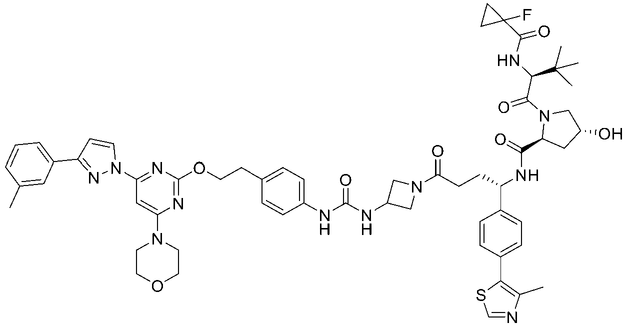 | 0.001-0.1 |
| **75** | 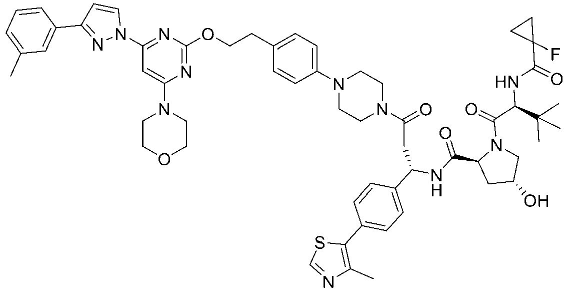 | 0.001-0.1 |
| **76** | 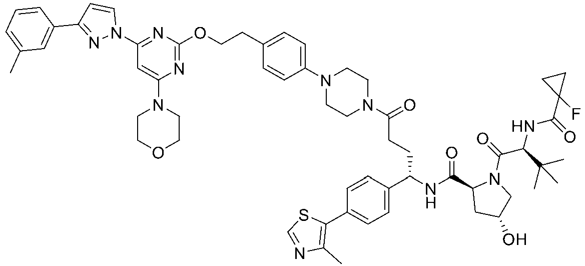 | 0.001-0.1 |
| **77** | 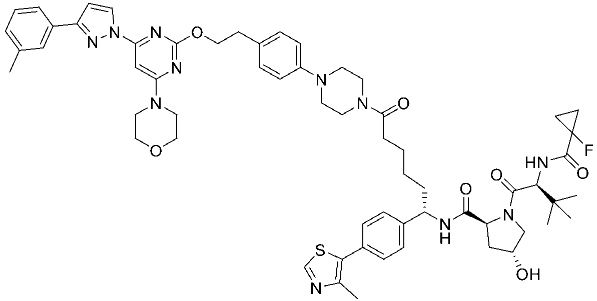 | 0.001-0.1 |
| **78** | 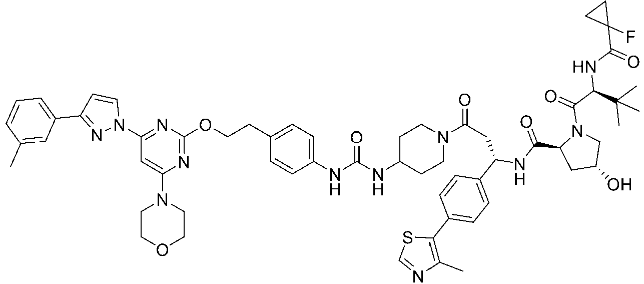 | 0.001-0.1 |
| **79** | 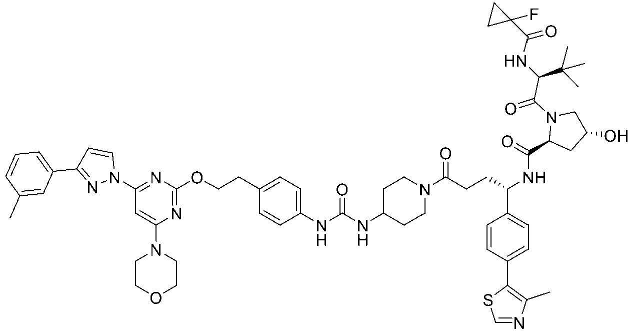 | 0.001-0.1 |
| **80** | 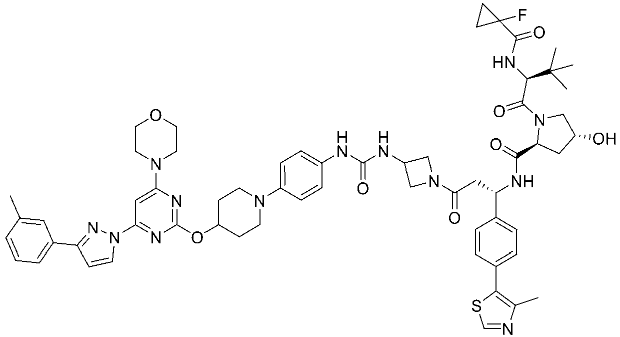 | 0.001-0.1 |
| **81** | 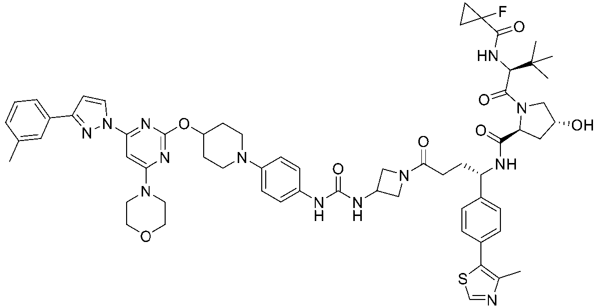 | 0.001-0.1 |
| **82** | 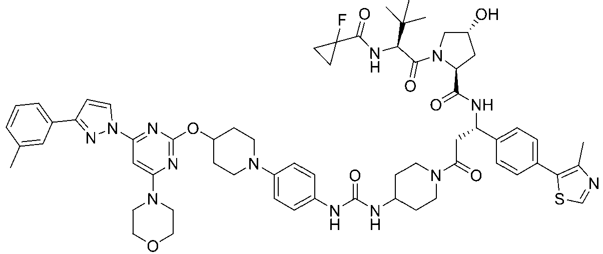 | 0.001-0.1 |
| **83** | 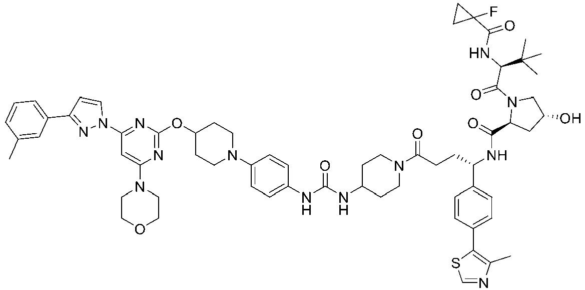 | 0.001-0.1 |
| **84** | 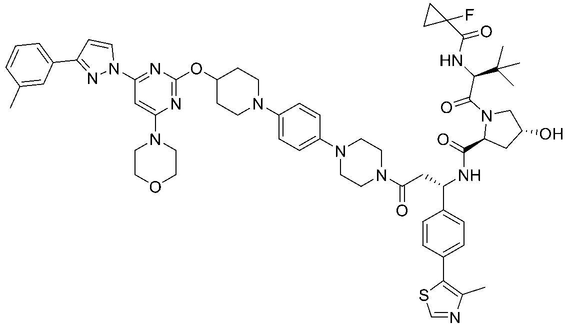 | 0.001-0.1 |
| **85** | 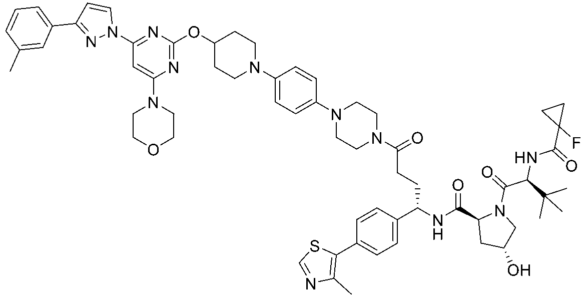 | 0.001-0.1 |
| **86** | 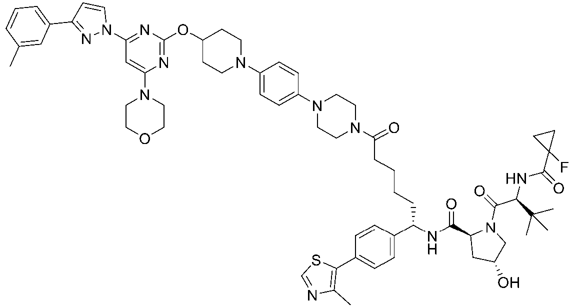 | 0.001-0.1 |
| **87** | 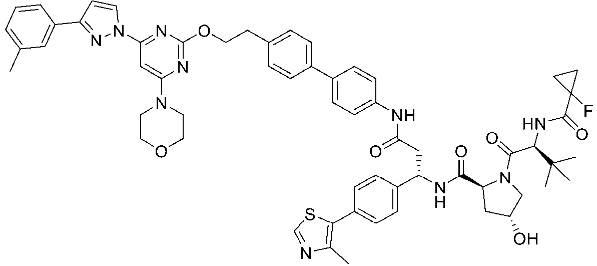 | 0.001-0.1 |
| **88** | 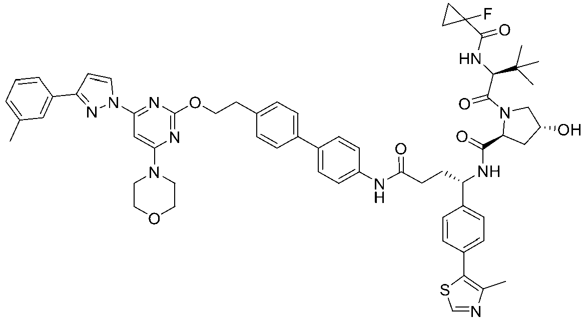 | 0.001-0.1 |
| **89** | 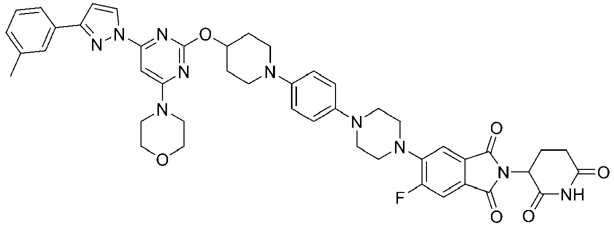 | 0.1-10 |
| **90** | 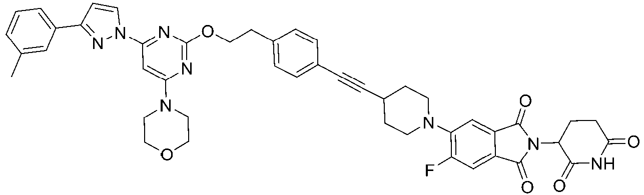 | 0.1-10 |
| **91** | 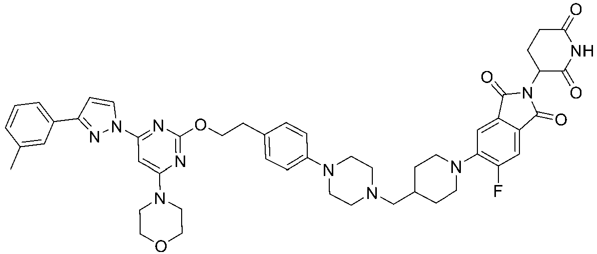 | 0.1-10 |
| **92** | 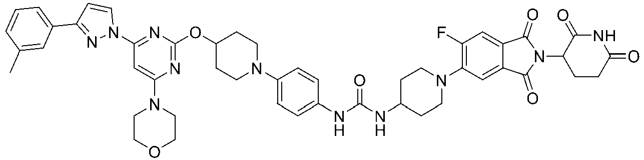 | 0.1-10 |
| **93** | 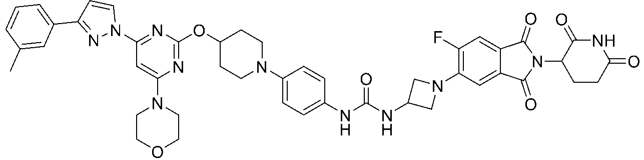 | 0.1-10 |
| **94** | 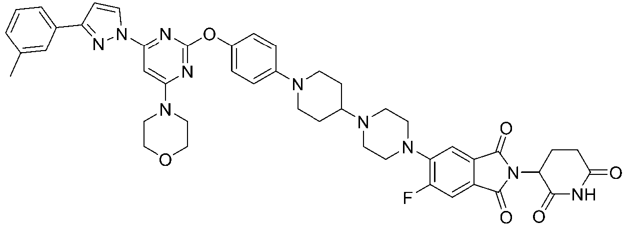 | 0.1-10 |
| **95** | 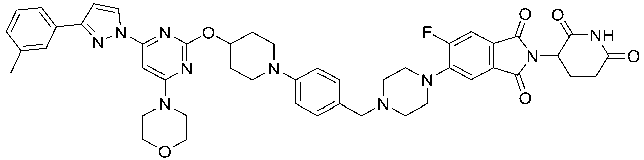 | 0.1-10 |
| **96** | 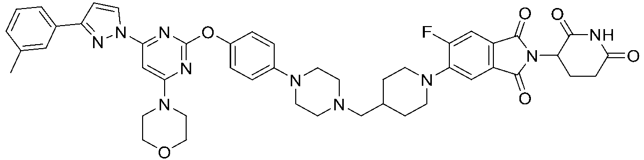 | 0.1-10 |

The results show that each compound of the present invention can achieve good PIKfyve protein degradation activity on VCaP and DU145 cells.

All documents mentioned herein are incorporated by reference in the present application, just like each document being individually incorporated by reference. In addition, it should be understood that after reading the above teachings in the present invention, those skilled in the art can make various alterations or modifications to the present invention, and these equivalent forms also fall within the scope defined by the appended claims of the present application.

## Claims

1. A compound represented by formula (I), or a pharmaceutically acceptable salt thereof, or a stereoisomer thereof, or a prodrug molecule thereof, 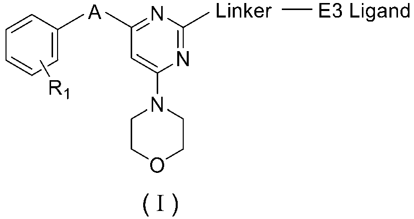 wherein
R₁ is selected from the group consisting of: H, halogen, cyano, a substituted or unsubstituted C₁-C₆ alkyl, a substituted or unsubstituted C₃-C₆ cycloalkyl, and a substituted or unsubstituted C₁-C₆ alkoxy;
A is selected from the group consisting of: -NH-, 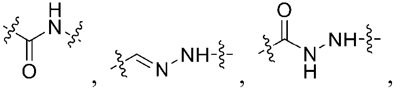 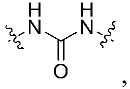 a 6-10-membered aryl, and a 5-10-membered heteroaryl; the A is each independently optionally substituted with 0-3 R₂, the R₂ is selected from the group consisting of: H, halogen, a substituted or unsubstituted C₁-C₆ alkyl, a substituted or unsubstituted C₃-C₆ cycloalkyl, and a substituted or unsubstituted C₁-C₆ alkoxy;
Linker has a structure represented by a formula below: 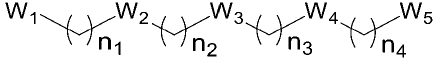
wherein W₁, W₂, W₃, W₄, and W₅ are each independently selected from the group consisting of: -O-, -S-, -NH-, -CH₂-, -CONH-, -NHCO-, -C=C-, -CH=CH-, -C(O)-, -P(=O)-, -S(O)₂-, -S(O)-, -P(O)₂(OH)-, -NH-S(O)-NH-, -C(O)O-, -OC(O)-, -NHCONH-, and a 3-12-membered ring having 0-4 heteroatoms; and n₁, n₂, n₃, and n₄ are each independently 0, 1, 2, 3, 4, 5, 6, 7, 8, 9, or 10; wherein the 3-12-membered ring may be a structure of a monocyclic ring, a spiro ring, a fused ring, or a bridged ring;
E3 Ligand is an E3 ubiquitin ligase ligand having a structure selected from the group consisting of: 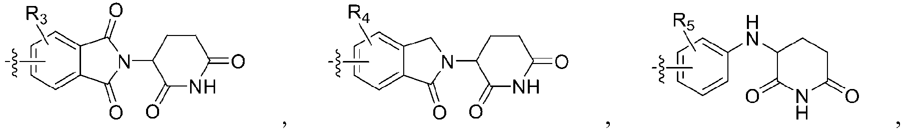 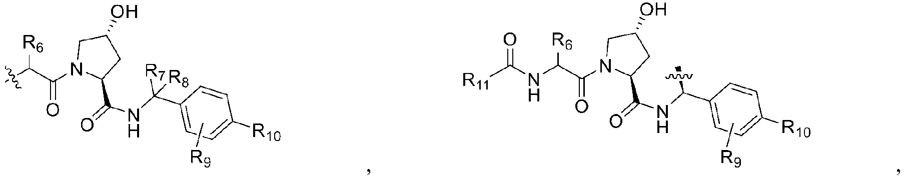 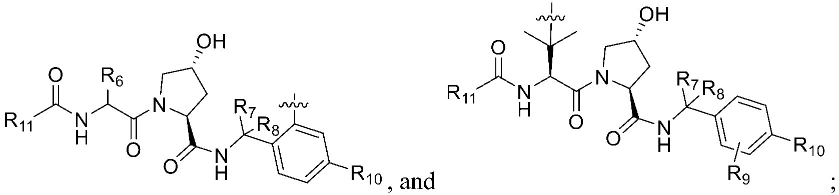
wherein R₃, R₄, R₅, R₆, R₇, R₈, R₉, and R₁₁ are each independently selected from the group consisting of: H, halogen, cyano, a substituted or unsubstituted C₁-C₆ alkyl, a substituted or unsubstituted C₃-C₆cycloalkyl, or a substituted or unsubstituted C₁-C₆ alkoxy; and
R₁₀ is selected from the group consisting of: 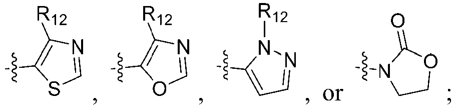 wherein each R₁₂ is each independently selected from the group consisting of: H, a substituted or unsubstituted C₁-C₆ alkyl, a substituted or unsubstituted C₃-C₆ cycloalkyl, and a substituted or unsubstituted C₁-C₆ alkoxy;
wherein the "substituted" in the "substituted or unsubstituted" means that one or more H atoms on the group are substituted with a substituent selected from the group consisting of: halogen, a deuterium atom, hydroxyl, cyano, a C1-C3 alkyl, a C1-C3 alkoxy, a C1-C3 haloalkyl, a C1-C3 haloalkoxy, a C1-C3 aldehyde group, a C1-C3 carboxyl, and -SF₅.

2. The compound or the pharmaceutically acceptable salt thereof or the stereoisomer thereof or the prodrug molecule thereof according to claim 1, wherein the E3 Ligand has a structure selected from the group consisting of: 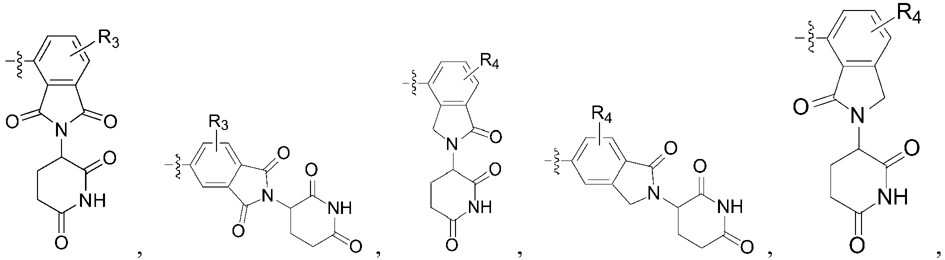 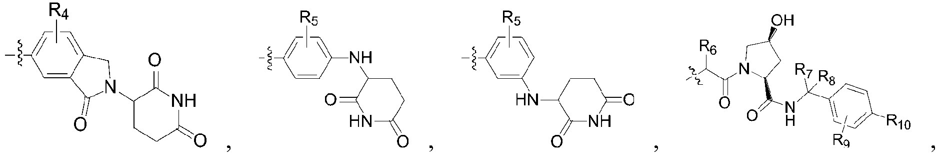 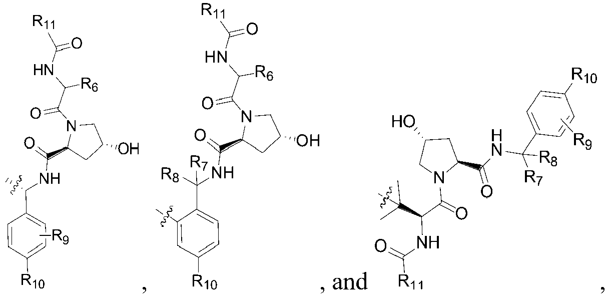 wherein the R₃, the R₄, the R₅, the R₆, the R₇, the R₈, the R₉, the R₁₀, and the R₁₁ are as defined in claim 1.

3. The compound or the pharmaceutically acceptable salt thereof or the stereoisomer thereof or the prodrug molecule thereof according to claim 1, wherein the Linker has a structure represented by a formula below: 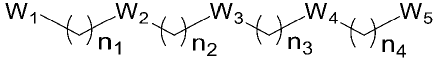 wherein the W₁, the W₂, the W₃, the W₄, and the W₅ are each independently selected from the group consisting of: -O-, -S-, -NH-, -CH₂-, -CONH-, -NHCO-, -C(O)-, -C(O)O-, -OC(O)-, - NHCONH-, and a 3-10-membered ring having 0-4 heteroatoms; and the n₁, the n₂, the n₃, and the n₄ are each independently 0, 1, 2, 3, 4, 5, 6, 7, 8, 9, or 10.

4. The compound or the pharmaceutically acceptable salt thereof or the stereoisomer thereof or the prodrug molecule thereof according to claim 1, wherein the Linker has a structure selected from the group consisting of: 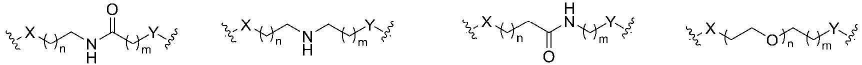 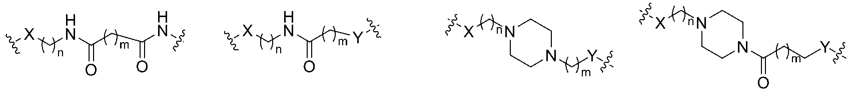 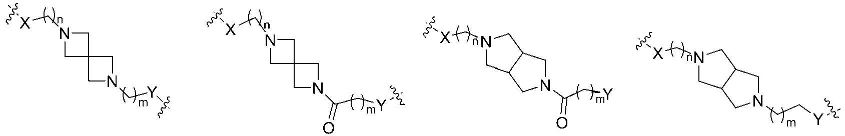 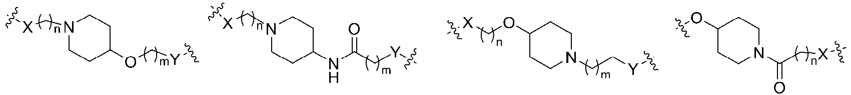 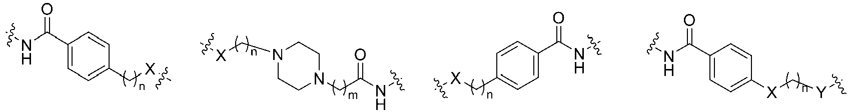 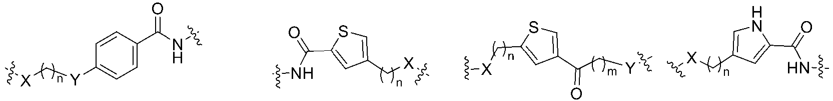 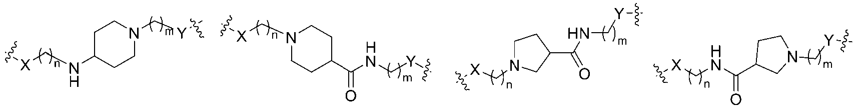 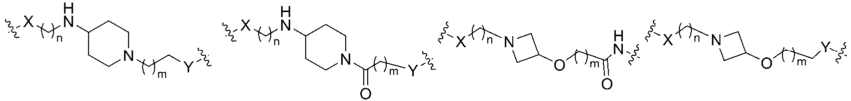 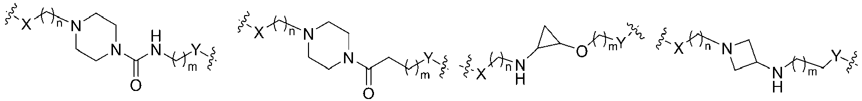 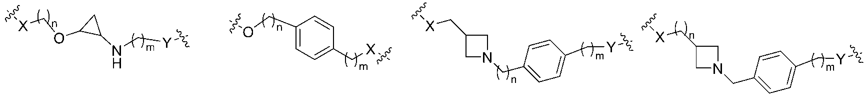 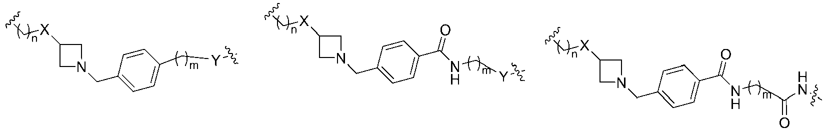 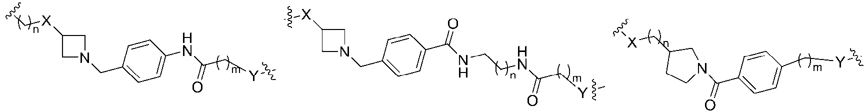 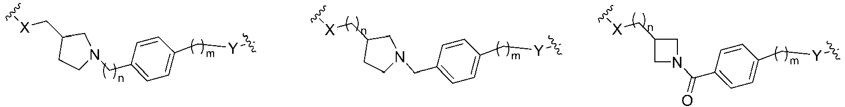 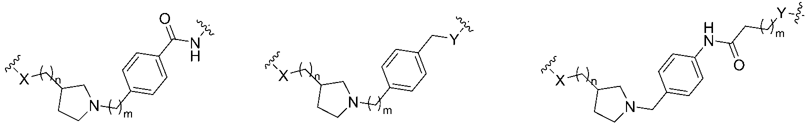 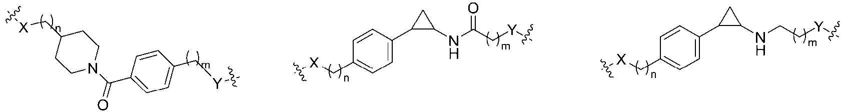 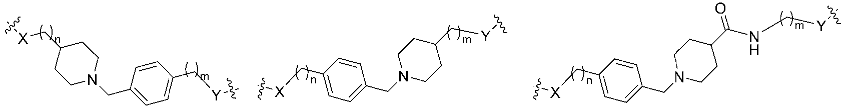 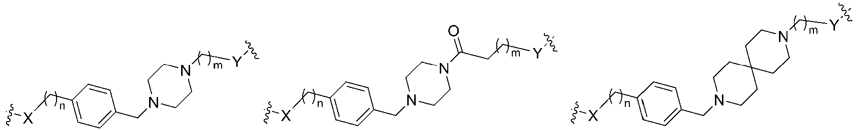 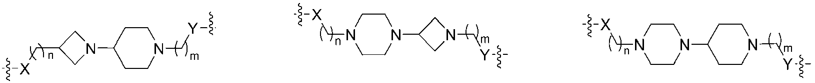 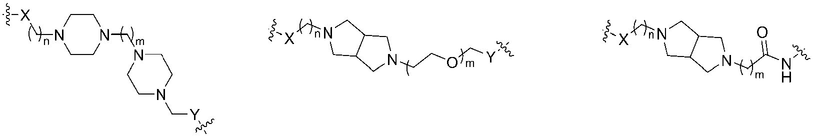 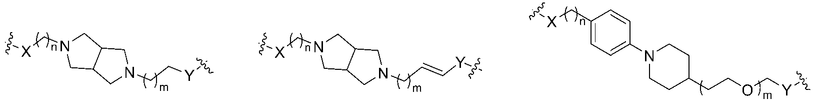 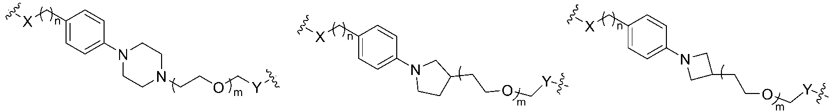 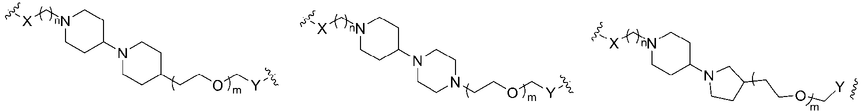 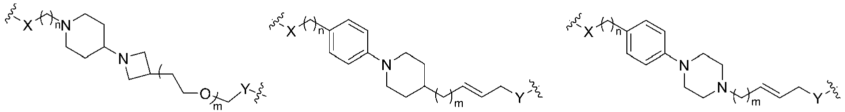 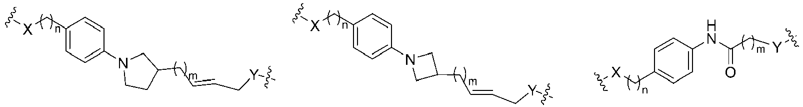 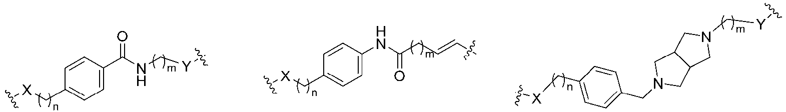 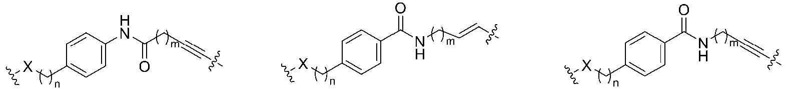 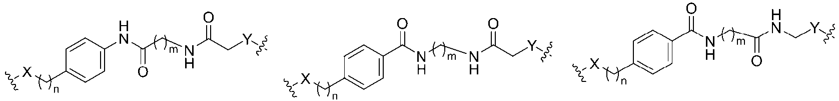 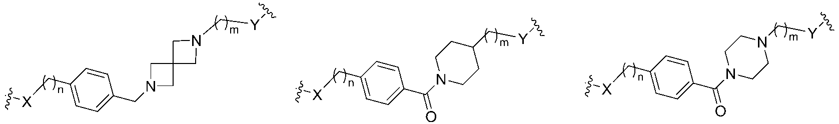 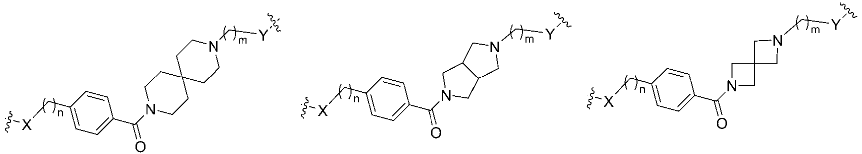 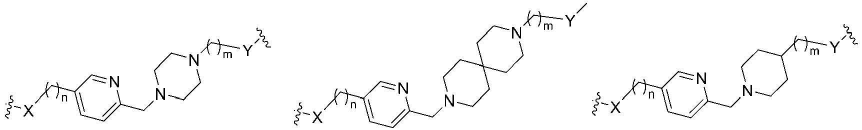 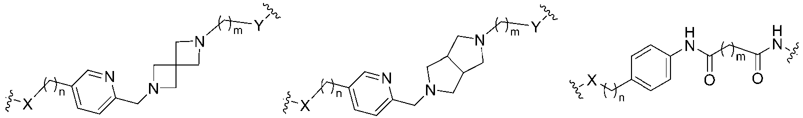 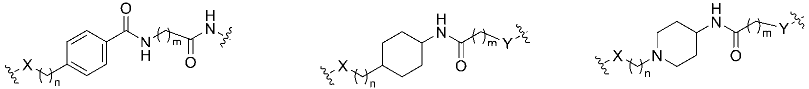 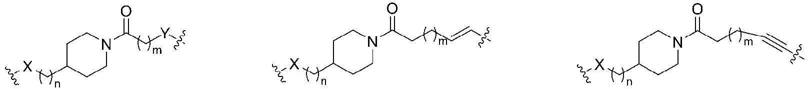 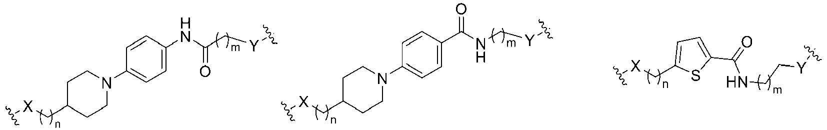 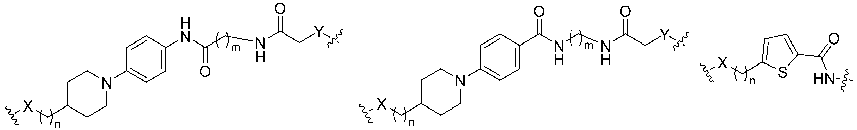 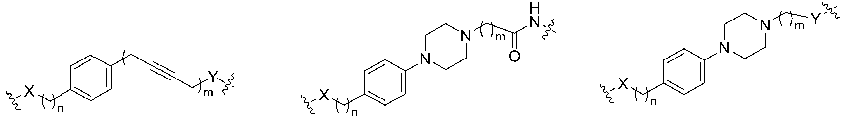 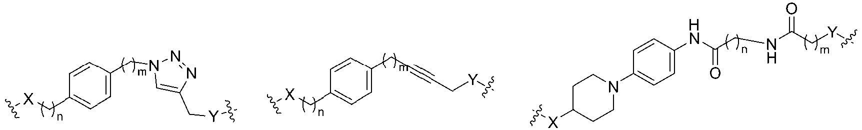 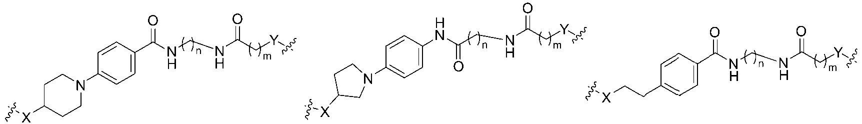 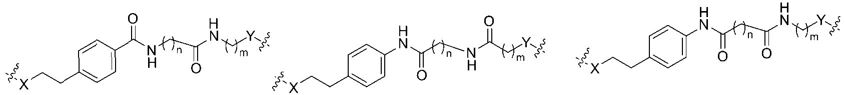 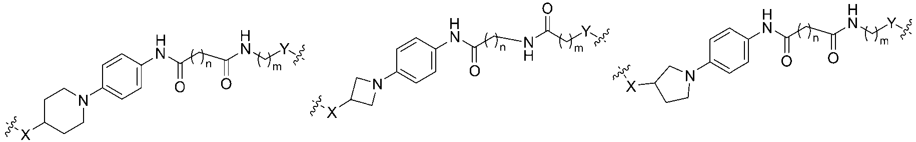 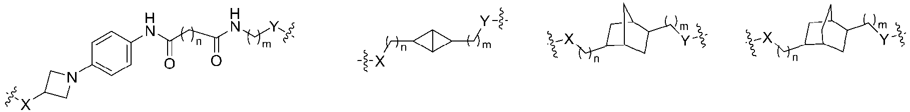 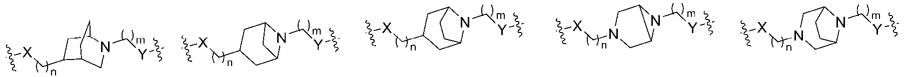 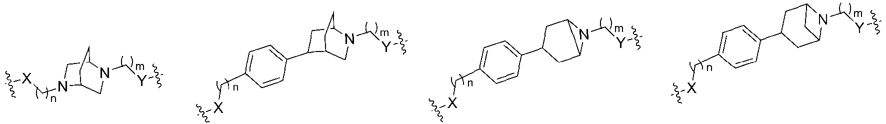 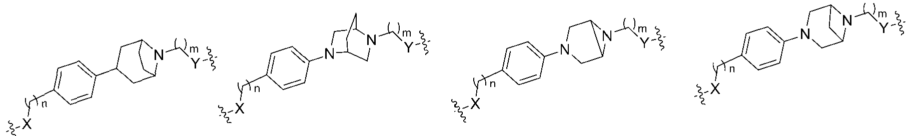 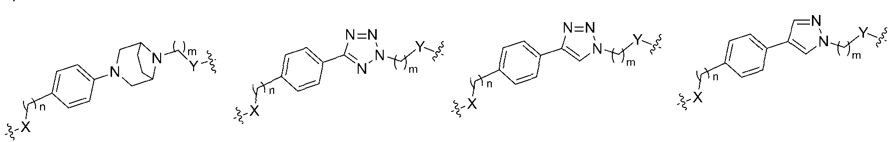 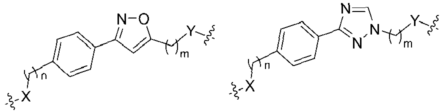 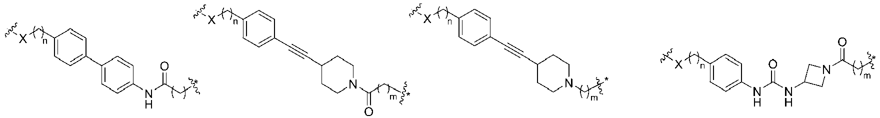 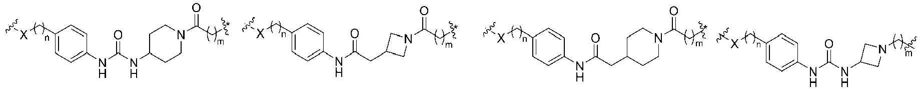 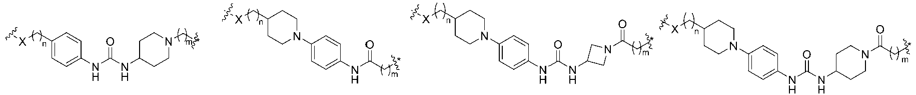 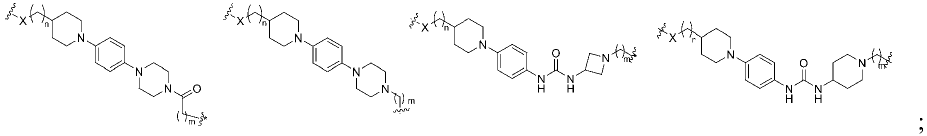 wherein each of X and Y is each independently selected from the group consisting of: -O-, -S-, -NH-, -CH₂-, and 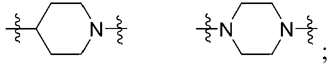 and each of n and m is each independently 0, 1, 2, 3, 4, 5, 6, 7, 8, 9, or 10.

5. The compound or the pharmaceutically acceptable salt thereof or the stereoisomer thereof or the prodrug molecule thereof according to claim 1, wherein the A is selected from the group consisting of: -NH-, 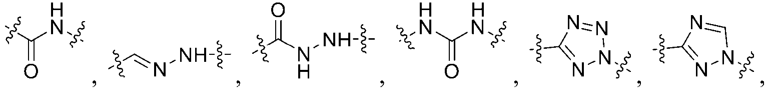 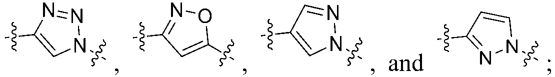 the A is each independently optionally substituted with 0-2 R₂, and the R₂ is as defined in claim 1.

6. The compound or the pharmaceutically acceptable salt thereof or the stereoisomer thereof or the prodrug molecule thereof according to claim 1, wherein the compound is selected from the group consisting of:

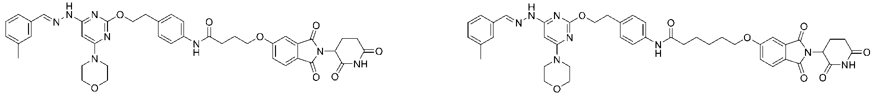

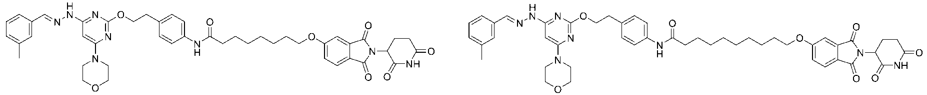

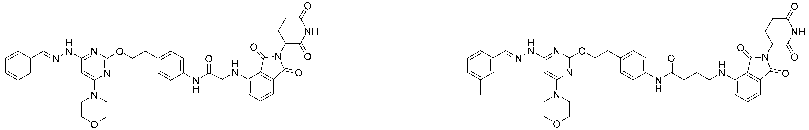

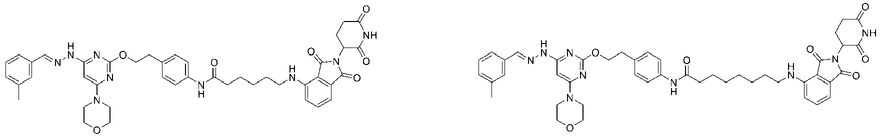

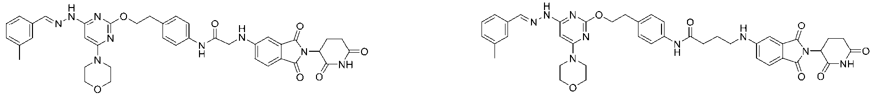

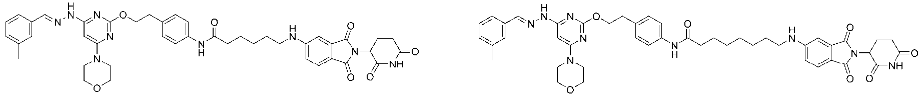

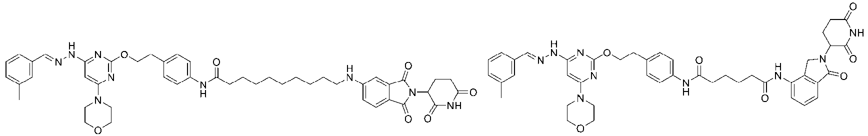

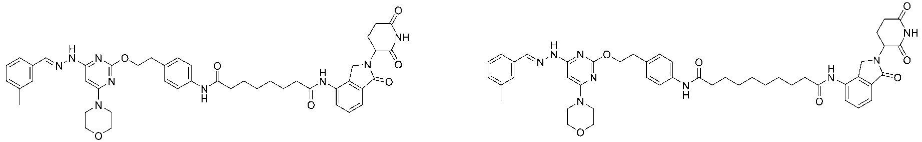

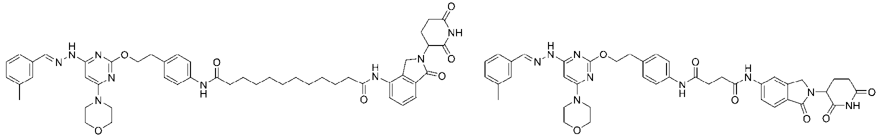

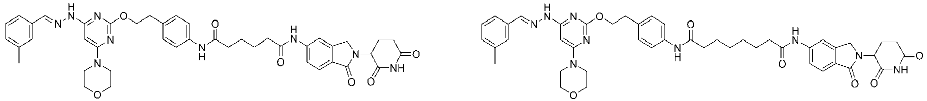

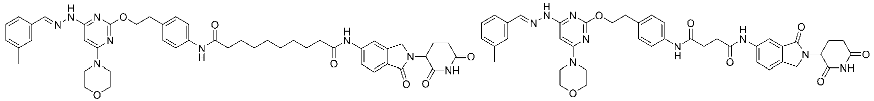

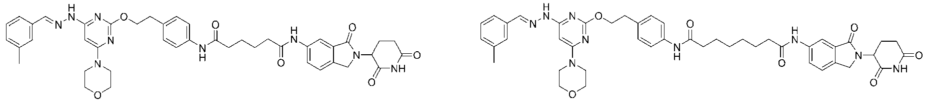

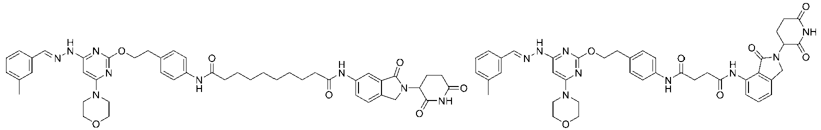

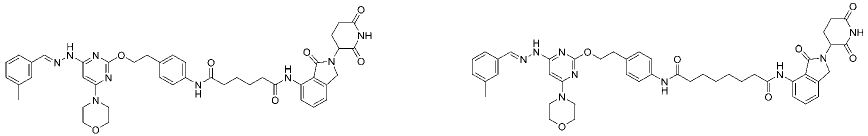

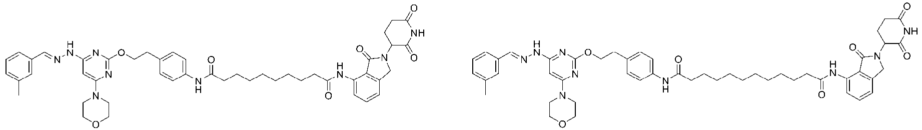

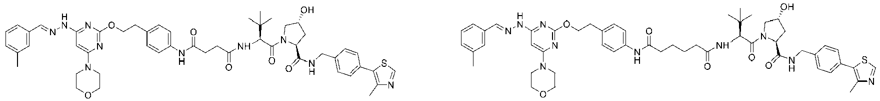

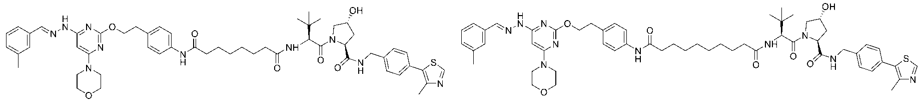

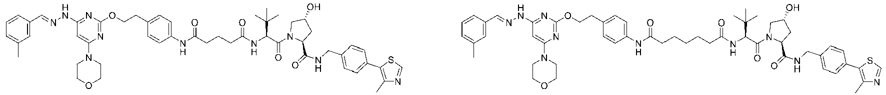

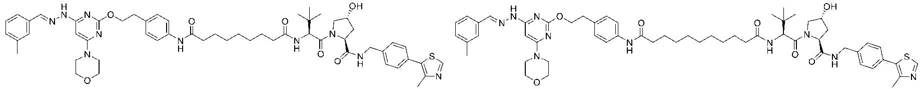

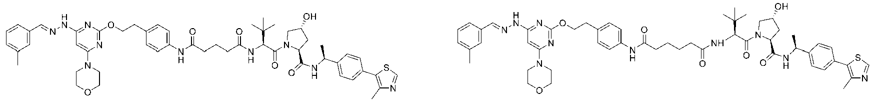

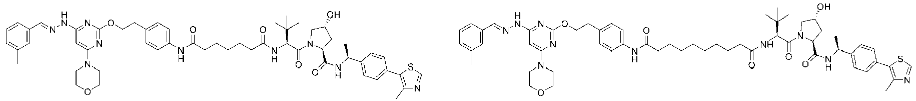

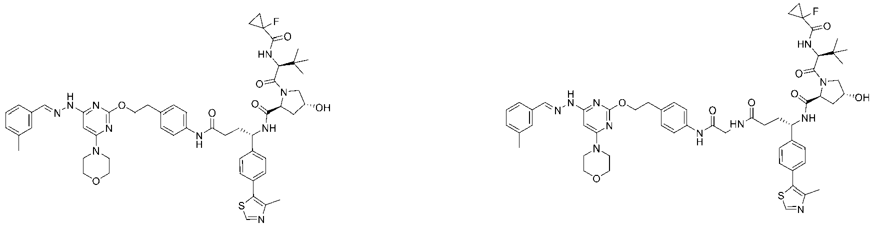

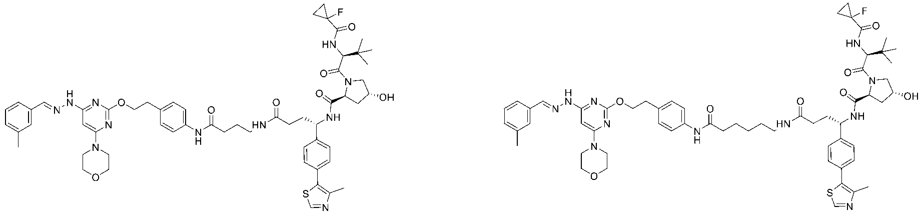

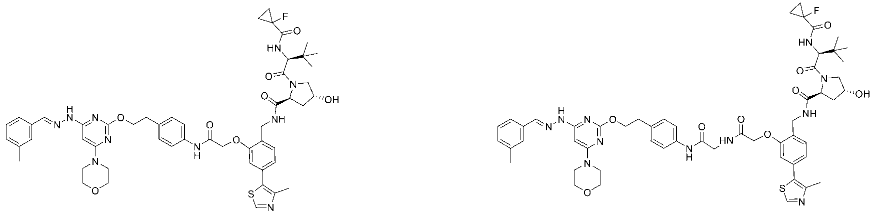

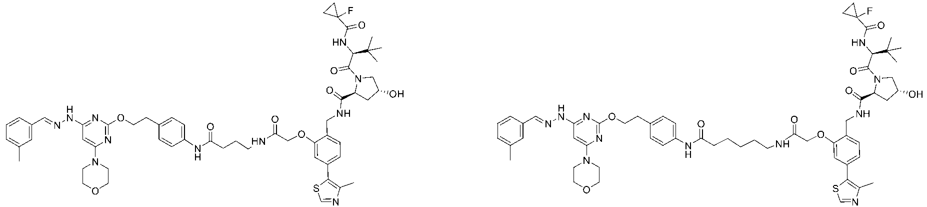

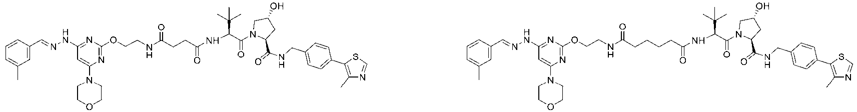

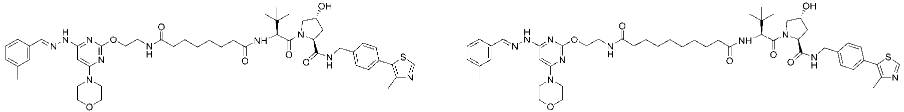

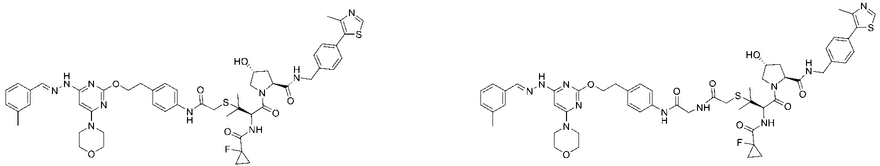

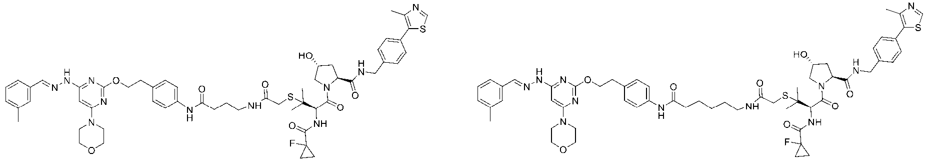

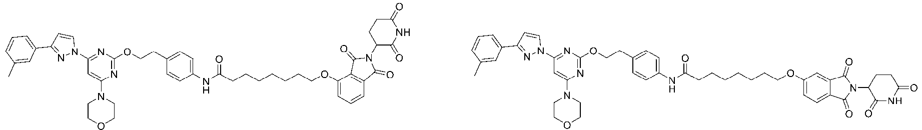

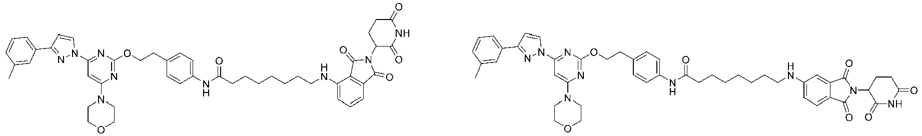

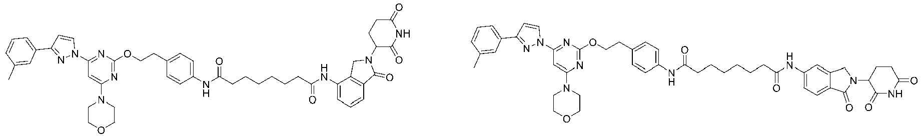

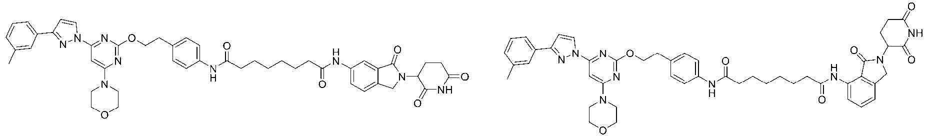

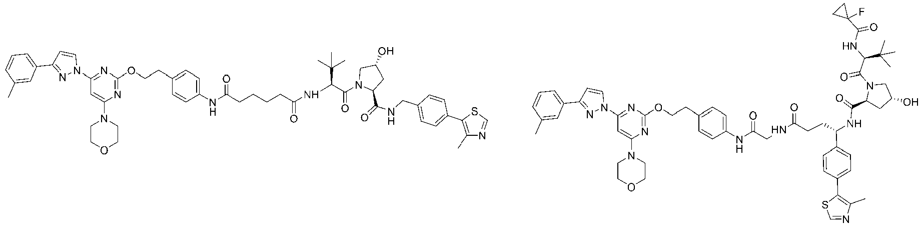

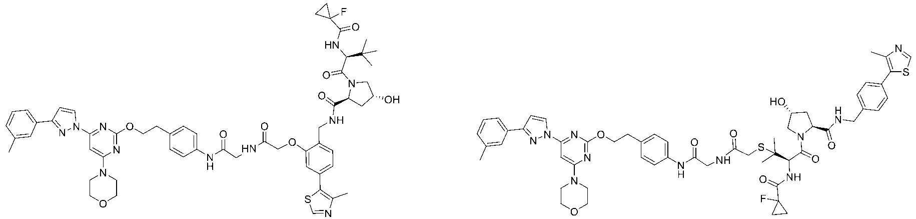

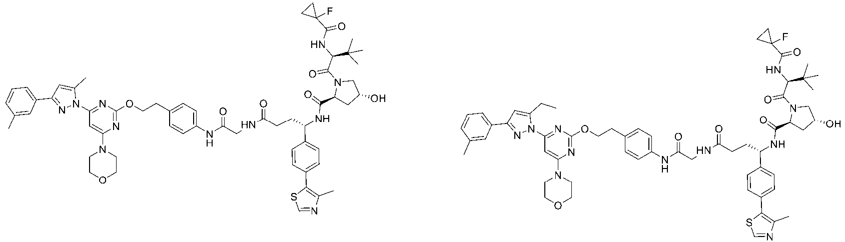

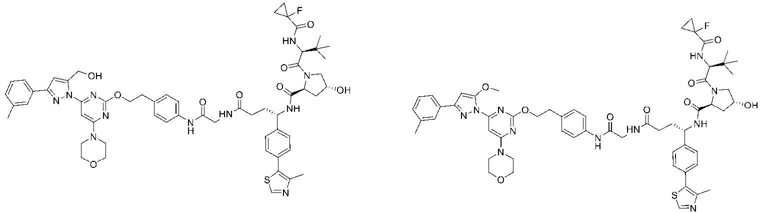

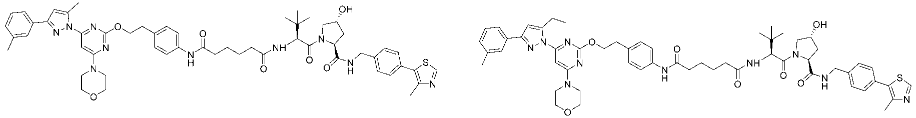

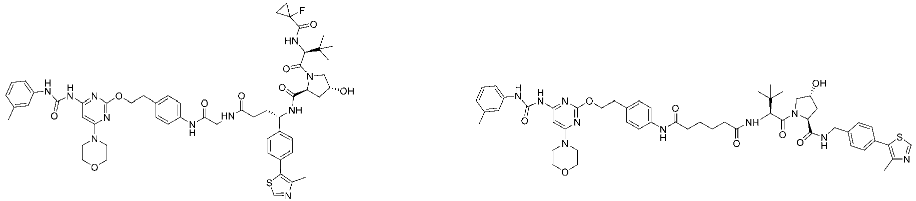

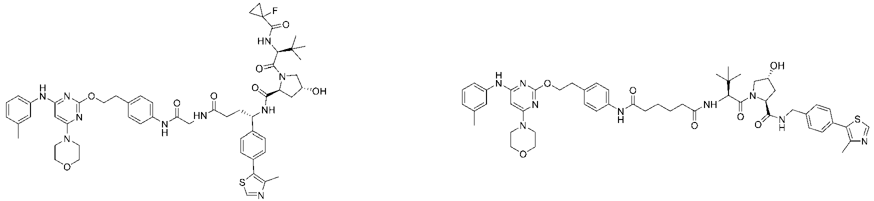

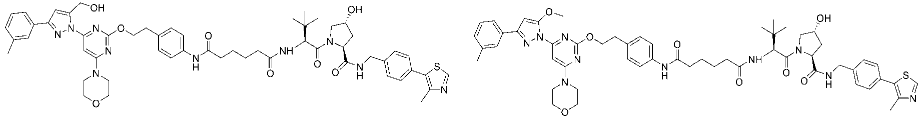

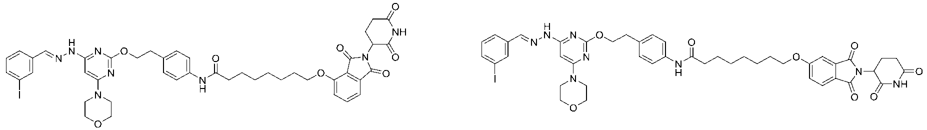

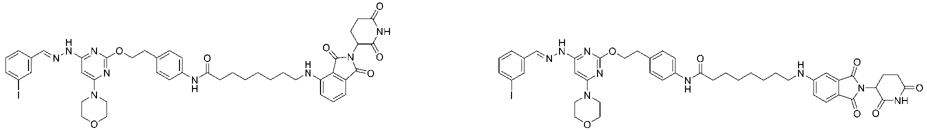

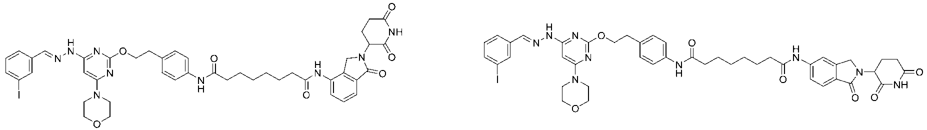

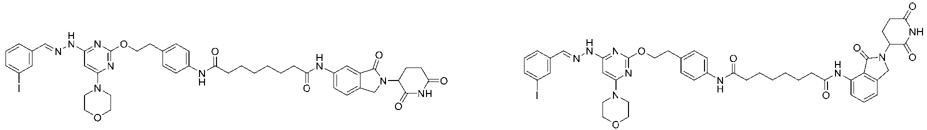

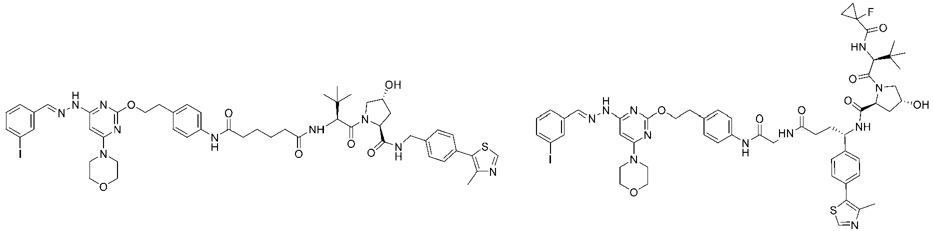

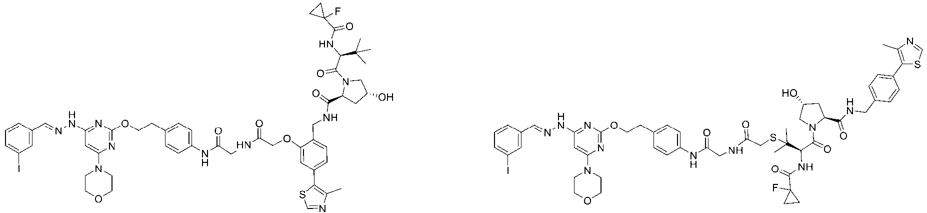

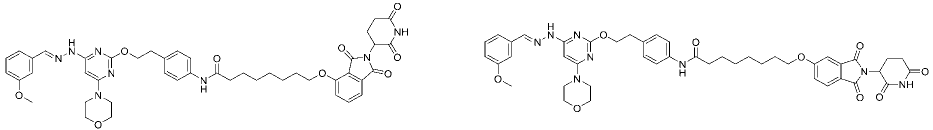

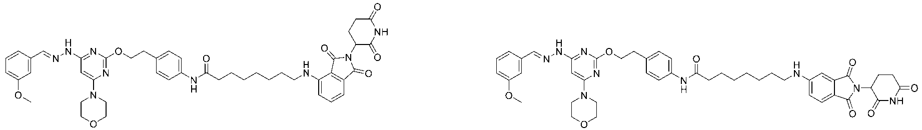

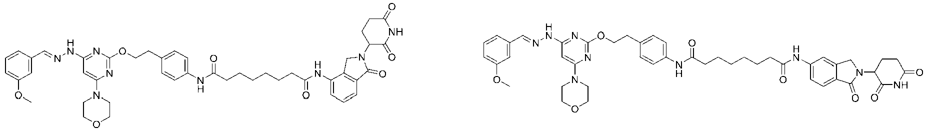

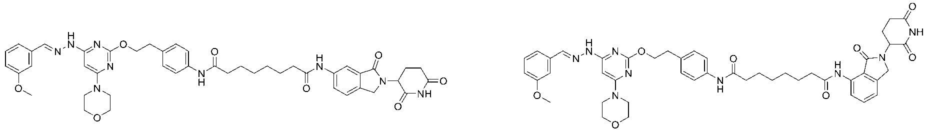

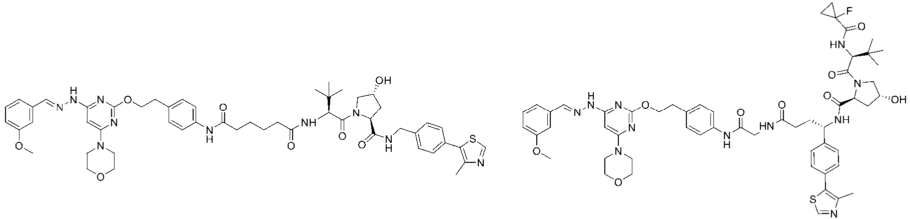

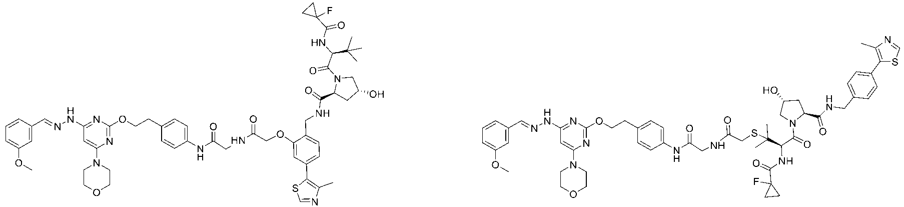

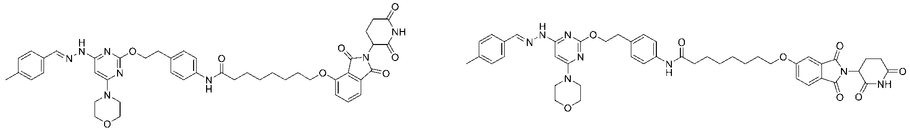

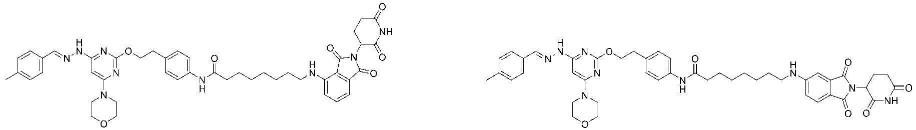

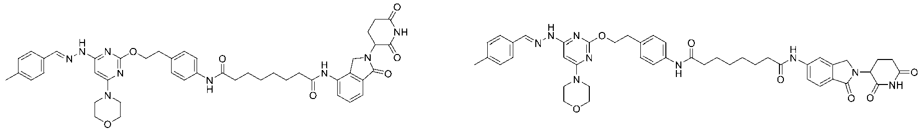

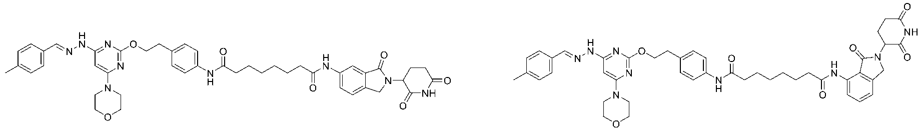

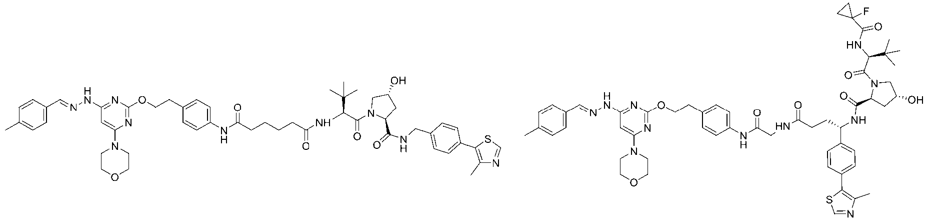

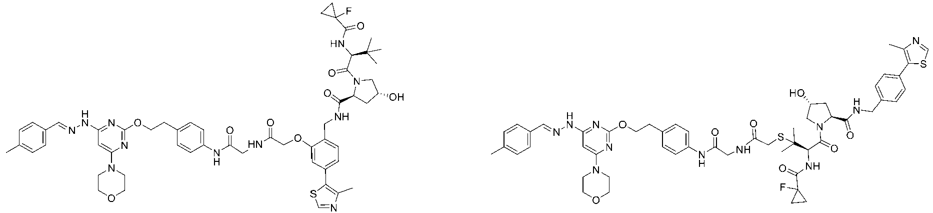

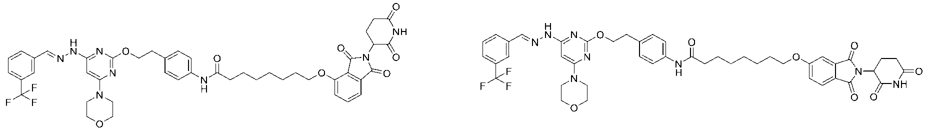

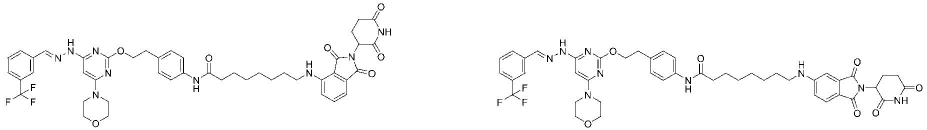

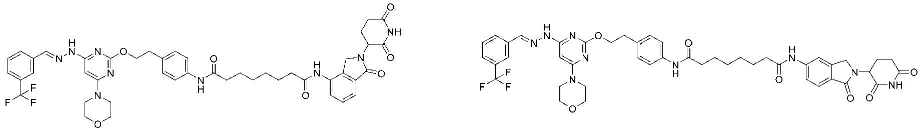

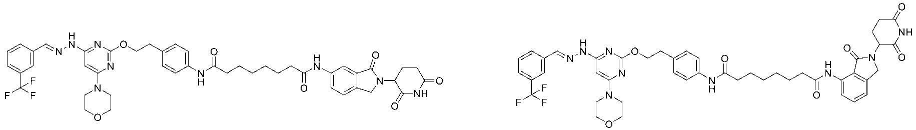

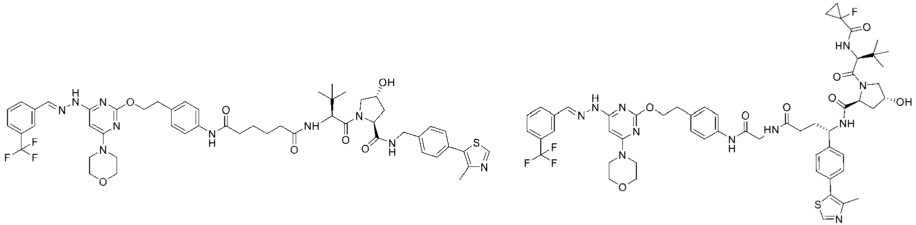

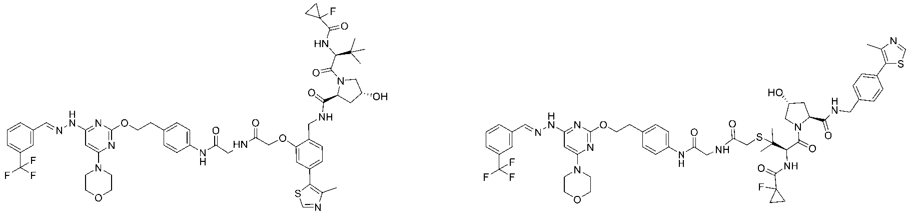

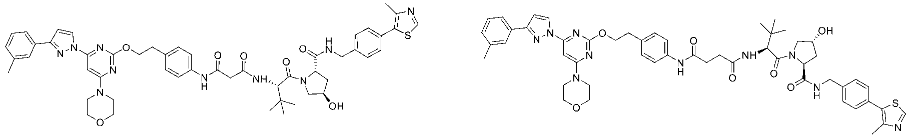

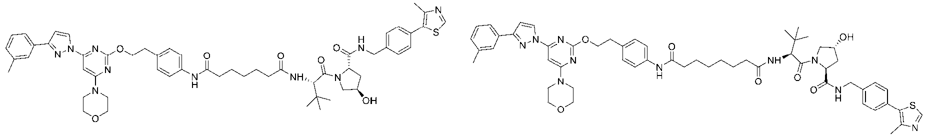

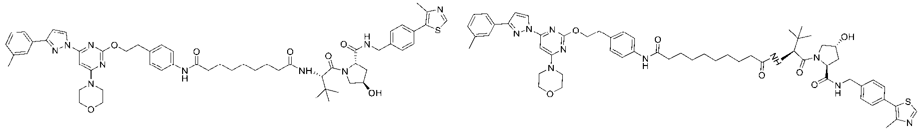

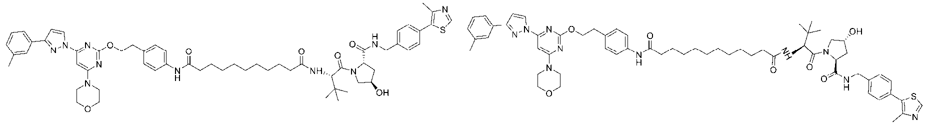

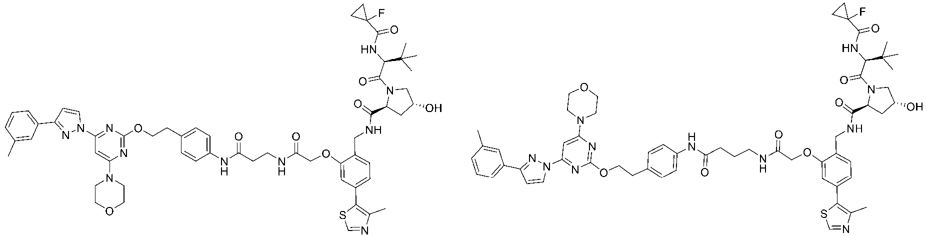

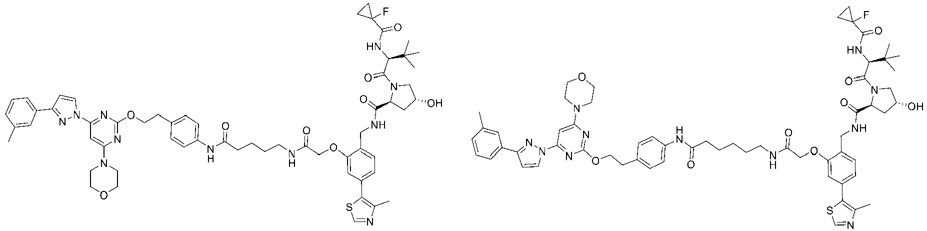

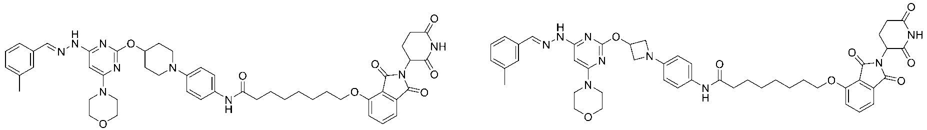

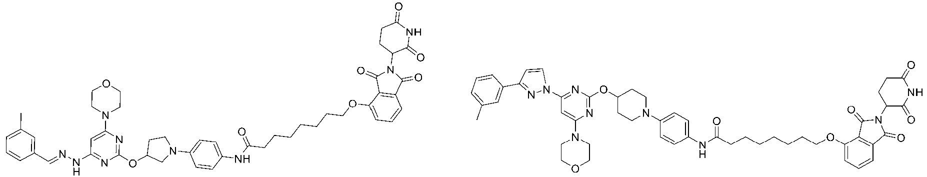

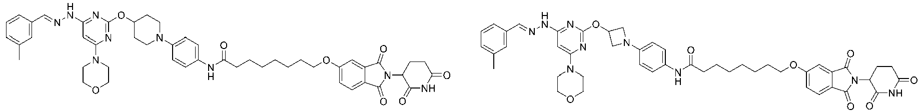

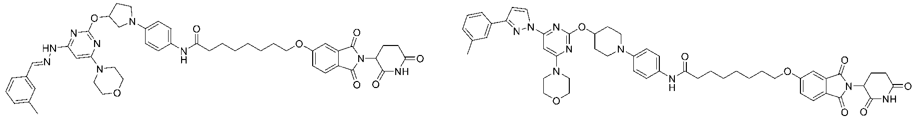

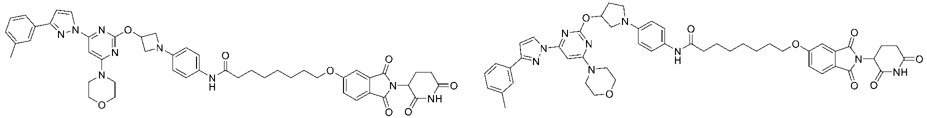

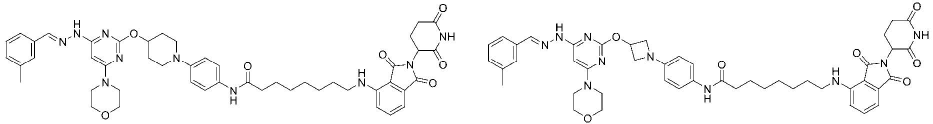

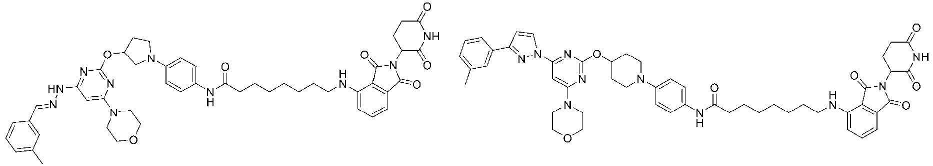

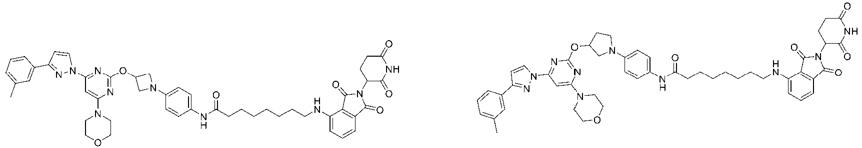

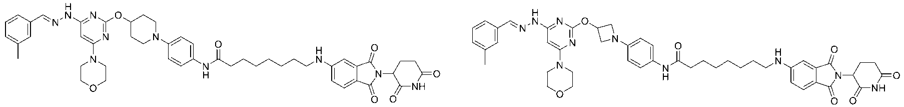

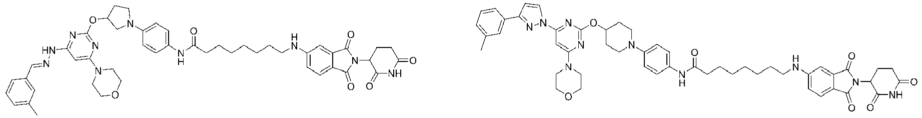

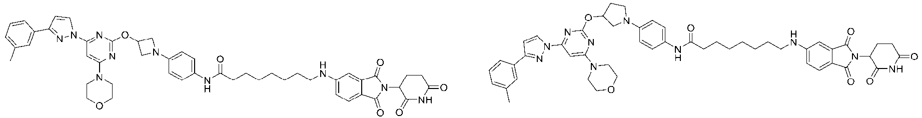

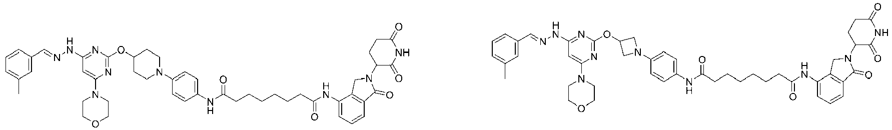

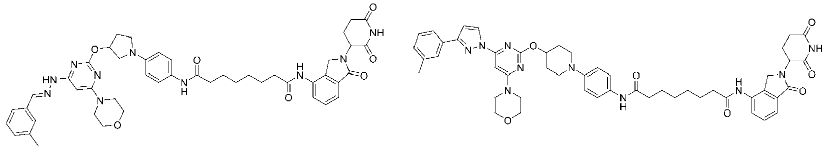

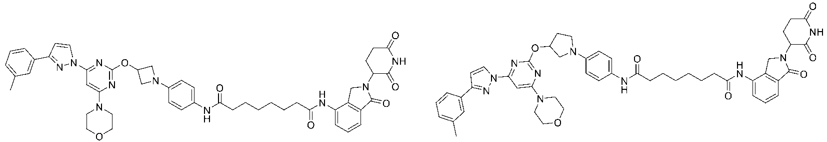

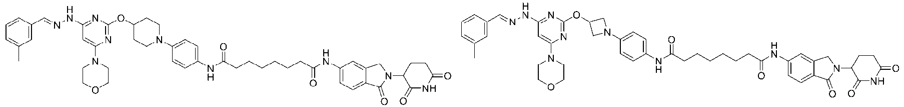

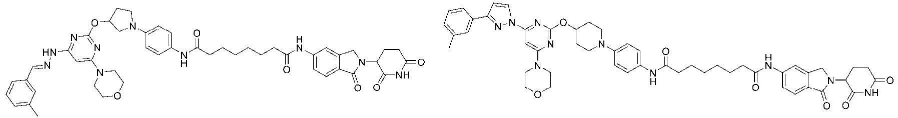

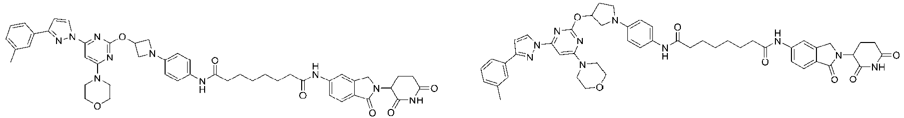

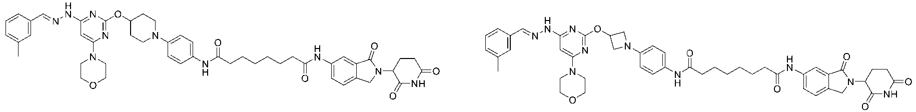

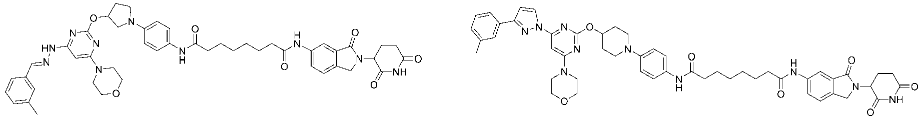

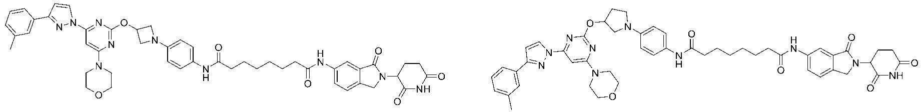

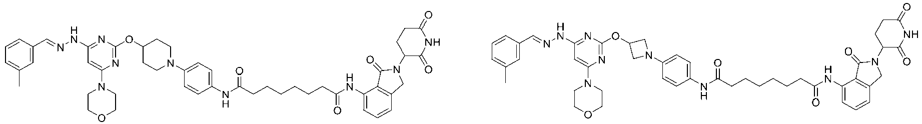

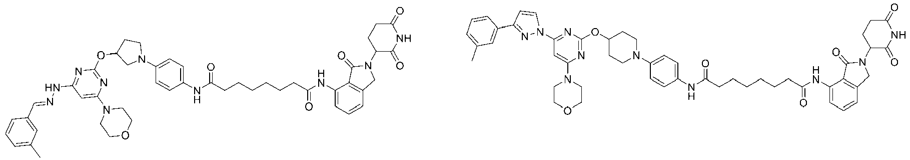

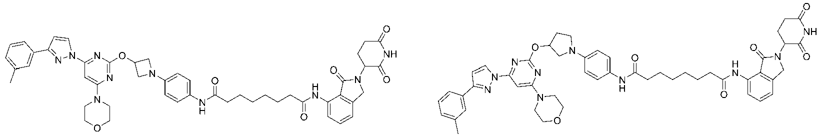

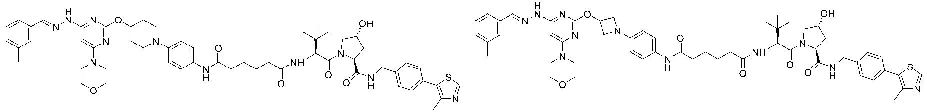

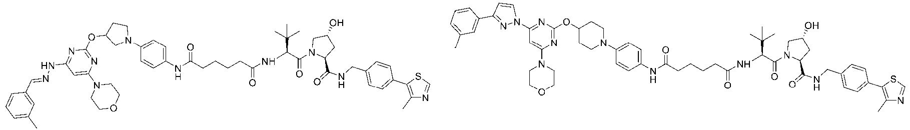

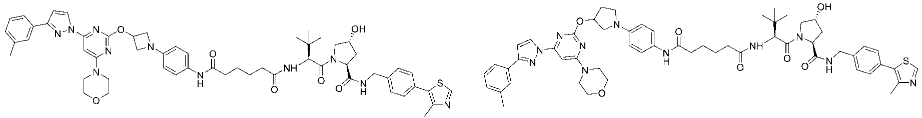

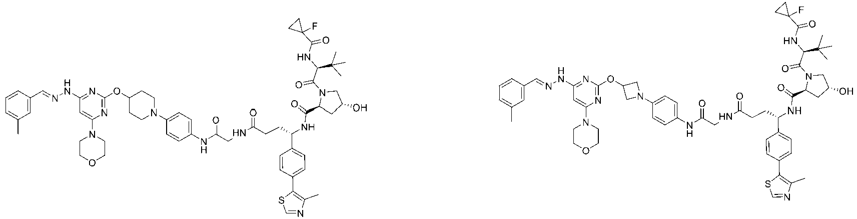

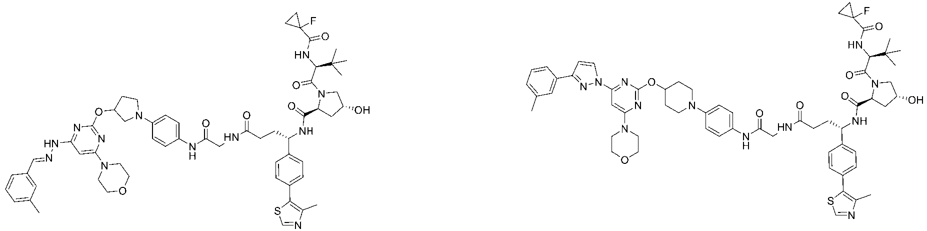

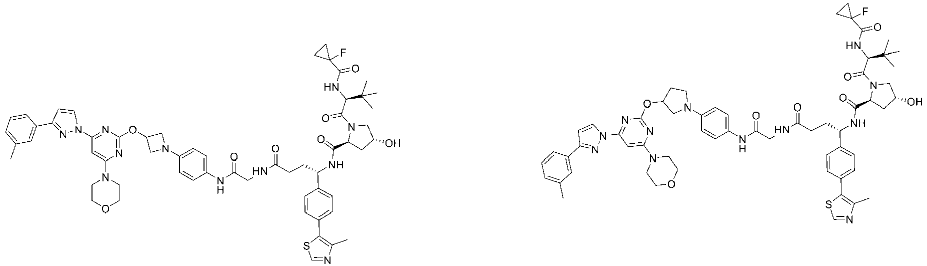

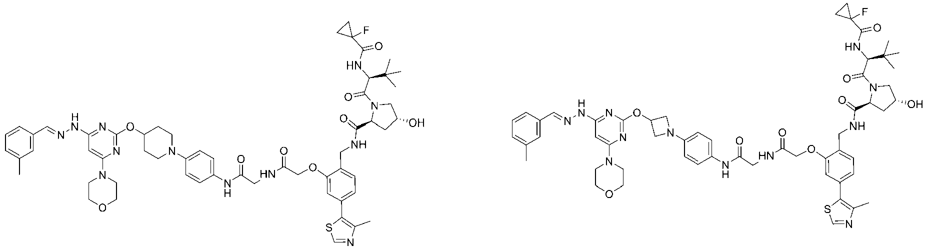

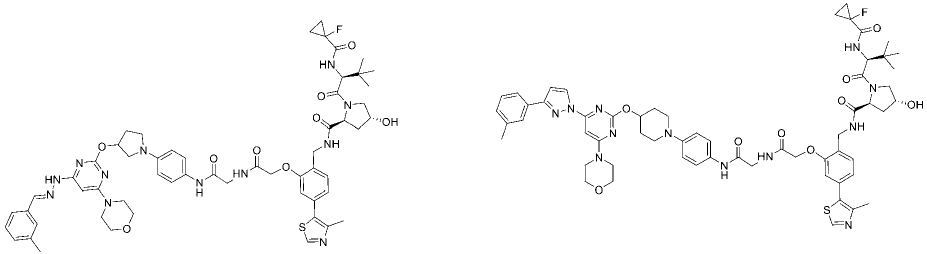

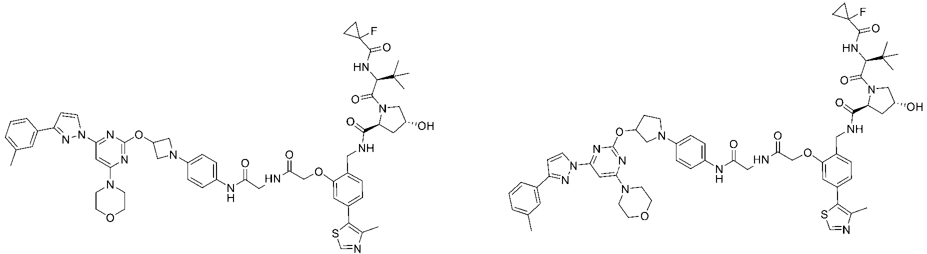

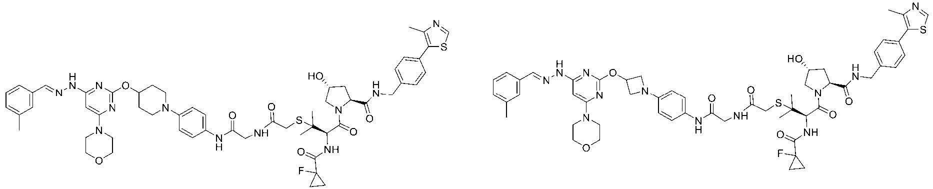

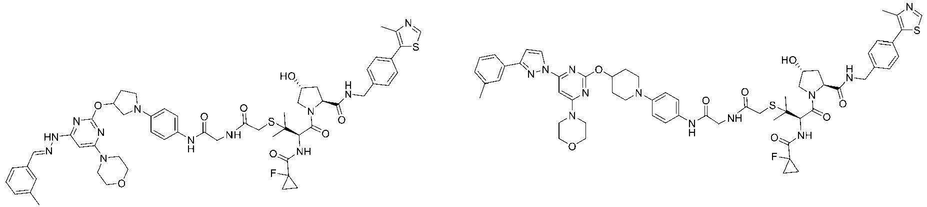

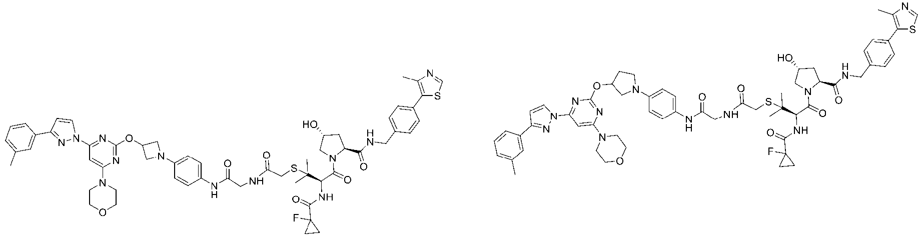

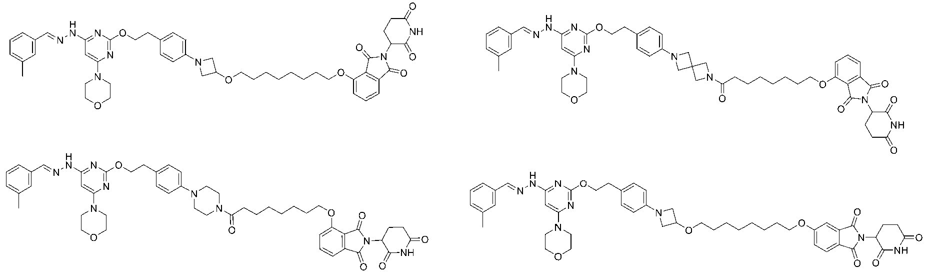

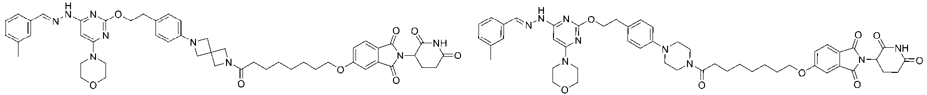

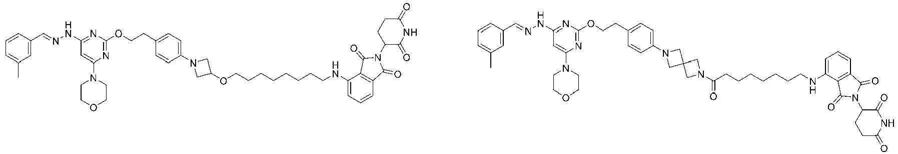

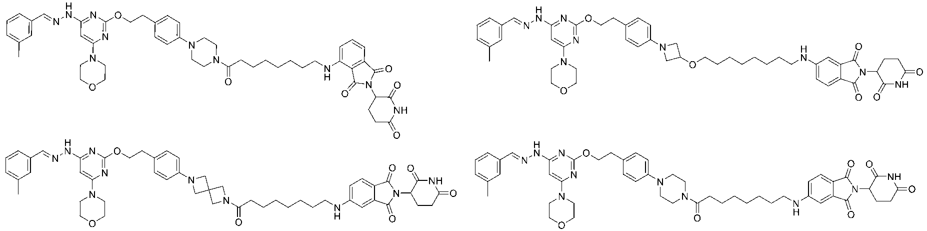

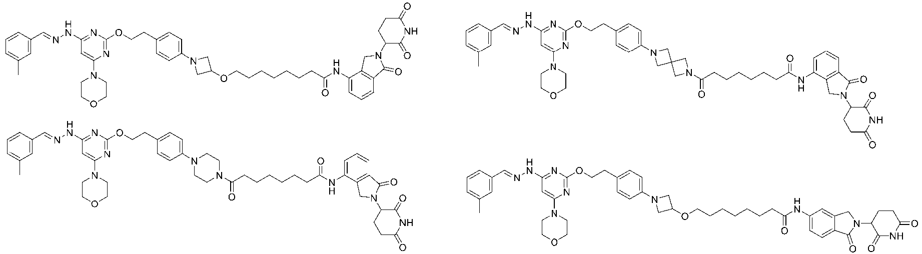

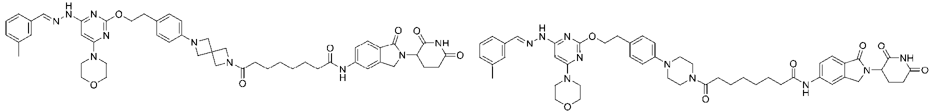

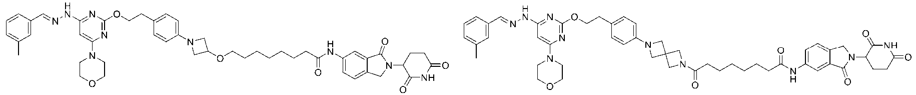

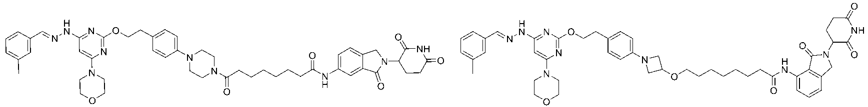

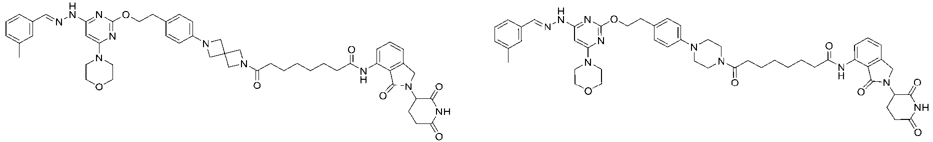

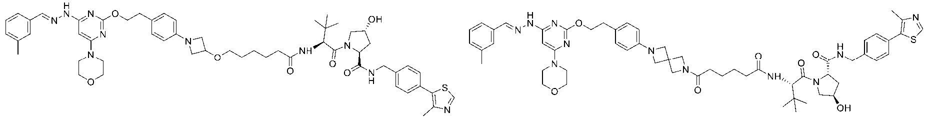

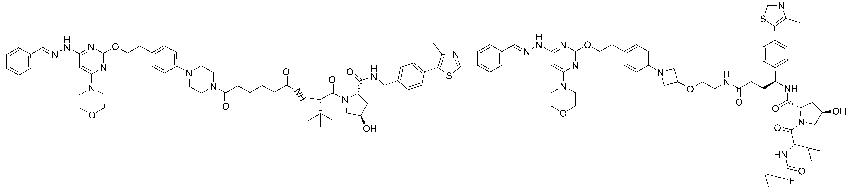

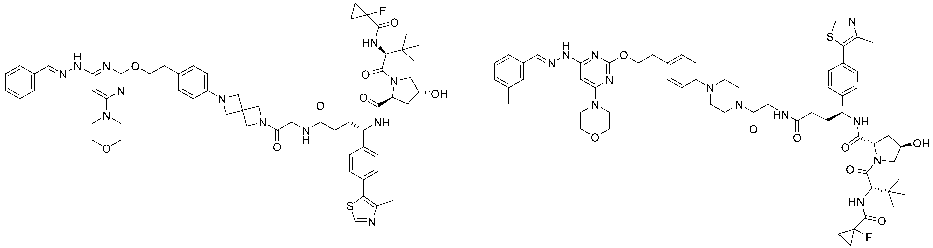

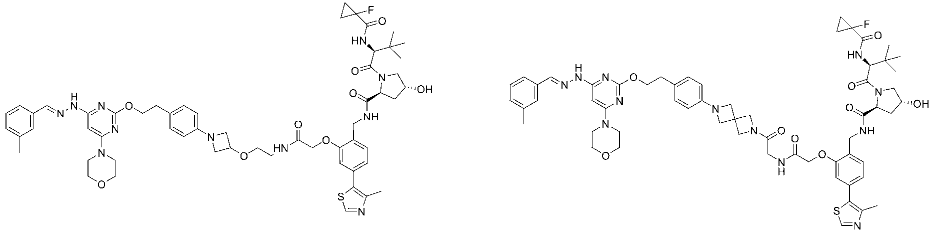

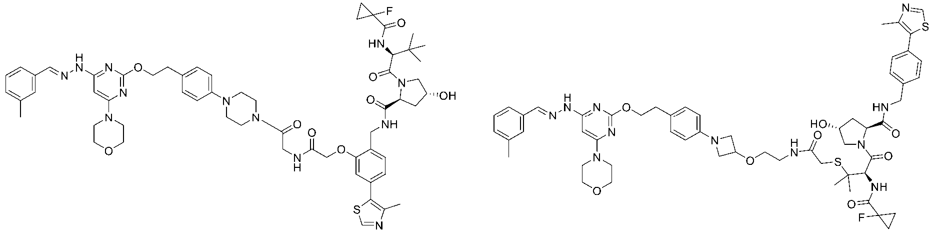

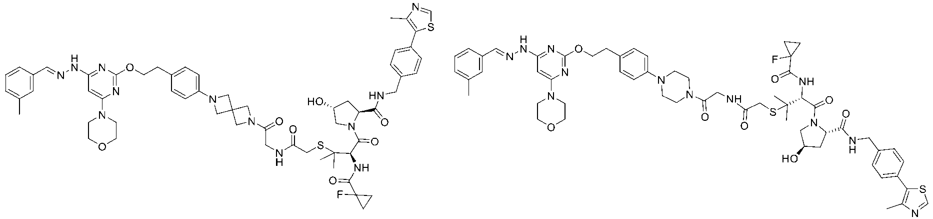

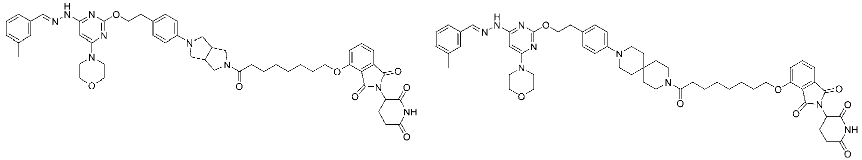

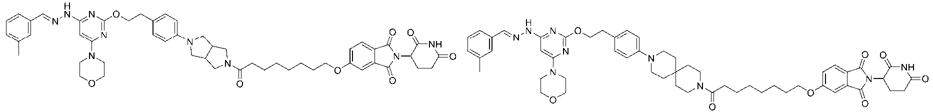

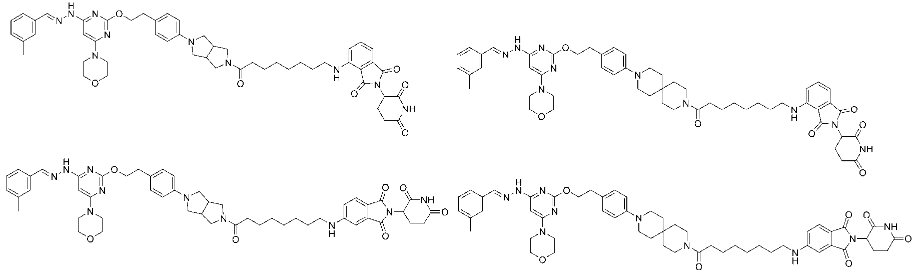

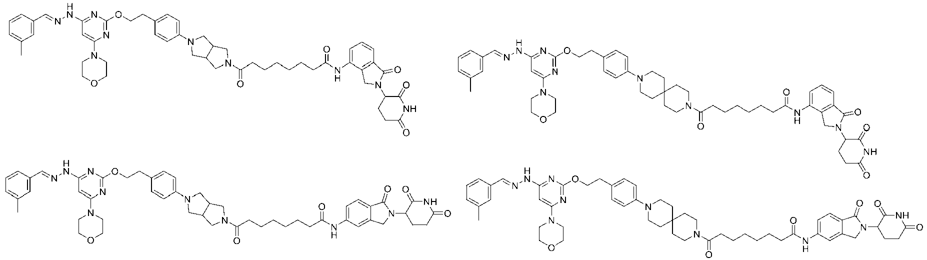

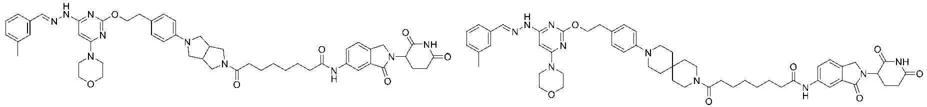

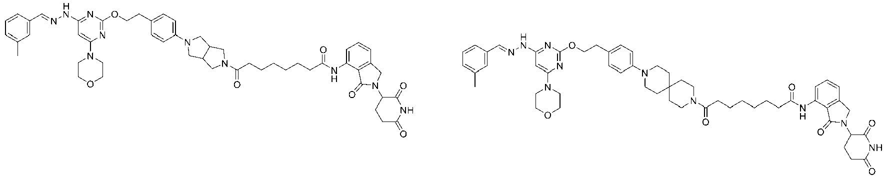

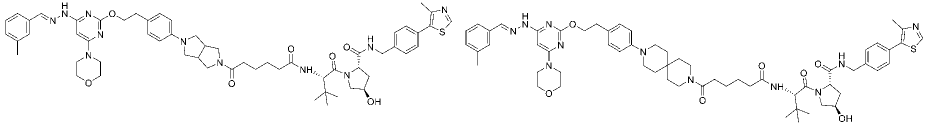

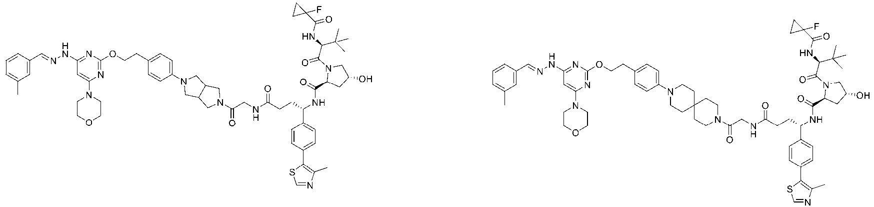

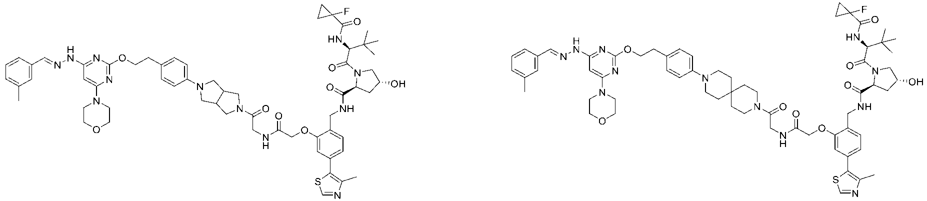

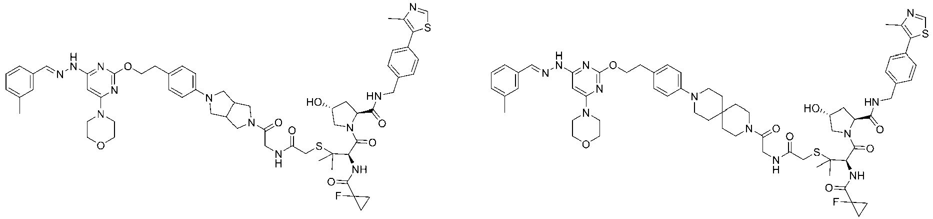

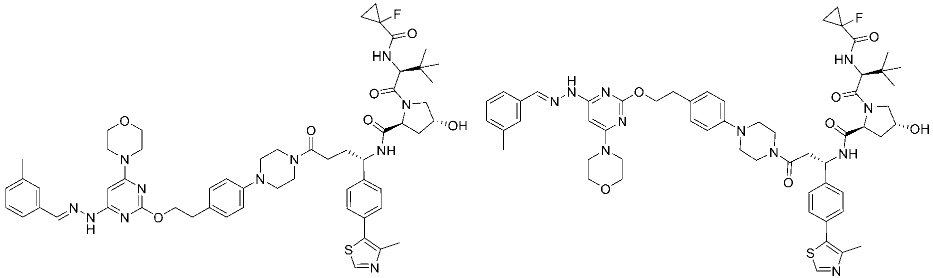

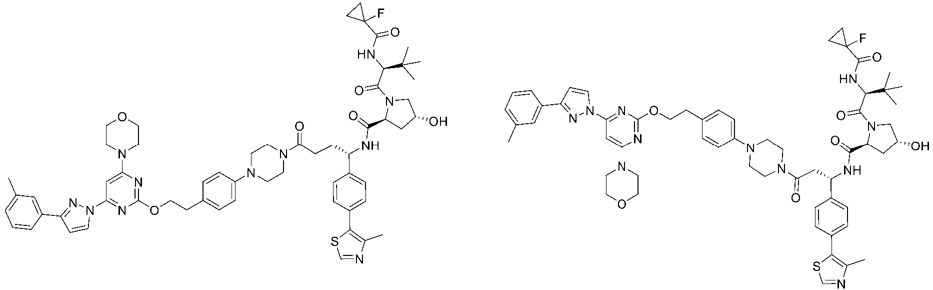

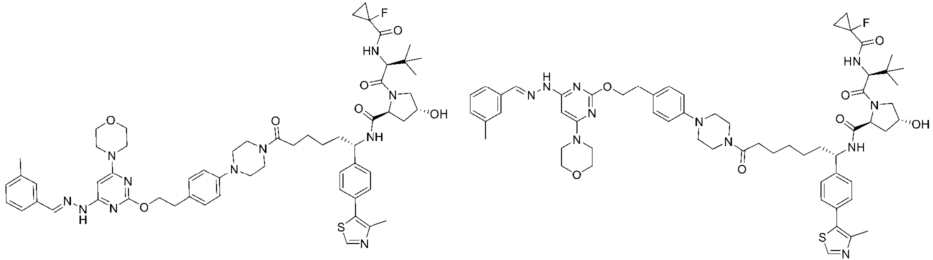

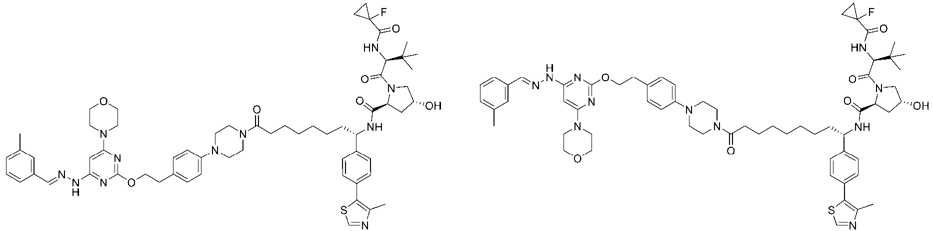

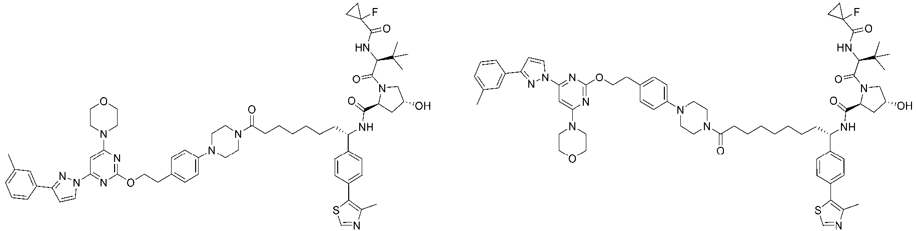

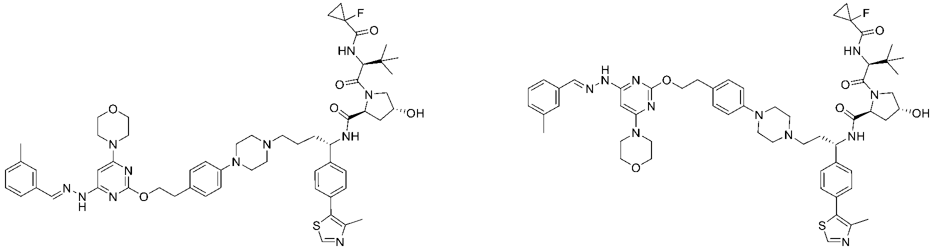

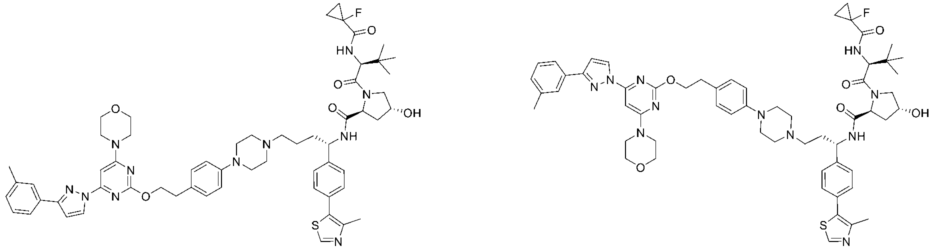

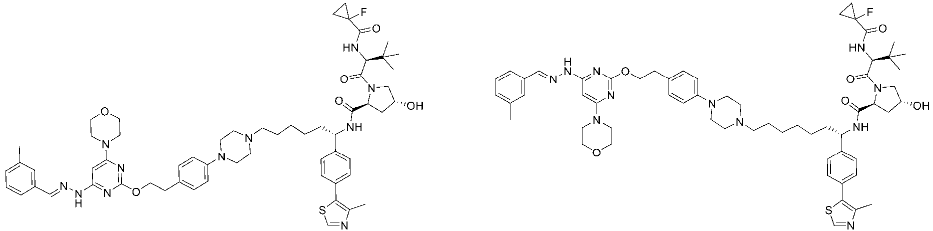

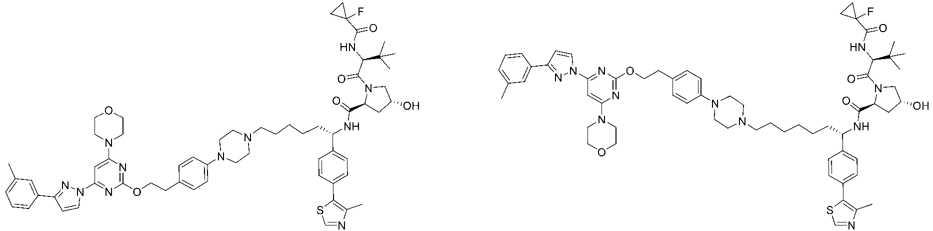

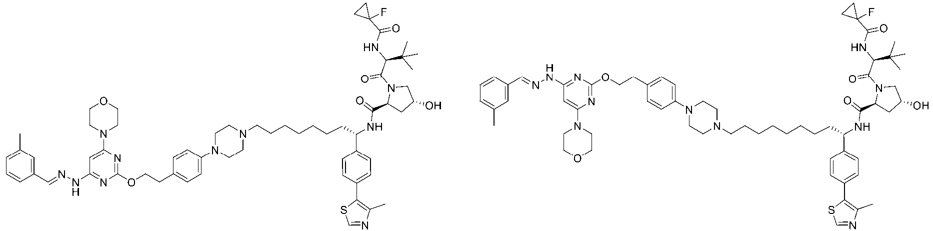

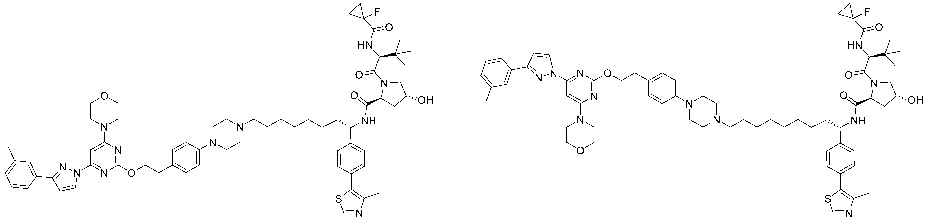

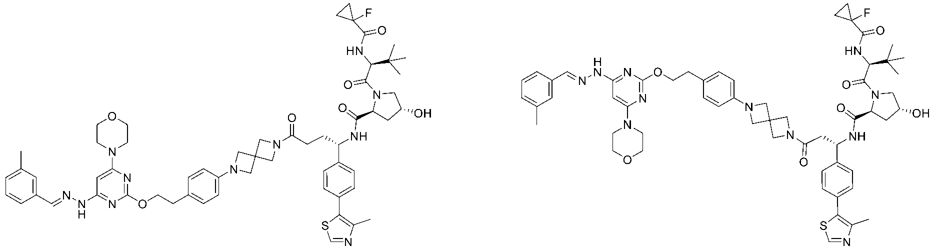

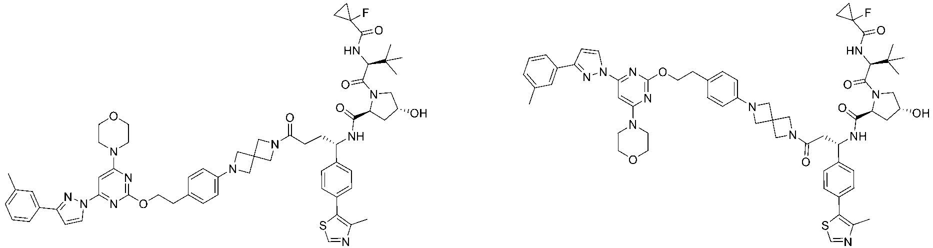

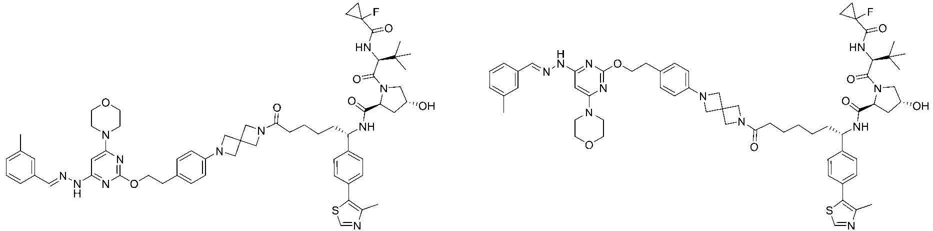

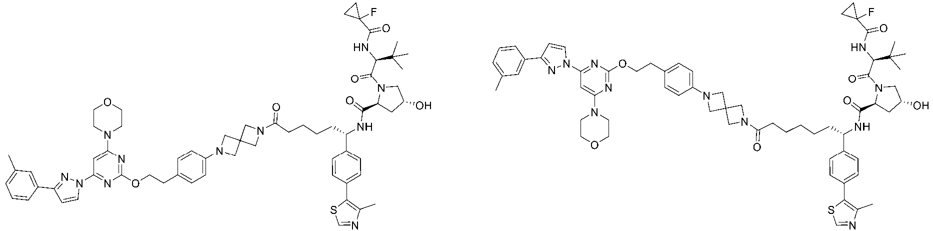

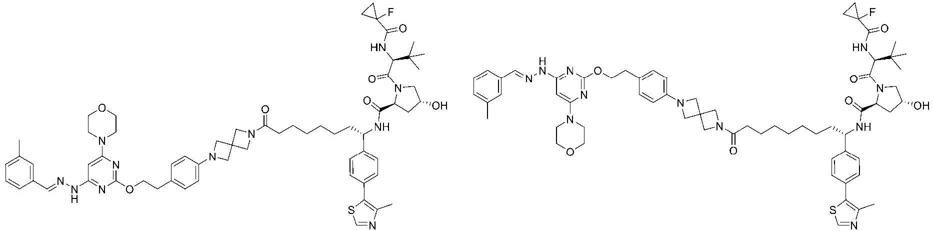

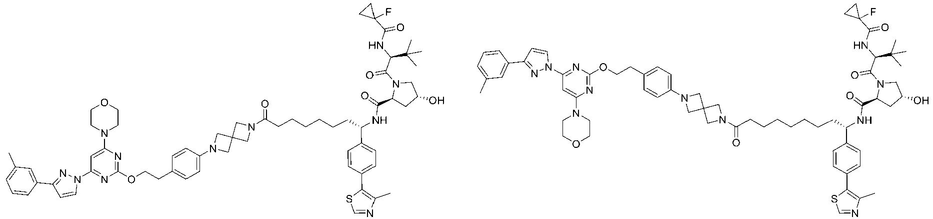

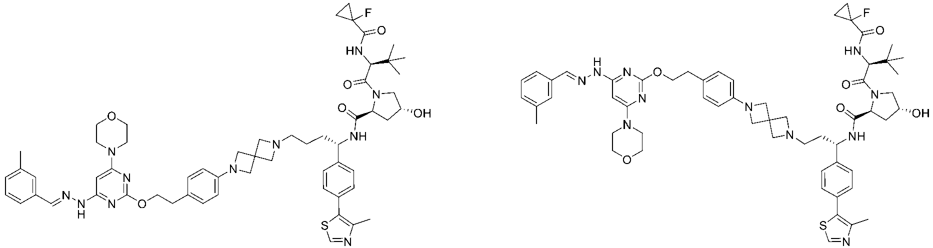

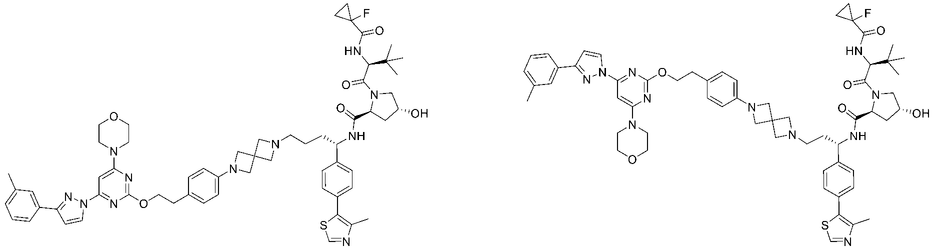

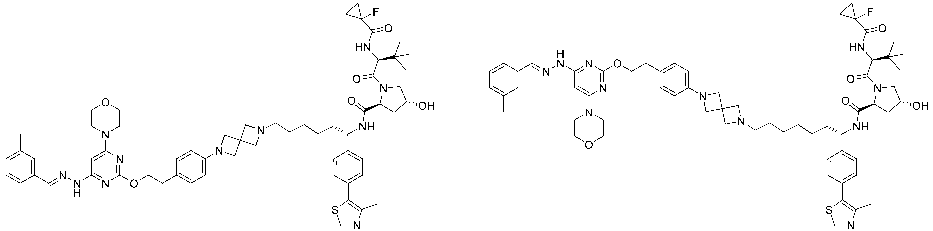

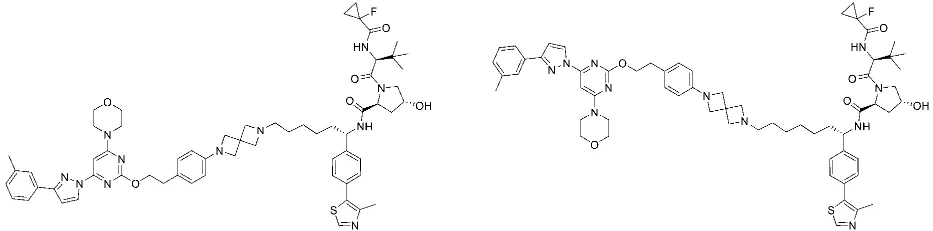

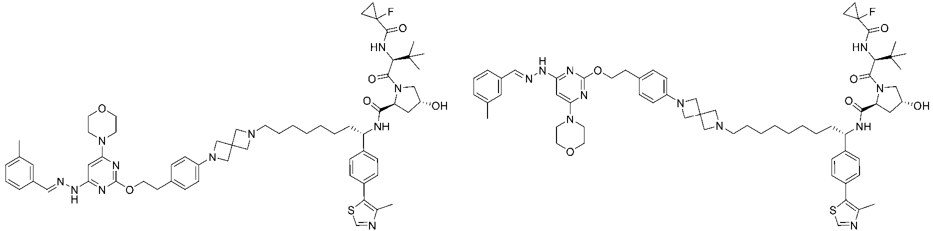

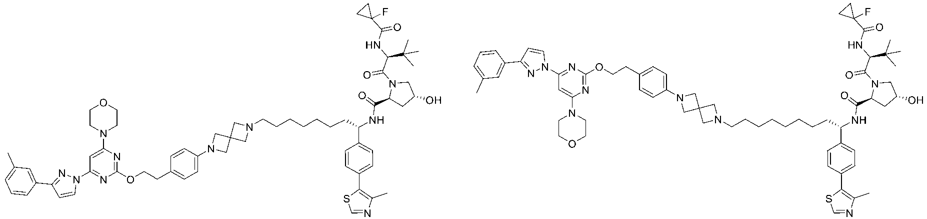

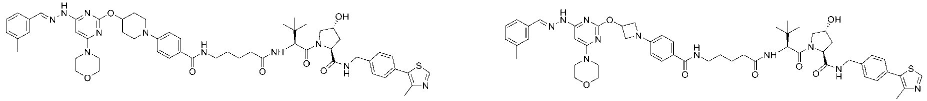

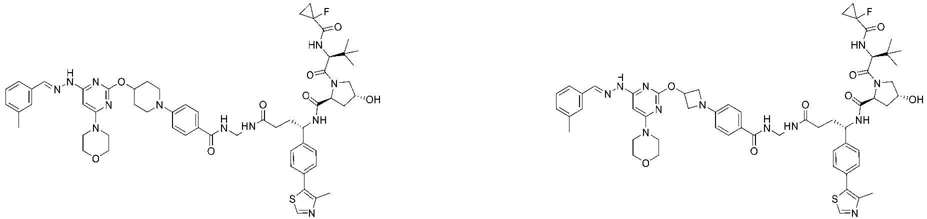

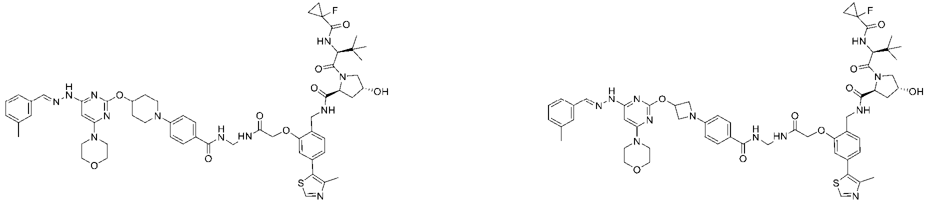

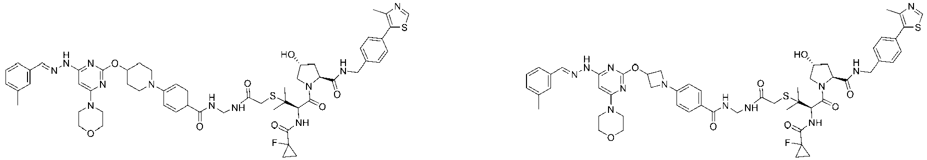

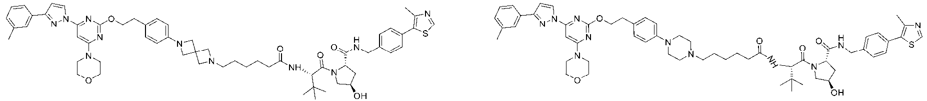

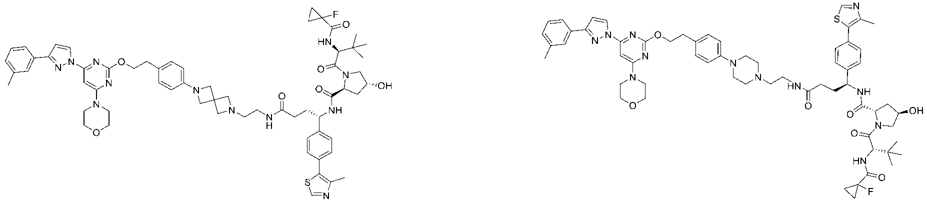

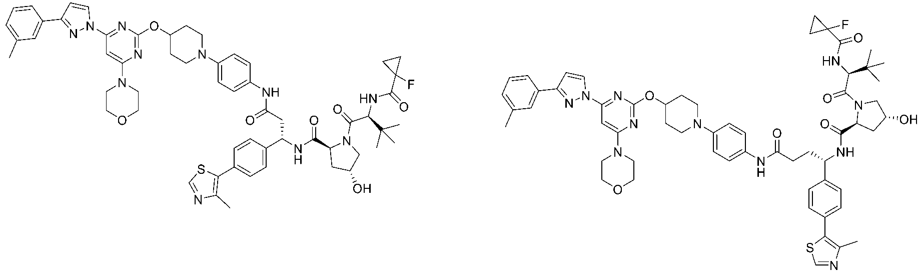

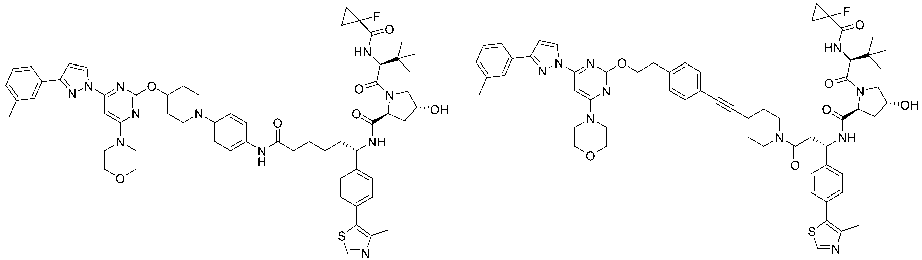

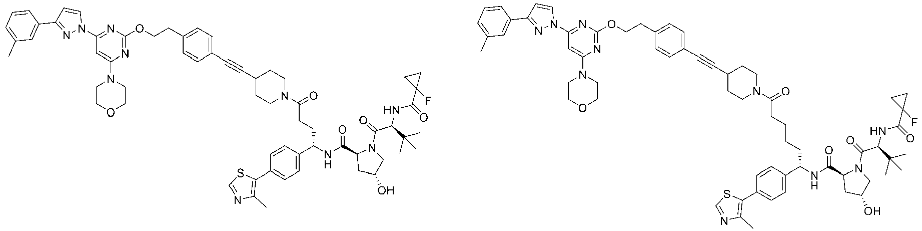

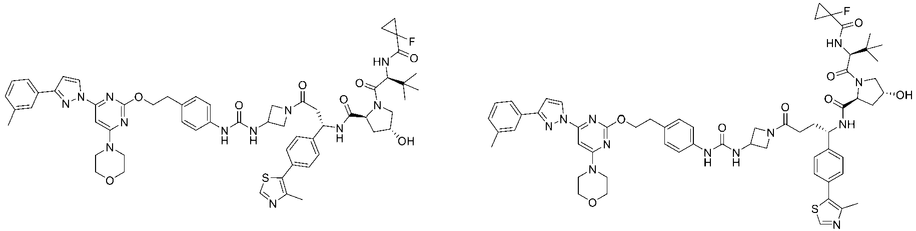

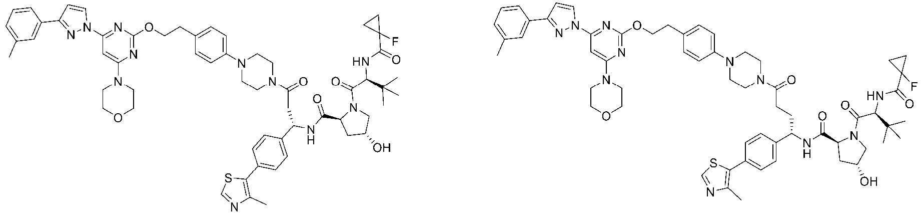

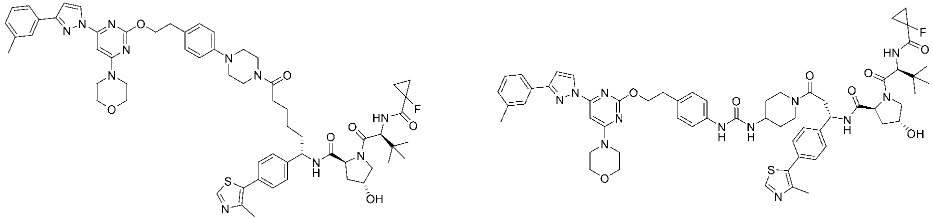

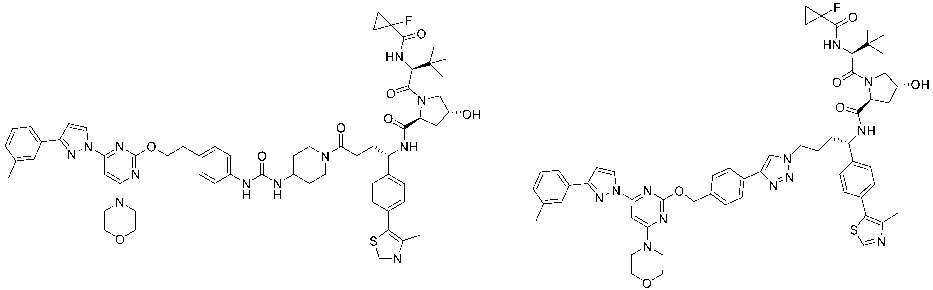

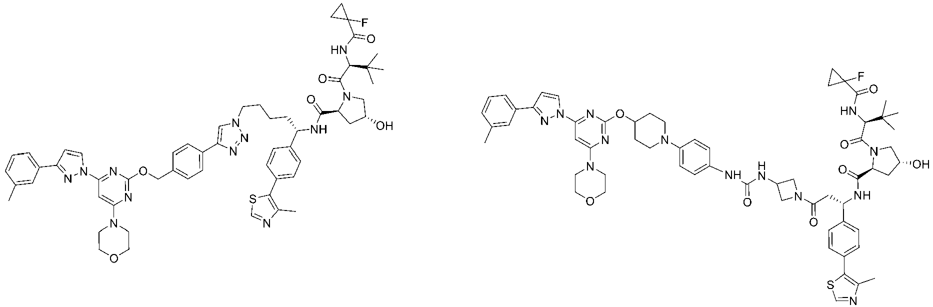

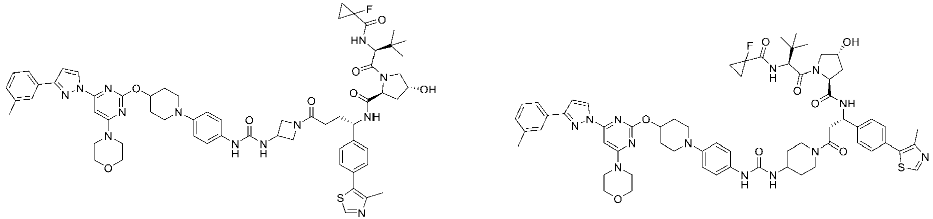

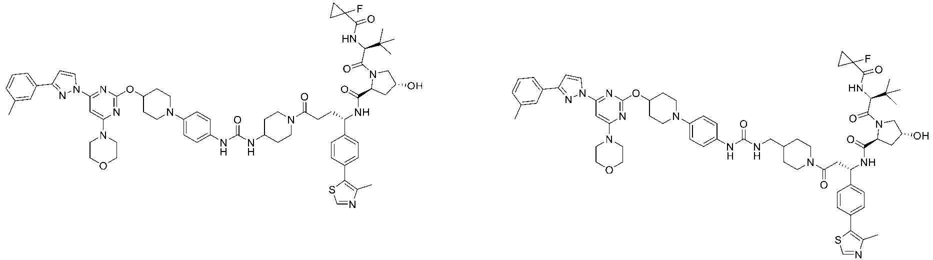

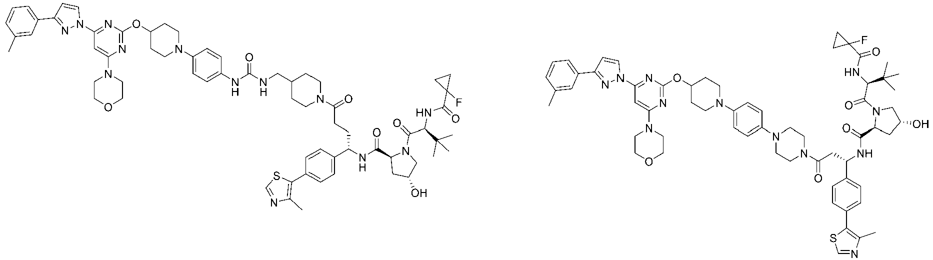

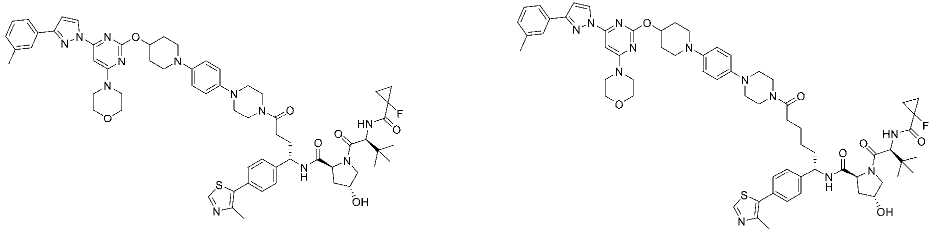

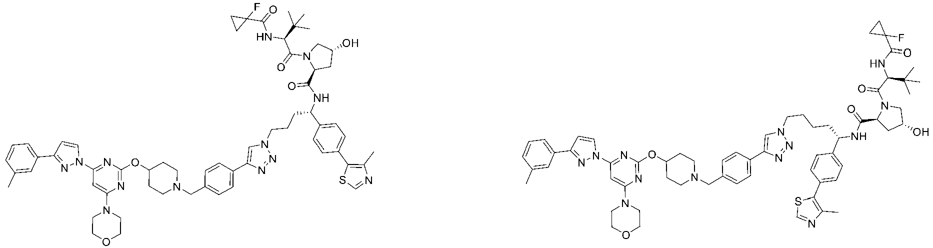

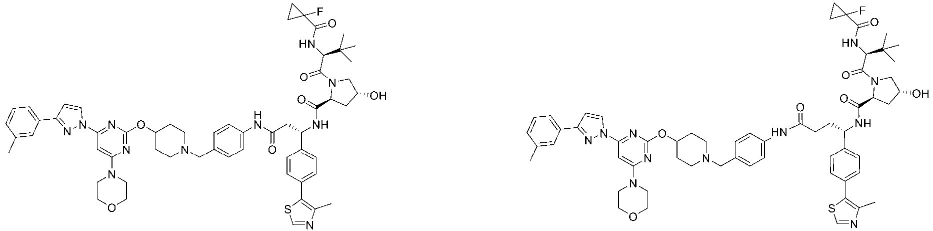

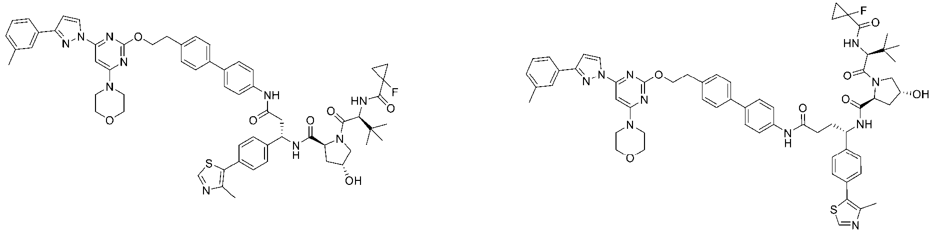

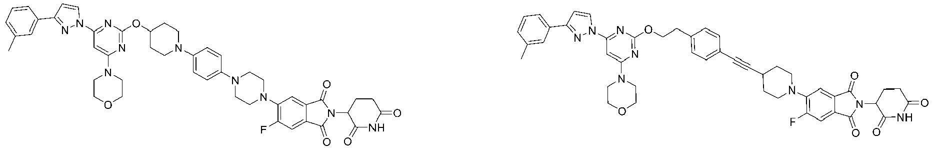

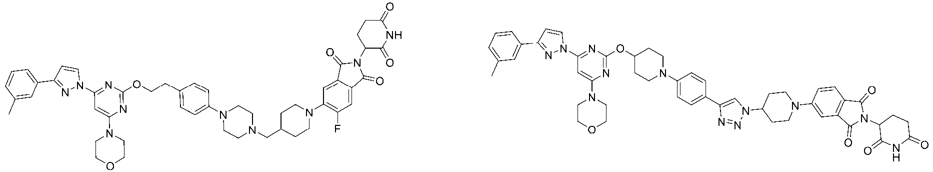

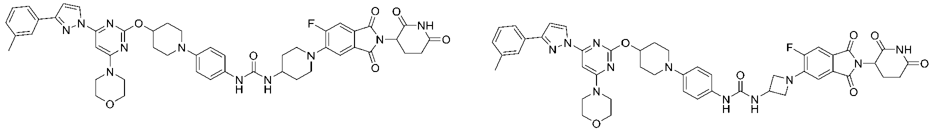

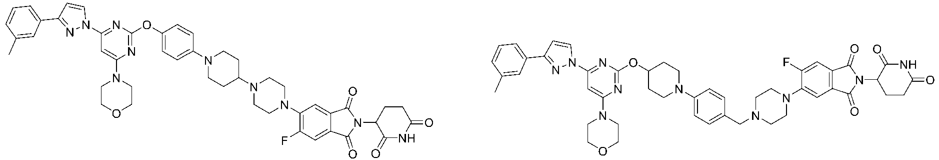

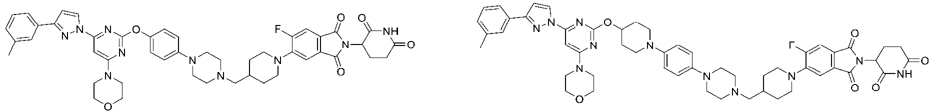

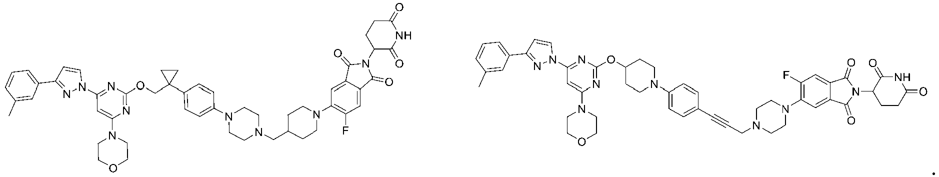

7. The compound or the pharmaceutically acceptable salt thereof or the stereoisomer thereof or the prodrug molecule thereof according to claim 1, wherein the compound is selected from the group consisting of: 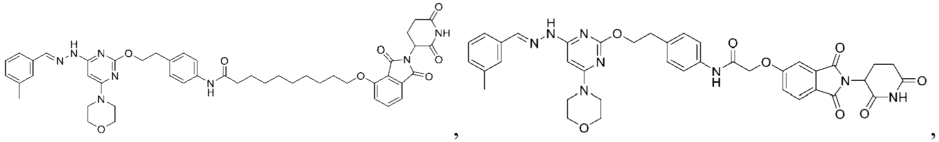 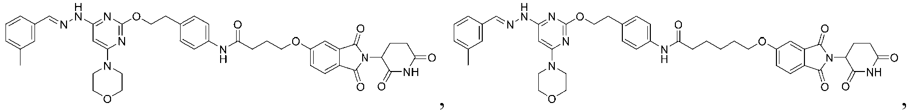 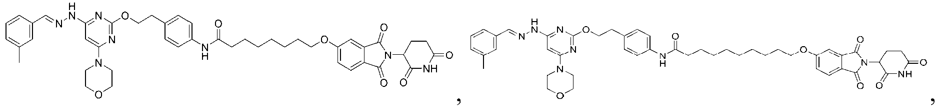 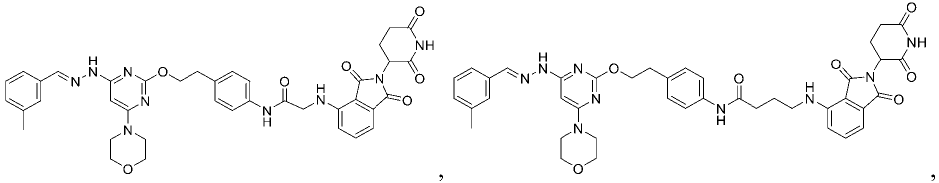 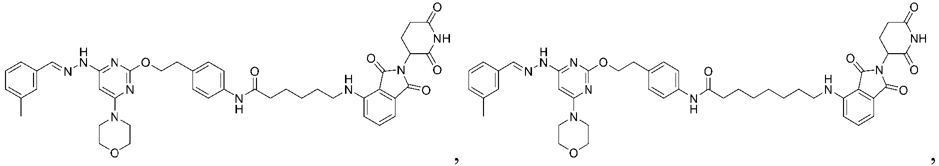 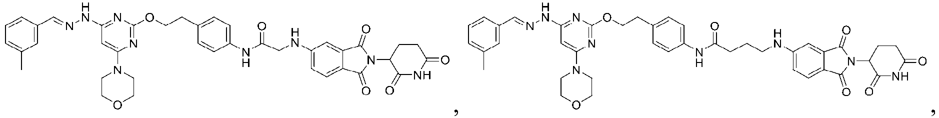 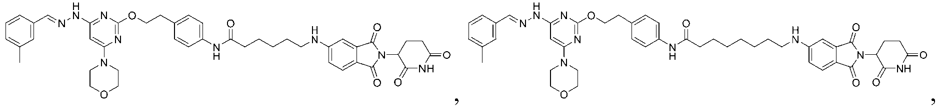 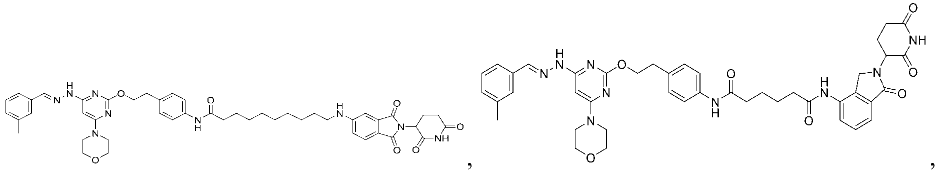 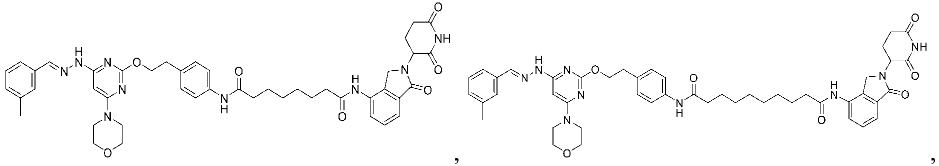 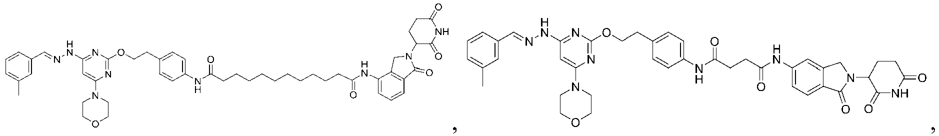 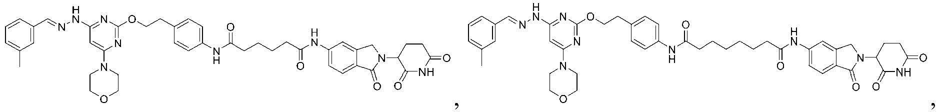 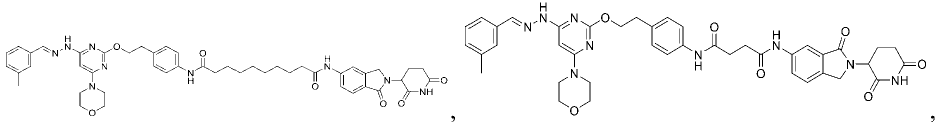 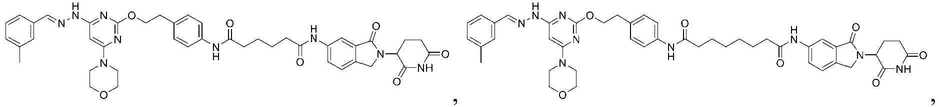 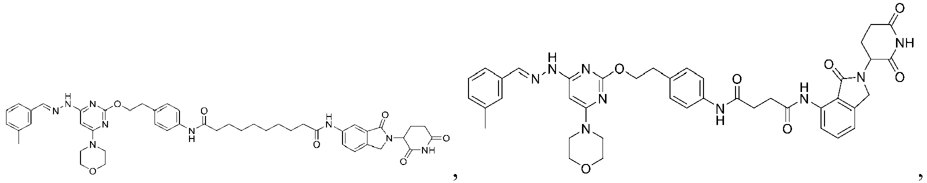 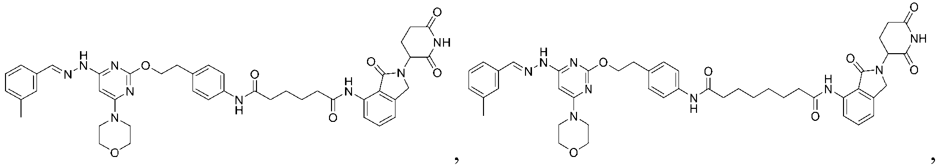 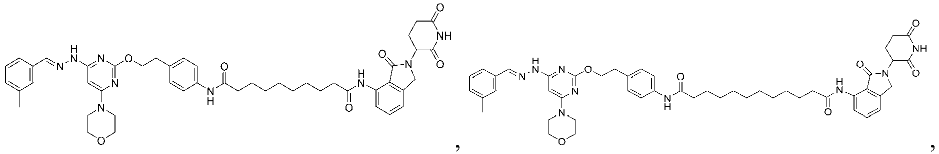 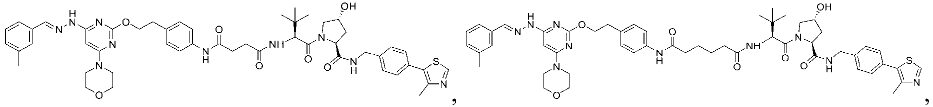 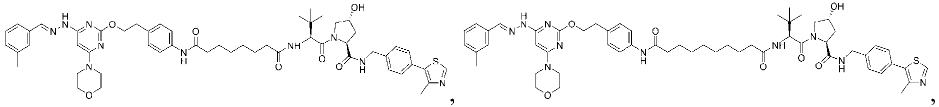 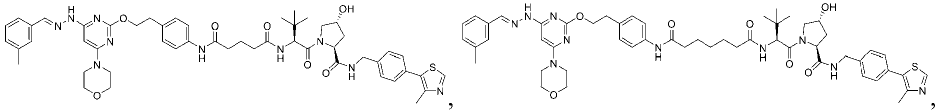 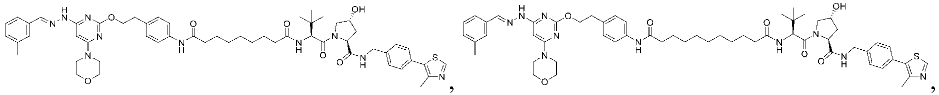 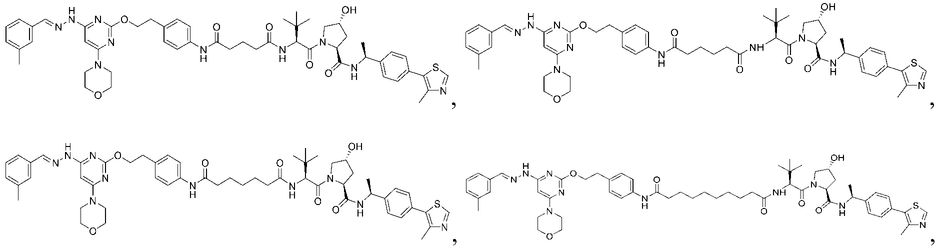 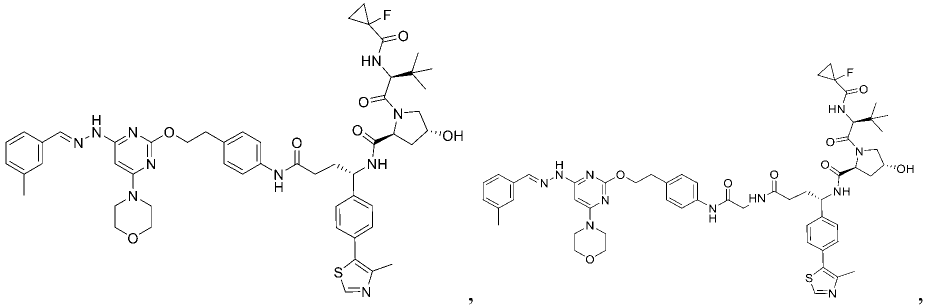 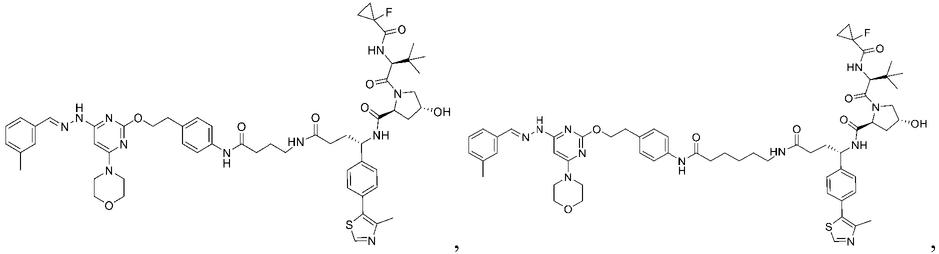 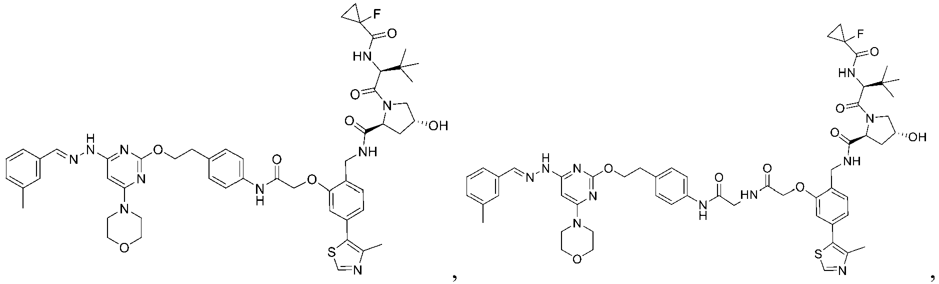 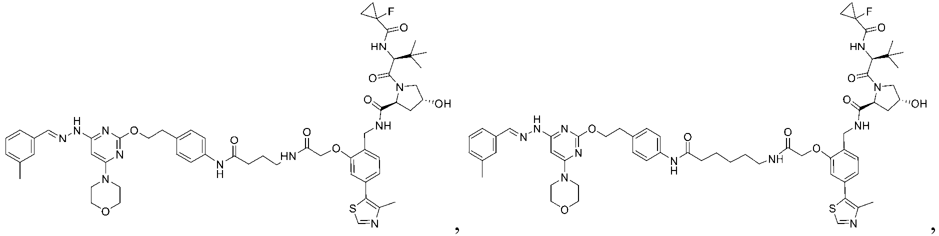 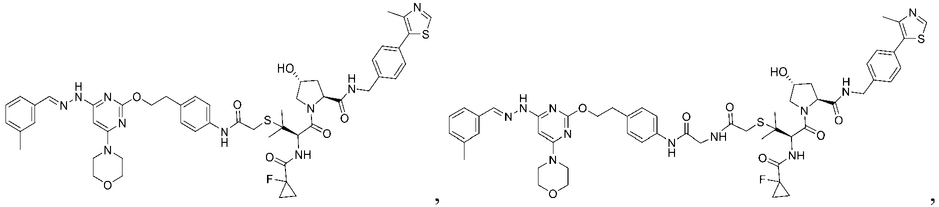 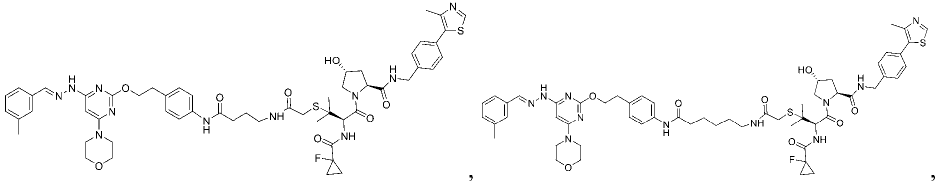 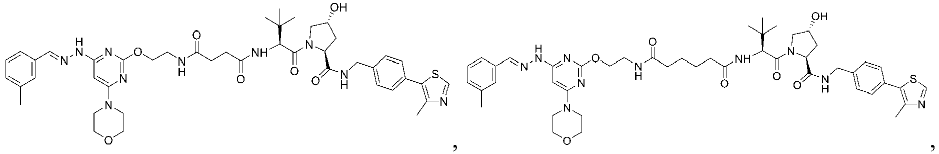 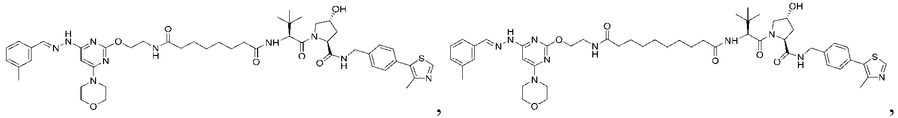 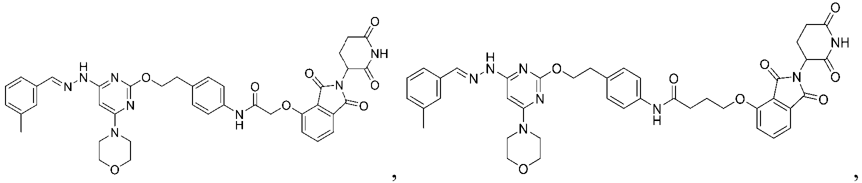 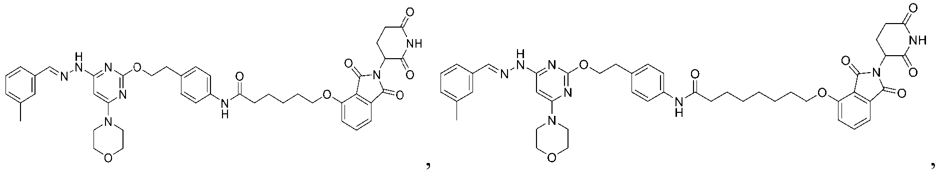 and

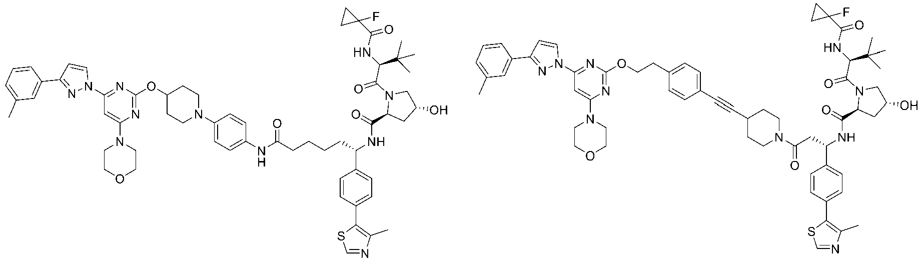

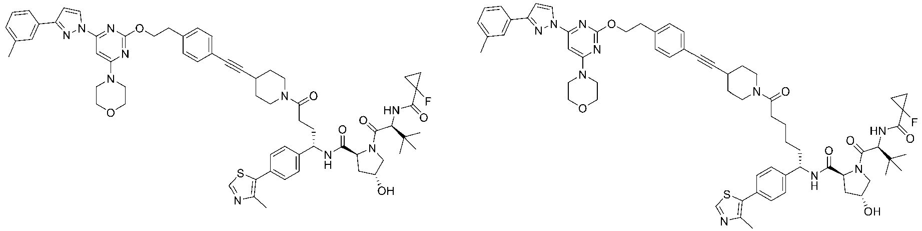

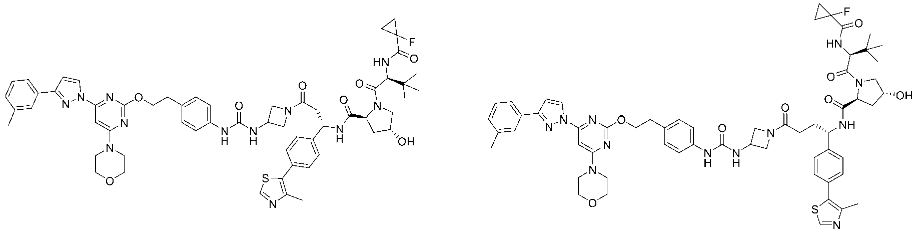

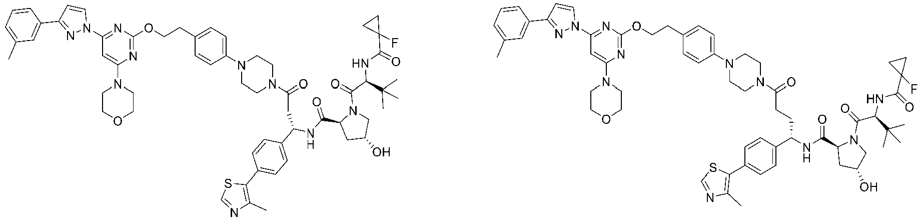

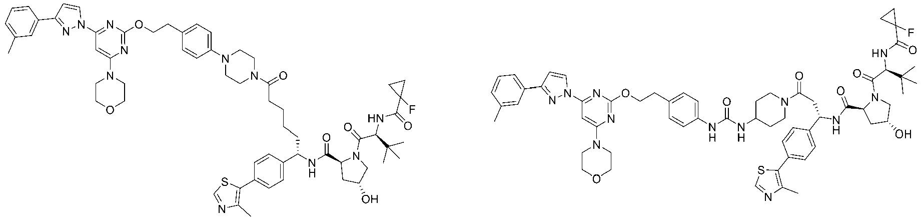

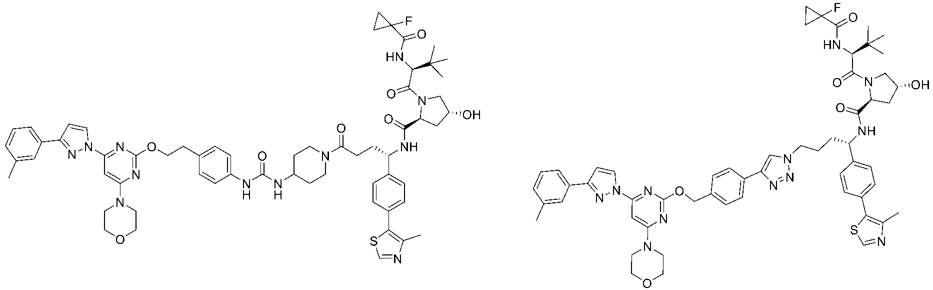

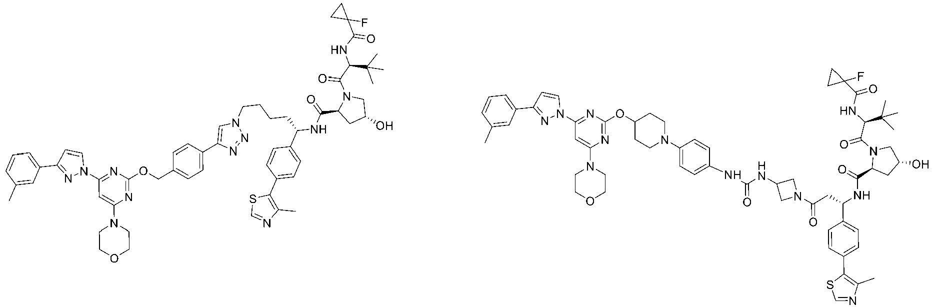

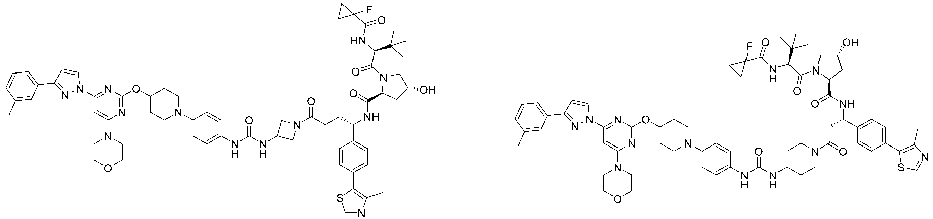

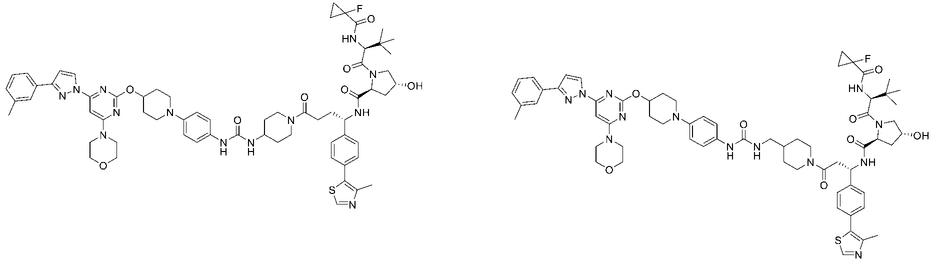

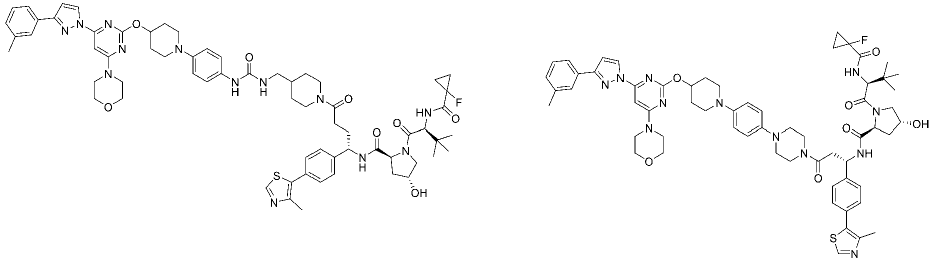

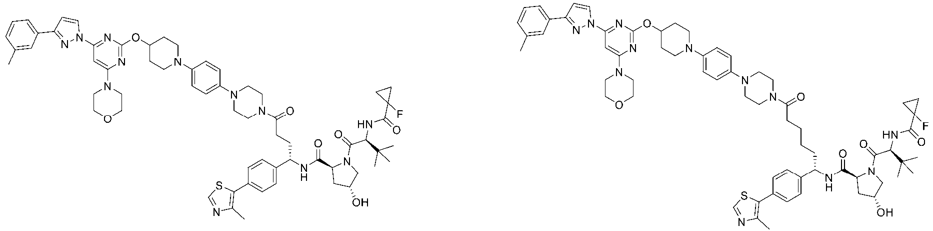

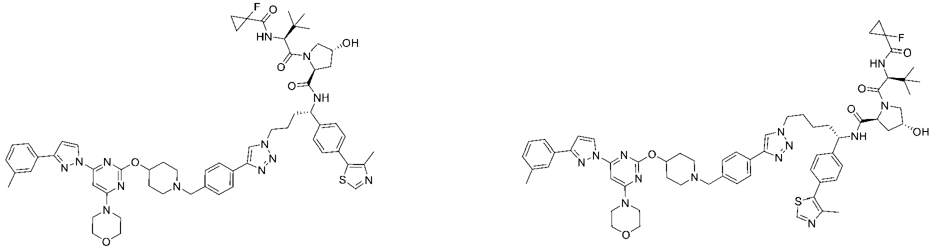

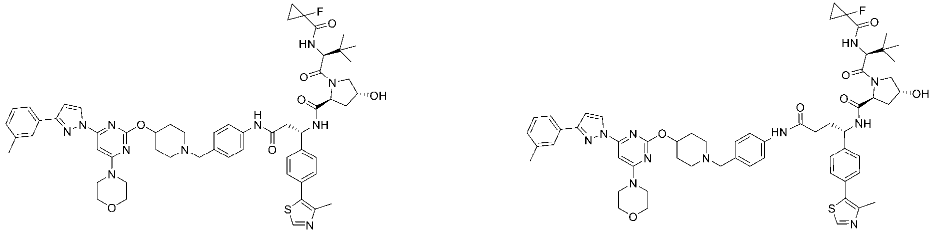

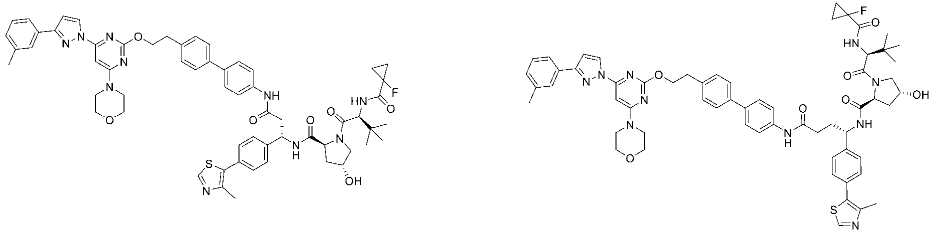

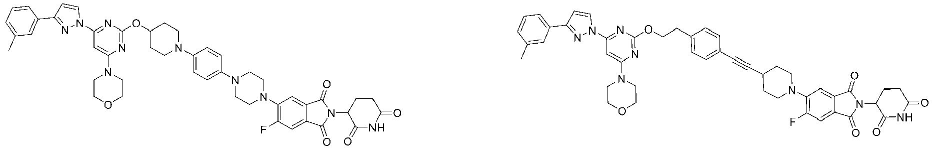

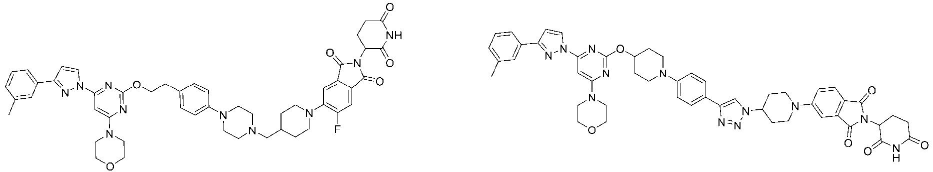

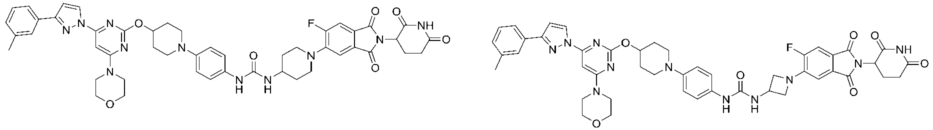

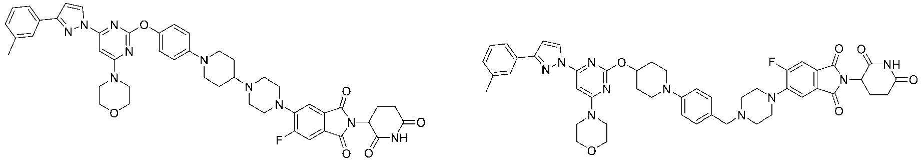

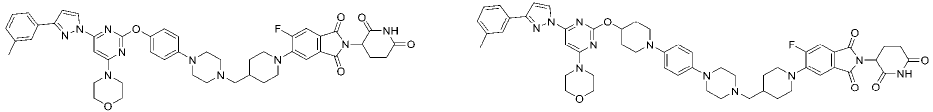

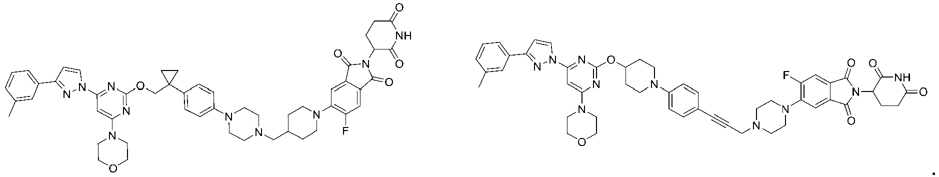

8. A pharmaceutical composition for treating and/or preventing a tumor, comprising:
(1) the compound or the pharmaceutically acceptable salt thereof or the stereoisomer thereof or the prodrug molecule thereof according to any one of claims 1-7; and
optionally (2) a pharmaceutically acceptable carrier.

9. Use of the compound or the pharmaceutically acceptable salt thereof, the stereoisomer thereof or the prodrug molecule thereof according to any one of claims 1-7 or the pharmaceutical composition according to claim 8 for treating and/or preventing diseases or disorders related to aberrant expression of PIKfyve, wherein the diseases or disorders are preferably selected from the group consisting of tumor, autoimmune disease, virus infection, and neurodegenerative disease.

10. The use according to claim 9, wherein the diseases or disorders are selected from the group consisting of: hematological tumor, gastrointestinal stromal tumor, histiocytic lymphoma, non-small cell lung cancer, small cell lung cancer, lung adenocarcinoma, lung squamous cell carcinoma, pancreatic cancer, breast cancer, prostate cancer, liver cancer, skin cancer, epithelial cell carcinoma, colorectal cancer, renal cancer, gastric cancer, head and neck cancer, and nasopharyngeal cancer.

11. A non-diagnostic and non-therapeutic method for degrading PIKfyve in a subject in need thereof, comprising administering to the subject the compound or the pharmaceutically acceptable salt thereof or the stereoisomer thereof or the prodrug molecule thereof according to any one of claims 1-7 or the pharmaceutical composition according to claim 7.

12. The method according to claim 11, wherein the subject is a human.
